# EUROPÄISCHE PATENTSCHRIFT

(11) **EP 2 240 487 B1**
(45) Veröffentlichungstag und Bekanntmachung des Hinweises auf die Patenterteilung: **14.12.2011**
(21) Anmeldenummer: 09708289.5
(22) Anmeldetag: 23.01.2009
(51) Int. Cl.: C07D 487/04, A61K 31/5025, A61P 7/02

(54) **TRIAZOLOPYRIDAZINE ALS PAR1-INHIBITOREN, IHRE HERSTELLUNG UND VERWENDUNG ALS ARZNEIMITTEL**
TRIAZOLOPYRIDAZINES AS PAR1 INHIBITORS, PRODUCTION THEREOF, AND USE AS MEDICAMENTS
TRIAZOLOPYRIDAZINES EN TANT QU'INHIBITEURS DE PAR1, LEUR PRODUCTION ET LEUR UTILISATION EN TANT QUE MÉDICAMENTS

(30) Priorität: 05.02.2008 EP 08290112
(43) Veröffentlichungstag der Anmeldung: 20.10.2010
(73) Patentinhaber: SANOFI, 75013 Paris (FR)
(72) Erfinder: HEINELT, Uwe, 65926 Frankfurt am Main (DE); WEHNER, Volkmar, 65926 Frankfurt am Main (DE); HERRMANN, Matthias, 65926 Frankfurt am Main (DE); SCHOENAFINGER, Karl, 63755 Alzenau (DE); STEINHAGEN, Henning, 65843 Sulzbach (DE); SCHEIPER, Bodo, 65926 Frankfurt am Main (DE)
(74) Vertreter: Then, Johann
(86) Internationale Anmeldenummer: PCT/EP2009/000406
(87) Internationale Veröffentlichungsnummer: WO 2009/097970

(56) Entgegenhaltungen:
- EP-A- 1 391 451
- EP-A- 1 394 152
- CHACKALAMANNIL S ET AL: "Thrombin receptor (PAR-1) antagonists as novel antithrombotic agents" EXPERT OPINION ON THERAPEUTIC PATENTS, ASHLEY PUBLICATIONS, GB, Bd. 16, Nr. 4, 1. April 2006 (2006-04-01), Seiten 493-505, XP002415653 ISSN: 1354-3776

## Beschreibung

Die Erfindung betrifft neue Verbindungen der Formel I 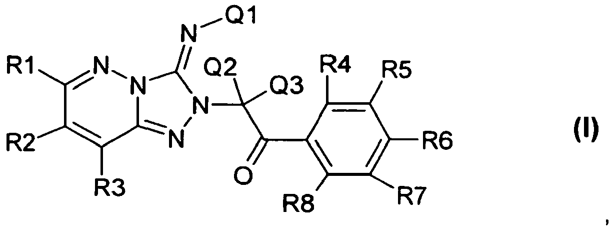 wobei R1, R2, R3, R4, R5, R6, R7, R8, Q1, Q2 und Q3 die unten bezeichnete Bedeutung haben. Die Verbindungen der Formel I haben antithrombotische Aktivität und inhibieren insbesondere den Protease-aktivierten Rezeptor 1 (PAR1). Die Erfindung betrifft ferner ein Verfahren zur Herstellung der Verbindung der Formel I und deren Verwendung als Arzneimittel.

Der Protease-aktivierte Rezeptor 1 (PAR1) ist ein Thrombin Rezeptor, der zur Klasse der G Protein-gekoppelten Rezeptoren (GPCR) gehört. Das Gen für PAR1 liegt auf Chromosom 5q13, besteht aus zwei Exonen und deckt eine Region von etwa 27 kb ab.

PAR1 wird unter anderem in Endothelzellen, glatten Muskelzellen, Fibroblasten, Neuronen und humanen Blutplättchen exprimiert. Auf Blutplättchen ist PAR1 ein wichtiger Rezeptor der Signalübertragung welcher an der Initiation der Aggregation von Blutplättchen beteiligt ist.

Die Aktivierung der PARs erfolgt über die proteolytische Abspaltung eines Teils des N-Terminus der PARs, wodurch eine neue N-terminale Sequenz freigelegt wird, die dann den Rezeptor aktiviert (Pharmacol Rev 54:203-217, 2002).

Die Blutgerinnung ist ein für das Überleben von Säugetieren wesentlicher Vorgang der Kontrolle des Blutstroms. Der Vorgang der Gerinnung und der nachfolgenden Auflösung des Gerinnsels nach erfolgter Wundheilung setzt nach einer Gefäßschädigung ein und lässt sich in vier Phasen einteilen:
1. Die Phase der vaskulären Konstriktion: Hierdurch wird der Blutverlust in das geschädigte Areal vermindert.
2. Die nächste Phase ist die der Plättchenadhäsion an das freigelegte Kollagen im Subendothel. Diese primäre Adhäsion an die Matrix aktiviert die Plättchen, die daraufhin verschiedene Aktivatoren sekretieren, die zur Verstärkung der Aktivierung führen. Diese Aktivatoren stimulieren zudem die weitere Rekrutierung neuer Plättchen zum Ort der Gefäßschädigung und fördern die Plättchenaggregation. Die Plättchen aggregieren an der Stelle des Gefäßwandschadens und bilden ein noch lockeres Plättchengerinnsel. Weiterhin führt die Aktivierung der Plättchen zur Präsentation von Phosphatidylserin und Phosphatidylinositol entlang der Zellmembranoberflächen. Die Exposition dieser Phospholipide ist zur Bindung und Aktiverung von Multienzymkomplexen der Blutgerinnungskaskade essentiell.
3. Das anfänglich noch lockere Plättchenaggregat wird durch Fibrin vernetzt. Wenn der Thrombus lediglich Plättchen und Fibrin enthält, handelt es sich um einen weißen Thrombus. Sind zusätzlich rote Blutkörperchen vorhanden, handelt es sich um einen roten Thrombus.
4. Nach Wundheilung wird der Thrombus durch die Einwirkung des Proteins Plasmin aufgelöst.

Zwei alternative Wege führen zur Bildung eines Fibringerinnsels, der intrinsische und der extrinsische Weg. Diese Wege werden durch unterschiedliche Mechanismen eingeleitet, in späterer Phase konvergieren sie jedoch zu einer gemeinsamen Wegstrecke der Gerinnungskaskade. Die Bildung eines roten Thrombus oder eines Gerinnsels auf dem Boden einer Gefäßwandabnormität ohne Wunde ist das Resultat des intrinsischen Weges. Die Fibringerinnselbildung als Antwort auf einen Gewebsschaden oder eine Verletzung ist das Resultat des extrinsischen Weges. Beide Wege beinhalten eine größere Anzahl von Proteinen, die als Gerinnungsfaktoren bekannt sind.

Der intrinsische Weg erfordert die Gerinnungsfaktoren VIII, IX, X, XI und XII sowie Präkallekrein, hochmolekulares Kininogen, Calciumionen und Phospholipide aus Plättchen. Jedes dieser Proteine führt zur Aktivierung des Faktors X.

Der intrinsische Weg wird eingeleitet, wenn Präkallekrein, hochmolekulares Kininogen Faktor XI und XII an eine negativ geladene Oberfläche binden. Dieser Moment wird als Kontaktphase bezeichnet. Die Exposition gegenüber einem Gefäßwandkollagen ist der primäre Stimulus der Kontaktphase. Resultat der Vorgänge der Kontaktphase ist die Umwandlung von Präkallekrein in Kallekrein, das wiederum den Faktor XII aktiviert. Faktor XIIa hydrolysiert weiteres Präkallekrein zu Kallekrein, so dass eine Aktivierung die Folge ist. Mit zunehmender Aktivierung von Faktor XII kommt es zur Aktivierung des Faktors XI, der zu einer Freisetzung von Bradykinin, einem Vasodilatator führt. Dadurch kommt es zur Beendigung der initialen Phase der Vasokonstriktion. Bradykinin entsteht aus dem hochmolekularen Kininogen. In Anwsenheit von Ca²⁺-Ionen aktiviert der Faktor XIa den Faktor IX. Faktor IX ist ein Proenzym, das Vitamin-K abhängige, c-Karboxiglutamat (GLA)-Reste enthält. Die Serinproteaseaktivität kommt nach Bindung von Ca²+-Ionen an diese GLA-Reste zum Tragen. Mehrere der Serinproteasen der Blutgerinnungskaskade (Faktoren II, VII, IX und X) enthalten derartige Vitamin-K-abhängige GLA-Reste. Faktor IXa spaltet den Faktor X und führt zur Aktivierung zum Faktor Xa. Voraussetzung für die Bildung von Faktor IXa ist die Bildung eines Proteasekomplexes aus von Ca²⁺-Ionen und den Faktoren VIIIa, IXa und X an der Oberfläche aktivierter Plättchen. Eine der Reaktionen aktivierter Plättchen ist die Präsentation von Phosphatidylserin und Phosphatidylinositol entlang der Oberflächen. Die Exposition dieser Phospholipide macht erst die Bildung des Proteasekomplexes möglich. Faktor VIII hat in diesem Vorgang die Funktion eines Rezeptors für die Faktoren 1Xa und X. Faktor VIII stellt daher einen Cofaktor in der Gerinnungskaskade dar. Die Aktivierung des Faktors VIII mit Bildung des Faktors VIIIa, dem eigentlichen Rezeptor, bedarf nur einer minimalen Menge von Thrombin. Mit Zunahme der Konzentration von Thrombin wird der Faktor VIIIa schließlich durch Thrombin weiter gespalten und inaktiviert. Diese duale Aktivität des Thrombins in Bezug zum Faktor VIII führt zu einer Selbstbegrenzung der Proteasekomplexbildung und damit zu einer Eingrenzung der Blutgerinnung.

Bei der Aktivierung von humanen Blutplättchen durch Thrombin spielen PAR1 und PAR4 eine zentrale Rolle; die Aktivierung dieser Rezeptoren führt in Blutplättchen zu morphologischen Veränderungen, Freisetzung von ADP und Aggregation der Blutplättchen (Nature 413:26-27, 2001).

Inhibitoren von PAR 1 werden beispielsweise in den Europäischen Patentanmeldungen EP1391451 oder EP1394152, den amerikanischen Patentanmeldungen US 6,063,847 und US 2004/0152736 sowie der internationalen Anmeldung WO 03/089428 beschrieben.

Die Verbindungen der Formel I zeigen ein hohe spezifisch Inhibierung des Protease-aktivierten Rezeptor 1 und zeichnen sich im Vergleich mit Verbindungen aus EP1391451 durch eine bessere Membrangängigkeit, beispielsweise durch Zellen der Darmwand, aus, die zu einer besseren Bioverfügbarkeit beitragen kann.

Die Verbindungen der Formel I eignen sich daher für die prophylaktische als auch für die therapeutische Anwendung am Menschen, die an Erkrankungen leiden, die mit Thrombosen, Embolien, Hyperkoagulabilität oder fibrotischen Veränderungen einhergehen. Beispiele solcher Erkrankungen sind Thrombose, tiefe Venenthrombose, Lungenembolien, Gehirninfarkt, Herzinfarkt, Bluthochdruck, Entzündliche Erkrankungen, Rheuma, Asthma, Glomerulonephritis oder Osteoporose. Die Verbindungen der Formel I können zur Sekundär-Prävention eingesetzt werden und eignen sich sowohl für eine akute als auch für eine Langzeittherapie.

Die Verbindungen der Formel I sind auch in Kombination mit Wirkstoffen einsetzbar, die über andere antithrombotische Prinzipien wie PAR 1 wirken.
1) Die Erfindung betrifft daher eine Verbindung der Formel I 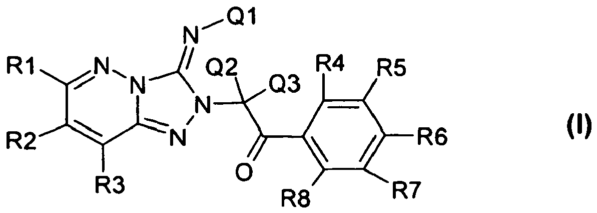 und/oder alle stereoisomeren oder tautomeren Formen der Verbindung der Formel 1 und/oder Gemische dieser Formen in jedem Verhältnis, und/oder ein physiologisch verträgliches Salz der Verbindung der Formel I, wobei
   - Q1: für Wasserstoffatom, -(C₁-C₆)-Alkyl, -(C₃-C₆)-Cycloalkyl, -OH, -O-(C₁-C₆)-Alkyl oder -O-(C₃-C₆)-Cycloalkyl steht, wobei Alkyl und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können,
   - Q2 und Q3: gleich oder verschieden sind und unabhängig voneinander für Wasserstoffatom, -(C₁-C₆)-Alkyl oder -(C₃-C₆)-Cycloalkyl stehen, wobei in Alkyl oder Cycloalkyl die Wasserstoffatome ganz oder teilweise durch Fluor ersetzt sein können,
   - R1, R2 und R3: gleich oder verschieden sind und unabhängig voneinander für Wasserstoffatom, -(C₁-C₆)-Alkyl, -(C₃-C₆)-Cycloalkyl, -O-(C₁-C₈)-Alkyl, -O-(C₃-C₆)-Cycloalkyl, -(C₀-C₄)-Alkylen-C(O)-N(R11)-R12, -(C₀-C₄)-Alkylen-C(O)-O-R11, -(C₀-C₄)-Alkylen-C(O)-R11, -(C₀-C₄)-Alkylen-N(R11)-R12, -(C₀-C₄)-Alkylen-N(R11)-C(O)-R12, Halogen, OH, -CN, -NO₂, -SO₂CH₃, -SO₂CF₃, -SF₅, -Si[-(C₁-C₄)-Alkyl]₃, -(C₁-C₆)-Alkylen-O-(C₁-C₆)-Alkyl, -O-(C₁-C₆)-Alkylen-O-(C₁-C₆)-Alkyl, -O-(C₀-C₄)-Alkylen-(C₅-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -O-(C₁-C₄)-Alkylen-(C₃-C₆)-Cycloalkyl, -(C₄-C₁₅)-Het, wobei Het unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₁-C₆)-Alkylen-O-(C₁-C₆)-Alkylen-O-(C₁-C₆)-Alkyl, stehen, wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist,
   -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder
   -O-(C₃-C₆)-Cycloalkyl, substituiert sind, oder wobei in Alkyl, Alkylen oder Cycloalkyl die Wasserstoffatome ganz oder teilweise durch Fluor ersetzt sein können, oder
   - R1 und R2 oder R2 und R3: bilden zusammen mit den Ringatomen, an die sie jeweils gebunden sind, 5- bis 8-gliedrigen Ring, wobei der Ring nur aus Kohlenstoffatomen besteht oder 1, 2 oder 3 dieser Atome durch N-, O- oder S-Atome ersetzt sind, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, wobei im 5- bis 8-gliedrigen gebildeten Ring, in Alkyl oder Cycloalkyl die Wasserstoffatome ganz oder teilweise durch Fluor ersetzt sein können,
   - R11 und R12: unabhängig voneinander für Wasserstoffatom, -(C₁-C₆)-Alkyl, -(C₃-C₆)-Cycloalkyl, -(C₀-C₄)-Alkylen-(C₆-₁₄)-Aryl, wobei Aryl unsubstituiert oder ein-, zwei- oder dreifach unabhängig voneinander durch -O-(C₁-₆)-Alkyl, -(C₁-C₄)-Alkyl, OH, -(C₃-C₆)-Cycloalkyl oder -O-(C₃-C₆)-Cycloalkyl, substituiert ist,
   -(C₀-C₄)-Alkylen-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆-Alkyl oder -O-(C₃-C₆)-Cycloalkyl substituiert ist,
   -SO₂CH₃ oder -SO₂CF₃ stehen,
   wobei in Alkyl, Alkylen oder Cycloalkyl die Wasserstoffatome ganz oder teilweise durch Fluor ersetzt sein können, oder
   - R11 und R12: in den Fragmenten "N(R11)-R12" und "N(R11)-C(O)-R12" für einen 5-bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH,
   -O(C₁-C₆)-Alkyl oder -O(C₃-C₆)-Cycloalkyl, substituiert ist, wobei im 5- bis 8-gliedrigen gebildeten Ring, in Alkyl oder Cycloalkyl die Wasserstoffatome ganz oder teilweise durch Fluor ersetzt sein können,
   - R4, R5, R6, R7 und R8: gleich oder verschieden sind und unabhängig voneinander für Wasserstoffatom, -(C₁-C₆)-Alkyl, -(C₃-C₆)-Cycloalkyl, OH, -CN, -NO₂, -O(C₁-C₈)-Alkyl, -O(C₃-C₆)-Cycloalkyl, -(C₀-C₄)-Alkylen-(CO)-N(R21)-R22,-SO₂CH₃, -SO₂CF₃, -(C₀-C₄)-Alkylen-C(O)-O-R21, Halogen, -SF₅, -(C₀-C₄)-Alkylen-C(O)-R21, -(C₀-C₄)-Alkylen-N(R21)-R22,
   -(C₀-C₄)-Alkylen-N(R21)-C(O)-R22, -(C₁-C₆)-Alkylen-O-(C₁-C₆)-Alkyl, -(C₀-C₆)-Alkylen-O-(C₁-C₆)-Alkylen-O-(C₁-C₆)-Alkyl, -Si[-(C₁-C₄)-Alkyl]₃, -(C₀-C₆)-Alkylen-O-(C₁-C₄)-Alkylen-(C₃-C₆)-Cycloalkyl, -(C₀-C₆)-Alkylen-O-(C₀-C₆)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, oder -(C₄-C₁₅)-Het stehen, wobei Het unsubstituiert oder ein-, zwei- oder dreifach unabhängig voneinander durch -O-(C₁-C₆)-Alkyl,
   -(C₁-C₄)-Alkyl, OH, -(C₃-C₆)-Cycloalkyl oder -O-(C₃-C₆)-Cycloalkyl, substituiert ist,
   wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist,
   -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder
   -O-(C₃-C₆)-Cycloalkyl, substituiert sind,
   wobei in Alkyl, Alkylen oder Cycloalkyl die Wasserstoffatome ganz oder teilweise durch Fluor ersetzt sein können, oder
   - R4 und R5, R5 und R6, R6 und R7 oder R7 und R8: bilden zusammen mit den Ringatomen, an die sie jeweils gebunden sind, einen 5- bis 8-gliedrigen Ring, wobei der Ring nur aus Kohlenstoffatomen besteht oder 1, 2 oder 3 dieser Atome durch N-, O- oder S-Atome ersetzt sind, wobei der Ring unsubstituiert oder ein- oder zweifach unabhängig voneinander durch
   -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei im 5- bis 8-gliedrigen gebildeten Ring, in Alkyl oder Cycloalkyl die Wasserstoffatome ganz oder teilweise durch Fluor ersetzt sein können,
   - R21 und R22: unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist,
   -(C₀-C₄)-Alkylen-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist,
   -SO₂CH₃ oder -SO₂CF₃ stehen,
   wobei in Alkyl, Alkylen oder Cycloalkyl die Wasserstoffatome ganz oder teilweise durch Fluor ersetzt sein können, oder
   - R21 und R22: in den Fragmenten "N(R21)-R22" und "N(R21)-C(O)-R22" für einen 5-bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH,
   -O-(C₁-C₆)-Alkyl oder -O-(C₃-C₆)-Cycloalkyl, substituiert ist, wobei im 5- bis 8-gliedrigen gebildeten Ring, in Alkyl oder Cycloalkyl die Wasserstoffatome ganz oder teilweise durch Fluor ersetzt sein können.
2) Bevorzugt ist eine Verbindung der Formel I, wobei
   - Q1, Q2 und Q3: gleich oder verschieden sind und unabhängig voneinander für Wasserstoffatom, -(C₁-C₆)-Alkyl oder -(C₃-C₆)-Cycloalkyl stehen, wobei in Alkyl oder Cycloalkyl die Wasserstoffatome ganz oder teilweise durch Fluor ersetzt sein können,
   - R1, R2 und R3: gleich oder verschieden sind und unabhängig voneinander für Wasserstoffatom, -(C₁-C₆)-Alkyl, -(C₃-C₆)-Cycloalkyl, -O-(C₁-C₈)-Alkyl, -O-(C₃-C₆)-Cycloalkyl, -(C₀-C₄)-Alkylen-C(O)-N(R11)-R12, -(C₀-C₄)-Alkylen-C(O)-O-R11, -(C₀-C₄)-Alkylen-C(O)-R11, -(C₀-C₄)-Alkylen-N(R11)-R12, -(C₀-C₄)-Alkylen-N(R11)-C(O)-R12, Halogen, OH, -CN, -NO₂, -SO₂CH₃, -SO₂CF₃, -SF₅, -Si[-(C₁-C₄)-Alkyl]₃, -(C₁-C₆)-Alkylen-O-(C₁-C₆)-Alkyl, -O-(C₁-C₆)-Alkylen-O-(C₁-C₆)-Alkyl, -O-(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl substituiert ist,
   -O-(C₁-C₄)-Alkylen-(C₃-C₆)-Cycloalkyl, -(C₄-C₁₅)-Het, wobei Het unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder
   -O-(C₁-C₆)-Alkylen-O-(C₁-C₆)-Alkylen-O-(C₁-C₆)-Alkyl, stehen, wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist,
   -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder
   -O-(C₃-C₆)-Cycloalkyl, substituiert sind, oder
   wobei in Alkyl, Alkylen oder Cycloalkyl die Wasserstoffatome ganz oder teilweise durch Fluor ersetzt sein können,
   mit der Maßgabe, das mindestens ein R1, R2 oder R3 nicht Wasserstoffatom ist oder
   - R1 und R2 oder R2 und R3: bilden zusammen mit den Ringatomen, an die sie jeweils gebunden sind, einen 5- bis 8-gliedrigen Ring, wobei der Ring nur aus Kohlenstoffatomen besteht oder 1, 2 oder 3 dieser Atome durch N-, O- oder S-Atome ersetzt sind, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, wobei im 5- bis 8-gliedrigen Ring, in Alkyl oder Cycloalkyl die Wasserstoffatome ganz oder teilweise durch Fluor ersetzt sein können,
   - R11 und R12: unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist,
   -(C₀-C₄)-Alkylen-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalky substituiert ist,
   -SO₂CH₃ oder -SO₂-CF₃ stehen,
   wobei in Alkyl, Alkylen oder Cycloalkyl die Wasserstoffatome ganz oder teilweise durch Fluor ersetzt sein können, oder
   - R11 und R12: in den Fragmenten "N(R11)-R12" und "N(R11)-C(O)-R12" für einen 5-bis 8-gliedrigen Ring stehen, der gemeinsam mit dem Stickstoffatom "N" oder der Gruppe "N-(CO)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH,
   -O-(C₁-C₆)-Alkyl oder -O-(C₃-C₆)-Cycloalkyl substituiert ist, wobei in Alkyl oder Cycloalkyl die Wasserstoffatome ganz oder teilweise durch Fluor ersetzt sein können,
   - R4, R5, R6, R7 und R8: gleich oder verschieden sind und unabhängig voneinander für Wasserstoffatom, -(C₁-C₆)-Alkyl, -(C₃-C₆)-Cycloalkyl, OH, -CN, -NO₂, -O-(C₁-C₈)-Alkyl, -O-(C₃-C₆)-Cycloalkyl, -(C₀-C₄)-Alkylen-(CO)-N(R21)-R22,-SO₂CH₃, -SO₂CF₃, -(C₀-C₄)-Alkylen-C(O)-O-R21, Halogen, -SF₅. -(C₀-C₄)-Alkylen-C(O)-R21, -(C₀-C₄)-Alkylen-N(R21)-R22, -(C₀-C₄)-Alkylen-N(R21)-C(O)-R22, -(C₁-C₆)-Alkylen-O-(C₁-C₆)-Alkyl, -(C₀-C₆)-Alkylen-O-(C₁-C₆)-Alkylen-O-(C₁-C₆)-Alkyl, -Si[-(C₁-C₄)-Alkyl]₃, -(C₀-C₆)-Alkylen-O-(C₁-C₄)-Alkylen-(C₃-C₆)-Cycloalkyl, -(C₀-C₆)-Alkylen-O-(C₀-C₆)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, oder -(C₄-C₁₅)-Het stehen, wobei Het unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist,
   wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist,
   -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder
   -O-(C₃-C₆)-Cycloalkyl, substituiert sindt, oder
   wobei in Alkyl, Alkylen oder Cycloalkyl die Wasserstoffatome ganz oder teilweise durch Fluor ersetzt sein können,
   mit der Maßgabe, das mindestens ein R4, R5, R6, R7 oder R8 nicht Wasserstoffatom ist, oder
   - R4 und R5, R5 und R6, R6 und R7 oder R7 und R8: bilden zusammen mit den Ringatomen, an die sie jeweils gebunden sind, einen 5- bis 8-gliedrigen Ring, wobei der Ring nur aus Kohlenstoffatomen besteht oder 1, 2 oder 3 dieser Atome durch N-, O- oder S-Atome ersetzt sind, wobei der Ring unsubstituiert oder ein- oder zweifach unabhängig voneinander durch
   -(C₁-C₄)-Alkyl, -(C₃-C₆)-Cycloalkyl, OH, -O-(C₁-C₆)-Alkyl oder
   -O-(C₃-C₆)-Cycloalkyl, substituiert ist, wobei im 5- bis 8-gliedrigen gebildeten Ring, in Alkyl oder Cycloalkyl die Wasserstoffatome ganz oder teilweise durch Fluor ersetzt sein können,
   - R21 und R22: unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist,
   -(C₀-C₄)-Alkylen-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist,
   -SO₂CH₃ oder -SO₂CF₃ stehen,
   wobei in Alkyl, Alkylen oder Cycloalkyl die Wasserstoffatome ganz oder teilweise durch Fluor ersetzt sein können, oder
   - R21 und R22: in den Fragmenten "N(R21)-R22" und "N(R21)-C(O)-R22" für einen 5-bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH,
   -O-(C₁-C₆)-Alkyl oder -O-(C₃-C₆)-Cycloalkyl, substituiert ist, wobei im 5- bis 8-gliedrigen gebildeten Ring, in Alkyl oder Cycloalkyl die Wasserstoffatome ganz oder teilweise durch Fluor ersetzt sein können.
3) Besonders bevorzugt ist eine Verbindung der Formel I, wobei
   - Q1, Q2 und Q3: gleich oder verschieden sind und unabhängig voneinander für Wasserstoffatom, -(C₁-C₆)-Alkyl oder -(C₃-C₆)-Cycloalkyl stehen, wobei in Alkyl oder Cycloalkyl die Wasserstoffatome ganz oder teilweise durch Fluor ersetzt sein können,
   - R1, R2 und R3: gleich oder verschieden sind und unabhängig voneinander für Wasserstoffatom, -(C₁-C₆)-Alkyl, -(C₃-C₆)-Cycloalkyl, -O-(C₁-C₈)-Alkyl, -O-(C₃-C₆)-Cycloalkyl, -(C₀-C₄)-Alkylen-C(O)-N(R11)-R12, -(C₀-C₄)-Alkylen-C(O)-O-R11, -(C₀-C₄)-Alkylen-C(O)-R11, -(C₀-C₄)-Alkylen-N(R11)-R12, -(C₀-C₄)-Alkylen-N(R11)-C(O)-R12, Halogen, OH, -CN, -NO₂, -SO₂CH₃, -Si[-(C₁-C₄)-Alkyl]₃, -(C₁-C₆)-Alkylen-O-(C₁-C₆)-Alkyl, -O-(C₁-C₆)-Alkylen-O-(C₁-C₆)-Alkyl, -O-(C₀-C₄)-Alkylen-(C₆-C₁₄)-Aryl, -O-(C₁-C₄)-Alkylen-(C₃-C₄)-Cycloalkyl, -(C₄-C₁₅)-Het oder -O-(C₁-C₆)-Alkylen-O-(C₁-C₆)-Alkylen-O-(C₁-C₆)-Alkyl stehen,
   wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert sind, oder wobei in Alkyl, Alkylen oder Cycloalkyl die Wasserstoffatome ganz oder teilweise durch Fluor ersetzt sein können,
   mit der Maßgabe, das mindestens ein R1, R2 oder R3 nicht Wasserstoffatom ist oder
   - R1 und R2 oder R2 und R3: bilden zusammen mit den Ringatomen, an die sie jeweils gebunden sind, einen Ring ausgewählt aus der Gruppe 2,3,5,6,7,8-hexa-hydro-1,2,3a,4,5,8-hexaaza-cyclopenta[b]naphthalin; 2,6,7,8-tetrahydro-3H-5-oxa-1,2,3a,4,8-pentaaza-cyclopenta[b]naphthalin; 2,3,6,7-tetrahydro-5,8-dioxa-1,2,3a,4-tetraaza-cyclopenta[b]naphthalin; 2,3,6,7-tetrahydro-5H-8-oxa-1,2,3a,4,5-pentaaza-cyclopenta[b]naphthalin; 2,6,7,8-tetrahydro-3H-5-thia-1,2,3a,4,8-pentaaza-cyclopenta[b]naphthalin; 2,3,6,7,8,9-hexahydro-1,2,3a,4,6,9-hexaaza-cyclopenta[a]naphthalin; 2,3-dihydro-5,7-dioxa-1,2,3a,4-tetraaza-s-indacin; 2,6,7,8-tetrahydro-3H-cyclopenta[e][1,2,4]triazolo[4,3-b]pyridazin; 2,7,8,9-tetrahydro-3H-cyclopenta[d][1,2,4]triazolo[4,3-b]pyridazin und 2,3,6a,9a-tetrahydro-[1,3]dioxolo[4,5-d][1,2,4]triazolo[4,3-b]pyridazin, wobei der Ring unsubstituiert oder ein- oder zweifach unabhängig voneinander durch-(C₁-C₄)-Alkyl, -(C₃-C₆)-Cycloalkyl, OH, -O-(C₁-C₆)-Alkyl oder -O-(C₃-C₆)-Cycloalkyl substituiert ist, wobei in Alkyl oder Cycloalkyl die Wasserstoffatome ganz oder teilweise durch Fluor ersetzt sein können,
   - R11 und R12: unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, -(C₀-C₄)-Alkylen-(C₄-C₁₅)-Het, -SO₂CH₃ oder -SO₂CF₃ stehen, wobei in Alkyl, Alkylen oder Cycloalkyl die Wasserstoffatome ganz oder teilweise durch Fluor ersetzt sein können, oder
   - R11 und R12: in den Fragmenten "N(R11)-R12" und "N(R11)-C(O)-R12" für einen 5-bis 8-gliedrigen Ring stehen, ausgewählt aus der Gruppe Azetidinyl, Pyrrolidinyl, Piperidinyl, Piperazinyl, Azepinyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3- onyl, wobei der Ring unsubstituiert oder ein- oder zweifach unabhängig voneinander durch -(C₁-C₄)-Alkyl, -(C₃-C₆)-Cycloalkyl, OH,
   -O-(C₁-C₆)-Alkyl oder -O-(C₃-C₆)-Cycloalkyl substituiert ist, wobei in Alkyl oder Cycloalkyl die Wasserstoffatome ganz oder teilweise durch Fluor ersetzt sein können,
   - R4, R5, R6, R7 und R8: gleich oder verschieden sind und unabhängig voneinander für Wasserstoffatom, -(C₁-C₆)-Alkyl, -(C₃-C₆)-Cycloalkyl, OH, -CN, -NO₂, -O-(C₁-C₈)-Alkyl, -O-(C₃-C₆)-Cycloalkyl, -(C₀-C₄)-Alkylen-(CO)-N(R21)-R22,-SO₂CH₃, -SO₂CF₃, -(C₀-C₄)-Alkylen-C(O)-O-R21, Halogen, -SF₅, -(C₀-C₄)-Alkylen-C(O)-R21, -(C₀-C₄)-Alkylen-N(R21)-R22, -(C₀-C₄)-Alkylen-N(R21)-C(O)-R22, -(C₁-C₆)-Alkylen-O-(C₁-C₆)-Alkyl, -(C₀-C₆)-Alkylen-O-(C₁-C₆)-Alkylen-O-(C₁-C₆)-Alkyl, -Si[-(C₁-C₄)-Alkyl]₃, -(C₀-C₆)-Alkylen-O-(C₁-C₄)-Alkylen-(C₃-C₆)-Cycloalkyl, -(C₀-C₆)-Alkylen-O-(C₀-C₆)-Alkylen-(C₆-C₁₄)-Aryl oder -(C₄-C₁₅)-Het stehen, wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalky substituiert ist,
   -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder
   -O-(C₃-C₆)-Cycloalkyl, substituiert ist, oder
   wobei in Alkyl, Alkylen oder Cycloalkyl die Wasserstoffatome ganz oder teilweise durch Fluor ersetzt sein können,
   mit der Maßgabe, das mindestens ein R4, R5, R6, R7 oder R8 nicht Wasserstoffatom ist, oder
   - R4 und R5, R5 und R6, R6 und R7 oder R7 und R8: bilden zusammen mit den Ringatomen, an die sie jeweils gebunden sind, einen 5- bis 8-gliedrigen Ring ausgewählt aus der Gruppe 2,3-Dihydro-benzo[1,4]dioxin; 3,4-Dihydro-2H-benzo[1,4]oxazin; 1,2,3,4-Tetrahydro-quinoxalin; Benzo[1,3]dioxol; 3,4-Dihydro-2H-benzo[1,4]thiazin und 2,3,4,5-Tetrahydro-1H-benzo[b][1,4]diazepin, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können,
   - R21 und R22: unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, -(C₀-C₄)-Alkylen-(C₄-C₁₅)-Het, -SO₂CH₃ oder -SO₂CF₃ stehen, wobei in Alkyl, Alkylen oder Cycloalkyl die Wasserstoffatome ganz oder teilweise durch Fluor ersetzt sein können, oder
   - R21 und R22: in den Fragmenten "N(R21)-R22" und "N(R21)-C(O)-R22" für einen 5-bis 8-gliedrigen Ring stehen, ausgewählt aus der Gruppe Azetidinyl, Pyrrolidinyl, Piperidinyl, Piperazinyl, Azepinyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können.
4) Die Erfindung betrifft ferner eine Verbindung der Formel I, wobei
   - Q1, Q2 und Q3: gleich sind und für jeweils ein Wasserstoffatom stehen,
   - R1, R2 und R3: gleich oder verschieden sind und unabhängig voneinander für Wasserstoffatom, -(C₁-C₆)-Alkyl, -O-(C₁-C₈)-Alkyl, -O-(C₃-C₆)-Cycloalkyl, -(C₀-C₄)-Alkylen-C(O)-N(R11)-R12, -(C₀-C₄)-Alkylen-N(R11)-R12, Halogen, -O-(C₁-C₆)-Alkylen-O-(C₁-C₆)-Alkyl, -O-(C₀-C₄)-Alkylen-Phenyl oder -O-(C₁-C₆)-Alkylen-O-(C₁-C₆)-Alkylen-O-(C₁-C₆)-Alkyl, stehen, wobei Alkyl oder Alkylen jeweils unsubstituiert oder ein- oder zweifach durch,-O-(C₁-C₆)-Alkyl substituiert ist, wobei in Alkyl oder Alkylen die Wasserstoffatome ganz oder teilweise durch Fluor ersetzt sein können,
   mit der Maßgabe, das mindestens ein R1. R2 oder R3 nicht Wasserstoffatom ist,
   - R11 und R12: unabhängig voneinander für Wasserstoffatom oder -(C₁-C₆)-Alkyl stehen, oder
   - R11 und R12: in dem Fragment "N(R11)-R12" für einen 5- bis 8-gliedrigen Ring stehen, ausgewählt aus der Gruppe Azetidinyl, Pyrrolidinyl, Piperidinyl, Piperazinyl, Azepinyl, Imidazolyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl,
   - R4, R5, R6, R7 und R8: gleich oder verschieden sind und unabhängig voneinander für Wasserstoffatom, -(C₁-C₆)-Alkyl, OH, -O-(C₁-C₈)-Alkyl, Halogen, -SF₅, -(C₀-C₄)-Alkylen-N(R21)-R22, -(C₀-C₄)-Alkylen-N(R21)-C(O)-R22, -CF₃, -(C₀-C₆)-Alkylen-O-(C₁-C₆)-Alkylen-O-(C₁-C₆)-Alkyl,
   -(C₀-C₆)-Alkylen-O-(C₁-C₄)-Alkylen-(C₃-C₆)-Cycloalkyl, oder
   -(C₀-C₆)-Alkylen-O-(C₁-C₆)-Alkylen-Phenyl stehen,
   wobei Alkyl oder Alkylen jeweils unsubstituiert oder ein- oder zweifach durch -O-(C₁-C₆)-Alkyl substituiert ist,
   mit der Maßgabe, das mindestens ein R4, R5, R6, R7 oder R8 nicht Wasserstoffatom ist, oder
   - R4 und R5, R5 und R6, R6 oder R7 oder R7 und R8: bilden zusammen mit den Ringatomen, an die sie jeweils gebunden sind, einen fünf- bis achtgliedrigen Ring ausgewählt aus der Gruppe 2,3-Dihydro-benzo[1,4]dioxin; 3,4-Dihydro-2H-benzo[1,4]oxazin; 1,2,3,4-Tetrahydro-quinoxalin; Benzo[1,3]dioxol; 3,4-Dihydro-2H-benzo[1,4]thiazin und 2,3,4,5-Tetrahydro-1 H-benzo[b][1,4]diazepin, wobei der Ring unsubstituiert oder ein- oder zweifach durch -(C₁-C₄)-Alkyl substituiert ist,
   - R21 und R22: unabhängig voneinander für Wasserstoffatom oder -(C₁-C₆)-Alkyl stehen, oder
   - R21 und R22: in dem Fragment "N(R21)-R22" für einen 5- bis 8-gliedrigen Ring stehen, ausgewählt aus der Gruppe Azetidinyl, Pyrrolidinyl, Piperidinyl, Piperazinyl, Azepinyl, Imidazolyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl.
5) Außerordentlich bevorzugt sind Verbindungen der Formel I, wobei folgende Verbindungen umfasst sind, 2-(6-Chlor-3-imino-[1,2,4]triazolo[4,3-b]pyridazin-2-yl)-1-(3,5-di-tert-butyl-4-hydroxyphenyl)-ethanon, 1-(3-tert-Butyl-4-methoxy-5-morpholin-4-yl-phenyl)-2-(3-imino-6-isopropoxy-[1,2,4]triazolo[4,3-b]pyridazin-2-yl)-ethanon, 1-(3-tert-Butyl-4-methoxy-5-morpholin-4-yl-phenyl)-2-(3-imino-6-methoxy-[1,2,4]triazolo[4,3-b]pyridazin-2-yl)-ethanon, 2-(6-Ethoxy-3-imino-[1,2,4]triazolo[4,3-b]pyridazin-2-yl)-1-(4-methoxy-3-morpholin-4-yl-5-trifluoromethyl-phenyl)-ethanon, N-[3-[2-(6-Ethoxy-3-imino-[1,2,4]triazolo[4,3-b]pyridazin-2-yl)-acetyl]-5-(pentafluor-sulfanyl)-phenyl]-N-methyl-acetamid, N-[3-[2-(6-Ethoxy-3-imino-[1,2,4]triazolo[4,3-b]pyridazin-2-yl)-acetyl]-5-(pentafluorsulfanyl)-phenyl]-acetamid als Trifluoressigsäuresalz, 1-(3-tert-Butyl-4-methoxy-5-morpholin-4-yl-phenyl)-2-(6-ethoxy-3-imino-[1,2,4]triazolo[4,3-b]pyridazin-2-yl)-ethanon als Trifluoressigsäuresalz, 1-(3-tert-Butyl-4-methoxy-5-morpholin-4-yl-phenyl)-2-(6-cyclopentyloxy-3-imino-[1,2,4]triazolo[4,3-b]pyridazin-2-yl)-ethanon, 1-(3-tert-Butyl-4-methoxy-5-morpholin-4-yl-phenyl)-2-(6-cyclobutoxy-3-imino-[1,2,4]triazolo[4,3-b]pyridazin-2-yl)-ethanon, 1-(3-tert-Butyl-4-methoxy-5-morpholin-4-yl-phenyl)-2-(3-imino-6-phenoxy-[1,2,4]triazolo[4,3-b]pyridazin-2-yl)-ethanon, 2-(6-Benzyloxy-3-imino-[1,2,4]triazolo[4,3-b]pyridazin-2-yl)-1-(3-tert-butyl-4-methoxy-5-morpholin-4-yl-phenyl)-ethanon, 1-(3-tert-Butyl-4-methoxy-5-morpholin-4-yl-phenyl)-2-(6-cyclohexyloxy-3-imino-[1,2,4]triazolo[4,3-b]pyridazin-2-yl)-ethanon, 1-(3-tert-Butyl-4-methoxy-5-morpholin-4-yl-phenyl)-2-[3-imino-6-(2,2,2-trifluoro-ethoxy)-[1,2,4]triazolo[4,3-b]pyridazin-2-yl]-ethanon, 1-(3-tert-Butyl-4-methoxy-5-morpholin-4-yl-phenyl)-2-[6-(1-ethyl-propoxy)-3-imino-[1,2,4]triazolo[4,3-b]pyridazin-2-yl]-ethanon als Trifluoressigsäuresalz, 1-(3-tert-Butyl-4-methoxy-5-morpholin-4-yl-phenyl)-2-(6-cyclopropylmethoxy-3-imino-[1,2,4]triazolo[4,3-b]pyridazin-2-yl)-ethanon, 2-[6-(1-Ethyl-propoxy)-3-imino-[1,2,4]triazolo[4,3-b]pyridazin-2-yl]-1-[3-methylamino-5-(pentafluor-sulfanyl)-phenyl]-ethanon, 2-[6-(1-Ethyl-propoxy)-3-imino-[1,2,4]triazolo[4,3-b]pyridazin-2-yl]-1-[3-methoxy-5-(pentafluor-sulfanyl)-phenyl]-ethanon, 1-(3-tert-Butyl-5-ethoxymethyl-phenyl)-2-[6-(1-ethyl-propoxy)-3-imino-[1,2,4]triazolo[4,3-b]pyridazin-2-yl]-ethanon, 1-(3-tert-Butyl-5-cyclopropylmethoxymethyl-phenyl)-2-[6-(1-ethyl-propoxy)-3-imino-[1,2,4]triazolo[4,3-b]pyridazin-2-yl]-ethanon, 1-(3-tert-Butyl-4,5-diethoxy-phenyl)-2-[6-(1-ethyl-propoxy)-3-imino-[1,2,4]triazolo[4,3-b]pyridazin-2-yl)-ethanon, 1-(3-tert-Butyl-5-ethoxy-phenyl)-2-[6-(1-ethyl-propoxy)-3-imino-[1,2,4]triazolo[4,3-b]pyridazin-2-yl]-ethanon, 1-(3-tert-Butyl-5-propoxymethyl-phenyl)-2-[6-(1-ethyl-propoxy)-3-imino-[1,2,4]triazolo[4,3-b]pyridazin-2-yl]-ethanon, 1-(3-tert-Butyl-4,5-bis-cyclopropylmethoxy-phenyl)-2-[6-(1-ethyl-propoxy)-3-imino-[1,2,4]triazolo[4,3-b]pyridazin-2-yl]-ethanon, 2-[6-(1-Ethyl-propoxy)-3-imino-[1,2,4]triazolo[4,3-b]pyridazin-2-yl]-1-(3-methoxy-5-trifluoromethyl-phenyl)-ethanon, 1-(3-tert-Butyl-5-methoxy-phenyl)-2-[6-(1-ethyl-propoxy)-3-imino-[1,2,4]triazolo[4,3-b]pyridazin-2-yl]-ethanon, 1-(3-tert-Butyl-5-cyclopropylmethoxy-phenyl)-2-[6-(1-ethyl-propoxy)-3-imino-[1,2,4]triazolo[4,3-b]pyridazin-2-yl]-ethanon, 1-(3-tert-Butyl-5-cyclobutylmethoxy-phenyl)-2-[6-(1-ethyl-propoxy)-3-imino-[1,2,4]triazolo[4,3-b]pyridazin-2-yl]-ethanon, 1-(3-Benzyloxymethyl-5-tert-butyl-phenyl)-2-[6-(1-ethyl-propoxy)-3-imino-[1,2,4]triazolo[4,3-b]pyridazin-2-yl]-ethanon, 1-(3-Cyclohexylmethoxy-4,5-dimethoxy-phenyl)-2-[6-(1-ethyl-propoxy)-3-imino-[1,2,4]triazolo[4,3-b]pyridazin-2-yl]-ethanon, 2-(6-Butoxy-3-imino-[1,2,4]triazolo[4,3-b]pyridazin-2-yl)-1-(3-tert-butyl-5-methoxymethyl-phenyl)-ethanon, 1-(3-Chlor-5-methoxy-phenyl)-2-[6-(1-ethyl-propoxy)-3-imino-[1,2,4]triazolo[4,3-b]pyridazin-2-yl]-ethanon, 1-(8-tert-Butyl-4-methyl-3,4-dihyd ro-2H-benzo[1,4]oxazin-6-yl)-2-[6-(1 1-ethyl-propoxy)-3-imino-[1,2,4]triazolo[4,3-b]pyridazin-2-yl]-ethanon als Trifluoressigsäuresalz, 1-(3-tert-Butyl-4-methoxy-5-morpholin-4-yl-phenyl)-2-(3-imino-6-piperidin-1-yl-[1,2,4]triazolo[4,3-b]pyridazin-2-yl)-ethanon, 1-(3-tert-Butyl-4-methoxy-5-morpholin-4-yl-phenyl)-2-(6-diethylamino-3-imino-[1,2,4]triazolo[4,3-b]pyridazin-2-yl)-ethanon, 2-[6-(1-Ethyl-propoxy)-3-imino-[1,2,4]triazolo[4,3-b]pyridazin-2-yl]-1-(3-isopropyl-5-methoxy-phenyl)-ethanon, 1-(3-Cyclohexylmethoxy-5-ethoxy-phenyl)-2-[6-(1-ethyl-propoxy)-3-imino-[1,2,4]triazolo[4,3-b]pyridazin-2-yl]-ethanon, 1-(3-Brom-5-methoxy-phenyl)-2-[6-(1-ethyl-propoxy)-3-imino-[1,2,4]triazolo[4,3-b]pyridazin-2-yl]-ethanon, 1-[3-(3,3-Dimethyl-butoxy)-5-methoxy-phenyl]-2-[6-(1-ethyl-propoxy)-3-imino-[1,2,4]triazolo[4,3-b]pyridazin-2-yl]-ethanon, 1-[3-(3,3-Dimethyl-butoxy)-5-ethoxy-phenyl]-2-[6-(1-ethyl-propoxy)-3-imino-[1,2,4]triazolo[4,3-b]pyridazin-2-yl]-ethanon, 1-(3-Cyclohexylmethoxy-5-methoxy-phenyl)-2-[6-(1-ethyl-propoxy)-3-imino-[1,2,4]triazolo[4,3-b]pyridazin-2-yl]-ethanon, 1-(3-Brom-4,5-dimethoxy-phenyl)-2-[6-(1-ethyl-propoxy)-3-imino-[1,2,4]triazolo[4,3-b]pyridazin-2-yl]-ethanon, 2-(6-Diethylamino-3-imino-[1, 2,4]triazolo[4,3-b]pyridazin-2-yl)-1-[3-methoxy-5-(pentafluor-sulfanyl)-phenyl]-ethanon, 1-(3-tert-Butyl-4-methoxy-5-morpholin-4-yl-phenyl)-2-(3-imino-6-morpholin-4-yl-[1,2,4]triazolo[4,3-b]pyridazin-2-yl)-ethanon, 1-(3-tert-Butyl-4-methoxy-5-morpholin-4-yl-phenyl)-2-(6-chloro-3-imino-8-methyl-[1,2,4]triazolo[4,3-b]pyridazin-2-yl)-ethanon, 1-(3-tert-Butyl-4-methoxy-5-morpholin-4-yl-phenyl)-2-(6-imidazol-1-yl-3-imino-[1,2,4]triazolo[4,3-b]pyridazin-2-yl)-ethanon, 1-(5-Brom-2,3-dimethoxy-phenyl)-2-[6-(1-ethyl-propoxy)-3-imino-[1,2,4]triazolo[4,3-b]pyridazin-2-yl]-ethanon, 1-(3-tert-Butyl-5-methoxy-phenyl)-2-{3-imino-6-[2-(2-methoxy-ethoxy)-ethoxy]-[1,2,4]triazolo[4,3-b]pyridazin-2-yl}-ethanon, 1-(3-Chlor-4,5-dimethoxy-phenyl)-2-[6-(1-ethyl-propoxy)-3-imino-[1,2,4]triazolo[4,3-b]pyridazin-2-yl]-ethanon, 1-(3-tert-Butyl-4-methoxy-5-morpholin-4-yl-phenyl)-2-(6-diethylamino-3-imino-8-methyl-[1,2,4]triazolo[4,3-b]pyridazin-2-yl)-ethanon als Trifluoressigsäuresalz, 1-[3-tert-Butyl-5-(2-methoxy-ethoxy)-phenyl]-2-[6-(1-ethyl-propoxy)-3-imino-[1,2,4]triazolo[4,3-b]pyridazin-2-yl]-ethanon, 1-(3-tert-Butyl-5-methoxy-phenyl)-2-[3-imino-6-(2-methoxy-ethoxy)-[1,2,4]triazolo[4,3-b]pyridazin-2-yl]-ethanon, 1-(3-tert-Butyl-5-methoxymethyl-phenyl)-2-[3-imino-6-(2-methoxy-ethoxy)-[1,2,4]triazolo[4,3-b]pyridazin-2-yl]-ethanon, 2-[6-(1-Ethyl-propoxy)-3-imino-[1,2,4]triazolo[4,3-b]pyridazin-2-yl]-1-[3-morpholin-4-yl-5-(pentafluor-sulfanyl)-phenyl]-ethanon, 1-(3-tert-Butyl-4-methoxy-5-morpholin-4-yl-phenyl)-2-(6-ethoxy-3-imino-8-methyl-[1,2,4]triazolo[4,3-b]pyridazin-2-yl)-ethanon, 2-[6-(1-Ethyl-propoxy)-3-imino-8-methyl-[1,2,4]triazolo[4,3-b]pyridazin-2-yl]-1-(5-methoxy-3-pentafluorsulfanyl-phenyl)-ethanon, 1-(3-tert-Butyl-4-methoxy-5-morpholin-4-yl-phenyl)-2-(6-chlor-3-imino-[1,2,4]triazolo[4,3-b]pyridazin-2-yl)-ethanon, 1-[3-tert-Butyl-5-(2-methoxy-ethoxymethyl)-phenyl]-2-[6-(1-ethyl-propoxy)-3-imino-[1,2,4]triazolo[4,3-b]pyridazin-2-yl]-ethanon, 2-[6-(1-Ethyl-propoxy)-3-imino-[1,2,4]triazolo[4,3-b]pyridazin-2-yl]-1-[3-(2-methoxy-ethoxy)-5-(pentafluoro-sulfanyl)-phenyl]-ethanon, 2-(6-Ethoxy-3-imino-8-methyl-[1,2,4]triazolo[4,3-b]pyridazin-2-yl)-1-[3-methoxy-5-(pentafluor-sulfanyl)-phenyl]-ethanon, 2-[6-(1-Ethyl-propoxy)-3-imino-[1,2,4]triazolo[4,3-b]pyridazin-2-yl]-1-[3-(3-methoxy-propoxy)-5-(pentafluoro-sulfanyl)-phenyl]-ethanon, 2-[2-(3-tert-Butyl-4-methoxy-5-morpholin-4-yl-phenyl)-2-oxo-ethyl]-6-chlor-3-imino-2,3-dihydro-[1,2,4]triazolo[4,3-b]pyridazin-8-carbonsäure-diethylamid, 6-Chlor-3-imino-2-{2-[3-methoxy-5-(pentafluorsulfanyl)-phenyl]-2-oxo-ethyl}-2,3-dihydro-[1,2,4]triazolo[4,3-b]pyridazin-8-carbonsäure-diethylamid, 2-(6-Ethyl-3-imino-[1,2,4]triazolo[4,3-b]pyridazin-2-yl)-1-[3-methoxy-5-(pentafluorsulfanyl)-phenyl]-ethanon, 1-(3-tert-Butyl-4-methoxy-5-morpholin-4-yl-phenyl)-2-(6-ethyl-3-imino-[1,2,4]triazolo[4,3-b]pyridazin-2-yl)-ethanon, 1-[3-tert-Butyl-5-(3-methoxy-propoxy)-phenyl]-2-[6-(1-ethyl-propoxy)-3-imino-8-methyl-[1,2,4]triazolo[4,3-b]pyridazin-2-yl]-ethanon, 1-[3-tert-Butyl'5-(2-methoxy-ethoxy)-phenyl]-2-[6-(1 1-ethyl-propoxy)-3-imino-8-methyl-[1,2,4]triazolo[4,3-b]pyridazin-2-yl]-ethanon, 1-(3-tert-Butyl-5-cyclopropylmethoxy-phenyl)-2-[6-(1-ethyl-propoxy)-3-imino-8-methyl-[1,2,4]triazolo[4,3-b]pyridazin-2-yl]-ethanon, 1-(3-tert-Butyl-5-methoxy-phenyl)-2-[6-(1-ethyl-propoxy)-3-imino-8-methyl-[1,2,4]triazolo[4,3-b]pyridazin-2-yl]-ethanon, 1-[3-tert-Butyl-5-(3-methoxy-propoxy)-phenyl]-2-[6-(1-ethyl-propoxy)-3-imino-7-methyl-[1,2,4]triazolo[4,3-b]pyridazin-2-yl]-ethanon, 2-(6-Chlor-3-imino-7,8-dimethyl-[1,2,4]triazolo[4,3-b]pyridazin-2-yl)-1-[3-methoxy-5-(pentafluorsulfanyl)-phenyl]-ethanon, 1-(3-tert-Butyl-4-methoxy-5-morpholin-4-yl-phenyl)-2-(6-chlor-3-imino-7,8-dimethyl-[1,2,4]triazolo[4,3-b]pyridazin-2-yl)-ethanon, 6-Chlor-3-imino-2-{2-[3-methoxy-5-(pentafluor-sulfanyl)-phenyl]-2-oxo-ethyl}-2,3-dihydro-[1,2,4]triazolo[4,3-b]pyridazin-7-carbonsäure-diethylamid, 2-[2-(3-tert-Butyl-4-methoxy-5-morpholin-4-yl-phenyl)-2-oxo-ethyl]-6-chlor-3-imino-2,3-dihydro-[1,2,4]triazolo[4,3-b]pyridazine-7-carbonsäure-diethylamid, 1-[3-tert-Butyl-5-(3-methoxy-propoxy)-phenyl]-2-(6-ethoxy-3-imino-7-methyl-[1,2,4]triazolo[4,3-b]pyridazin-2-yl)-ethanon oder 1-[3-tert-Butyl-5-(2-methoxy-ethoxy)-phenyl]-2-[6-(1-ethyl-propoxy)-3-imino-7-methyl-[1,2,4]triazolo[4,3-b]pyridazin-2-yl]-ethanon. Unter dem Begriff "(C₁-C₄)-Alkyl" oder "(C₁-C₆)-Alkyl" werden Kohlenwasserstoffreste verstanden, deren Kohlenstoffkette geradkettig oder verzweigt ist und 1 bis 4 Kohlenstoffatome oder 1 bis 6 Kohlenstoffatome enthält, beispielsweise Methyl, Ethyl, Propyl, Isopropyl, Butyl, Isobutyl, sec-Butyl, tertiär-Butyl, Pentyl, Isopentyl, Neopentyl, 1-Ethylpropyl, Hexyl, 2,3-Dimethylbutyl oder Neohexyl.
Unter dem Begriff "-(C₀-C₄)-Alkylen" oder "-(C₁-C₆)-Alkylen" werden
Kohlenwasserstoffreste verstanden, deren Kohlenstoffkette geradkettig oder verzweigt ist und 1 bis 4 oder 1-6 Kohlenstoffatome enthält, beispielsweise Methylen, Ethylen, 1-Methylmethylen, Propylen, 1-Methylethylen, Butylen, 1-Propylmethylen, 1-Ethyl-1-methylmethylen, 1,2-Dimethylethylen, 1,1-Dimethylmethylen, 1-Ethylethylen, 1-Methylpropylen, 2-Methylpropylen, Pentylen, 1-Methylbutylen, Hexylen, 1-Methylpentylen. "-C₀-Alkylen" ist eine kovalente Bindung.

Unter dem Begriff "-O-(C₁-C₆)-Alkyl" oder "O-(C₁-C₈)-Alkyl" werden Alkoxyreste verstanden, deren Kohlenstoffkette geradkettig oder verzweigt ist und 1 bis 6 oder 1 bis 8 Kohlenstoffatome enthält, beispielsweise Methoxy, Ethoxy, Propoxy, Iso-Propoxy, Butoxy, Iso-Butoxy, tertiär-Butoxy, 1-Pentoxy, 2-Pentoxy, 3-Pentoxy, 1-Hexoxy, 2-Hexoxy, 3-Hexoxy, 1-Heptoxy, 2-Heptoxy, 3-Heptoxy, 4-Heptoxy, 2,4-Dimethyl-pentan-3-oxy, 1-Octoxy, 2-Octoxy, 3-Octoxy, 2,2,4-Trimethyl-pentan-3-oxy, 2,3,4-Trimethylpentan-3-oxy oder 4-Octoxy.

Unter dem Begriff "(C₃-C₆)-Cycloalkyl" werden Reste verstanden wie Verbindungen, die sich von 3- bis 6-gliedrige Monocyclen wie Cyclopropan, Cyclobutan, Cyclopentan oder Cyclohexan herleiten.

Unter dem Begriff "-O-(C₃-C₆)-Cycloalkyl" werden Cycloalkoxy-Reste verstanden wie Verbindungen, die sich von 3- bis 6-gliedrige Monocyclen wie Cyclopropoxy, Cyclobutoxy, Cyclopentoxy oder Cyclohexoxy herleiten.

Unter dem Begriff "-(C₆-C₁₄)-Aryl" werden aromatische Kohlenwasserstoffreste verstanden mit 6 bis 14 Kohlenstoffatomen im Ring. -(C₆-C₁₄)-Arylreste sind beispielsweise Phenyl, Naphthyl, zum Beispiel 1-Naphthyl, 2-Naphthyl, Anthryl oder Fluorenyl. Naphthylreste und insbesondere Phenylreste sind bevorzugte Arylreste. Unter dem Begriff "Het" werden Ringsysteme verstanden mit 4 bis 15 Kohlenstoffatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten. Beispiele für diese Ringsysteme sind die Reste Acridinyl, Azepinyl, Azetidinyl, Benzimidazolyl, Benzofuranyl, Benzothiofuranyl, Benzothiophenyl, Benzoxazolyl, Benzthiazolyl, Benztriazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochinolinyl, Isochromanyl, Isoindazolyl, Isoindolinyl, Isoindolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxothiolanyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadiazinyl, 1,2,3-Thiadiazolyl, 1,2,4-Thiadiazolyl, 1,2,5-Thiadiazolyl, 1,3,4-Thiadiazolyl, Thianthrenyl, Thiazolidinyl, Thiazolinyl, Thiazolyl, Thienyl, Thienoimidazolyl, Thienooxazolyl, Thienopyrrol, Thienopyridin, Thienothiazolyl, Thienothiophenyl, Thiomorpholinyl, Triazinyl, 1,2,3-Triazolyl, 1,2,4-Triazolyl, 1,2,5-Triazolyl, 1,3,4-Triazolyl oder Xanthenyl.

Unter dem Begriff "R1 und R2 oder R2 und R3 bilden zusammen mit den Ringatomen, an die sie jeweils gebunden sind, einen 5- bis 8-gliedrigen Ring, wobei der Ring nur aus Kohlenstoffatomen besteht oder 1, 2 oder 3 dieser Atome durch N-, O- oder S-Atome ersetzt sind" werden beispielsweise Ringsysteme wie 2,3,5,6,7,8-hexahydro-1,2,3a,4,5,8-hexaaza-cyclopenta[b]naphthalin; 2,6,7,8-tetrahydro-3H-5-oxa-1,2,3a,4,8-pentaaza-cyclopenta[b]naphthalin; 2,3,6,7-tetrahydro-5,8-dioxa-1,2,3a,4-tetraazacyclopenta[b]naphthalin; 2,3,6,7-tetrahydro-5H-8-oxa-1,2,3a,4,5-pentaazacyclopenta[b]naphthalin; 2,6,7,8-tetrahydro-3H-5-thia-1,2,3a,4,8-pentaazacyclopenta[b]naphthalin; 2,3,6,7,8,9-hexahydro-1,2,3a,4,6,9-hexaaza-cyclopenta[a]-naphthalin; 2,3-dihydro-5,7-dioxa-1,2,3a,4-tetraaza-s-indacin; 2,6,7,8-tetrahydro-3H-cyclopenta[e][1,2,4]triazolo[4,3-b]pyridazin; 2,7,8,9-tetrahydro-3H-cyclopenta[d] [1,2,4]triazolo[4,3-b]pyridazin oder 2,3,6a,9a-tetrahydro-[1,3]dioxolo[4,5-d][1,2,4]triazolo[4,3-b]pyridazin verstanden.

Unter dem Begriff "R4 und R5, R5 und R6, R6 oder R7 oder R7 und R8 bilden zusammen mit den Ringatomen, an die sie jeweils gebunden sind, einen 5- bis 8-gliedrigen Ring, wobei der Ring nur aus Kohlenstoffatomen besteht oder 1, 2 oder 3 dieser Atome durch N-, O- oder S-Atome ersetzt sind" werden beispielsweise Ringsysteme wie 2,3-Dihydro-benzo[1,4]dioxin; 3,4-Dihydro-2H-benzo[1,4]oxazin; 1,2,3,4-Tetrahydro-quinoxalin; Benzo[1,3]dioxol; 3,4-Dihydro-2H-benzo[1,4]thiazin oder 2,3,4,5-Tetrahydro-1H-benzo[b][1,4]diazepin verstanden.

Unter den Begriffen "R11 und R12 in den Fragmenten "N(R11)-R12" und "N(R11)-C(O)-R12" für einen 5- bis 8-gliedrigen Ring stehen, der gemeinsam mit dem Stickstoffatom "N" oder der Gruppe "N-(CO)" gebildet wird, wobei cyclische Amine, Imide oder Lactame gebildet werden, die bis zu 2 weitere Heteroatome aus der Gruppe N, O oder S enthalten" oder "R11 und R12 in den Fragmenten "N(R21)-R22" und "N(R21)-C(O)-R22" für einen 5- bis 8-gliedrigen Ring stehen, der gemeinsam mit dem Stickstoffatom "N" oder der Gruppe "N-(CO)" gebildet wird, wobei cyclische Amine, Imide oder Lactame gebildet werden, die bis zu 2 weitere Heteroatome aus der Gruppe N, O oder S enthalten" werden beispielsweise Ringsysteme wie cyclische Amine verstanden wie Azetidinyl, Pyrrolidinyl, Piperidinyl, Piperazinyl, Azepinyl, Imidazolyl, Morpholinyl oder Thiomorpholinyl, bei den Imiden Reste wie Pyrrolidin-2,5-dionyl, Piperidin-2,6-dionyl, Piperazin-2,6-dionyl, Morpholin-3,5-dionyl, und bei den Lactamen Reste wie Pyrrolidin-2-onyl, Piperidin-2-onyl, Piperazin-2-onyl, Morpholin-3-onyl.

Unter der Umschreibung "Alkyl, Alkylen oder Cycloalkyl 1 n der die Wasserstoffatome ganz oder teilweise durch Fluor ersetzt sein" wird ein partiell oder vollständig fluorierter Alkyl-, Alkylen- oder Cycloalkylrest verstanden, der sich beispielsweise für Alkyl von folgenden Resten -CF₃, -CHF₂, -CH₂F, -CHF-CF₃, -CHF-CHF₂, -CHF-CH₂F, -CH₂-CF₃, -CH₂-CHF₂, -CH₂-CH₂F, -CF₂-CF₃, -CF₂-CHF₂, -CF₂-CH₂F, -CH₂-CHF-CF₃, -CH₂-CHF-CHF₂, -CH₂-CHF-CH₂F, -CH₂-CH₂-CF₃, -CH₂-CH₂-CHF₂, -CH₂-CH₂-CH₂F, -CH₂-CF₂-CF₃, -CH₂-CF₂-CHF₂, -CH₂-CF₂-CH₂F, -CHF-CHF-CF₃, -CHF-CHF-CHF₂, -CHF-CHF-CH₂F, -CHF-CH₂-CF₃, -CHF-CH₂-CHF₂, -CHF-CH₂-CH₂F, -CHF-CF₂-CF₃, -CHF-CF₂-CHF₂, -CHF-CF₂-CH₂F, -CF₂-CHF-CF₃, -CF₂-CHF-CHF₂, -CF₂-CHF-CH₂F, -CF₂-CH₂-CF₃, -CF₂-CH₂-CHF₂, -CF₂-CH₂-CH₂F, -CF₂-CF₂-CF₃, -CF₂-CF₂-CHF₂, -CF₂-CF₂-CH₂F, -CH(CF₃)₂, -CH(CHF₂)₂, -CH(CFH₂)₂, -CH(CFH₂)(CHF₂), -CH(CFH₂)(CF₃), -CH(CFH₂)(CH₃), -CH(CHF₂)(CH₃), -CH(CF₃)(CH₃), -CF(CF₃)₂, -CF(CHF₂)₂, -CF(CFH₂)₂, -CF(CFH₂)(CHF₂), -CF(CFH₂)(CF₃), -CF(CFH₂)(CH₃), -CF(CHF₂)(CH₃), oder -CF(CF₃)(CH₃), sowie die weiteren möglichen Kombinationen für Butyl, Pentyl und Hexyl, die wie Propyl auch verzweigt sein können,
für Alkylen beispielsweise von folgenden Resten -CF₂-, -CHF-, -CHF-CF₂-, -CHF-CHF-, -CHF-CH₂-, -CF₂-CF₂- oder -CF₂-CH₂F, sowie die weiteren möglichen Kombinationen für Propylen, Butylen, Pentylen und Hexylen, die auch verzweigt sein können, und für Cycloalkyl beispielsweise von den Resten 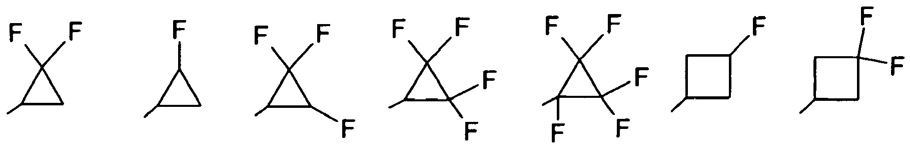 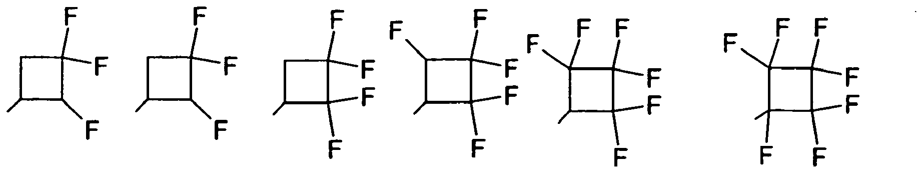 sowie den analogen größeren Ringen Cyclopentyl und Cyclohexyl ableitet.

Unter dem Begriff "Halogen" wird Fluor, Chlor, Brom oder Jod verstanden, bevorzugt sind Fluor, Chlor oder Brom, insbesondere Fluor oder Chlor.

Oben beschriebene Begriffe sind auch beliebig kombinierbar wie beispielsweise in "-(C₀-C₆)-Alkylen-O-(C₀-C₆)-Alkylen-(C₆-C₁₄)-Aryl" geschehen.

Funktionelle Gruppen der verwendeten Intermediate, beispielsweise Amino- oder Carboxylgruppen in der Verbindung der Formel I, können dabei durch geeignete Schutzgruppen maskiert werden. Geeignete Schutzgruppen für Aminofunktionen sind beispielweise die t-Butoxycarbonyl-, die Benzyloxycarbonyl- oder die Phtalolylgruppe sowie die Trityl- oder Tosylschutzgruppe. Geeignete Schutzgruppen für die Carboxylfunktion sind beispielweise Alkyl-, Aryl- oder Arylalkylester. Schutzgruppen können durch wohlbekannte oder hier beschriebene Techniken eingeführt und entfernt werden (siehe Greene, T.W., Wuts, P.G.M., Protective Groups in Organic Synthesis (1999), 3rd Ed., Wiley-Interscience, oder Kocienski, P. J., Protecting Groups (2004), 3rd Ed., Thieme. Der Begriff Schutzgruppe kann auch entsprechende polymergebundene Schutzgruppen umfassen.
Die erfindungsgemäßen Verbindungen können nach wohlbekannten Verfahren oder nach hier beschriebenen Verfahren hergestellt werden.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, dass man
a) eine Verbindung der Formel II, 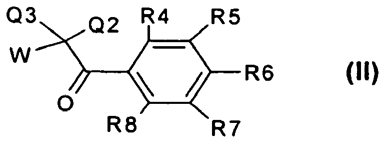 wobei R4, R5, R6, R7, R8, Q2 und Q3 wie in Formel I definiert sind und W für Chlorid, Bromid, Mesylat oder Tosylat steht, mit einer Verbindung der Formel III, 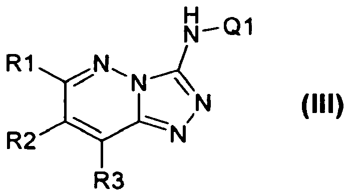 wobei R1, R2, R3 und Q1 wie in Formel I definiert sind, mit oder ohne Basenzugabe in einem Lösungsmittel zu einer Verbindung der Formel I umsetzt, oder
b) eine Verbindung der Formel VII, 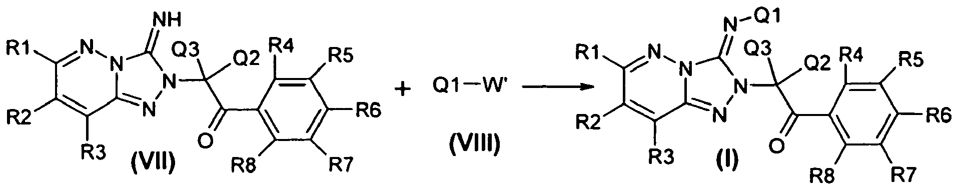 wobei R1, R2, R3, R4, R5, R6, R7, R8, Q1, Q2 und Q3 wie in Formel I definiert sind, mit einer Verbindung Q-W', wobei W' Chlorid, Bromid, Mesylat, Tosylat, Methylsulfat oder eine ähnliche gute Fluchtgruppe bedeutet, mit oder ohne Basenzugabe zu einer Verbindung der Formel I umsetzt, oder
c) die nach den Verfahren a) oder b) hergestellte Verbindung der Formel I entweder in freier Form isoliert oder aus physiologisch unverträglichen Salzen freisetzt oder im Falle des Vorliegens von sauren oder basischen Gruppen in physiologisch verträgliche Salze umwandelt, oder
d) eine nach den Verfahren a) oder b) hergestellte Verbindung der Formel I, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt.

Die Erfindung betrifft ferner ein Verfahren zur Herstellung der Verbindung der Formel I nach Schema 1.

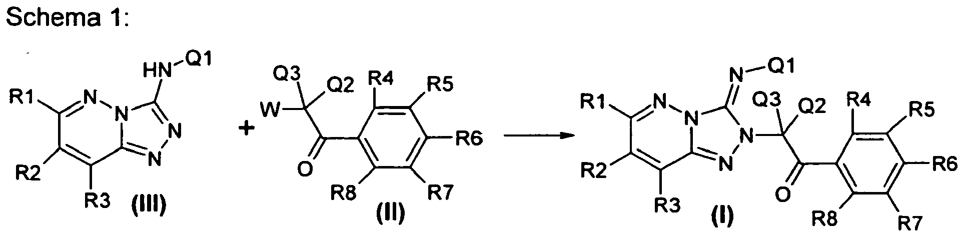

Die Edukte II und III, wobei III gegebenenfalls in Form eines Salzes vorliegt, werden dabei bei Raumtemperatur oder leicht erhöhter Temperatur von 40 °C bis 60 °C vorteilhafterweise, wenn III als Salz vorliegt, in Gegenwart einer Base, bevorzugt Hünig-Base, in einem Lösungsmittel, bevorzugt Dimethylformamid (DMF) oder Dioxan, zu der Verbindung der Formel I umgesetzt. Die Reste R1, R2, R3, R4, R5, R6, R7, R8, Q1, Q2 und Q3 sind wie in Formel I definiert, W entspricht einer guten Fluchtgruppe wie Chlorid, Bromid, Mesylat oder Tosylat, bevorzugt Bromid oder Mesylat.

Die Verbindungen der Formel I können teilweise auch in isomeren Formen auftreten, wobei Q1 in der folgenden Teilformel von Formel I entweder (E) oder (Z) konfiguriert sein kann:

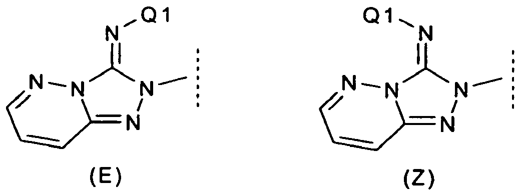

Die unter diesen Reaktionsbedingungen je nach Substitutionsmuster in unterschiedlichen Anteilen ebenfalls gebildeten 1-substituierten Triazolopyridazinium-Salze (A) lassen sich chromatographisch oder durch Kristallisation abtrennen. Vorteilhaft ist die Trennung über Kieselgel mit Dichlormethan-Methanol als Laufmittelgemisch.

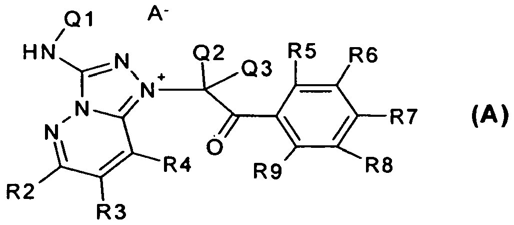

Verbindungen der Formel 11 sind käuflich oder lassen sich nach literaturbekannten Verfahren beispielsweise ausgehend von den korrespondierenden Acetophenonen X oder X' gewinnen (siehe beispielsweise: Phosphorus and Sulfur and the Related Elements (1985), 25(3), 357 oder Tetrahedron Letters (1984), 25(34), 3715). Die wohlbekannten Verbindungen vom Typ X lassen sich beispielsweise an der Acetylgruppe unter anderem mit elementarem Brom oder Chlor, Tribromid-Derivaten wie Phenyl-trimethylammonium-tribromid, 1,3-Dichlordimethylhydantoin,N-Chlor- oder N-Bromsuccinimid funktionalisieren. Verbindungen von Typ X' lassen sich beispielsweise mit Mesyl- oder Tosylchlorid in die Verbindungen vom Typ II überführen.

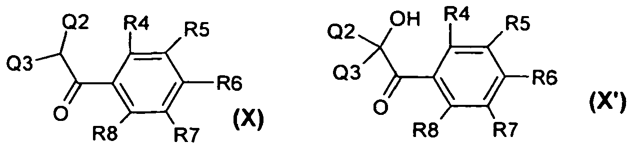

Verbindungen der Formel III sind käuflich oder lassen sich nach literaturbekannten Verfahren gewinnen. Als Vorstufen eignen sich dabei Verbindungen vom Typ XX, die in Gegenwart von Bromcyan, Chlorcyan oder Tosylcyanid zu Verbindungen vom Typ III cyclisiert werden können und die auch in der tautomeren Form vom Typ XXa vorliegen können.

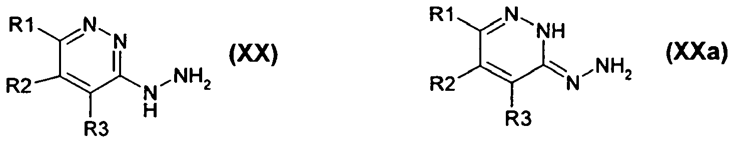

Die Verbindung der Formel XX wie Pyridazin-3-yl-hydrazin und die Verbindung der Formel XXa wie [2H-Pyridazin-(3E)-yliden]-hydrazin sind tautomere Formen. Wenn im folgenden nur eine Schweibweise benutzt wird, bedeutet dies, dass auch die andere tautomere Form mit offenbart wird.

Alternativ können Verbindungen vom Typ XX auch mit Isothiocyanaten vom Typ XXV zu den Thioharnstoffen vom Typ XXVI umgesetzt werden. Letztere lassen sich nach Aktivierung des Schwefels beispielsweise mit Tosylchlorid, einem Carbodiimid, Bromessigsäureethylester oder Quecksilberoxid in die Verbindungen vom Typ der Formel III überführen. Dabei haben die Reste R1, R2 und R3 die oben angegebene Bedeutung und Q1' entspricht Q1 oder einer Schutzgruppe wie FMOC (Fluoren-9-yl-methyloxycarbonyl), die nach Ringschluss wieder abgespalten werden kann, so dass Verbindungen mit Q1 gleich Wasserstoff zugänglich werden.

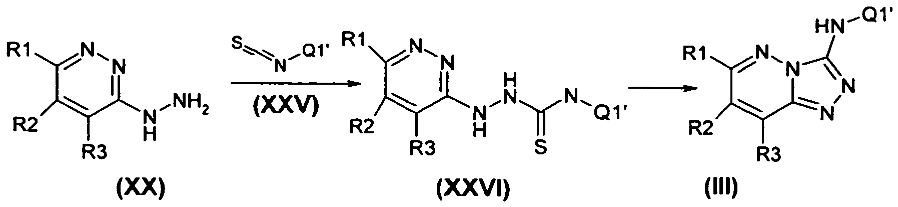

Verbindungen vom Typ XX lassen sich durch Einbau von Hydrazin in Verbindungen vom Typ XXI gewinnen, die mit verschiedensten Substitutionsmustern käuflich sind. Dabei haben die Reste R1, R2 und R3 die oben angegebene Bedeutung und LG steht für eine gute Abgangsgruppe wie Fluor, Chlor, Brom, Iod, Mesylat, Tosylat, Triflat oder Nonaflat.

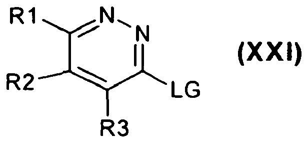

Einen Zugang zu den Chlor-Verbindungen vom Typ XXI' stellt beispielsweise die Umsetzung von Maleinsäureanhydriden vom Typ XXIII mit Hydrazin-Hydrochlorid zu den Verbindungen vom Typ XXII dar, gefolgt von der Umsetzung mit Phosphoroxychlorid zum Dichlorid XXI' und mit Hydrazin zu den Verbindungen vom Typ XXa und XXb.

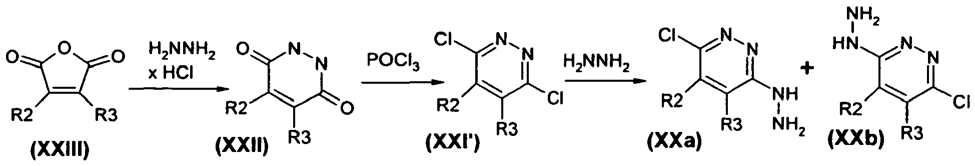

Eine nach Schema 1 hergestellte Verbindung der Formel I, oder eine geeignete Vorstufe der Formel I, die aufgrund ihrer chemischen Struktur in enantiomeren Formen auftritt, kann durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren und Abspaltung der chiralen Hilfsgruppen in die reinen Enantiomeren aufgetrennt werden (Verfahren c), oder die nach Schema 1 hergestellte Verbindung der Formel I kann entweder in freier Form isoliert oder im Falle des Vorliegens von sauren oder basischen Gruppen in physiologisch verträgliche Salze umwandelt werden (Verfahren d).

Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze oder Hydrochloride, Sulfate, Hemisulfate, Methylsulfonate, p-Toluolsulfonate, alle möglichen Phosphate sowie Salze der Aminosäuren, natürlicher Basen oder Carbonsäuren wie Lactate, Citrate, Tartrate, Acetate, Adipinate, Fumarate, Gluconate, Glutamate, Maleinate oder Pamoate.

Die Herstellung physiologisch verträglicher Salze aus zur Salzbildung befähigten Verbindungen der Formel I, einschließlich deren stereoisomeren Formen, gemäß Verfahrensschritt c) erfolgt in an sich bekannter Weise. Enthalten Verbindungen der Formel I saure Funktionalität, so lassen sich mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze bilden. Basische Gruppen der Verbindungen der Formel I, bilden mit Säuren Säureadditionssalze. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon- 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron-, Palmitin- oder Trifluoressigsäure in Frage.

Im Verfahrensschritt d) wird die Verbindung der Formel I, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel I zur Salzbildung befähigt ist, kann auch eine fraktionierte Kristallisation der mit einer optisch aktiven Base oder Säure als Hilfsstoff gebildeten diastereomeren Salze durchgeführt werden. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L-Milchsäure sowie (+) und (-)-Mandelsäure in die reinen Enantiomeren überführen. Auch kann man chirale Verbindungen, die Alkohol- oder Amin-Funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Weiterhin ergibt sich bei einigen der erfindungsgemäßen Verbindungen die Möglichkeit, zur Herstellung der Gerüststrukturen diastereo- oder enantiomerenreine Ausgangsprodukte einzusetzen. Dadurch können auch andere oder vereinfachte Verfahren zur Aufreinigung der Endprodukte eingesetzt werden. Diese Ausgangsprodukte wurden zuvor nach literatur-bekannten Verfahren enantiomeren- oder diastereomerenrein hergestellt. Das kann insbesondere bedeuten, dass in der Synthese der Grundgerüste entweder enantioselektive Verfahren zum Einsatz kommen, oder aber eine Enantiomeren- (oder Diastereomeren-) Trennung auf früher Synthesestufe und nicht erst auf der Stufe der Endprodukte durchgeführt wird. Ebenso kann eine Vereinfachung der Trennungen dadurch erreicht werden, dass zwei- oder mehrstufig vorgegangen wird.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen beispielsweise zur Prophylaxe, Sekundär-Prävention und Therapie all solcher Erkrankungen, die durch eine Hemmung des Protease-aktivierten Rezeptors 1 (PAR1) behandelbar sind. So eignen sich die erfindungsgemäßen Verbindungen sowohl für eine prophylaktische als auch für eine therapeutische Anwendung am Menschen. Sie eignen sich sowohl für eine akute Behandlung als auch für eine Langzeittherapie. Die Verbindungen der Formel I können eingesetzt werden in Patienten, die an Störungen des Wohlbefindens oder Krankheiten leiden, die mit Thrombosen, Embolien, Hyperkoagulabilität, fibrotischen Veränderungen oder entzündlichen Erkrankungen einhergehen.

Dazu gehören der Myokardinfarkt, die Angina pectoris und alle anderen Formen des akuten Koronarsyndroms, der Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und Bypass-Operationen. Weiterhin können die Verbindungen der Formel I eingesetzt werden bei allen Eingriffen, die zu einem Kontakt des Blutes mit Fremdoberflächen führen wie bei Dialysepatienten und Patienten mit Verweilkathetern. Verbindungen der Formel I können eingesetzt werden, um die Thrombosegefahr nach chirurgischen Eingriffen wie bei Knie- und Hüftgelenksoperationen zu reduzieren.

Verbindungen der Formel I eignen für die Behandlung von Patienten mit disseminierter intravaskulärer Koagulation, Sepsis und anderen intravaskulären Ereignissen, die mit einer Entzündung einhergehen. Weiterhin eignen sich Verbindungen der Formel I für die Prophylaxe und Behandlung von Patienten mit Atherosklerose, Diabetes und dem metabolischen Syndrom und deren Folgen. Störungen des hämostatischen Systems (beispielsweise Fibrinablagerungen) wurden impliziert in Mechanismen, die zu Tumorwachstum und Tumormetastasierung führen, sowie bei entzündlichen und degenerativen Gelenkserkrankungen wie der rheumatoiden Arthritis und der Arthrose.

Verbindungen der Formel I eignen sich zur Verlangsamung oder Verhinderung solcher Prozesse.

Weitere Indikationen für den Einsatz der Verbindungen der Formel I sind fibrotische Veränderungen der Lunge wie die chronische obstruktive Lungenerkrankung, das adult respiratory distress syndrome (ARDS) und des Auges wie Fibrinablagerungen nach Augenoperationen. Verbindungen der Formel I eignen sich auch zur Verhinderung und/oder Behandlung von Narbenbildung.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation. Eine Beschichtung von Stents mit Verbindungen der Formel I und anderen Oberflächen, die im Körper mit Blut in Kontakt kommen, ist möglich.

Die Erfindung betrifft auch ein Verfahren zur Herstellung eines Arzneimittels, das dadurch gekennzeichnet ist, dass man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formel I, Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Verbindungen der Formel I können sowohl als Monotherapie als auch in Kombination oder gemeinsam mit allen Antithrombotika (Antikoagulanzien und Plättchenaggregationshemmer), Thrombolytika (Plasminogenaktivatoren jeglicher Art), anderen profibrinolytisch wirksamen Substanzen, Blutdrucksenkern, Regulatoren des Blutzuckers, Lipidsenkern und Antiarrhythmika verabreicht werden. Als Plättchenaggregationshemmer kommen dabei Cyclooxygenase 1-Inhibitoren wie Aspirin, irreversible P2Y₁₂₋Antagonisten wie Clopidogrel oder Prasugrel, reversible P2Y₁₂-Antagonisten wie Cangrelor oder AZD6140 und Thromboxan A₂/Prostaglandin H₂-Antagonisten wie Terutroban in Frage. Additive Effekte von PAR 1-Blockade in Kombination mit P2Y₁₂-Blockade konnten beispielweise bereits gezeigt werden (Eur. Heart J. 2007, 28, Abstract Supplement, 188).

### Beispiele

Endprodukte wurden in der Regel durch eine chromatographische Methode mit Ultraviolett- und-massenspektroskopischer Detektion (LCUV/ESI-MS-Kopplung) sowie ¹H-NMR charakterisiert. Beschrieben sind die Verbindungen durch Angabe der zugehörigen Retentionszeit im Ionenstrom (LCMS-rt) und des entsprechenden [M+H]⁺-Signals bei positiver lonisiation im zugehörigen Massenspektrum. Konnte kein [M+H]⁺-Massensignal erhalten werden, wurden alternativ die ¹H-NMR-Daten angegeben.

Verwendete Abkürzungen sind entweder erläutert oder entsprechen den üblichen Konventionen.

Kieselgeltrennungen wurden manuell (Flash-Chromatographie) oder unterstützt durch halbautomatische Kartuschen-Systeme wie Companion (CombiFlash) oder Flashmaster II (Jones Chromatography) durchgeführt. Soweit nicht anders angegeben, wurden chromatographische Trennungen an Kieselgel mit Ethylacetat/Heptan-, Dichlormethan/Ethanol- oder Dichlormethan/Methanol-Gemischen als Laufmittel durchgeführt.

Das Abdampfen von Lösungsmitteln geschah in der Regel unter vermindertem Druck bei 35 °C bis 45 °C am Rotationsverdampfer und wird mit, "eingeengt", "einrotiert" "zur Trockne gebracht", "vom Lösungsmittel befreit", "Lösungsmittel entfernt bzw. abgezogen" oder ähnlichen Ausdrucksweisen umschrieben.

Die LCUV/MS-Analysen wurden unter folgenden Bedingungen durchgeführt:

| Methode A (=Met-a): | |
|---|---|
| System: | Agilent 1100 HPLC-System gekoppelt an 1100 LC/MSD |
| Säule: | YMC J'shere ODS H80 20x2,1 mm, Packungsmaterial 4 µm |
| Laufmittel: | ACN:H₂O+0,05% TFA (Fluss 1 ml/min) |
| Gradient: | 4:96 (0 min) → 95:5 (2 min) → 95:5 (2,4 min) → 4:96 (2,45 min) |
| Ionisierung: | ESI⁺ |
| | |

| Methode B (=Met-b): | |
|---|---|
| System: | Agilent 1200 HPLC-System gekoppelt an 6120 LC/MS |
| Säule: | Luna C18, 10 x 2,0 mm, Packungsmaterial 3 µm |
| Laufmittel: | ACN:H₂O+0,05% TFA (Fluss 1,1 ml/min) |
| Gradient: | 7:93 (0 min) → 95:5 (1 min) → 95:5 (1,45 min) → 7:93 (1,5 min) |
| Ionisierung: | ESI⁺ |
| | |

| Methode C (=Met-c): | |
|---|---|
| System: | Agilent 1200 HPLC-System gekoppelt an 6120 LC/MS |
| Säule: | Luna C18, 10 x 2,0 mm, Packungsmaterial 3 µm |
| Laufmittel: | ACN:H₂O+0,05% TFA (Fluss 1,1 ml/min) |
| Gradient: | 1:99 (0 min) → 7:93 (0,3 min) → 95:5 (1,3 min) → 95:5 (1,75 min) → 1:99 (1,8 min) |
| Ionisierung: | ESI⁺ |
| | |

| Methode D (=Met-d): | |
|---|---|
| System: | Waters: 1525 pump, 996 PDA, LCT classic TOF-MS |
| Säule: | Waters XBridge C18 4,6 x 50 mm; 2,5 µM |
| Laufmittel: | ACN+0,05 % TFA : H₂O+0,05 % TFA (Fluss 1,3 ml/min), 40°C |
| Gradient: | 5:95 (0 min) → 5:95 (0,3 min) → 95:5 (3,5 min) → 95:5 (4 min) |
| Ionisierung: | ESI⁺ |

| Methode E (=Met-e): | |
|---|---|
| System: | Waters: 1525 pump, 996 PDA, LCT classic TOF-MS |
| Säule: | Waters XBridge C18; 4,6 x 50 mm; 2,5 µM |
| Laufmittel: | ACN+0,05 % TFA : H₂O+0,05 % TFA (Fluss 1,7 ml/min), 40°C |
| Gradient: | 5:95 (0 min) → 5:95 (0,2 min) → 95:5 (2,4 min) → 95:5 (3,2 min) → 5:95 (3,3 min) → 5:95 (4,0 min) |
| Ionisierung: | ESI⁺ |
| | |

| Methode F (=Met-f): | |
|---|---|
| System: | Waters: 1525 pump, 996 PDA, LCT classic TOF-MS |
| Säule: | YMC J'shere, 33 x 2 mm, 4 µM |
| Laufmittel: | ACN+0,05 % TFA : H₂O+0,05 % TFA (Fluss 1,3 ml/min) |
| Gradient: | 5:95 (0 min) → 5:95 (2,5 min) → 95:5 (3 min) |
| Ionisierung: | ESI⁺ |
| | |

Präparative HPLC-Reinigungen an Reversed-Phase-(RP)-Kieselgel wurden mit den folgenden Methoden durchgeführt:

| Methode A (=Met-A): | |
|---|---|
| Säule: | Merck (Darmstadt, Deutschland) Purosphere® RP18 25x250 mm, 10 µm |
| Laufmittel: | ACN:H₂O+0,05%TFA (Fluss 25 ml/min) |
| Gradient: | 10:90 (0 min) → 90:10 (40 min) |

| Methode B (=Met-B) | |
|---|---|
| Säule: | Merck Purosphere® RP18 25x250 mm, 10 µm |
| Laufmittel: | ACN:H₂O+0,05%TFA (Fluss 25 ml/min) |
| Gradient: | 0:100 (0 min) → 0:100 (5 min) → 20:80 (20 min) |
| | |

| Methode C (=Met-C) | |
|---|---|
| Säule: | Agilent Prep-C18, 30 x 250mm, 10 µm |
| Laufmittel: | ACN:H₂O+0,05%TFA (Fluss 75 ml/min) |
| Gradient: | 10:90 (0 min) → 90:10 (12,5 min) → 90:10 (15min) → 10:90 (15,5 min) → 10:90 (17.5 min) |
| | |

| Methode D (=Met-D): | |
|---|---|
| Säule: | Merck (Darmstadt, Deutschland) Purosphere® RP18 25x250 mm, 10 µm |
| Laufmittel: | ACN:H₂O+0,05% konz. HCl (Fluss 25 ml/min) |
| Gradient: | 10:90 (0 min) → 90:10 (40 min) |
| | |

| Methode E (=Met-E) | |
|---|---|
| Säule: | Merck Purosphere® RP18 25x250 mm, 10 µm |
| Laufmittel: | ACN:H₂O+0,05%HCl (Fluss 25 ml/min) |
| Gradient: | 0:100 (0 min) → 0:100 (5 min) → 20:80 (20 min) |
| | |

| Methode F (=Met-F) | |
|---|---|
| Säule: | Agilent Prep-C18, 30 x 250mm, 10 µm |
| Laufmittel: | ACN:H₂O+0,05%TFA (Fluss 75 ml/min) |
| Gradient: | 0:100 (0 min) → 0:100 (5 min) → 20:80 (20min) |

Die Umsetzungen fanden in Standard-Reaktionsapparaturen wie Ein- oder Mehrhalskolben statt, die wenn nicht anders beschrieben, dem Bedarf angemessen 5 ml bis 2000 ml Volumen fassten und je nach Anforderung mit Septum, Stopfen, Kühler, Rührer oder anderen Ausrüstungsgegenständen bestückt waren. Wenn nicht anders erwähnt, fanden alle Reaktionen unter Argon als Schutzgas statt und wurden mit Magnetrührern gerührt. Mikrowellenreaktionen wurden im Emrys Optimizer von Personal Chemistry in dem Bedarf angepassten Gefäßen von 0,5 ml bis 10 ml Fassungsvermögen durchgeführt.

Lösungsmittel wie Dichlormethan, Ethanol, Dimethylformamid, Methanol, Isopropanol und Ähnliche wurden als "trockene" Lösungsmittel gekauft und auch so in den Reaktionen eingesetzt, ohne dass das jeweils explizit erwähnt ist.

### Verwendete Abkürzungen:

- abs.: absolut
- ACN: Acetonitril
- Boc: Butoxycarbonyl
- Bsp.: Beispiel
- DCM: Dichlormethan
- DIPEA: N,N-Diisopropylethylamin (Hünig-Base)
- DMF: Dimethylformamid
- DMSO: Dimethylsulfoxid
- EE: Essigsäureethylester
- eq.: Äquivalente
- EtOH: Ethanol
- h: Stunde(n)
- HPLC: Hochleistungsflüssigkeitschromatographie
- Hünig-Base: N, N-Diisopropyl-N-ethylamin
- LCMS-rt: Retentionszeit der Verbindung im Ionenstrom der Flüssigkeitschromatographie
- LCUV/MS: Flüssigkeitschromatographie Ultraviolett-/Massenspektroskopie
- NMP: 1-Methyl-2-pyrrolidon
- MeOH: Methanol
- MtB-Ether: tert.-Butyl-methylether
- MW: Mikrowelle
- RF: Rückfluss
- RT: Raumtemperatur (20 °C bis 25 °C)
- rt: Retentionszeit
- TEA: Triethylamin
- TFA: Trifluoressigsäure
- THF: Tetrahydrofuran
- TOTU: O-[(Ethoxycarbonyl)cyanomethylenamino]-N,N,N',N'-tetramethyluronium-tetrafluorborat

### Synthese der Bausteine der Westhälfte:

### W1

### W1.001

### 6-Ethoxy-[1,2,4]triazolo[4,3-b]-pyridazin-3-ylamin

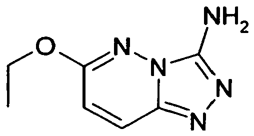

6-Chlor-[1,2,4]triazolo[4,3-b]pyridazin-3-ylamin (W2.001a, 616 mg) wurde in absolutem Ethanol (40 ml) gelöst und protionsweise mit festem Natriumethylat (990 mg) versetzt. Nach 2 h Rühren bei 55°C wurde Wasser zugegeben und die wässrige Phase dreimal mit Dichlormethan extrahiert. Die vereinigten Extrakte wurden über Natriumsulfat getrocknet, filtriert und eingeengt. Es wurden 709 mg Rohprodukt der gewünschten Verbindung in ausreichender Reinheit erhalten.

| | |
|---|---|
| LCMS-rt: 0,51 min | [M+H]⁺: 180,1 (Met-a) |

Analog wurden synthetisiert:

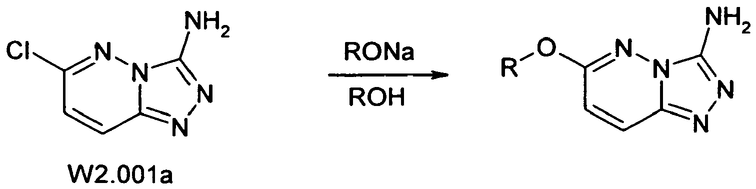

| Nummer | R | LCMS-rt | [M+H]⁺ | Bemerkung: |
|---|---|---|---|---|
| W1.002 | Methyl- | 0,22 | 166,1 (Met-a) | W2.001a (100 mg); Natrium-methylat-Lösung; Produkt: 95 mg |
| W1.003 | 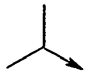 | 0,69 | 194,1 (Met-a) | W2.001a (100 mg); Natrium-isopropylat-Lösung; Produkt: 84 mg |

### W1.004

### 6-Cyclobutoxy-[1,2,4]triazolo[4,3-b]pyridazin-3-ylamin

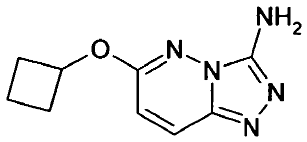

Cyclobutanol (2,43 ml) wurde bei RT unter Rühren vorgelegt und mit einem Eisbad annähernd auf 0°C gekühlt. Anschließend wurde portionsweise mit Natriumhydrid (146 mg) versetzt. Die dabei entstehende Suspension wurde 30 min auf 55°C erwärmt und portionsweise mit 6-Chlor-[1,2,4]triazolo[4,3-b]pyridazin-3-ylamin-Hydrobromid (W2.001, 200 mg), suspendiert in Cyclobutanol (5 ml), versetzt. Nach 1,5 h Rühren bei 55°C wurde über Nacht bei RT stehen gelassen und anschließend mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 136 mg der Titelverbindung als Feststoff erhalten.

| | |
|---|---|
| LCMS-rt: 0,82 min | [M+H]⁺: 206,2 (Met-a) |

Analog wurden die folgenden Bausteine dargestellt:

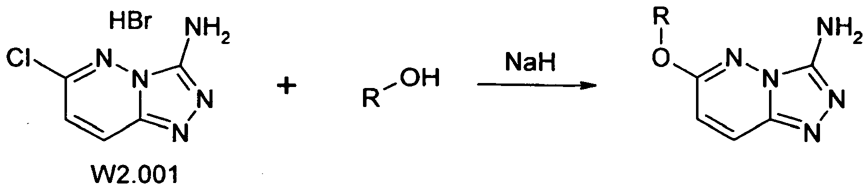

| Nummer | R | LCMS -rt | [M+H]⁺ | Bemerkung: |
|---|---|---|---|---|
| W1.005 | 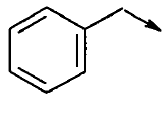 | 0,89 min | 242,1 (Met-a) | W2.001a (100 mg) in NMP gelöst eingesetzt; Rohprodukt per Chromatographie über Kieselgel mit Dichlormethan/Methanol-Gradienten gereinigt; Produkt: 48 mg |
| W1.006 | 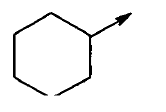 | 0,95 min | 234,1 (Met-a) | W2.001 (200 mg); Rohprodukt über präparative HPLC (Met-A) gereinigt; Produkt: 47 mg (TFA-Salz) |
| W1.007 | 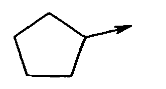 | 0,88 min | 220,1 (Met-a) | W2.001 (463 mg); Rohprodukt über präparative HPLC (Met-A) gereinigt; Produkt: 15 mg (TFA-Salz) |
| W1.008 | 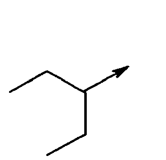 | 0,92 min | 222,1 (Met-a) | W2.001a (194 mg) im Gemisch aus 10 ml DMF und 5 ml 3-Pentanol suspendiert eingesetzt; Rohprodukt per Chromatographie über Kieselgel mit Dichlormethan/Methanol-Gradienten gereinigt; Produkt: 155 mg |

### W1.009

### 6-Cyclopropylmethoxy-[1,2,4]triazolo[4,3-b]pyridazin-3-ylamin

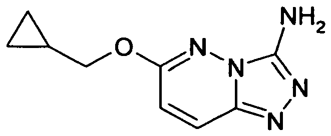

Cyclopropylmethanol (2,64 ml) wurde in DMF (35 ml) vorgelegt, unter Argon mit Natriumhydrid (795 mg) versetzt und 1 h bei 40°C gerührt. Anschließend wurde 6-Chlor-[1,2,4]triazolo[4,3-b]pyridazin-3-ylamin-Hydrobromid (W2.001; 1,66 g), gelöst in DMF (35 ml), zugetropft. Nach 1 h wurde mit Wasser versetzt und viermal mit Dichlormethan ausgeschüttelt. Die vereinigten organischen Phasen wurden über Magnesiumsulfat getrocknet und einrotiert. Der Rückstand wurde mit MtB-Ether verrieben, abgesaugt und getrocknet. Es wurden 720 mg der Titelverbindung erhalten.

| | |
|---|---|
| LCMS-rt: 0,80 min | [M+H]⁺: 206,1 (Met-f) |

Analog wurden die folgenden Bausteine dargestellt:

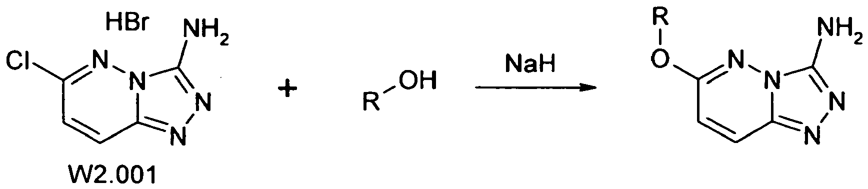

| Nummer | R | LCMS-RT [min] | [M+H]⁺ | Bemerkung: |
|---|---|---|---|---|
| W1.010 | 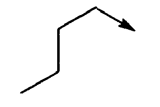 | 0,91 | 208,2 (Met-a) | W2.001 (1,75 g); 1,5 h bei 45°C gerührt; Produkt: 820 mg |
| W1.011 | 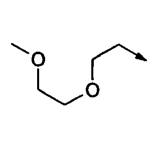 | 0,63 | 254,1 (Met-a) | W2.001 (2,5 g); Rohprodukt über Kieselgelkartusche (120 g, Gradient; Dichlormethan/Methanol 0-20 % in 60 min) gereinigt; Produkt: 1,08 g |
| W1.012 | 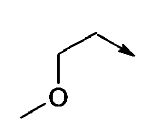 | 0,52 | 210,1 (Met-a) | W2.001 (2,5 g); Rohprodukt über Kieselgelkartusche (40 g, Gradient; Dichlormethan/Methanol 0-20 % in 60 min) gereinigt; Produkt: 1,04 g |

### W1.013

### 6-Phenoxy-[1,2,4]triazolo[4,3-b]pyridazin-3-ylamin

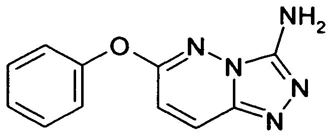

6-Chlor-[1,2,4]triazolo[4,3-b]pyridazin-3-ylamin-Hydrobromid (W2.001; 200 mg) wurde in NMP (4 ml) gelöst vorgelegt. Danach wurde bei RT Natrium-Phenolat (185 mg) eingetragen. Nach einer Stunde Rühren bei RT wurde zur Vervollständigung der Reaktion 2 h auf 55°C erwärmt. Anschließend wurde mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Das Rohprodukt wurde über Kieselgel mit einem Dichlormethan/Methanol-Gradienten aufgereinigt. Es wurden 38 mg der Titelverbindung als Feststoff erhalten.

| | |
|---|---|
| LCMS-rt: 0,80 min | [M+H]⁺: 228,1 (Met-a) |

### W1.014

### 6-(2,2,2-Trifluorethoxy)-[1,2,4]triazolo[4,3-b]pyridazin-3-ylamin

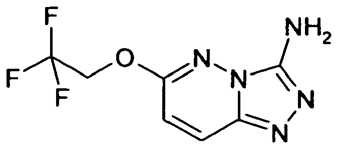

2,2,2-Trifluorethanol (1 ml), Natriumhydrid (86 mg) und 6-Chlor-[1,2,4]triazolo[4,3-b]pyridazin-3-ylamin-Hydrobromid (W2.001, 100 mg) wurden entsprechend W1.004 umgesetzt. Es wurden 87 mg der Titelverbindung als Feststoff erhalten.

| | |
|---|---|
| LCMS-rt: 0,68 min | [M+H]⁺: 234,1 (Met-a) |

### W1.020

### 6-Piperidin-1-yl-[1,2,4]triazolo[4,3-b]pyridazin-3-ylamin

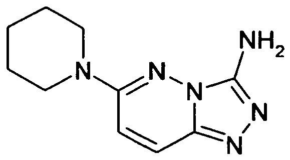

6-Chlor-[1,2,4]triazolo[4,3-b]pyridazin-3-ylamin-Hydrobromid (W2.001; 100 mg) wurde in Wasser (1 ml) vorgelegt und mit Piperidin (260 µl) unter Rühren versetzt. Danach wurde 1 h auf Rückfluss erhitzt und nach Erkalten vom Lösungsmittel befreit. Anschließend wurde mit Wasser versetzt und der dabei gebildete Feststoff abgesaugt und getrocknet. Die Mutterlauge ließ man zur Trockne kommen und versetzte mit wenig Wasser. Der dabei gewonnene Feststoff wurde abgesaugt und getrocknet. Das Filtrat wurde dann dreimal mit Dichlormethan extrahiert. Die vereinigten organischen Phasen wurden über Natriumsulfat getrocknet und nach Abfiltrieren des Trockenmittels im Vakuum zur Trockne gebracht. Die drei erhaltenen Feststofffraktionen wurden vereinigt und ergaben 56 mg der Titelverbindung.

| | |
|---|---|
| LCMS-rt: 0,77 min | [M+H]⁺: 219,1 (Met-a) |

Analog wurde dargestellt:

| Nummer | | LCMS-rt | [M+H]⁺ | Bemerkung: |
|---|---|---|---|---|
| W1.021 | 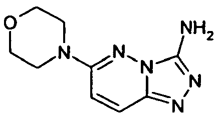 | 0,55 min | 221,1 (Met-a) | W2.001 (150 mg); 5 h Rückfluss; trockene Rohprodukt mit Wasser versetzt und direkt mit DCM extrahiert. Produkt: 81 mg |

### W1.022

### N*6*,N*6*-Diethyl-[1,2,4]triazolo[4,3-b]pyridazin-3,6-diamin

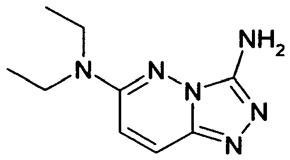

6-Chlor-[1,2,4]triazolo[4,3-b]pyridazin-3-ylamin-Hydrobromid (W2.001; 150 mg) wurde in Wasser (5 ml) vorgelegt und mit Diethylamin (814 µl) unter Rühren versetzt. Danach wurde 16 h auf Rückfluss erhitzt und anschließend weiteres Diethylamin (407 µl) zugesetzt. Nach weiteren 12 h Rückfluss wurde vom Lösungsmittel befreit, mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 67 mg der Titelverbindung erhalten.

| | |
|---|---|
| LCMS-rt: 0,72 min | [M+H]⁺: 207,1 (Met-a) |

### W1.023

### 6-Imidazol-1-yl-[1,2,4]triazolo[4,3-b]pyridazin-3-ylamin

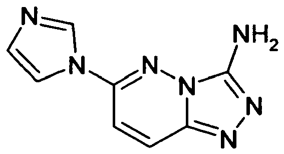

(6-Imidazol-1-yl-pyridazin-3-yl)-hydrazin (500 mg) wurde in einer Mischung aus Essigsäure (4 ml) und Wasser (8 ml) unter Zusatz von Natriumacetat (232 mg) bei RT unter Rühren vorgelegt. Danach kühlte man auf 0°C und trug Bromcyan (330 mg) portionsweise ein. Nach 2,5 h Rühren ließ man über Nacht stehen. Nach Kühlen auf 0°C wurde mit 10 M Natriumhydroxid-Lösung alkalisch gestellt und mehrmals mit EE extrahiert. Die vereinigten EE-Phasen trocknete man über Natriumsulfat, filtrierte und engte ein. Es wurden 66 mg der Titelverbindung isoliert.

Weiteres Produkt befand sich noch in der wässrigen Phase, die gefriergetrocknet und beiseite gestellt wurde. LCMS-rt: 1,46 min [M+H]⁺: 202,1 (Met-d)

### W1.025

### 6-Ethyl-[1,2,4]triazolo[4,3-b]pyridazin-3-ylamin

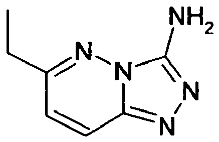

(6-Ethyl-pyridazin-3-yl)-hydrazin-Hydrochlorid (2 g) wurde in einer Mischung aus Ethanol (30 ml) und Wasser (6 ml) unter Rühren bei RT vorgelegt. Danach tropfte man vorsichtig Bromcyan zu (2,4 g in 7,5 ml Ethanol und 1,5 ml Wasser gelöst). Nach einer Stunde Rühren bei RT ließ man über Nacht stehen und Rührte am nächsten Tag 4 weitere Stunden. Dann wurde das Lösungsmittel abgezogen und der Rückstand mittels präparativer HPLC (Met-C) gereinigt. Die sauberen Produktfraktionen wurden vereinigt, im Vakuum vom Acetonitril befreit, mit gesättigter Kaliumcarbonat-Lösung alkalisch gestellt und mehrmals mit Dichlormethan extrahiert. Die vereinigten organischen Phasen wurden über Natriumsulfat getrocknet, filtriert und eingeengt. Es wurden 830 mg der Titelverbindung erhalten.

Die wässrige Kaliumcarbonatphase wurde ebenfalls gefriergetrocknet, danach mit wenig Wasser aufgenommen und fünfmal mit Dichlormethan extrahiert. Die vereinigten organischen Phasen wurden über Natriumsulfat getrocknet, filtriert und eingeengt. Es wurden weitere 180 mg der Titelverbindung erhalten.

| | |
|---|---|
| LCMS-rt: 0,32 min | [M+H]⁺: 164,1 (Met-a) |

### W1.030

### 6-Ethoxy-8-methyl-[1,2,4]triazolo[4,3-b]pyridazin-3-ylamin

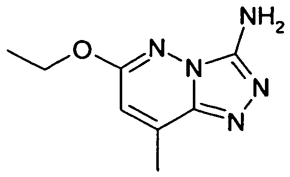

6-Chlor-8-methyl-[1,2,4]triazolo[4,3-b]pyridazin-3-ylamin-Trifluoracetat (W2.002b; 50 mg) wurde in Ethanol (3,5 ml) unter Rühren bei RT gelöst. Danach tropfte man unter Eiskühlung Natriumethylat zu (25 mg in 1,5 ml Ethanol gelöst). Nach 2,5 h Rühren bei RT wurde auf 60°C erwärmt und nach 2,5 h ein weiteres Äquivalent Natriumethylat-Lösung zugegeben. Nach Stehen über Nacht wurde erneut auf 60°C erwärmt, zwei weitere Äquivalente Natriumethylat-Lösung zugegeben und 4 h gerührt. Anschleißend wurde mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 27 mg der Titelverbindung erhalten. LCMS-rt: 0,67 min [M+H]⁺: 194,1 (Met-a)

### Alternativ:

6-Chlor-8-methyl-[1,2,4]triazolo[4,3-b]pyridazin-3-ylamin-Hydrobromid (W2.002; 2,5 g) wurde in Ethanol (50 ml) gelöst, unter Argon mit Natriumethylat (3,86 g) versetzt und 1 h bei 60°C gerührt. Dann wurde zur Trockne gebracht und der Rückstand über eine 40 g Kieselgelkartusche (Dichlormethan-Ethanol-Gradient 0-20 % in 40 min) aufgereinigt. Die sauberen Produktfraktionen wurden vereinigt und zur Trockne gebracht. Es wurden 1,76 g der Titelverbindung erhalten.

| | |
|---|---|
| LCMS-rt: 1,48 min | [M+H]⁺: 194,1 (Met-e) |

### W1.031

### 6-(1-Ethyl-propoxy)-8-methyl-[1,2,4]triazolo[4,3-b]pyridazin-3-ylamin

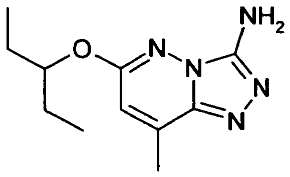

3-Pentanol (10,2 ml) wurde bei RT unter Rühren vorgelegt und mit einem Eisbad annähernd auf 0°C gekühlt. Anschließend wurde portionsweise mit Natriumhydrid (511 mg) versetzt. Die dabei entstandene Suspension wurde 30 min auf 55°C erwärmt und portionsweise mit 6-Chlor-8-methyl-[1,2,4]triazolo[4,3-b]pyridazin-3-ylamin-Trifluoracetat (W2.002b; 500 mg), suspendiert in 3-Pentanol (5 ml) und DMF (10 ml), versetzt. Nach 1,5 h Rühren bei 55°C wurde über Nacht bei RT stehen gelassen, das DMF im Vakuum entfernt, der Rückstand anschließend mit Wasser versetzt und viermal mit Dichlormethan extrahiert. Die vereinigten organischen Phasen wurden über Natriumsulfat getrocknet und nach Abfiltrieren des Trockenmittels im Vakuum zur Trockne gebracht. Der Rückstand wurde mittels präparativer HPLC (Met-A) gereinigt, die sauberen Produktfraktionen vereinigt, im Vakuum vom Acetonitril befreit, mit gesättigter Kaliumcarbonat-Lösung alkalisch gestellt und mehrmals mit Dichlormethan extrahiert. Die vereinigten organischen Phasen wurden über Natriumsulfat getrocknet, filtriert und eingeengt. Es wurden 198 mg der Titelverbindung erhalten. LCMS-rt: 1,24 min [M+H]⁺: 236,2 (Met-a)

### W1.032

### 6-(2-Methoxy-1-methoxymethyl-ethoxy)-8-methyl-[1,2,4]triazolo[4,3-b]pyridazin-3-ylamin

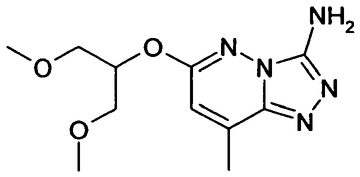

1,3-Dimethoxy-propan-2-ol (12,96 ml) wurde in DMF (25 ml) vorgelegt, unter Argon mit Natriumhydrid (681 mg) versetzt und 1 h bei 40°C gerührt. Anschließend 6-Chlor-[1,2,4]triazolo[4,3-b]pyridazin-3-ylamine-Hydrobromid (W2.002; 1,5 g), gelöst in DMF (25 ml), innerhalb von 20 min zugetropft. Nach 1 h wurde mit Wasser versetzt und viermal mit Dichlormethan ausgeschüttelt. Die vereinigten organischen Phasen wurden über Magnesiumsulfat getrocknet und einrotiert. Der Rückstand wurde mit MtB-Ether verrieben, abgesaugt und getrocknet. Es wurden 764 mg der Titelverbindung isoliert.

| | |
|---|---|
| LCMS-rt: 0,74 min | [M+H]⁺: 268,1 (Met-a) |

### W1.033

### 6-Cyclopropylmethoxy-8-methyl-[1,2,4]triazolo[4,3-b]pyridazin-3-ylamin

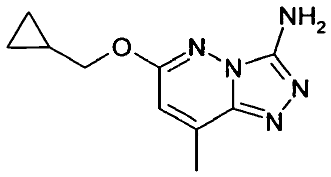

Cyclopropyl-methanol (2,99 ml) wurde in DMF (30 ml) vorgelegt, unter Argon mit Natriumhydrid (907 mg) versetzt und 1 h bei 50°C gerührt. Anschließend wurde 6-Chlor-8-methyl-[1,2,4]triazolo[4,3-b]pyridazin-3-ylamin-Hydrobromid (W2.002; 2,0 g) in DMF (30 ml) gelöst und ein Äquivalent der Alkoholat-Lösung zugegeben. Nach einer Stunde Rühren bei 50°C wurden zwei weitere Äquivalente Alkoholat-Lösung zugegeben und weiter bei 50°C gerührt. Dann wurde das Reaktionsgemisch zur Trockne gebracht und über Kieselgel (80 g Kartusche, Dichlormethan/Methanol-Gradient von 0-10% in 40 min) aufgereinigt. Es wurden 620 mg der Titelverbindung erhalten. LCMS-rt: 0,57 min [M+H]⁺: 220,1 (Met-b)

### W1.034

### 8-Methyl-6-[2-(tetrahydro-pyran-2-yloxy)-ethoxy]-[1,2,4]triazolo[4,3-b]pyridazin-3-ylamin

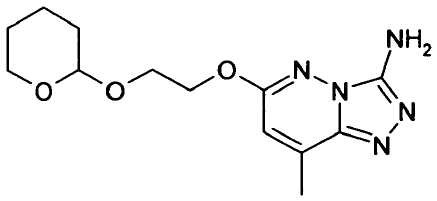

2-(Tetrahydro-pyran-2-yloxy)-ethanol (3,85 ml) wurde in DMF (25 ml) vorgelegt, unter Argon mit Natriumhydrid (681 mg) versetzt und 0,5 h bei 40°C gerührt. Anschließend wurde 6-Chlor-8-methyl-[1,2,4]triazolo[4,3-b]pyridazin-3-ylamin-Hydrobromid (W2.002; 1,5 g), gelöst in DMF (25 ml), zugetropft. Nach 40 min wurde mit Wasser versetzt und viermal mit Dichlormethan ausgeschüttelt. Zur Beseitigung des überschüssigen Alkohols wurde zunächst über ein kurze Kieselgelsäule mit MtB-Ether und anschließend mit Dichlormethan/Methanol 8:2 chromatographiert. Die sauberen Produktfraktionen wurden vereinigt und zur Trockne gebracht. Es wurden 678 mg der Titelverbindung erhalten.

| | |
|---|---|
| LCMS-rt: 0,88 min | [M+H]⁺: 294,1 (Met-a) |

### W1.035

### 8-Methyl-6-(3-methyl-oxetan-3-ylmethoxy)-[1,2,4]triazolo[4,3-b]pyridazin-3-ylamin

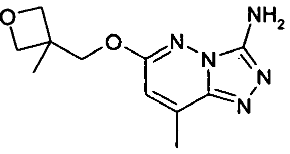

(3-Methyl-oxetan-3-yl)-methanol (1,74 g) wurde in DMF (30 ml) vorgelegt, unter Argon mit Natriumhydrid (408 mg) versetzt und 0,5 h bei 45°C gerührt. Anschließend 6-Chlor 8-methyl-[1,2,4]triazolo[4,3-b]pyridazin-3-ylamin-Hydrobromid (W2.002; 1,5 g) in DMF (30 ml) gelöst und ein Äquivalent der Alkoholat-Lösung (10 ml) zugegeben. Nach 30 min Rühren bei 45°C wurden weitere 0,5 Äquivalente Alkoholat-Lösung zugegeben, gefolgt von je 0,5 eq. nach weiteren 30 und 60 min. Dann wurde mit Wasser versetzt, das Reaktionsgemisch zur Trockne gebracht und über Kieselgel (80 g Kartusche, Dichlormethan/Methanol-Gradient von 0-20% in 60 min) aufgereinigt. Es wurden 758 mg der Titelverbindung erhalten.

| | |
|---|---|
| LCMS-rt: 0,49 min | [M+H]⁺: 250,1 (Met-b) |

Analog wurde dargestellt:

| Nummer | | LCMS-rt | [M+H]⁺: | Bemerkung: |
|---|---|---|---|---|
| W1.036 | 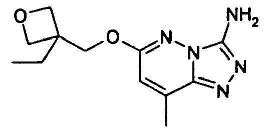 | 0,57 min | 264,1 (Met-b) | W2.002 (1 g); Produkt: 550 mg |
| W1.037 | 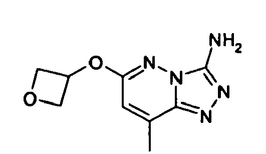 | 0,62 min | 222,1 (Met-a) | W2.002 (1 g); Produkt: 605 mg |

### W1.040

### N*6*,N*6*-Diethyl-8-methyl-[1,2,4]triazolo[4,3-b]pyridazin-3,6-diamin

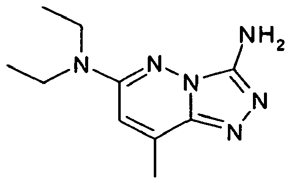

6-Chlor-8-methyl-[1,2,4]triazolo[4,3-b]pyridazin-3-ylamin-Hydrobromid (W2.002; 250 mg) wurde in DMF abs. (2 ml) gelöst und mit Diethylamin (7 ml) versetzt. Danach stellte man das Reaktionsgemisch bei 80°C für 11 Tage unter Rühren in den Heizblock. Dann wurde das Lösungsmittel abgezogen und der Rückstand mit wenig Wasser versetzt und dreimal mit Dichlormethan extrahiert. Die vereinigten organischen Phasen wurden über Natriumsulfat getrocknet, filtriert und eingeengt. Der Rückstand wurde mittels präparativer HPLC (Met-A) gereinigt, die sauberen Produktfraktionen vereinigt, im Vakuum vom Acetonitril befreit, mit gesättigter Kaliumcarbonat-Lösung alkalisch gestellt und mehrmals mit Dichlormethan extrahiert. Die vereinigten organischen Phasen wurden über Natriumsulfat getrocknet, filtriert und eingeengt. Es wurden 100 mg der Titelverbindung erhalten.

| | |
|---|---|
| LCMS-RT: 0,70 min | [M+H]⁺: 221,2 (Met-b) |

### W1.041 und W1.041a

### 8-Methyl-6-pyrrolidin-1-yl-[1,2,4]triazolo[4,3-b]pyridazin-3-ylamin und 8-Methyl-6-pyrrolidin-1-yl-[1,2,4]triazolo[4,3-b]pyridazin-3-ylamin-Trifluoracetat

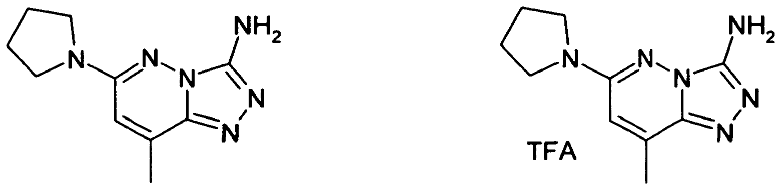

6-Chlor-8-methyl-[1,2,4]triazolo[4,3-b]pyridazin-3-ylamin-Hydrobromid (W2.002; 250 mg) wurde in DMF (2 ml) gelöst und mit Pyrrolidin (2 ml) versetzt. Danach stellte man das Reaktionsgemisch bei 80°C 1,5 h unter Rühren in den Heizblock. Anschließend wurde das Lösungsmittel abgezogen und der Rückstand mit wenig Wasser versetzt und dreimal mit Dichlormethan extrahiert. Die vereinigten organischen Phasen wurden über Natriumsulfat getrocknet, filtriert und eingeengt. Beim Lösen des Rückstands in Acetonitril/Wasser + 0,05 % TFA fiel ein Niederschlag aus, der abgesaugt und getrocknet wurde. Es wurden 40 mg 8-Methyl-6-pyrrolidin-1-yl-[1,2,4]triazolo[4,3-b]pyridazin-3-ylamin erhalten. Die Mutterlauge wurde mittels präparativer HPLC (Met-A) gereinigt, die sauberen Produktfraktionen vereinigt, im Vakuum vom Acetonitril befreit und gefriergetrocknet. Es wurden 11 mg 8-Methyl-6-pyrrolidin-1-yl-[1,2,4]triazolo[4,3-b]pyridazin-3-ylamin als Trifluoressigsäuresalz erhalten. 8-Methyl-6-pyrrolidin-1-yl-[1,2,4]triazolo[4,3-b]pyridazin-3-ylamin

| | |
|---|---|
| LCMS-rt: 0,76 min | [M+H]⁺: 219,1 (Met-b) |

### W1.045 und W1.046

### 6-Ethoxy-8-trifluormethyl-[1,2,4]triazolo[4,3-b]pyridazin-3-ylamin und 6-Ethoxy-8-(ethoxy-difluor-methyl)-[1,2,4]triazolo[4,3-b]pyridazin-3-ylamin

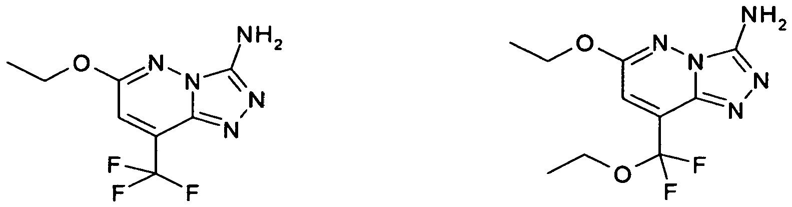

6-Chlor-8-trifluormethyl-[1,2,4]triazolo[4,3-b]pyridazin-3-ylamin (W2.004; 65 mg) wurde in Ethanol (6 ml) vorgelegt und mit Natriumethylat (21 mg) versetzt. Das Reaktionsgemisch rührte 4 h bei 50 °C. Nach Abkühlen wurde mit Wasser versetzt und eingeengt. Der Rückstand wurde in Essigsäureethylester aufgenommen und dreimal mit Wasser gewaschen. Die Essigsäureethylester-Phase wurde über Natriumsulfat getrocknet, filtriert und eingeengt. Das Rohprodukt wurde mittels präparativer HPLC (Met-E) aufgereinigt. Die jeweils sauberen, Produkt enthaltenden Fraktionen wurden vereinigt, vom Acetonitril befreit, mit Natriumhydrogencarbonat auf pH 9 gestellt und dreimal mit Essigsäureethylester extrahiert. Die vereinigten organischen Extrakte wurden über Natriumcarbonat getrocknet, filtriert und eingeengt. Es wurden 15 mg 6-Ethoxy-8-trifluormethyl-[1,2,4]triazolo[4,3-b]pyridazin-3-ylamin und 23 mg 6-Ethoxy-8-(ethoxy-difluor-methyl)-[1,2,4]triazolo[4,3-b]pyridazin-3-ylamin erhalten. 6-Ethoxy-8-trifluormethyl-[1,2,4]triazolo[4,3-b]pyridazin-3-ylamin, W1.045

| | |
|---|---|
| LCMS-rt: 0,70 min | [M+H]⁺: 248,1 (Met-b) |

### 6-Ethoxy-8-(ethoxy-difluormethyl)-[1,2,4]triazolo[4,3-b]pyridazin-3-ylamin, W1.046

| | |
|---|---|
| LCMS-rt: 0,82 min | [M+H]⁺: 272,2 (Met-b) |

### W1.050

### 6-Ethoxy-7-methyl-[1,2,4]triazolo[4,3-b]pyridazin-3-ylamin

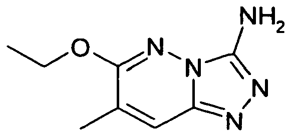

6-Chlor-7-methyl-[1,2,4]triazolo[4,3-b]pyridazin-3-ylamin (W2.005; 1 g) wurde in Ethanol (50 ml) gelöst, unter Argon mit Natriumethylat (2,2 g) versetzt und 1 h bei 60°C gerührt. Dann wurde zur Trockne gebracht und der Rückstand über eine 40 g Kieselgelkartusche (Dichlormethan-Ethanol-Gradient 0-20 % in 40 min) aufgereinigt. Die sauberen Produktfraktionen wurden vereinigt und zur Trockne gebracht. Es wurden 480 mg der Titelverbindung erhalten.

| | |
|---|---|
| LCMS-rt: 2,07 min | [M+H]⁺: 194,1 (Met-d) |

### W1.055

### 6-Ethoxy-7,8-dimethyl-[1,2,4]triazolo[4,3-b]pyridazin-3-ylamin

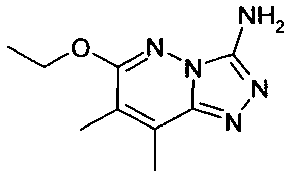

6-Chlor-7,8-dimethyl-[1,2,4]triazolo[4,3-b]pyridazin-3-ylamin-Hydrobromid (W2.006; 4 g) wurde in Ethanol (120 ml) gelöst, unter Argon mit Natriumethylat (5,86 g) versetzt und 1 h bei 60°C gerührt. Dann wurde zur Trockne gebracht und der Rückstand über eine 40 g Kieselgelkartusche (Dichlormethan-Ethanol-Gradient 0-20 % in 40 min) aufgereinigt. Die sauberen Produktfraktionen wurden vereinigt und zur Trockne gebracht. Es wurden 3,24 g der Titelverbindung erhalten, die noch Restmengen Dichlormethan enthielt. LCMS-rt: 0,53 min [M+H]⁺: 208,1 (Met-b)

### W1.056

### 6-((S)-sec-Butoxy)-7,8-dimethyl-[1,2,4]triazolo[4,3-b]pyridazin-3-ylamin

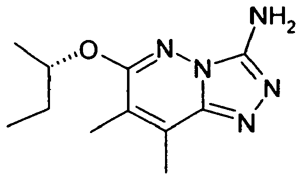

(S)-Butan-2-ol (1,7 ml) wurde bei RT unter Rühren vorgelegt und mit einem Eisbad annähernd auf 0°C gekühlt. Anschließend wurde portionsweise mit Natriumhydrid (85 mg) versetzt. Die dabei entstehende Suspension wurde 1,5 h auf 55°C erwärmt und dann portionsweise mit 6-Chlor-7,8-dimethyl-[1,2,4]triazolo[4,3-b]pyridazin-3-ylamin (W2.006a, 100 mg), suspendiert in (S)-Butan-2-ol (2 ml) und DMF (2 ml), versetzt. Nach 1 h Rühren bei 55°C wurde mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 105 mg der Titelverbindung in ausreichender Reinheit isoliert. LCMS-rt: 0,81 min
[M+H]⁺: 236,2 (Met-b)

### W1.057

### 6-((R)-sec-Butoxy)-7,8-dimethyl-[1,2,4]triazolo[4,3-b]pyridazin-3-ylamin

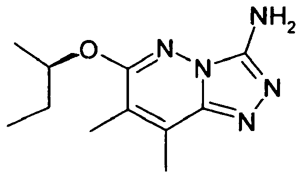

(R)-Butan-2-ol (0,5 ml) wurde analog W1.056 mit Natriumhydrid (85 mg) deprotoniert und mit 6-Chlor-7,8-dimethyl-[1,2,4]triazolo[4,3-b]pyridazin-3-ylamin (W2.006a, 100 mg), suspendiert in DMF (2 ml), umgesetzt und aufgearbeitet. Es wurden 75 mg der Titelverbindung in ausreichender Reinheit isoliert.

| | |
|---|---|
| LCMS-rt: 0,81 min | [M+H]⁺: 236,2 (Met-b) |

### W1.058

### 6-Isopropoxy-7,8-dimethyl-[1,2,4]triazolo[4,3-b]pyridazin-3-ylamin

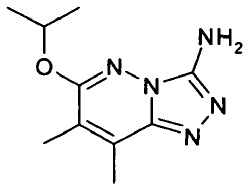

Isopropanol (27 ml) wurde analog W1.056 mit Natriumhydrid (1,3 g) deprotoniert und mit 6-Chlor-7,8-dimethyl-[1,2,4]triazolo[4,3-b]pyridazin-3-ylamin (W2.006a, 1 g), gelöst in Isopropanol (10 ml) und DMF (10 ml), umgesetzt und aufgearbeitet. Es wurden 847 mg der Titelverbindung in ausreichender Reinheit isoliert.

| | |
|---|---|
| LCMS-rt: 0,74 min | [M+H]⁺: 222,2 (Met-b) |

### W1.059

### 7,8-Dimethyl-6-propoxy-[1,2,4]triazolo[4,3-b]pyridazin-3-ylamin

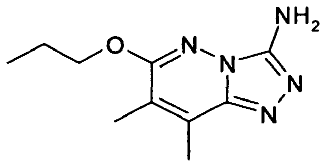

1-Propanol (28 ml) wurde analog W1.056 mit Natriumhydrid (1,3 g) deprotoniert und mit 6-Chlor-7,8-dimethyl-[1,2,4]triazolo[4,3-b]pyridazin-3-ylamin (W2.006a, 1 g), gelöst in 1-Propanol (10 ml) und DMF (10 ml), umgesetzt und aufgearbeitet. Es wurden 647 mg der Titelverbindung in ausreichender Reinheit isoliert.

| | |
|---|---|
| LCMS-rt: 0,75 min | [M+H]⁺: 222,2 (Met-b) |

### W1.060 und W1.061

### 6-(1-Ethyl-propoxy)-7,8-dimethyl-[1,2,4]triazolo[4,3-b]pyridazin-3-ylamin und 6-Methoxy-7,8-dimethyl-[1,2,4]triazolo[4,3-b]pyridazin-3-ylamin

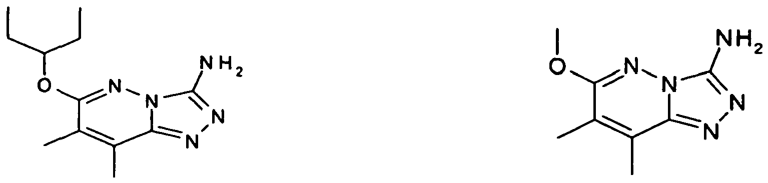

3-Pentanol (1,5 ml) wurde bei RT unter Rühren vorgelegt und mit einem Eisbad annähernd auf 0°C gekühlt. Anschließend wurde portionsweise mit Natriumhydrid (117 mg) versetzt. Die dabei entstehende Suspension wurde 1,5 h auf 55°C erwärmt und dann portionsweise mit 6-Chlor-7,8-dimethyl-[1,2,4]triazolo[4,3-b]pyridazin-3-ylamin (W2.006a, 100 mg), suspendiert in 3-Pentanol (1 ml) und DMF (1,5 ml), versetzt. Nach 2 h Rühren bei 55°C wurde über Nacht bei RT stehen gelassen, mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Der Rückstand wurde mittels präparativer HPLC (Met-A) gereinigt, die sauberen Produktfraktionen vereinigt, im Vakuum vom Acetonitril befreit, mit gesättigter Kaliumcarbonat-Lösung alkalisch gestellt und mehrmals mit Dichlormethan extrahiert. Die vereinigten organischen Phasen wurden über Natriumsulfat getrocknet, filtriert und eingeengt. Es wurden 79 mg 6-(1-Ethyl-propoxy)-7,8-dimethyl-[1,2,4]triazolo[4,3-b]pyridazin-3-ylamin sowie 10 mg 6-Methoxy-7,8-dimethyl-[1,2,4]triazolo[4,3-b]pyridazin-3-ylamin erhalten, das durch Verunreinigung mit Methanol im Reaktionsgemisch entstand.

### 6-(1-Ethyl-propoxy)-7,8-dimethyl-[1,2,4]triazolo[4,3-b]pyridazin-3-ylamin, W1.060

| | |
|---|---|
| LCMS-rt: 1,30 min | [M+H]⁺: 250,2 (Met-a) |

### 6-Methoxy-7,8-dimethyl-[1,2,4]triazolo[4,3-b]pyridazin-3-ylamin, W1.061

| | |
|---|---|
| LCMS-rt: 0,88 min | [M+H]⁺: 194,1 (Met-a) |

### W1.062

### 6-Cyclopropylmethoxy-7,8-dimethyl-[1,2,4]triazolo[4,3-b]pyridazin-3-ylamin

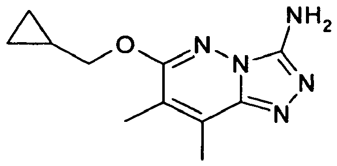

Cyclopropyl-methanol (2,84 ml) wurde in DMF (20 ml) vorgelegt, unter Argon mit Natriumhydrid (862 mg) versetzt und 1 h bei 50°C gerührt. Anschließend wurde 6-Chlor-7,8-dimethyl-[1,2,4]triazolo[4,3-b]pyridazin-3-ylamin-Hydrobromid (W2.006, 2 g) in DMF (20 ml) gelöst und ein Äquivalent der Alkoholat-Lösung zugegeben. Nach einer Stunde Rühren bei 50°C wurden zwei weitere Äquivalente Alkoholat-Lösung zugegeben und weiter bei 50°C gerührt. Dann wurde das Reaktionsgemisch zur Trockne gebracht und über Kieselgel (40 g Kartusche, Dichlormethan/Methanol-Gradient von 0-10% in 40 min) aufgereinigt. Es wurden 625 mg der Titelverbindung erhalten. LCMS-rt: 0,62 min[M+H]⁺: 234,1 (Met-b)

### W1.065

### N*6*,N*6*-Diethyl-7,8-dimethyl-[1,2,4]triazolo[4,3-b]pyridazine-3,6-diamin

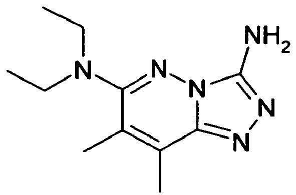

6-Chlor-7,8-dimethyl-[1,2,4]triazolo[4,3-b]pyridazin-3-ylamin (W2.006a, 800 mg und 650 mg auf 2 Mikrowellengefäße verteilt), wurde in jeweils 12 ml Diethylamin gelöst vorgelegt und mit 1-Butyl-3-methylimidazolium-hexafluorphosphat (150 µl) versetzt. Anschließend wurde bei 180°C 4 h in die Mikrowelle gestellt. Um die Reaktion zu vervollständigen wurde erneut bei 180°C 4 h in die Mikrowelle gestellt und dann über Nacht stehen gelassen. Die beiden Reaktionsgemische wurden in einem Rundkolben vereinigt und zur Trockne gebracht. Der Rückstand wurde mit Wasser versetzt und dreimal mit Dichlormethan extrahiert. Die vereinigten organischen Phasen wurden über Natriumsulfat getrocknet, filtriert und eingeengt. Der Rückstand wurde mittels präparativer HPLC (Met-F) gereinigt, die sauberen Produktfraktionen vereinigt, im Vakuum vom Acetonitril befreit, mit gesättigter Kaliumcarbonat-Lösung alkalisch gestellt und mehrmals mit Dichlormethan extrahiert. Die vereinigten organischen Phasen wurden über Natriumsulfat getrocknet, filtriert und eingeengt. Es wurden 900 mg der Titelverbindung erhalten.

| | |
|---|---|
| LCMS-rt: 0,77 min | [M+H]⁺: 235,2 (Met-b) |

### W1.066

### 7,8-Dimethyl-6-pyrrolidin-1-yl-[1,2,4]triazolo[4,3-b]pyridazin-3-ylamin

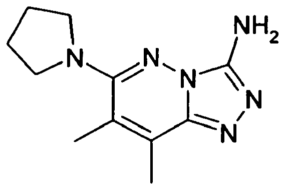

6-Chlor-7,8-dimethyl-[1,2,4]triazolo[4,3-b]pyridazin-3-ylamin (W2.006a, 500 mg) wurde in Pyrrolidin (5 ml) bei RT unter Rühren vorgelegt. Dann erwärmte man 4 h auf 65°C. Danach wurde das Pyrrolidin abgezogen, der Rückstand mit Wasser aufgenommen und dreimal mit Dichlormethan extrahiert. Die vereinigten organischen Phasen wurden über Natriumsulfat getrocknet, filtriert und eingeengt. Es wurden 438 mg der Titelverbindung erhalten.

| | |
|---|---|
| LCMS-rt: 0,72 min | [M+H]⁺: 233,2 (Met-b) |

### W1.070

### 6-Ethoxy-7-ethyl-8-methyl-[1,2,4]triazolo[4,3-b]pyridazin-3-ylamin

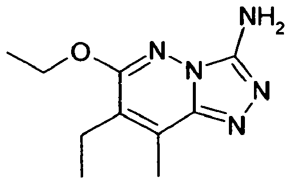

6-Chlor-7-ethyl-8-methyl-[1,2,4]triazolo[4,3-b]pyridazin-3-ylamin (W2.007; 380 mg) wurde in Ethanol (45 ml) bei RT unter Rühren vorgelegt, mit Natriumethylat (244 mg) versetzt und 3 h auf 55°C erwärmt. Dann wurde ein weiteres Äquivalent Natriumethylat zugegeben und nochmals 3 h bei 55°C gerührt. Nach Stehenlassen übers Wochenende wurde zur Trockne gebracht, der Rückstand mit Wasser aufgenommen und dreimal mit Dichlormethan extrahiert. Die vereinigten organischen Phasen wurden über Natriumsulfat getrocknet, filtriert und eingeengt. Es wurden 387 mg der Titelverbindung erhalten.

| | |
|---|---|
| LCMS-rt: 0,76 min | [M+H]⁺_{:} 222,2 (Met-b) |

### W1.071

### 7-Ethyl-6-(1-ethyl-propoxy)-8-methyl-[1,2,4]triazolo[4,3-b]pyridazin-3-ylamin

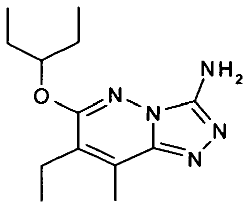

3-Pentanol (2,5 ml) wurde bei RT unter Rühren vorgelegt. Danach wurde unter Eiskühlung Natriumhydrid (91 mg) zugegeben. Nach 2,5 h Rühren bei 55°C tropfte man 6-Chlor-7-ethyl-8-methyl-[1,2,4]triazolo[4,3-b]pyridazin-3-ylamin (W2.007; 120 mg), gelöst in 3-Pentanol (2 ml) und DMF (4 ml), zu. Nach 1 h Rühren ließ man über Nacht bei RT stehen, versetzte das Reaktionsgemisch mit Wasser und Dichlormethan und extrahierte anschließend noch dreimal mit Dichlormethan. Die vereinigten organischen Phasen wurden über Natriumsulfat getrocknet und nach Abfiltrieren des Trockenmittels im Vakuum zur Trockne gebracht. Es wurden 126 mg der Titelverbindung erhalten. LCMS-rt: 0,90 min [M+H]⁺: 264,2 (Met-b)

### W1.075

### 6-Ethoxy-8-ethyl-[1,2,4]triazolo[4,3-b]pyridazin-3-ylamin

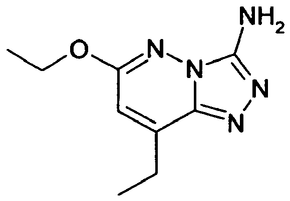

6-Chlor-8-ethyl-[1,2,4]triazolo[4,3-b]pyridazin-3-ylamin (W2.003; 700 mg) wurde in Ethanol (100 ml) unter Rühren gelöst. Danach versetzte man das Reaktionsgemisch mit Natriumethylat (724 mg) und rührte 2 h bei RT. Anschließend wurde 3 h auf 45°C erwärmt. Nach Abziehen des Lösungsmittels wurde der Rückstand mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 670 mg der Titelverbindung erhalten.

| | |
|---|---|
| LCMS-rt: 0,64 min | [M+H]⁺: 208,2 (Met-b) |

### W1.076

### 6-Ethoxy-8-ethyl-7-methyl-[1,2,4]triazolo[4,3-b]pyridazin-3-ylamin

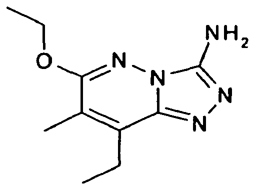

6-Chlor-8-ethyl-7-methyl-[1,2,4]triazolo[4,3-b]pyridazin-3-ylamin (W2.008, 273 mg) wurde in Ethanol abs. (20 ml) unter Rühren gelöst. Danach versetzte man das Reaktionsgemisch mit Natriumethylat (176 mg) und rührte 3,5 h bei RT. Nach Stehen übers Wochenende wurde das Lösungsmittels abgezogen, der Rückstand mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 230 mg der Titelverbindung erhalten.

| | |
|---|---|
| LCMS-rt: 0,75 min | [M+H]⁺: 222,2 (Met-b) |

### W1.080 und W1.081

### 6-Ethoxy-8-isopropyl-[1,2,4]triazolo[4,3-b]pyridazin-3-ylamin und 2-(3-Amino-6-ethoxy-[1,2,4]triazolo[4,3-b]pyridazin-8-yl)-propan-2-ol

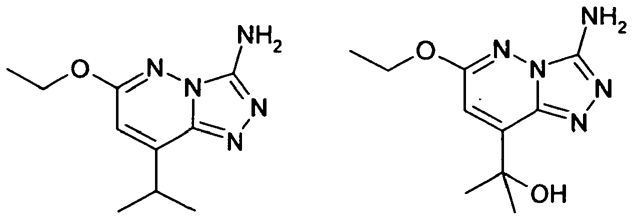

6-Chlor-8-isopropyl-[1,2,4]triazolo[4,3-b]pyridazin-3-ylamin (W2.009, 77 mg) wurde in Ethanol (8 ml) unter Rühren bei RT vorgelegt und mit Natriumethylat (50 mg) versetzt. Nach 4,5 h Rühren ließ man über Nacht stehen und zog dann das Lösungsmittel ab. Der Rückstand wurde mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 52 mg der Titelverbindungen als Gemisch erhalten (laut ¹H-NMR ~70:30 zugunsten von 6-Ethoxy-8-isopropyl-[1,2,4]triazolo[4,3-b]pyridazin-3-ylamin).

### 6-Ethoxy-8-isopropyl-[1,2,4]triazolo[4,3-b]pyridazin-3-ylamin, W1.080

| | |
|---|---|
| LCMS-rt: 0,76 min | [M+H]⁺: 222,2 (Met-b) |

### 2-(3-Amino-6-ethoxy-[1,2,4]triazolo[4,3-b]pyridazin-8-yl)-propan-2-ol, W1.081

| | |
|---|---|
| LCMS-rt: 0,40 min | [M+H]⁺: 238,1 (Met-b) |

### W1.082

### 8-Cyclopropyl-6-ethoxy-[1,2,4]triazolo[4,3-b]pyridazin-3-ylamin

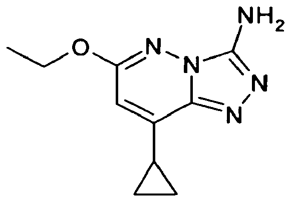

6-Chlor-8-cyclopropyl-[1,2,4]triazolo[4,3-b]pyridazin-3-ylamin (W2.010, 77 mg) wurde in Ethanol (10 ml) unter Rühren bei RT vorgelegt und mit Natriumethylat (67 mg) versetzt. Nach 4,5 h Rühren ließ man über Nacht stehen und zog dann das Lösungsmittel ab. Der Rückstand wurde mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 91 mg der Titelverbindung erhalten

| | |
|---|---|
| LCMS-rt: 0,71 min | [M+H]⁺: 220,2 (Met-b) |

### W1.085

### 6-Ethoxy-7-ethyl-[1,2,4]triazolo[4,3-b]pyridazin-3-ylamin

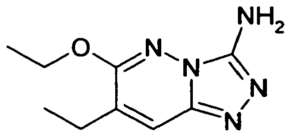

6-Chlor-7-ethyl-[1,2,4]triazolo[4,3-b]pyridazin-3-ylamin-Trifluoracetatsalz (W2.011, 82 mg) wurde analog W1.075 umgesetzt und aufgearbeitet. Es wurden 77 mg der Titelverbindung erhalten. LCMS-rt: 0,69 min [M+H]⁺: 208,2 (Met-b)

### W1.086

### 7-Cyclopropyl-6-ethoxy-[1,2,4]triazolo[4,3-b]pyridazin-3-ylamin

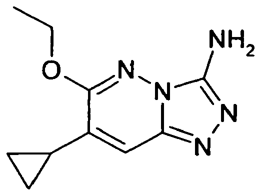

6-Chlor-7-cyclopropyl-[1,2,4]triazolo[4,3-b]pyridazin-3-ylamin (W2.012, 30 mg) wurde analog W1.075 umgesetzt und aufgearbeitet. Es wurden 31 mg der Titelverbindung erhalten. LCMS-rt: 0,70 min [M+H]⁺: 220,2 (Met-b)

### W1.087

### 6-Ethoxy-7-isopropyl-[1,2,4]triazolo[4,3-b]pyridazin-3-ylamin

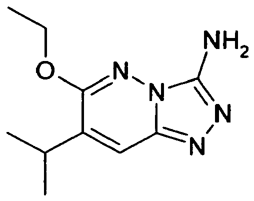

6-Chlor-7-isopropyl-[1,2,4]triazolo[4,3-b]pyridazin-3-ylamin (W2.013, 30 mg) wurde analog W1.075 umgesetzt und aufgearbeitet. Es wurden 31 mg der Titelverbindung erhalten. LCMS-rt: 0,76 min [M+H]⁺: 222,2 (Met-b)

### W1.090

### 6-Ethoxy-7,8-diethyl-[1,2,4]triazolo[4,3-b]pyridazin-3-ylamin

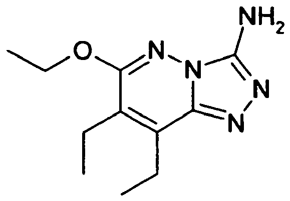

6-Chlor-7,8-diethyl-[1,2,4]triazolo[4,3-b]pyridazin-3-ylamin (W2.014, 0,1 g), wurde in Ethanol (13 ml) unter Rühren und Argon gelöst. Danach versetzte man das Reaktionsgemisch mit Natriumethylat (61 mg) und rührte 1 h bei RT, dann 30 min bei 55°C. Anschließend wurden weitere 2 Äquivalente Natriumethylat zugegeben und 4,5 h bei 55°C gerührt. Nachdem erneut 1 Äquivalent Natriumethylat zugegeben worden war, wurde 2,5 h bei 55°C gerührt und übers Wochenende stehen gelassen. Dann wurde das Lösungsmittel abgezogen, der Rückstand mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 92 mg der Titelverbindung erhalten.

| | |
|---|---|
| LCMS-rt: 0,80 min | [M+H]⁺: 236,2 (Met-b) |

### W1.091

### 6-Ethoxy-7,8-diisopropyl-[1,2,4]triazolo[4,3-b]pyridazin-3-ylamin

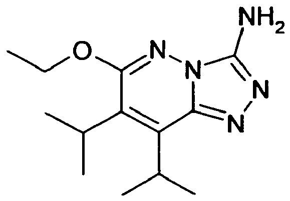

6-Chlor-7,8-diisopropyl-[1,2,4]triazolo[4,3-b]pyridazin-3-ylamin (W2.015, 0,1 g) wurde in Ethanol (15 ml) unter Rühren und Argon gelöst. Danach versetzte man das Reaktionsgemisch mit Natriumethylat (110 mg) und rührte 1 h bei RT, dann 1 h bei 55°C. Anschließend wurde über Nacht Stehen gelassen, dann erneut 7,5 h bei 55°C gerührt und danach das Lösungsmittel abgezogen, der Rückstand mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 100 mg der Titelverbindung erhalten. LCMS-rt: 0,90 min [M+H]⁺: 264,2 (Met-b)

### W1.092

### 7,8-Dicyclopropyl-6-ethoxy-[1,2,4]triazolo[4,3-b]pyridazin-3-ylamin

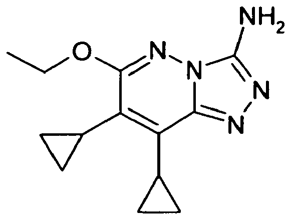

6-Chlor-7,8-dicyclopropyl-[1,2,4]triazolo[4,3-b]pyridazin-3-ylamin (W2.016, 0,1 g), wurde in Ethanol (20 ml) unter Rühren und Argon gelöst. Danach versetzte man das Reaktionsgemisch mit Natriumethylat (110 mg) und rührte 1 h bei RT, dann 1 h bei 55°C. Anschließend wurde über Nacht Stehen gelassen und dann 7,5 h auf Rückfluss erhitzt. Nach Stehen übers Wochenende wurde das Lösungsmittel abgezogen, der Rückstand mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 100 mg der Titelverbindung erhalten.

| | |
|---|---|
| LCMS-rt: 0,84 min | [M+H]⁺: 260,2 (Met-b) |

### W1.095

### 6-Ethoxy-8,9-dihydro-7H-cyclopenta[d][1,2,4]triazolo[4,3-b]pyridazin-3-ylamin

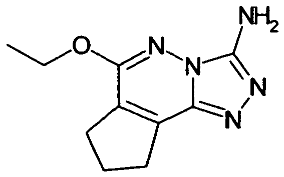

6-Chlor-8,9-dihydro-7H-cyclopenta[d][1,2,4]triazolo[4,3-b]pyridazin-3-ylamin-Hydrobromid (W2.017, 1,0 g) wurde in Ethanol (50 ml) unter Rühren und Argon gelöst. Danach versetzte man das Reaktionsgemisch mit Natriumethylat (1,41 g) und rührte 1 h bei 60°C. Nach Abziehen des Lösungsmittels wurde der Rückstand über eine 40 g Kieselgelkartusche (Dichlormethan-Ethanol-Gradient 0-20 % in 60 min) aufgereinigt. Die sauberen Produktfraktionen wurden vereinigt und zur Trockne gebracht. Es wurden 662 mg der Titelverbindung erhalten.

| | |
|---|---|
| LCMS-rt: 0,56 | [M+H]⁺: 220,1 (Met-b) |

### W1.096

### 6-Ethoxy-7,8,9,10-tetrahydro-[1,2,4]triazolo[3,4-a]phthalazin-3-ylamin

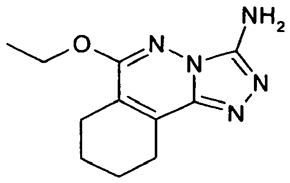

6-Chlor-7,8,9,10-tetrahydro-[1,2,4]triazolo[3,4-a]phthalazin-3-ylamin (W2.018, 1,0 g) wurde in Ethanol (50 ml) unter Rühren und Argon gelöst. Danach versetzte man das Reaktionsgemisch mit Natriumethylat (1,34 g) und rührte 1 h bei 60°C. Nach Abziehen des Lösungsmittels wurde der Rückstand über eine 40 g Kieselgelkartusche (Dichlormethan-Ethanol-Gradient 0-20 % in 40 min) aufgereinigt. Die sauberen Produktfraktionen wurden vereinigt und zur Trockne gebracht. Es wurden 491 mg der Titelverbindung erhalten.

| | |
|---|---|
| LCMS-rt: 0,95 | [M+H]⁺: 234,1 (Met-a) |

### W1.100

### 3-Amino-6-ethoxy-[1,2,4]triazolo[4,3-b]pyridazin-7-carbosäurediethylamid

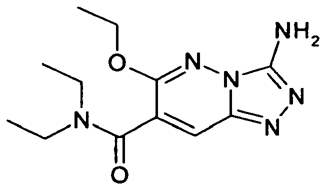

3-Amino-6-chlor-[1,2,4]triazolo[4,3-b]pyridazin-7-carbonsäurediethylamid (W2.019, 50 mg) wurde in Ethanol (5 ml) vorgelegt und unter Rühren mit Natriumethylat (28 mg) versetzt. Nach 7 h Rühren bei RT und Stehenlassen über Nacht wurde das Lösungsmittel abgezogen und dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 51 mg der Titelverbindung erhalten. LCMS-rt: 1,00 min [M+H]⁺: 279,2 (Met-b)

### W1.101

### 3-Amino-6-ethoxy-[1,2,4]triazolo[4,3-b]pyridazin-8-carbonsäurediethylamid

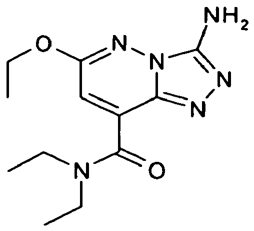

3-Amino-6-chlor-[1,2,4]triazolo[4,3-b]pyridazin-8-carbonsäurediethylamid (W2.020, 50 mg) wurde analog W1.100 dargestellt. Es wurden 51 mg der Titelverbindung erhalten.

| | |
|---|---|
| LCMS-rt: 1,04 min | [M+H]⁺: 279,2 (Met-b) |

### W1.102

### 6-Ethoxy-N*7*,N*7*-diethyl-[1,2,4]triazolo[4,3-b]pyridazin-3,7-diamin

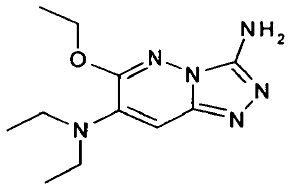

6-Chlor-N*7*,N*7*-diethyl-[1,2,4]triazolo[4,3-b]pyridazin-3,7-diamin (W2.021, 38 mg) wurde in Ethanol (7 ml) vorgelegt und mit Natriumethylat (24 mg) versetzt. Das Reaktionsgemisch rührte 4 h bei RT. Es wurde dann noch 2 h bei 45 ° C gerührt. Anschließend wurde zur Trockne gebracht und der Rückstand mit Wasser versetzt, dreimal mit EE extrahiert und die vereinigten EE-Phasen mit Magnesiumsulfat getrocknet, filtriert und eingeengt. Es wurden 30 mg der Titelverbindung als Rohprodukt erhalten, das sauber genug für die nächste Umsetzung war.

| | |
|---|---|
| LCMS-rt: 1,13 min | [M+H]⁺: 251,2 (Met-a) |

### W1.105

### 8-Ethyl-6-(1-ethyl-propoxy)-[1,2,4]triazolo[4,3-b]pyridazin-3-ylamin

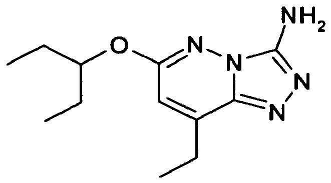

3-Pentanol (1,2 ml) wurde bei RT unter Rühren vorgelegt. Danach wurde unter Eiskühlung Natriumhydrid (77 mg) zugegeben. Nach 2,5 h Rühren bei 55°C tropfte man 6-Chlor-8-ethyl-[1,2,4]triazolo[4,3-b]pyridazin-3-ylamin (W2.003, 60 mg), gelöst in 3-Pentanol (1,5 ml) und DMF (4 ml), zu. Nach 2 h Rühren ließ man über Nacht bei RT stehen, versetzte das Reaktionsgemisch mit Wasser und Dichlormethan und extrahierte anschließend noch dreimal mit Dichlormethan. Die vereinigten organischen Phasen wurden über Natriumsulfat getrocknet und nach Abfiltrieren des Trockenmittels im Vakuum zur Trockne gebracht. Es wurden 70 mg der Titelverbindung erhalten.

| | |
|---|---|
| LCMS-rt: 0,87 min | [M+H]⁺: 250,2 (Met-b) |

### W1.106

### 7,8-Diethyl-6-(1-ethyl-propoxy)-[1,2,4]triazolo[4,3-b]pyridazin-3-ylamin

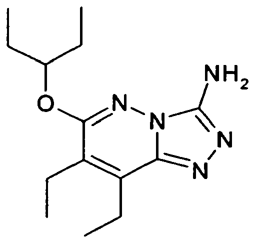

6-Chlor-7,8-diethyl-[1,2,4]triazolo[4,3-b]pyridazin-3-ylamin (W2.014, 100 mg) wurde analog W1.071 umgesetzt und aufgearbeitet. Es wurden 110 mg der Titelverbindung erhalten. LCMS-rt: 0,93 min [M+H]⁺: 278,2 (Met-b)

### W1.107

### 6-(1-Ethyl-propoxy)-8-isopropyl-[1,2,4]triazolo[4,3-b]pyridazin-3-ylamin

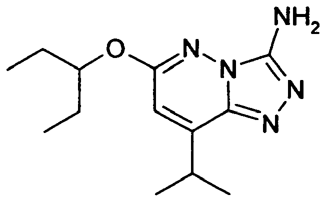

6-Chlor-8-isopropyl-[1,2,4]triazolo[4,3-b]pyridazin-3-ylamin (W2.009, 80 mg) wurde analog W1.071 umgesetzt und aufgearbeitet. Das Rohprodukt wurde dann mittels präparativer HPLC (Met-A) gereinigt, die sauberen Produktfraktionen vereinigt, im Vakuum vom Acetonitril befreit, mit gesättigter Kaliumcarbonat-Lösung alkalisch gestellt und mehrmals mit Dichlormethan extrahiert. Die vereinigten organischen Phasen wurden über Natriumsulfat getrocknet, filtriert und eingeengt. Es wurden 51 mg der Titelverbindung erhalten.

| | |
|---|---|
| LCMS-rt: 0,91 min | [M+H]⁺: 264,2 (Met-b) |

### W1.108

### 6-(1-Ethyl-propoxy)-7,8-diisopropyl-[1,2,4]triazolo[4,3-b]pyridazin-3-ylamin

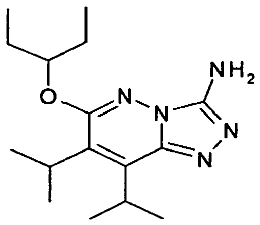

6-Chlor-7,8-diisopropyl-[1,2,4]triazolo[4,3-b]pyridazin-3-ylamin (W2.015, 100 mg) wurde analog W1.107 umgesetzt, aufgearbeitet und gereinigt. Es wurden 72 mg der Titelverbindung erhalten. LCMS-rt: 1,01 min [M+H]⁺: 306,3 (Met-b)

### W1.109

### 8-Cyclopropyl-6-(1-ethyl-propoxy)-[1,2,4]triazolo[4,3-b]pyridazin-3-ylamin

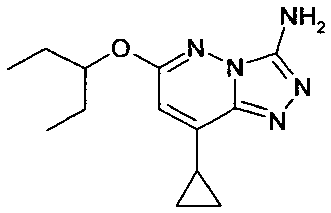

6-Chlor-8-cyclopropyl-[1,2,4]triazolo[4,3-b]pyridazin-3-ylamin (W2.010, 100 mg) wurde analog W1.107 umgesetzt, aufgearbeitet und gereinigt. Es wurden 74 mg der Titelverbindung erhalten. LCMS-rt: 0,89 min [M+H]⁺: 262,2 (Met-b)

### W1.110

### 7,8-Dicyclopropyl-6-(1-ethyl-propoxy)-[1,2,4]triazolo[4,3-b]pyridazin-3-ylamin

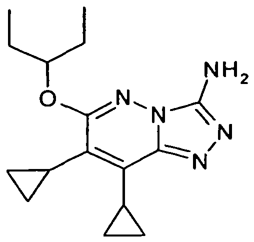

6-Chlor-7,8-dicyclopropyl-[1,2,4]triazolo[4,3-b]pyridazin-3-ylamin (W2.016, 100 mg) wurde analog W1.107 umgesetzt, aufgearbeitet und gereinigt. Es wurden 35 mg der Titelverbindung erhalten. LCMS-rt: 0,95 min [M+H]⁺: 302,2 (Met-b)

### W1.111

### 6-(1-Ethyl-propoxy)-7-methyl-[1,2,4]triazolo[4,3-b]pyridazin-3-ylamin

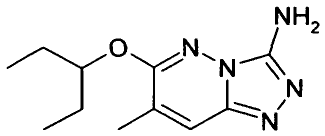

3-Pentanol (30 ml) wurden unter Argon vorgelegt, mit Natriumhydrid (1,48 g) versetzt und 1 h bei 50°C gerührt. Anschließend wurde 6-Chlor-7-methyl-[1,2,4]triazolo[4,3-b]pyridazin-3-ylamin (W2.005, 1,88 g), gelöst in 3-Pentanol (130 ml) und DMF (60 ml) innerhalb 1 h langsam zugetropft. Nach 1 h Rühren wurde das Gemisch aufkonzentriert, der Rückstand mit Wasser versetzt und viermal mit Dichlormethan extrahiert. Die vereinigten organischen Phasen wurden über Magnesiumsulfat getrocknet, filtriert und eingeengt. Der Rückstand wurde über eine 80 g Kieselgelkartusche (Dichlormethan-Methanol-Gradient 0-10 % in 40 min) aufgereinigt. Die sauberen Produktfraktionen wurden vereinigt und zur Trockne gebracht. Es wurden 1,25 g der Titelverbindung erhalten.

| | |
|---|---|
| LCMS-rt: 2,60 min | [M+H]⁺: 236,1 (Met-e) |

### W1.112

### 7-Ethyl-6-(1-ethyl-propoxy)-[1,2,4]triazolo[4,3-b]pyridazin-3-ylamin

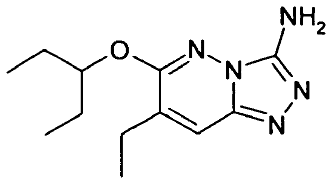

6-Chlor-7-ethyl-[1,2,4]triazolo[4,3-b]pyridazin-3-ylamin (W2.011, 150 mg) wurde analog W1.107 umgesetzt, aufgearbeitet und gereinigt. Es wurden 103 mg der Titelverbindung erhalten. LCMS-rt: 0,87 min [M+H]⁺: 250,2 (Met-b)

### W1.113

### 6-(1-Ethyl-propoxy)-7-isopropyl-[1,2,4]triazolo[4,3-b]pyridazin-3-ylamin

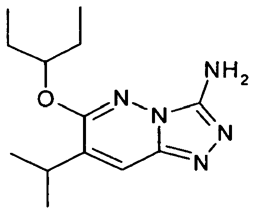

6-Chlor-7-isopropyl-[1,2,4]triazolo[4,3-b]pyridazin-3-ylamin (W2.013, 100 mg) wurde analog W1.071 umgesetzt und aufgearbeitet. Es wurden 132 mg der Titelverbindung erhalten, die noch mit etwas DMF verunreinigt war.

| | |
|---|---|
| LCMS-rt: 0,90 min | [M+H]⁺: 264,2 (Met-b) |

### W1.114

### 7-Cyclopropyl-6-(1-ethyl-propoxy)-[1,2,4]triazolo[4,3-b]pyridazin-3-ylamin

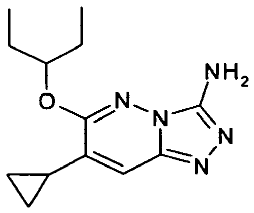

6-Chlor-7-cyclopropyl-[1,2,4]triazolo[4,3-b]pyridazin-3-ylamin (W2.012, 100 mg) wurde analog W1.071 umgesetzt und aufgearbeitet. Es wurden 116 mg der Titelverbindung erhalten.

| | |
|---|---|
| LCMS-rt: 0,87 min | [M+H]⁺: 262,2 (Met-b) |

### W1.115

### 6-(1-Ethyl-propoxy)-8,9-dihydro-7H-cyclopenta[d][1,2,4]triazolo[4,3-b]pyridazin-3-ylamin

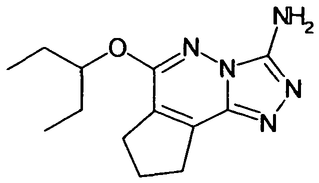

3-Pentanol (20 ml) wurden unter Argon vorgelegt, mit Natriumhydrid (991 mg) versetzt und 1 h bei 50°C gerührt. Anschließend wurde 6-Chlor-8,9-dihydro-7H-cycclpenta[d][1,2,4]triazolo[4,3-b]pyridazin-3-ylamin-Hydrobromid (W2.017, 2,0 g), gelöst in 3-Pentanol (20 ml) und DMF (100 ml), innerhalb 1 h langsam zugetropft. Nach 1 h Rühren wurde das Gemisch aufkonzentriert, der Rückstand mit Wasser versetzt und viermal mit Dichlormethan extrahiert. Die vereinigten organischen Phasen wurden über Magnesiumsulfat getrocknet, filtriert und eingeengt. Der Rückstand über eine 40 g Kieselgelkartusche (Dichlormethan-Methanol-Gradient 0-10 % in 50 min) aufgereinigt. Die sauberen Produktfraktionen wurden vereinigt und zur Trockne gebracht. Es wurden 1,04 g der Titelverbindung erhalten.

| | |
|---|---|
| LCMS-rt: 2,82 min | [M+H]⁺: 262,1 (Met-e) |

### W1.116

### 6-(1-Ethyl-propoxy)-7,8,9,10-tetrahydro-[1,2,4]triazolo[3,4-a]phthalazin-3-ylamin

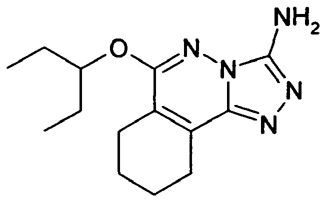

3-Pentanol (20 ml) wurden unter Argon vorgelegt, mit Natriumhydrid (946 mg) versetzt und 1 h bei 50°C gerührt. Anschließend wurde 6-Chlor-7,8,9,10-tetrahydro-[1,2,4]triazolo[3,4-a]phthalazin-3-ylamin-Hydrobromid (W2.018, 1,88 g), gelöst in 3-Pentanol (20 ml) und DMF (100 ml), innerhalb 1 h langsam zugetropft. Nach 1 h Rühren wurde das Gemisch aufkonzentriert, der Rückstand mit Wasser versetzt und viermal mit Dichlormethan extrahiert. Die vereinigten organischen Phasen wurden über Magnesiumsulfat getrocknet, filtriert und eingeengt. Der Rückstand über eine 40 g Kieselgelkartusche (Dichlormethan-Methanol-Gradient 0-10 % in 50 min) aufgereinigt. Die sauberen Produktfraktionen wurden vereinigt und zur Trockne gebracht. Es wurden 0,8 g der Titelverbindung erhalten.

| | |
|---|---|
| LCMS-rt: 1,15 min | [M+H]⁺: 276,1 (Met-a) |

### W1.120

### 6-Cyclopropylmethoxy-8,9-dihydro-7H-cyclopenta[d][1,2,4]triazolo[4,3-b]pyridazin-3-ylamin

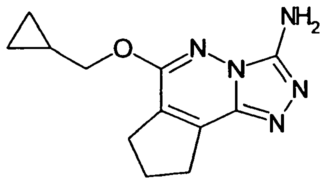

Cyclopropyl-methanol (1,78 ml) wurde in DMF (25 ml) vorgelegt. Anschließend wurde unter Argon mit Natriumhydrid (541 mg) versetzt und 0,5 h bei 45°C gerührt. Anschließend wurde 6-Chlor-8,9-dihydro-7H-cyclopenta[d][1,2,4]triazolo[4,3-b]pyridazin-3-ylamin-Hydrobromid (W2.017, 1,31 g) in DMF (20 ml) gelöst und bei 45°C portionsweise über 90 min 11,25 ml der Alkoholat-Lösung zugegeben. Dann wurde noch eine Stunde bei 45 °C gerührt. Nach Zugabe von etwas Wasser wurde zur Trockne gebracht und über Kieselgel (80 g Kartusche, Dichlormethan/Methanol-Gradient von 0-20% in 60 min) aufgereinigt. Es wurden 528 mg der Titelverbindung erhalten. LCMS-rt: 0,63 min [M+H]⁺: 246,1 (Met-b)

### W1.125

### 6,7-Diethoxy-[1,2,4]triazolo[4,3-b]pyridazin-3-ylamin

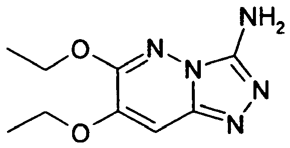

(5,6-Diethoxy-pyridazin-3-yl)-hydrazin (W3.120; 50 mg) wurde in einer Mischung aus Ethanol (3,5 ml) und Wasser (0,75 ml) bei RT unter Rühren vorgelegt. Danach tropfte man Bromcyan (55 mg, gelöst in 0,75 ml Ethanol und 0,15 ml Wasser) vorsichtig zu.

Nach 7 h Rühren ließ man über Nacht stehen. Danach wurden weitere 2 Äquivalente Bromcyan, gelöst in 0,75 ml Ethanol und 0,15 ml Wasser, zugegeben und noch weitere 2 h bei RT und dann 8 h bei 55°C gerührt. Nach Erkalten wurde das Lösungsmittel abgezogen und der Rückstand mit Wasser versetzt. Nachdem mit gesättigter Kaliumcarbonat-Lösung alkalisch gestellt worden war, wurde viermal mit Dichlormethan extrahiert. Die vereinigten organischen Phasen wurden über Natriumsulfat getrocknet und nach Abfiltrieren des Trockenmittels im Vakuum zur Trockne gebracht. Der Rückstand wurde mittels präparativer HPLC (Met-A) gereinigt. Die sauberen Produktfraktionen wurden vereinigt, im Vakuum vom Acetonitril befreit, mit gesättigter Kaliumcarbonat-Lösung alkalisch gestellt und viermal mit Dichlormethan extrahiert. Die vereinigten organischen Phasen wurden über Natriumsulfat getrocknet und nach Abfiltrieren des Trockenmittels im Vakuum zur Trockne gebracht. Es wurden 36 mg der Titelverbindung erhalten.

| | |
|---|---|
| LCMS-rt: 0,54 min | [M+H]⁺: 224,2 (Met-b) |

### W1.126

### 6,8-Diethoxy-[1,2,4]triazolo[4,3-b]pyridazin-3-ylamin

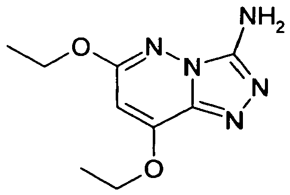

6-Chlor-8-methansulfonyl-[1,2,4]triazolo[4,3-b]pyridazin-3-ylamin (W2.126; 60 mg, 0,24 mmol) wurde in Ethanol (5 ml) gelöst, Natriumethylat (500 µl, 21 % in Ethanol) wurde zugegeben und die Mischung 5 h auf 50°C erhitzt. Die Mischung wurde konzentriert und der Rückstand durch Flash-Chromatographie (Dichlormethan:Methanol) gereinigt. Ausbeute: 54 mg

| | |
|---|---|
| LCMS-rt: 0,52 min | [M+H]⁺: 224,1 (Met-b) |

### W1.127

### 6,8-Dimethoxy-[1,2,4]triazolo[4,3-b]pyridazin-3-ylamin

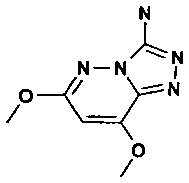

6-Chlor-8-methansulfonyl-[1,2,4]triazolo[4,3-b]pyridazin-3-ylamin (W2.126; 100 mg, 0.40 mmol) wurde in Methanol (10 ml) gelöst und Natriummethanolat-Lösung (0,75 ml, 4,04 mmol) zugegeben. Die Mischung wurde 3 h auf Rückfluß erhitzt, dann konzentriert und der Rückstand in Wasser gelöst und durch Zugabe von wässriger Salzsäure ein pH von 6 eingestellt. Es wurde mit EE extrahiert und die vereinigten organischen Phasen getrocknet und konzentriert. Der verbliebene Rückstand wurde durch Flash-Chromatographie (Dichlormethan:MeOH) gereinigt. Ausbeute: 30 mg

| | |
|---|---|
| LCMS-rt: 0,14 min | [M+H]⁺: 196,1 (Met-b) |

### W1.128

### 6-Ethoxy-8-isopropoxy-[1,2,4]triazolo[4,3-b]pyridazin-3-ylamin

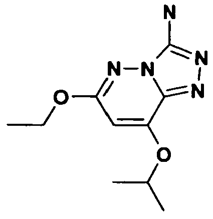

6,8-Diethoxy-[1,2,4]triazolo[4,3-b]pyridazin-3-ylamin (W1.126; 80 mg, 0,358 mmol) wurde in Isopropanol (10 ml) gelöst und Natriumisopropylat (297 mg) wurde zugegeben. Die Mischung wurde 1 h auf 85°C erhitzt, konzentriert und der Rückstand in Wasser gelöst und durch Zugabe von wässriger Salzsäure ein pH von 6 eingestellt. Es wurde mit Essigester extrahiert und die vereinigten organischen Phasen getrocknet und konzentriert. Der verbliebene Rückstand wurde durch Flash-Chromatographie (Dichlormethan:MeOH) gereinigt. Ausbeute: 58 mg.

| | |
|---|---|
| LCMS-rt: 0,58 min | [M+H]⁺: 238,2 (Met-b) |

### W1.130

### 6-Methansulfonyl-7,8-dimethyl-[1,2,4]triazolo[4,3-b]pyridazin-3-ylamin

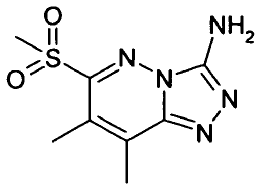

6-Chlor-7,8-dimethyl-[1,2,4]triazolo[4,3-b]pyridazin-3-ylamin-Hydrobromid (W2.006; 1,0 g) und Natriumsulfinat (916 mg) in DMF (6 ml) gelöst und bei 150°C 45 min. in der Mikrowelle gerührt. Nach Abziehen des Lösungsmittels wurde der Rückstand über Kieselgel (40 g Kartusche, Dichlormethan/Methanol-Gradient von 0-20% in 60 min) aufgereinigt. Es wurden 1,02 g der Titelverbindung erhalten.

| | |
|---|---|
| LCMS-rt: 0,33 min | [M+H]⁺: 242,1 (Met-b) |

Analog wurde dargestellt:

| Nummer | | LCMS-rt | [M+H]⁺ | Bemerkung: |
|---|---|---|---|---|
| W1.131 | 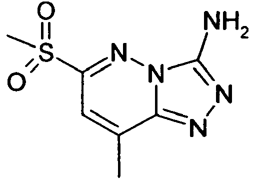 | 0,20 min | 175,1(Me t-b) | W2.002; 5,1 g; Sulfinat (4,9 g); 7 h bei 100°C; Produkt: 6,3 g |

### W1.135

### 3-Amino-7,8-dimethyl-[1,2,4]triazolo[4,3-b]pyridazine-6-carbonitril

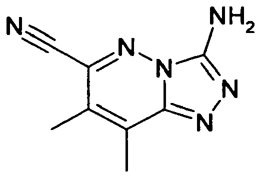

6-Methansulfonyl-7,8-dimethyl-[1,2,4]triazolo[4,3-b]pyridazin-3-ylamin (W1.130; 1,0 g) wurde in DMF (30 ml) gelöst und mit Kaliumcyanid (405 mg) versetzt. Nach 1 h Rühren bei 100°C gerührt wurde zur Trockne gebracht. Der Rückstand wurde in EE verrührt und über eine kurze Kieselgelsäule mit EE chromatographiert. Fraktionen einrotiert. Es wurden 560 mg der Titelverbindung erhalten.

| | |
|---|---|
| LCMS-rt: 0,27 min | [M+H]⁺: 189,1 (Met-b) |

Analog wurde dargestellt:

| Nummer | | LCMS-rt | [M+H]⁺ | Bemerkung: |
|---|---|---|---|---|
| W1.136 | 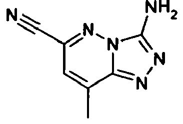 | 0,20 min | 175,1 (Met-b) | W1.131: 6,3 g; KCN (2,7 g); 7 h bei 100°C; Produkt: 870 mg |

### W1.140

### 3-Amino-7,8-dimethyl-[1,2,4]triazolo[4,3-b]pyridazin-6-carbonsäure-Hydrochlorid

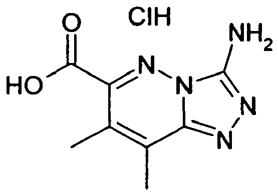

3-Amino-7,8-dimethyl-[1,2,4]triazolo[4,3-b]pyridazin-6-carbonitril (W1.135; 600 mg) wurde mit konzentrierte Salzsäure (20 ml) versetzt und 5 h am Rückfluss gehalten. Anschließend wurde die Salzsäure abgezogen, der Rückstand mit Wasser aufgenommen und gefriergetrocknet. Es wurden 790 mg der Titelverbindung erhalten, die für die nachfolgende Umsetzung von ausreichender Reinheit war.

| | |
|---|---|
| LCMS-rt: 0,19 min | [M+H]⁺: 189,1 (Met-c) |

Analog wurde dargestellt:

| Nummer | | LCMS-rt | [M+H]⁺: | Bemerkung: |
|---|---|---|---|---|
| W1.41 | 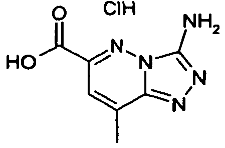 | 0,11 min | 194,1 (Met-b) | W1.136: 860 mg; HCl (30 ml); 2 h RF; Produkt: 1,7 g |

### W1.145

### 3-Amino-7,8-dimethyl-[1,2,4]triazolo[4,3-b]pyridazine-6-carbonsäuremethylester-Hydrochlorid

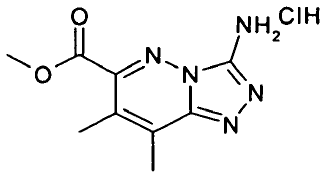

3-Amino-7,8-dimethyl-[1,2,4]triazolo[4,3-b]pyridazin-6-carbonsäure-Hydrochlorid (W1.140; 780 mg) wurde in Methanol (40 ml) gelöst und Thionylchlorid (1,9 ml) langsam zugetropft und dann bei 65°C gerührt. Nach 2,5 h wurde zur Trockne gebracht und der Rückstand über Kieselgel (24 g Kartusche, Dichlormethan/ Methanol-Gradient von 0-20% in 60 min) aufgereinigt. Es wurden 602 mg der Titelverbindung erhalten. LCMS-rt: 0,37 min [M+H]⁺: 222,1 (Met-b)

Analog wurde dargestellt:

| Nummer | | LCMS-rt | [M+H]⁺ | Bemerkung: |
|---|---|---|---|---|
| W1.146 | 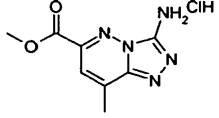 | 0,20 min | 208,1 (Met-b) | W1.141: 600 mg; Thionylchlorid (1,53 ml); 1,5 h 65°C; Produkt: 429 mg |

### W1.150

### 3-Amino-8-methyl-[1,2,4]triazolo[4,3-b]pyridazin-6-carbonsäuremethylamid

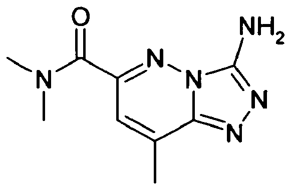

3-Amino-8-methyl-[1,2,4]triazolo[4,3-b]pyridazin-6-carbonsäuremethylester (W1.146; 210 mg) wurde in Methanol (4 ml) gelöst, auf 0°C gekühlt und langsam tropfenweise mit Dimethylamin (2,02 ml; 2 M in THF) versetzt und bei 0°C gerührt. Nach 3 h wurden weitere 5 Äquivalente Dimethylamin-Lösung zugesetzt und 4 h bei 40°C gerührt. Nach Stehen über Nacht wurden nochmals 5 Äquivalente Dimethylamin-Lösung zugesetzt und weitere 8 h bei 40°C gerührt. Nach erneutem Stehen über Nacht wurden weitere 5 Äquivalente Dimethylamin-Lösung zugesetzt und dann 30 min bei 100°C in der Mikrowelle gerührt. Anschließend wurde der Ansatz eingeengt und über Kieselgel (24 g Kartusche, Dichlormethan/Methanol-Gradient von 0-20% in 60 min) aufgereinigt. Es wurden 92 mg der Titelverbindung erhalten.

| | |
|---|---|
| LCMS-rt: 0,13 min | [M+H]⁺: 221,1 (Met-b) |

### W1.152

### 3-Amino-8-methyl-[1,2,4]triazolo[4,3-b]pyridazin-6-carbonsäure-ethylamid

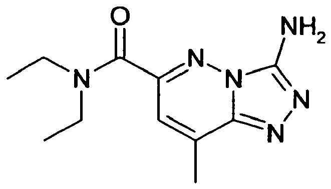

3-Amino-8-methyl-[1,2,4]triazolo[4,3-b]pyridazin-6-carbonsäure-Hydrochlorid (W1.141; 300 mg) wurde in DMF (6 ml) gelöst und mit O-[(Ethoxycarbonyl)-cyanomethylenamino]-N,N,N',N'-tetramethyluronium-tetrafluorborat (TOTU; 429 mg) und Diethylamin (573 mg) versetzt. Nach 2 h wurde das DMF abgezogen und der Rückstand über Kieselgel (24 g Kartusche, Dichlormethan/ Methanol-Gradient von 0-20% in 60 min) aufgereinigt. Das Produkt wurde mit Wasser aufgenommen und gefriergetrocknet. Um restliches Diethylamin zu entfernen wurde über eine kurze Säule (Dichlormethan/ Methanol 95:5) chromatographiert. Es wurden 112 mg der Titelverbindung erhalten, die noch mit Diethylamin verunreinigt war.

| | |
|---|---|
| LCMS-rt: 0,40 min | [M+H]⁺: 249,1 (Met-b) |

### W1.154

### 3-Amino-7,8-dimethyl-[1,2,4]triazolo[4,3-b]pyridazin-6-carbonsäure-ethylamid

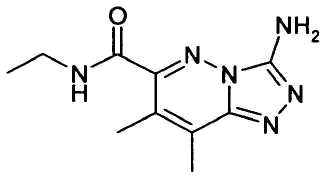

3-Amino-7,8-dimethyl-[1,2,4]triazolo[4,3-b]pyridazine-6-carbonsäuremethylester-Hydrochlorid (W1.145; 200 mg) wurde in Methanol gelöst, auf 0°C gekühlt, langsam tropfenweise mit Ethylamin (1,81 ml; 2 M in THF) versetzt und 6 h bei 0°C gerührt. Dann wurde ein weiteres Äquivalent Ethylamin nachgegeben und bei RT übers Wochenende stehen gelassen. Anschließend wurde der Ansatz eingeengt. und über Kieselgel (24 g Kartusche, Dichlormethan/Methanol-Gradient von 0-20% in 60 min) aufgereinigt. Es wurden 149 mg der Titelverbindung erhalten.

| | |
|---|---|
| LCMS-rt: 0,21 min | [M+H]⁺: 235,1 (Met-b) |

Analog wurde dargestellt:

| Nummer | | LCMS-rt | [M+H]⁺ | Bemerkung: |
|---|---|---|---|---|
| W1.155 | 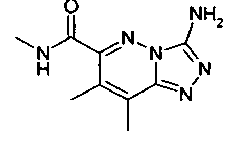 | 0,58 min | 221,1 (Met-c) | W1.145: 200 mg; Methylamin (2 M in THF); Produkt: 113 mg |
| W1.156 | 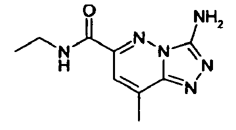 | 0,28 min | 221,1(Met-b) | W1.146: 210 mg; Ethylamin (4,7 eq.; 2 M in THF); Produkt: 164 mg |
| W1.157 | 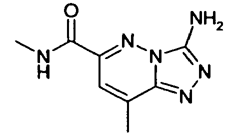 | 0,16 min | 207,1 (Met-b) | W1.146: 225 mg; Methylamin (4,7 eq.; 2 M in THF); Produkt: 113 mg |

### W1.165

### (6-Ethoxy-7,8-dimethyl-[1,2,4]triazolo[4,3-b]pyridazin-3-yl)-(2,2,2-trifluor-ethyl)-amin

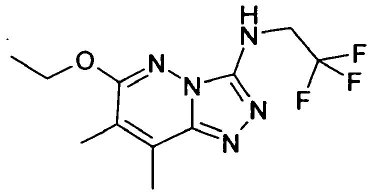

(6-Chlor-7,8-dimethyl-[1,2,4]triazolo[4,3-b]pyridazin-3-yl)-(2,2,2-trifluorethyl)-amin (W2.150; 330 mg) wurde in Ethanol (25 ml) vorgelegt und mit Natriumethylat (90 mg) versetzt. Das Reaktionsgemisch rührte 4 h bei 50 °C, dann wurde weiteres Natriumethylat (10 mg) zugesetzt und 3 h bei 50 °C weiter gerührt. Nach Stehen über Nacht wurde mit Wasser versetzt und zur Trockne gebracht. Der Rückstand wurde in EE aufgenommen und dreimal mit Wasser gewaschen. Die EE-Phase wurde über Natriumsulfat getrocknet, filtriert und eingeengt. Es wurden 340 mg der Titelverbindung erhalten. LCMS-rt: 0,82 min [M+H]⁺: 290,2 (Met-b)

Analog wurden dargestellt:

| Nummer | | LCMS-rt | [M+H]⁺ | Bemerkung: |
|---|---|---|---|---|
| W1.166 | 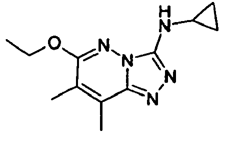 | 0,75 min | 248,2(Met-b) | W2.166; 690 mg; Produkt: 508 mg |
| W1.167 | 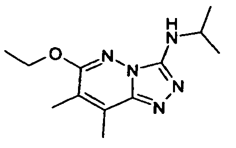 | 0,73 min | 236,2(Met-b) | W2.167; 500 mg; Produkt: 380 mg |
| W1.168 | 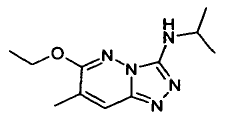 | 0,78 min | 250,2(Met-b) | W2.168; 78 mg; Produkt: 57 mg |
| W1.169 | 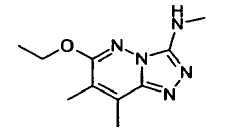 | 0,71 min | 222,2(Met-b) | W2.169; 50 mg; Produkt: 27 mg |
| W1.170 | 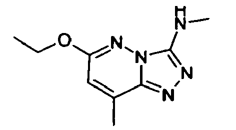 | 0,49 min | 208,2(Met-b) | W2.170; 105 mg; Produkt: 70 mg |

### W1.175

### [6-(1-Ethyl-propoxy)-7,8-dimethyl-[1,2,4]triazolo[4,3-b]pyridazin-3-yl]-methyl-amin

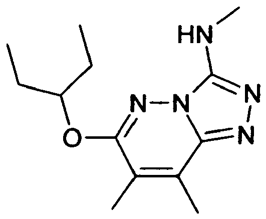

3-Pentanol (1,2 ml) wurde bei RT unter Rühren vorgelegt. Danach wurde unter Eiskühlung Natriumhydrid (77 mg) zugegeben. Nach 3 h Rühren bei 55°C tropfte man (6-Chlor-7,8-dimethyl-[1,2,4]triazolo[4,3-b]pyridazin-3-yl)-methyl-amin (W2.169; 50 mg), gelöst in 3-Pentanol (1 ml) und DMF (2 ml) zu. Nach 2 h Rühren ließ man über Nacht bei RT stehen, versetzte das Reaktionsgemisch mit Wasser und Dichlormethan und extrahierte anschließend noch dreimal mit Dichlormethan. Die vereinigten organischen Phasen wurden über Magnesiumsulfat getrocknet und nach Abfiltrieren des Trockenmittels im Vakuum zur Trockne gebracht. Es wurden 44 mg der Titelverbindung erhalten.

| | |
|---|---|
| LCMS-rt: 0,86 min | [M+H]⁺: 264,2 (Met-b) |

Analog wurde dargestellt:

| Nummer | | LCMS-rt | [M+H]⁺ | Bemerkung: |
|---|---|---|---|---|
| W1.176 | 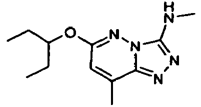 | 0,82 min | 250,2( Met-b) | W2.170:105 mg; Produkt: 60 mg |

Analog W1.130 wurden dargestellt:

| Nummer | | LCMS-rt | [M+H]⁺ | Bemerkung: |
|---|---|---|---|---|
| W1.189 | 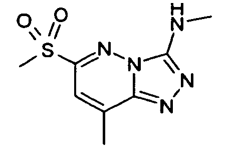 | 0,24 min | 242,1 (Met-b) | W2.170: 7,73 g; Produkt: 6,22 g |
| W1.190 | 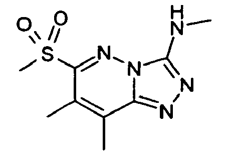 | 0,43 min | 256,0 (Met-b) | W2.169,08 g; Produkt: 4,84 g |
| W1.191 | 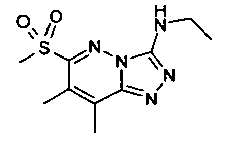 | 0,51 min | 270,1 (Met-b) | W2.191: 7,89 g; Produkt: 5,38 g |
| W1.192 | 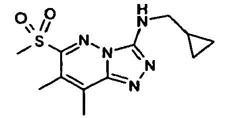 | 0,60 min | 296,1 (Met-b) | W2.192: 9,44 g; Produkt: 5,45 g |

Analog W1.135 wurden dargestellt:

| Nummer | | LCMS-rt | [M+H]⁺ | Bemerkung: |
|---|---|---|---|---|
| W1.199 | 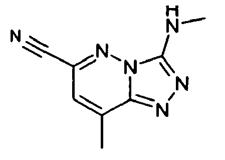 | 0,24 min | 189,1 (Met-b) | W1.189: 5,00 g; Produkt: 1,74 g |
| W1.200 | 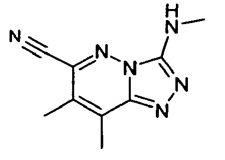 | 0,39 min | 203,1 (Met-b) | W1.190: 4,83 g; Produkt: 3,50 g |
| W1.201 | 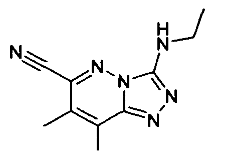 | 0,50 min | 217,1 (Met-b) | W1.191: 5,37 g; Produkt: 3,18 g |
| W1.202 | 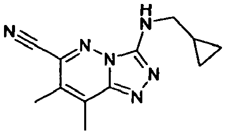 | 0,63 min | 243,1 (Met-b) | W1.192: 5,44 g; Produkt: 3,84 g |

Analog W1.140 wurden dargestellt:

| Nummer | | LCMS-rt | [M+H]⁺ | Bemerkung: |
|---|---|---|---|---|
| W1.209 | 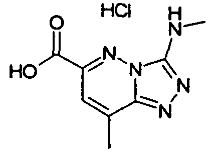 | 0,10 min | 208,1 (Met-b) | W1.199: 1,73 g; Produkt: 2,77 g |
| W1.210 | 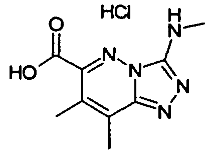 | 0,23 min | 222,1 (Met-c) | W1.200: 3,50 g; Produkt: 5,27 g |
| W1.211 | 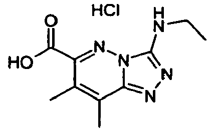 | 0,47 min | 236,1 (Met-c) | W1.201: 3,17 g; Produkt: 4,68 g |
| W1.212 | 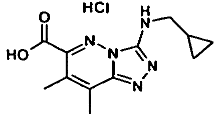 | 0,17 min | 262,1 (Met-b) | W1.202: 3,83 g; Produkt: 5,47 g |

Analog W1.145 wurden dargestellt:

| Nummer | | LCMS-rt | [M+H]⁺ | Bemerkung: |
|---|---|---|---|---|
| W1.219 | 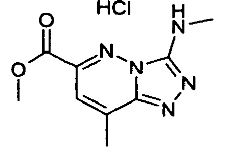 | 0,29 min | 222,1 (Met-b) | W1.210: 2,77 g; Produkt: 2,56 g |
| W1.220 | 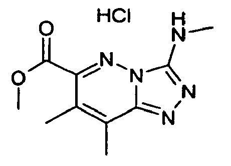 | 0,41 min | 236,1 (Met-b) | W1.210: 4,98 g; Produkt: 4,11 g |
| W1.221 | 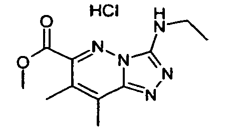 | 0,50 min | 250,1 (Met-b) | W1.211: 4,67 g; Produkt: 4,06 g |

### W1.250

### 7,8-Dimethyl-3-methylamino-[1,2,4]triazolo[4,3-b]pyridazin-6-carbonsäure-ethylamid

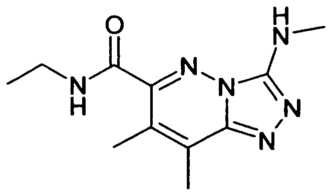

7,8-Dimethyl-3-methylamino-[1,2,4]triazolo[4,3-b]pyridazin-6-carbonsäuremethylester-Hydrochlorid (W1.220; 1,3 g) wurde in Methanol (30 ml) gelöst, auf 0°C gekühlt, langsam tropfenweise mit Ethylamin (11,44 ml; 2 M in THF) versetzt und 6 h bei 0°C gerührt. Dann wurden vier weitere Äquivalente Ethylamin nachgegeben und bei RT übers Wochenende stehen gelassen. Anschließend wurde der Ansatz eingeengt und über Kieselgel (40 g Kartusche, Dichlormethan/Methanol-Gradient von 0-20% in 60 min) aufgereinigt. Es wurden 1,12 g der Titelverbindung erhalten. LCMS-rt: 0,81 min [M+H]⁺: 249,1 (Met-b)

Analog wurden dargestellt:

| Nummer | | LCMS-rt | [M+H]⁺ | Bemerkung: |
|---|---|---|---|---|
| W1.251 | 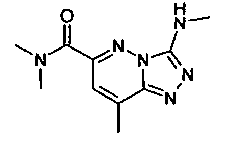 | 0,14 min | 235,1(Met-b) | W1.219: 770 mg; Dimethylamin (5+2 eq.; 2 M in THF); Produkt: 542 mg |
| W1.252 | 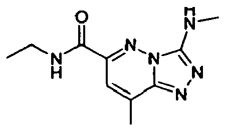 | 0,39 min | 235,1 (Met-b) | W1.219; 850 mg; Ethylamin (4,7 eq.; 2 M in THF); Produkt: 590 mg |
| W1.253 | 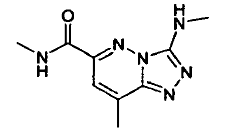 | 0,18 min | 221,1(Met-b) | W1.219.850 mg; Methylamin (4,7+2 eq.; 2 M in THF); Produkt: 530 mg |
| W1.254 | 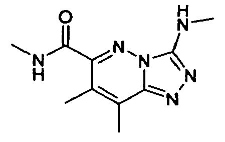 | 0,68 min | 235,1 (Met-b) | W1.220; 800 mg; Methylamin (4,7+2 eq.; 2 M in THF); Produkt: 629 mg |
| W1.255 | 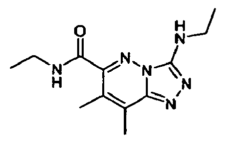 | 0,86 min | 263,1 (Met-b) | W1.221; 800 mg; Ethylamin (4,7+4 eq.; 2 M in THF); Produkt: 732 mg |
| W1.256 | 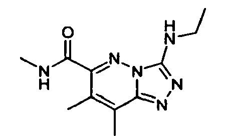 | 0,79 min | 249,1 (Met-b) | W1.221; 800 mg; Methylamin (4,7+2 eq.; 2 M in THF); Produkt: 612 mg |

W1.265 wurde analog W1.150 dargestellt:

| Nummer | | LCMS-rt | [M+H]⁺ | Bemerkung: |
|---|---|---|---|---|
| W1.265 | 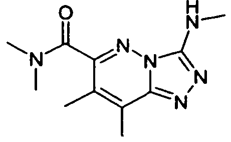 | 0,16 min | 249,1 (Met-b) | W1.220; 800 mg; Dimethylamin (5+5+5 eq., 2 M in THF); Produkt: 330 mg |

Analog W1.152 wurde dargestellt:

| Nummer | | LCMS-rt | [M+H]⁺ | Bemerkung: |
|---|---|---|---|---|
| W1.270 | 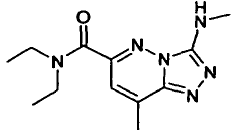 | 0,45 min | 263,1( Met-b) | W1.209; 860 mg; Diethylamin (1,81 ml); TOTU 1,16 g; Produkt: 251 mg (keine Gefriertrocknung und zusätzliche Säule) |

### W2.

### W2.001

### 6-Chlor-[1,2,4]triazolo[4,3-b]pyridazin-3-ylamin-Hydrobromid

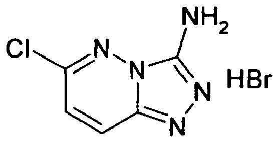

3-Chlorpyridazin-6-yl)-hydrazin (5 g) wurde in einer Mischung aus EtOH (90 ml) und Wasser (36 ml) bei RT unter Rühren gelöst. Danach tropfte man 5 M Bromcyan-Lösung (13 ml in Acetonitril) vorsichtig zu. Nach 4,5 h Rühren ließ man über Nacht stehen und gab am nächsten Tag unter Rühren weitere 5 M Bromcyan-Lösung (3 ml in Acetonitril) zu. Nach weiteren 4 h Rühren wurde der entstandene Niederschlag abgesaugt und getrocknet. Es wurden 6,1 g der Titelverbindung erhalten.

Die Mutterlauge wurde mit MtB-Ether versetzt und der dabei gebildete Niederschlag abgesaugt und getrocknet, so dass weitere 1,5 g der Titelverbindung erhalten wurden.

| | |
|---|---|
| LCMS-rt: 0,24 min | [M+H]⁺: 170,1 (Met-a) |

### W2.001a

### 6-Chlor-[1,2,4]triazolo[4,3-b]pyridazin-3-ylamin

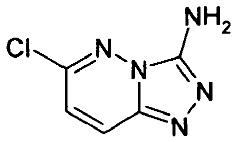

6-Chlor-[1,2,4]triazolo[4,3-b]pyridazin-3-ylamin-Hydrobromid (W2.001; 1,1 g) wurde mit viel Wasser aufgenommen und mit gesättigter Kaliumcarbonat-Lösung alkalisch gestellt. Der dabei ausfallende Niederschlag wurde abgesaugt und getrocknet (388 mg). Mehrmaliges Extrahieren der Mutterlauge mit Dichlormethan, Trockenen der vereinigten organischen Phasen über Natriumsulfat, Filtrieren und Einengen lieferte insgesamt weitere 228 mg Produkt.

| | |
|---|---|
| LCMS-rt: 0,24 min | [M+H]⁺: 170,1 (Met-a) |

### W2.002

### 6-Chlor-8-methyl-[1,2,4]triazolo[4,3-b]pyridazin-3-ylamin-Hydrobromid

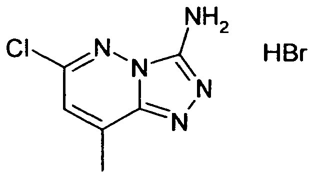

(6-Chlor-4-methyl-pyridazin-3-yl)-hydrazin (W3.002; 4,6 g) wurde in EtOH (330 ml) und Wasser (70 ml) unter Rühren bei RT vorgelegt. Danach tropfte man bei RT Bromcyan in einer Mischung aus EtOH (170 ml) und Wasser (30 ml) langsam zu. Nach 6 h Rühren bei RT wurde über Nacht stehen gelassen. Dann wurde zur Trockne gebracht und der Rückstand über Kieselgel (40 g Kartusche, DCM/Methanol-Gradient von 0-10% in 30 min) aufgereinigt. Es wurden 7,3 g der Titelverbindung erhalten.

| | |
|---|---|
| LCMS-rt: 0,17 min | [M+H]⁺: 184,1 (Met-b) |

### W2.002b

### 6-Chlor-8-methyl-[1,2,4]triazolo[4,3-b]pyridazin-3-ylamin-Trifluoressigsäuresalz

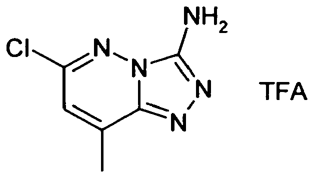

(6-Chlor-4-methyl-pyridazin-3-yl)-hydrazin (W3.002; 432 mg) wurde in einer Mischung aus Ethanol (15 ml) und Wasser (3 ml) unter Rühren bei RT vorgelegt. Danach tropfte man Bromcyan (581 mg, gelöst in 7 ml EtOH und 1,5 ml Wasser) vorsichtig zu. Nach 2 h Rühren ließ man über Nacht stehen. Danach wurde noch weitere 4 h bei RT und dann 2 h bei 50°C gerührt. Nach Erkalten über Nacht wurde das Lösungsmittel abgezogen und der Rückstand mittels präparativer HPLC (Met-A) gereinigt. Die sauberen Produktfraktionen wurden vereinigt, im Vakuum vom Acetonitril befreit und gefriergetrocknet. Es wurden 158 mg der Titelverbindung erhalten. LCMS-rt: 0,44 min
[M+H]⁺: 184,1 (Met-a)

### W2.003

### 6-Chlor-8-ethyl-[1,2,4]triazolo[4,3-b]pyridazin-3-ylamin

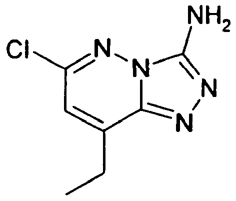

(6-Chlor-4-ethyl-pyridazin-3-yl)-hydrazin-Trifluoracetat (W3.003; 1,35 g) wurde in EtOH (25 ml) und Wasser (4 ml) unter Rühren bei RT vorgelegt. Danach tropfte man Bromcyan (997 mg), gelöst in EtOH/Wasser (5 / 2 ml) vorsichtig zu. Nach 8 h Rühren bei RT und Stehen über Nacht wurde nochmals 4 h bei 55°C gerührt. Anschließend wurde das Lösungsmittel eingeengt und der Rückstand mit Wasser versetzt. Nachdem mit gesättigter Kaliumcarbonat-Lösung alkalisch gestellt worden war, wurde dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 918 mg der Titelverbindung erhalten. LCMS-rt: 0,35 min [M+H]⁺: 198,1 (Met-b)

### W2.004

### 6-Chlor-8-trifluormethyl-[1,2,4]triazolo[4,3-b]pyridazin-3-ylamin

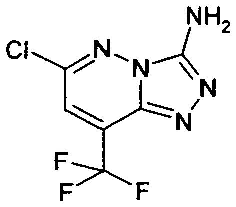

(6-Chlor-4-trifluormethyl-pyridazin-3-yl)-hydrazin-Hydrochlorid (W3.004; 85 mg) wurde analog W2.003 umgesetzt und aufgearbeitet. Allerdings rührte man 8 h bei RT und ließ dann zur Vervollständigung der Reaktion übers Wochenende stehen.

| | |
|---|---|
| LCMS-rt: 0,41 min | [M+H]⁺: 238,1 (Met-b) |

### W2.005 und W2.005a

### 6-Chlor-7-methyl-[1,2,4]triazolo[4,3-b]pyridazin-3-ylamin und 6-Chlor-7-methyl-[1,2,4]triazolo[4,3-b]pyridazin-3-ylamin-Hydrobromid

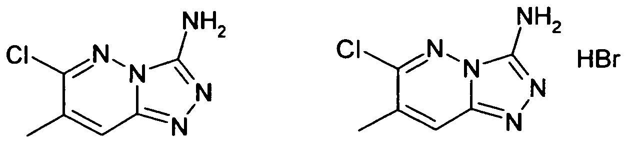

(6-Chlor-5-methyl-pyridazin-3-yl)-hydrazin (W3.005; 4,68g) wurde analog W2.002 umgesetzt und aufgearbeitet. Die Reinigung erfolgte über Kieselgel (80 g Kartusche, DCM/ Methanol-Gradient 0-10% in 60 min). Es wurden 1,88 g der freien Base isoliert. Erneutes Spülen des Kieselgels lieferte 2,67 g des Hydrobromids.

| | |
|---|---|
| LCMS-rt: 0,46 min | [M+H]⁺: 184,1 (Met-a) |

### W2.006

### 6-Chlor-7,8-dimethyl-[1,2,4]triazolo[4,3-b]pyridazin-3-ylamin-Hydrobromid

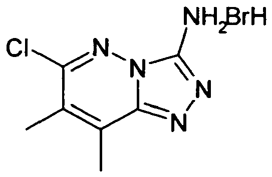

(6-Chlor-4,5-dimethyl-pyridazin-3-yl)-hydrazin (W3.006; 12 g) wurde in EtOH (240 ml) und Wasser (50 ml) unter Rühren bei RT vorgelegt. Danach tropfte man bei RT Bromcyan (14,7 g) in einer Mischung aus EtOH (120 ml) und Wasser (25 ml) langsam zu. Nach 6 h Rühren bei RT wurde über Nacht stehen gelassen. Dann wurde zur Trockne gebracht und der Rückstand über Kieselgel (80 g Kartusche, DCM/Methanol-Gradient von 0-20% in 60 min) aufgereinigt. Es wurden 19,4 g der Titelverbindung erhalten. LCMS-Rt: 0,28 min [M+H]⁺: 198,1 (Met-b)

### W2.006a

### 6-Chlor-7,8-dimethyl-[1,2,4]triazolo[4,3-b]pyridazin-3-ylamin

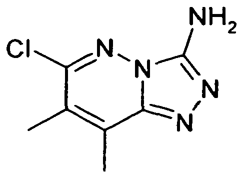

(6-Chlor-4,5-dimethyl-pyridazin-3-yl)-hydrazin (W3.006; 519 mg) wurde analog W2.007 umgesetzt und aufgearbeitet. Es wurden 570 mg der freien Base isoliert.

| | |
|---|---|
| LCMS-rt: 1,35 min | [M+H]⁺: 198,0 (Met-e) |

### W2.007

### 6-Chlor-7-ethyl-8-methyl-[1,2,4]triazolo[4,3-b]pyridazin-3-ylamin

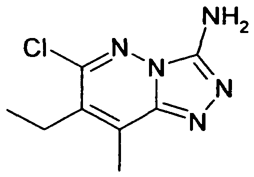

(6-Chlor-5-ethyl-4-methyl-pyridazin-3-yl)-hydrazin (W3.007; 340 mg) wurde Ethanol/Wasser (10/2 ml) unter Rühren bei RT gelöst. Danach tropfte man vorsichtig Bromcyan (386 mg, gelöst in 5 ml Ethanol und 1 ml Wasser) zu. Nach 5 h Rühren bei RT ließ man über Nacht stehen und zog dann das Lösungsmittel ab und versetzte den Rückstand mit Wasser. Nachdem mit gesättigter Kaliumcarbonat-Lösung alkalisch gestellt worden war, wurde dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 385 mg der Titelverbindung erhalten. LCMS-rt: 0,62 min [M+H]⁺: 212,1 (Met-b)

### W2.008

### 6-Chlor-8-ethyl-7-methyl-[1,2,4]triazolo[4,3-b]pyridazin-3-ylamin

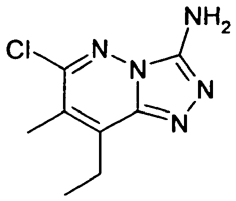

(6-Chlor-4-ethyl-5-methyl-pyridazin-3-yl)-hydrazin-Trifluoressigsäuresalz (W3.008; 340 mg) wurde in einer Mischung aus EtOH (12 ml) und Wasser (2 ml) bei RT unter Rühren vorgelegt. Danach tropfte man vorsichtig Bromcyan (240 mg), gelöst in 3 ml EtOH und 1 ml Wasser, zu und rührte 8 h. Nach Stehen über Nacht wurden 0,5 eq. Bromcyan-Lösung zugegeben und erneut 4,5 h bei RT und anschließend 2 h bei 55°C gerührt. Nach Stehen über Nacht wurde das Lösungsmittel abgezogen und der Rückstand mit Wasser versetzt. Nachdem mit gesättigter Kaliumcarbonat-Lösung alkalisch gestellt worden war, wurde dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 270 mg der Titelverbindung erhalten. LCMS-rt: 0,54 min [M+H]⁺: 212,1 (Met-b)

### W2.009

### 6-Chlor-8-isopropyl-[1,2,4]triazolo[4,3-b]pyridazin-3-ylamin

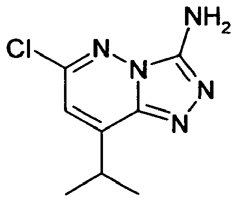

(6-Chlor-4-isopropyl-pyridazin-3-yl)-hydrazin-Trifluoressigsäuresalz (W3.009; 258 mg;) wurde in Ethanol/Wasser (6/1 ml) bei RT unter Rühren vorgelegt. Danach tropfte man Bromcyan (182 mg, gelöst in einer Mischung aus 1,5 ml EtOH und 0,5 ml Wasser) vorsichtig zu. Nach 5 Stunden Rühren ließ man über Nacht stehen, gab noch 1 eq. der Bromcyan-Lösung zu und Rührte über Tag. Nach Stehen über Nacht wurde das Lösungsmittel abgezogen und der Rückstand mit Wasser versetzt. Nachdem mit gesättigter Kaliumcarbonat-Lösung alkalisch gestellt worden war, wurde dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 180 mg der Titelverbindung in ausreichender Reinheit erhalten.

| | |
|---|---|
| LCMS-rt: 0,65 min | [M+H]⁺: 212,1 (Met-b) |

¹H-NMR (500 MHz, DMSO-d6) [ppm]: 6,98 (1 H), 6,63 (2 H), 3,37 (1 H), 1,35 (6 H)

### W2.010

### 6-Chlor-8-cyclopropyl-[1,2,4]triazolo[4,3-b]pyridazin-3-ylamin

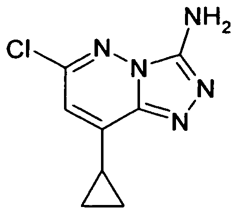

(6-Chlor-4-cyclopropyl-pyridazin-3-yl)-hydrazin-Trifluoessigsäuresalz (W3.010; 400 mg) wurde analog W2.009 umgesetzt und aufgearbeitet. Allerdings wurde ein weiteres Äquivalent Bromcyan zugegeben und zusätzlich 6 h bei 55°C gerührt. Es wurden 252 mg der Titelverbindung erhalten.

| | |
|---|---|
| LCMS-rt: 0,51 min | [M+H]⁺: 210,1 (Met-b) |

### W2.011

### 6-Chlor-7-ethyl-[1,2,4]triazolo[4,3-b]pyridazin-3-ylamin

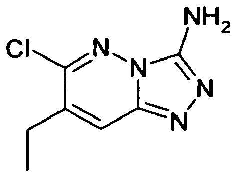

(6-Chlor-5-ethyl-pyridazin-3-yl)-hydrazin-Trifluoessigsäuresalz (W3.011; 169 mg) wurde analog W2.009 umgesetzt und aufgearbeitet. Es wurden 130 mg der Titelverbindung in ausreichender Reinheit erhalten.

| | |
|---|---|
| LCMS-rt: 0,33 min | [M+H]⁺: 198,1 (Met-b) |

### W2.012

### 6-Chlor-7-cyclopropyl-[1,2,4]triazolo[4,3-b]pyridazin-3-ylamin

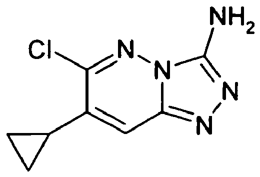

(6-Chlor-5-cyclopropyl-pyridazin-3-yl)-hydrazin-Trifluoessigsäuresalz (W3.012; 400 mg) wurde analog W2.008 umgesetzt und aufgearbeitet, wobei allerdings statt 0,5 ein weiteres Äquivalent Bromcyan-Lösung zugegeben wurde. Es wurden 260 mg der Titelverbindung in ausreichender Reinheit erhalten.

| | |
|---|---|
| LCMS-rt: 0,45 min | [M+H]⁺: 210,1 (Met-b) |

### W2.013

### 6-Chlor-7-isopropyl-[1,2,4]triazolo[4,3-b]pyridazin-3-ylamin

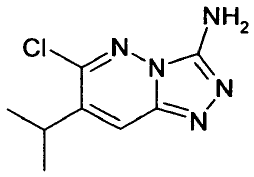

(6-Chlor-5-isopropyl-pyridazin-3-yl)-hydrazin-Trifluoessigsäuresalz (W3.013; 400 mg) wurde in Ethanol/Wasser (6/1 ml) bei RT unter Rühren vorgelegt. Danach tropfte man Bromcyan (353 mg, gelöst in einer Mischung aus 1 ml EtOH und 0,5 ml Wasser) vorsichtig zu. Nach 5 Stunden Rühren ließ man über Nacht stehen und rührte einen weiteren Tag. Nach Stehen über Nacht wurde das Lösungsmittel abgezogen und der Rückstand mit Wasser versetzt. Nachdem mit gesättigter Kaliumcarbonat-Lösung alkalisch gestellt worden war, wurde dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 400 mg der Titelverbindung in ausreichender Reinheit erhalten.

| | |
|---|---|
| LCMS-rt: 0,65 min | [M+H]⁺: 212,1 (Met-b) |

¹H-NMR (500 MHz, DMSO-d6) [ppm]: 7,97 (1 H), 6,61 (2 H), 3,11 (1 H), 1,25 (6 H)

### W2.014

### 6-Chlor-7,8-diethyl-[1,2,4]triazolo[4,3-b]pyridazin-3-ylamin

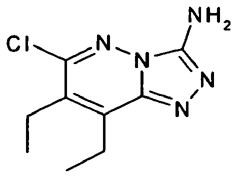

(6-Chlor-4,5-diethyl-pyridazin-3-yl)-hydrazin (W3.014; 2,0 g) wurde analog W2.007 umgesetzt und aufgearbeitet. Es wurden 2,20 g der Titelverbindung in ausreichender Reinheit erhalten. LCMS-rt: 0,72 min [M+H]⁺: 226,1 (Met-b)

### W2.015

### 6-Chlor-7,8-diisopropyl-[1,2,4]triazolo[4,3-b]pyridazin-3-ylamin

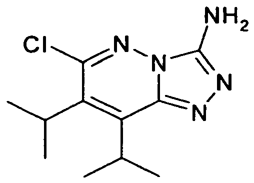

(6-Chlor-4,5-diisopropyl-pyridazin-3-yl)-hydrazin (W3.015; 274 mg) wurde analog W2.007 umgesetzt und aufgearbeitet. Es wurden 290 mg der Titelverbindung in ausreichender Reinheit erhalten.

| | |
|---|---|
| LCMS-rt: 0,87 min | [M+H]⁺: 254,2 (Met-b) |

### W2.016

### 6-Chlor-7,8-dicyclopropyl-[1,2,4]triazolo[4,3-b]pyridazin-3-ylamin

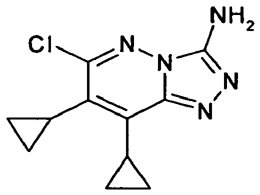

(6-Chlor-4,5-dicyclopropyl-pyridazin-3-yl)-hydrazin (W3.016; 346 mg) wurde analog W2.007 umgesetzt und aufgearbeitet. Es wurden 385 mg der Titelverbindung in ausreichender Reinheit erhalten.

| | |
|---|---|
| LCMS-rt: 0,81 min | [M+H]⁺: 250,1 (Met-b) |

### W2.017

### 6-Chlor-8,9-dihydro-7H-cyclopenta[d][1,2,4]triazolo[4,3-b]pyridazin-3-ylamin-Hydrobromid

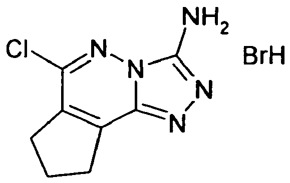

(4-Chlor-6,7-dihydro-5H-cyclopenta[d]pyridazin-1-yl)-hydrazin (W3.017; 3,85 g) wurde analog W2.006 umgesetzt, aufgearbeitet und gereinigt. Es wurden 5,44 g der Titelverbindung erhalten. LCMS-rt: 0,74 min [M+H]⁺: 210,1 (Met-a)

### W2.018

### 6-Chlor-7,8,9,10-tetrahydro-[1,2,4]triazolo[1,3,4]-alphthalazin-3-ylamin-Hydrobromid

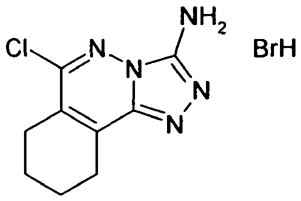

(4-Chlor-5,6,7,8-tetrahydro-phthalazin-1-yl)-hydrazin (W3.018; 4,00 g) wurde analog W2.006 umgesetzt, aufgearbeitet und gereinigt. Es wurden 5,16 g der Titelverbindung erhalten. LCMS-rt: 0,82 min [M+H]⁺: 224,1 (Met-a)

### W2.019

### 3-Amino-6-chlor-[1,2,4]triazolo[4,3-b]pyridazin-7-carbonsäurediethylamid

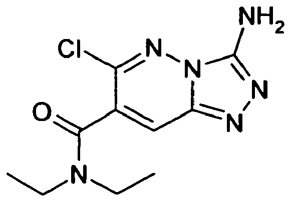

3-Chlor-6-hydrazin-pyridazin-4-carbonsäurediethylamid-Trifluoressigsäuresalz (W3.019; 390 mg) wurde analog W2.007 umgesetzt und aufgearbeitet. Es wurden 285 mg der Titelverbindung in ausreichender Reinheit erhalten.

| | |
|---|---|
| LCMS-rt: 0,94 min | [M+H]⁺: 269,1 (Met-a) |

### W2.020

### 3-Amino-6-chlor-[1,2,4]triazolo[4,3-b]pyridazin-8-carbonsäurediethylamid

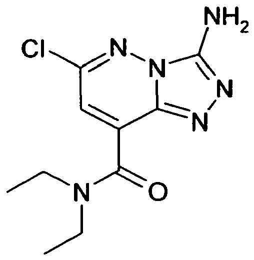

6-Chlor-3-hydrazin-pyridazin-4-carbonsäurediethylamid-Trifluoressigsäuresalz (W3.020; 320 mg) wurde analog W2.007 umgesetzt und aufgearbeitet. Es wurden 222 mg der Titelverbindung in ausreichender Reinheit erhalten.

| | |
|---|---|
| LCMS-rt: 0,93 min | [M+H]⁺: 269,1 (Met-a) |

### W2.021

### 6-Chlor-N*7*,N*7*-diethyl-[1,2,4]triazolo[4,3-b]pyridazin-3,7-diamin

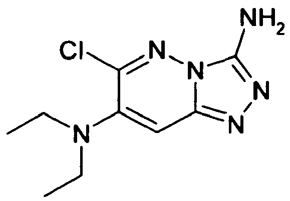

(3-Chlor-6-hydrazin-pyridazin-4-yl)-diethyl-amin (W3.021; 215 mg) wurde analog W2.007 umgesetzt und das Lösungsmittelgemisch abgezogen. Der Rückstand wurde mittels präparativer HPLC (Met-A) gereinigt. Die jeweils sauberen Produktfraktionen wurden vereinigt, im Vakuum vom Acetonitril befreit, mit Natriumhydrogencarbonat auf pH 9 gestellt und fünfmal mit DCM extrahiert. Die vereinigten organischen Phasen wurden über Magnesiumsulfat getrocknet und nach Abfiltrieren des Trockenmittels im Vakuum zur Trockne gebracht. Es wurden 94 mg der Titelverbindung in ausreichender Reinheit erhalten.

| | |
|---|---|
| LCMS-rt: 1,04 min | [M+H]⁺: 241,1 (Met-a) |

### W2.126

### 6-Chlor-8-methansulfonyl-[1,2,4]triazolo[4,3-b]pyridazin-3-ylamin

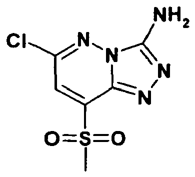

(6-Chlor-4-methanesulfonyl-pyridazin-3-yl)-hydrazin (W3.126; 200 mg, 0,9 mmol) wurde in Methanol (5 ml) gelöst, Bromcyan (360 µl, 3 M in DCM) wurde hinzugegeben und die Mischung bei RT 4 h gerührt. Die Mischung wurde konzentriert und der Rückstand durch Flash-Chromatographie (Dichlormethan:Methanol) gereinigt. Ausbeute: 111 mg.

| | |
|---|---|
| LCMS-rt: 0,51 min | [M+H]⁺: 227,0 (Met-c) |

### W2.150

### (6-Chlor-7,8-dimethyl-[1,2,4]triazolo[4,3-b]pyridazin-3-yl)-(2,2,2-trifluor-ethyl)-amin

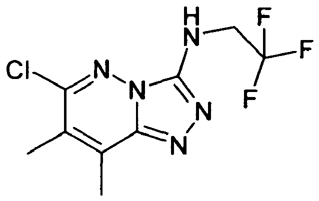

N*-(6-Chlor-4,5-dimethyl-pyridazin-3-yl)-amino-N-(2,2,2-trifluorethyl)-harnstoff (W3.150; 400 mg) wurde in Phosphoroxychlorid (10 ml) gelöst und unter Rühren auf 80 °C erwärmt. Nach 7 h Rühren bei 80°C wurde über Nacht stehen gelassen und dann das Phosphoroxychlorid abgezogen. Der Rückstand wurde in Wasser / DCM gelöst, mit Natriumhydrogencarbonat pH 9 gestellt und die Phasen getrennt. Die wässrige Phase wurde dreimal mit DCM extrahiert, die vereinigten Extrakte mit gesättigter Kochsalz-Lösung gewaschen, über Natriumcarbonat getrocknet, filtriert und eingeengt. Es wurden 330 mg der Titelverbindung erhalten.

| | |
|---|---|
| LCMS-rt: 0,83 min | [M+H]⁺: 280,1 (Met-b) |

Analog W2.169 wurden dargestellt:

| Nummer | | LCMS-rt | [M+H]⁺ | Bemerkung: |
|---|---|---|---|---|
| W2.166 | 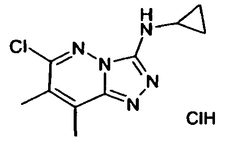 | 0,64 min | 238,1 (Met-b) | W3.166: 830 mg; Reinigung über HPLC (Met-D); Produkt: 690 mg |
| W2.167 | 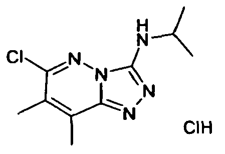 | 0,71 min | 240,2 (Met-b) | W3.168: 540 mg; Reinigung über HPLC (Met-D); Produkt: 507 mg |
| W2.168 | 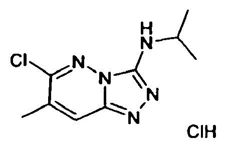 | 0,68 min | 226,1 (Met-b) | W3.168: 140 mg; Reinigung über HPLC (Met-D); Produkt: 82 mg |

### W2.169

### (6-Chlor-7,8-dimethyl-[1,2,4]triazolo[4,3-b]pyridazin-3-yl)-methyl-amin-Hydrobromid

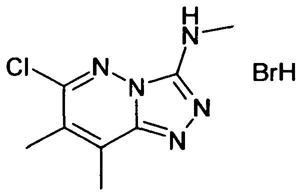

N'-(6-Chlor-4,5-dimethyl-pyridazin-3yl)-amino-N-methyl-thioharnstoff (W3.169; 11,25 g) wurde in Ethanol (400 ml) gelöst, mit Bromessigsäureethylester (5,57 ml) versetzt und unter Feuchtigkeitsauschluß unter Rückfluss gehalten. Nach 2 h wurde das Lösungsmittel abgezogen und der Rückstand über Kieselgel (120 g Kartusche, DCM/Methanol-Gradient von 0-10% in 60 min) aufgereinigt. Es wurden 10,9 g der Titelverbindung erhalten. LCMS-Rt: 0,35 min [M+H]⁺: 212,1 (Met-b)

Analog W2.169 wurden dargestellt:

| Nummer | | LCMS-rt | [M+H]⁺ | Bemerkung: |
|---|---|---|---|---|
| W2.170 | 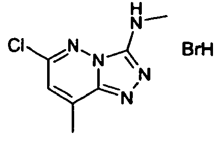 | 0,24 min | 198,1 (Met-b) | W3.170: 7,74 g; Produkt: 7,93 g |
| W2.191 | 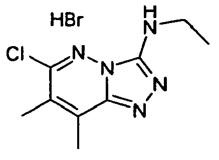 | 0,50 min | 226,1 (Met-b) | W3.191: 7,61 g; Produkt: 7,89 g |
| W2.192 | 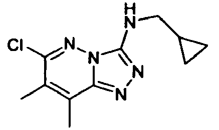 | 0,60 min | 252,1 (Met-b) | W3.192: 7,80 g; Produkt: 9,44 g |

### W3

### W3.002

### (6-Chlor-4-methyl-pyridazin-3-yl)-hydrazin

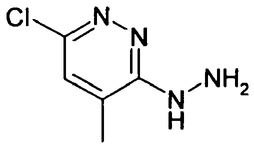

Synthetisiert analog US 4,578,464

| | |
|---|---|
| LCMS-rt: 0,21 min | [M+H]⁺: 159,1 (Met-a) |

### W3.003 und W3.011

### (6-Chlor-4-ethyl-pyridazin-3-yl)-hydrazin-Trifluoracetat und (6-Chlor-5-ethyl-pyridazin-3-yl)-hydrazin-Trifluoracetat

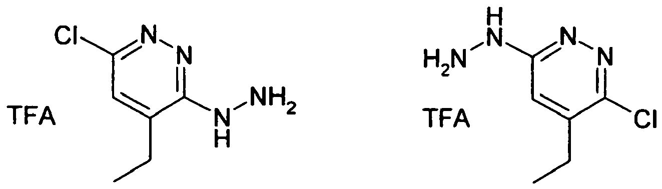

3,6-Dichlor-4-ethyl-pyridazin (W4.003, 2 x 2,4 g) wurde auf 2 Mikrowellengefäße verteilt und jeweils mit einer Mischung aus Hydrazin-monohydrat (6 ml) und Dioxan (7 ml) versetzt. Das Reaktionsgemisch wurde in der Mirkowelle 1 h bei 130 °C gehalten. Anschließend wurden die Inhalte der beiden Gefäße in einem Rundkolben vereinigt und zur Trockne gebracht. Der Rückstand wurde mit Wasser versetzt und dreimal mit Dichlormethan extrahiert. Die vereinigten organischen Phasen wurden über Natriumsulfat getrocknet und nach Abfiltrieren des Trockenmittels im Vakuum zur Trockne gebracht. Der Aufarbeitungsprozess wurde noch zweimal wiederholt. Der so erhaltene Rückstand wurde mittels präparativer HPLC (Methode A, aber Gradient von 100 % Wasser+0,05%TFA → 15% Acetonitril/85 % Wasser+0,05% TFA in 25 min) getrennt. Die jeweils sauberen Produktfraktionen wurden vereinigt, im Vakuum vom Acetonitril befreit und gefriergetrocknet. Es wurden 1,35 g (6-Chlor-4-ethyl-pyridazin-3-yl)-hydrazin als Trifluoracetat und 3,96 g (6-Chlor-5-ethyl-pyridazin-3-yl)-hydrazin als Trifluoracetat erhalten.

### (6-Chlor-4-ethyl-pyridazin-3-yl)-hydrazin als Trifluoracetat, W3.003

| | |
|---|---|
| LCMS-rt: 0,20 min | [M+H]⁺: 173,1 (Met-c) |

### (6-Chlor-5-ethyl-pyridazin-3-yl)-hydrazin als Trifluoracetat, W3.011

| | |
|---|---|
| LCMS-rt: 0,13 min | [M+H]⁺: 173,1 (Met-b) |

### W3.004 und W3.100

### (6-Chlor-4-trifluormethyl-pyridazin-3-yl)-hydrazin-Hydrochlorid und (6-Chlor-5-trifluormethyl-pyridazin-3-yl)-hydrazin-Hydrochlorid

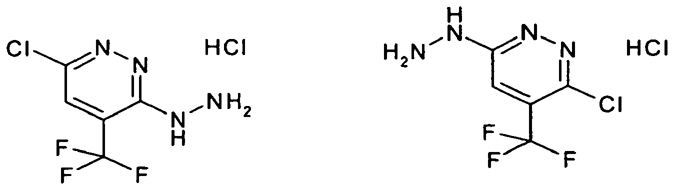

3,6-Dichlor-4-trifluormethyl-pyridazin (W4.004, 150 mg) wurde in Dioxan (3 ml) gelöst und nach Zugabe von Hydrazin-monohydrat (100 µl) bei RT gerührt. Nach 4 h wurde das Lösungsmittel abgezogen und der Rückstand in Wasser / ACN gelöst und mittel präparativer HPLC (Met-D) getrennt. Die jeweils sauberen Fraktionen wurden vereinigt, vom Acetonitril befreit und gefriergetrocknet. Es wurden 87 mg (6-Chlor-4-trifluormethyl-pyridazin-3-yl)-hydrazin-Hydrochlorid und 7 mg (6-Chlor-5-trifluormethylpyridazin-3-yl)-hydrazin-Hydrochlorid erhalten.

### (6-Chlor-4-trifluormethyl-pyridazin-3-yl)-hydrazin-Hydrochlorid, W3004

¹H-NMR (500 MHz, DMSO-d6) [ppm] (NHs nicht aufgeführt): 8,27 (1 H)

| | |
|---|---|
| LCMS-rt: 0,15 min | [M+H]⁺: 213,1 (Met-b) |

### (6-Chlor-5-trifluormethyl-pyridazin-3-yl)-hydrazin-Hydrochlorid, W3.100

¹H-NMR (500 MHz, DMSO-d6) [ppm] (NHs nicht aufgeführt): 7,73 (1 H)

| | |
|---|---|
| LCMS-rt: 0,37 min | [M+H]⁺: 173,1 (Met-c) |

### W3.005

### (6-Chlor-5-methyl-pyridazin-3-yl)-hydrazin

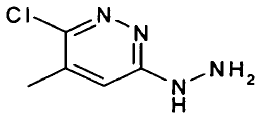

Synthetisiert analog US 4,578,464

| | |
|---|---|
| LCMS-rt: 0,26 min | [M+H]⁺: 159,1 (Met-a) |

### W3.006

### (6-Chlor-4,5-dimethyl-pyridazin-3-yl)-hydrazin

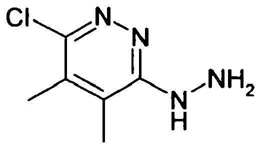

3,6-Dichlor-4,5-dimethyl-pyridazin (W4.006; 29,0 g) mit 160 ml Hydrazin-momohydrat-Lösung (160 ml) versetzt und 4 h auf 90°C unter Rühren erwärmt. Das Reaktionsgemisch wurde mit Wasser versetzt und der Niederschlag abgesaugt, mit Wasser gewaschen und über Phosphorpentoxid getrocknet. Es wurden 27,2 g der Titelverbindung erhalten. LCMS-rt: 0,15 min [M+H]⁺: 173,1 (Met-b)

### W3.007 und W3.008

### (6-Chlor-5-ethyl-4-methyl-pyridazin-3-yl)-hydrazin (auch als TFA-Salz) und (6-Chlor-4-ethyl-5-methyl-pyridazin-3-yl)-hydrazin-Trifluoressigsäuresatz

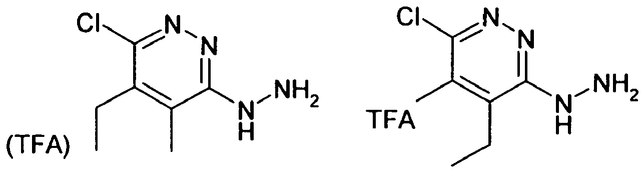

3,6-Dichlor-4-ethyl-5-methyl-pyridazin (W4.007; 1 g) wurde in Dioxan (8 ml) unter Zusatz von Hydrazin-monohydrat (2 ml) in einem Mikrowellengefäß bei RT vorgelegt. Danach wurde das Reaktionsgemisch in der Mikrowelle 1 h bei 140°C gehalten. Beim Stehen über Nacht fiel ein Niederschlag aus, der abgesaugt, gewaschen und getrocknet wurde. Es wurden 345 mg (6-Chlor-5-ethyl-4-methyl-pyridazin-3-yl)-hydrazin als freie Base erhalten.

Die Mutterlauge wurde bis zur Trockene eingeengt und der Rückstand mittels präparativer HPLC (Met-F) aufgereinigt. Die jeweils sauberen Produktfraktionen wurden vereinigt, im Vakuum vom Acetonitril befreit und gefriergetrocknet. Es wurden 340 mg 6-Chlor-4-ethyl-5-methyl-pyridazin-3-yl)-hydrazin-Trifluoressigsäuresalz und 239 mg (6-Chlor-5-ethyl-4-methyl-pyridazin-3-yl)-hydrazin als Trifluoracetat erhalten.

### (6-Chlor-5-ethyl-4-methyl-pyridazin-3-yl)-hydrazin -Trifluoressigsäuresalz, W3.007

| | |
|---|---|
| LCMS-rt: 0,25 min | [M+H]⁺: 187,1 (Met-b) |

### 6-Chlor-4-ethyl-5-methyl-pyridazin-3-yl)-hydrazin-Trifluoressigsäuresalz, W3.008

| | |
|---|---|
| LCMS-rt: 0,22 min | [M+H]⁺: 187,1 (Met-b) |

### W3.009 und W3.012

### (6-Chlor-4-isopropyl-pyridazin-3-yl)-hydrazin-Trifluoressigsäuresalz und (6-Chlor-5-isopropyl-pyridazin-3-yl)-hydrazin-Trifluoressigsäuresalz

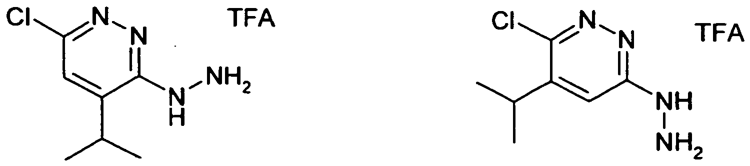

3,6-Dichlor-4-isopropyl-pyridazin (W4.009; 2,3 g) wurde analog W3.007 umgesetzt und anschließend zur Trockne gebracht. Nach präparativer Chromatographie (Met-F) wurden 260 mg (6-Chlor-4-isopropyl-pyridazin-3-yl)-hydrazin-Trifluoressigsäuresalz und 2,16 g (6-Chlor-5-isopropyl-pyridazin-3-yl)-hydrazin- Trifluoressigsäuresalz erhalten.

### (6-Chlor-4-isopropyl-pyridazin-3-yl)-hydrazin-Trifluoressigsäuresalz, W3.009

| | |
|---|---|
| LCMS-rt: 0,20 min | [M+H]⁺: 187,1 (Met-b) |

### (6-Chlor-5-isopropyl-pyridazin-3-yl)-hydrazin- Trifluoressigsäuresalz, W3.012

| | |
|---|---|
| LCMS-rt: 0,34 min | [M+H]⁺: 187,1 (Met-b) |

### W3.010 und W3.012

### (6-Chlor-4-cyclopropyl-pyridazin-3-yl)-hydrazin-Trifluoressigsäuresalz und (6-Chlor-5-cyclopropyl-pyridazin-3-yl)-hydrazin-Trifluoressigsäuresalz

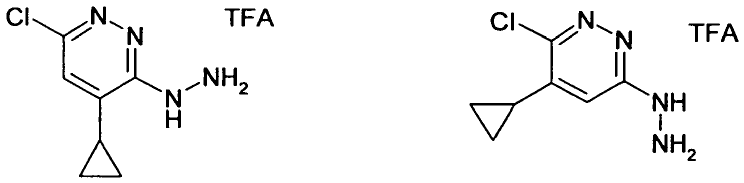

3,6-Dichlor-4-cyclopropyl-pyridazin (W4.010; 1,4 g) wurde analog W3.007 umgesetzt und anschließend zur Trockne gebracht. Nach präparativer Chromatographie (Met-F) wurden 805 mg (6-Chlor-4-cyclopropyl-pyridazin-3-yl)-hydrazin-Trifluoressigsäuresalz und 708 mg (6-Chlor-5-cyclopropyl-pyridazin-3-yl)-hydrazin erhalten.

### (6-Chlor-4-cyclopropyl-pyridazin-3-yl)-hydrazin-Trifluoressigsäuresalz, W3.010

| | |
|---|---|
| LCMS-rt: 0,15 min | [M+H]⁺: 185,1 (Met-b) |

### (6-Chlor-5-cyclopropyl-pyridazin-3-yl)-hydrazin-Trifluoressigsäuresalz, W3.012

| | |
|---|---|
| LCMS-rt: 0,22 min | [M+H]⁺: 185,1 (Met-b) |

### W3.011

### (6-Chlor-5-ethyl-pyridazin-3-yl)-hydrazin-Trifluoressigsäuresalz

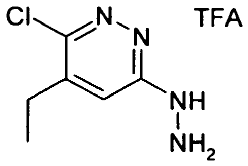

Siehe W3.003

### W3.012

### (6-Chlor-5-cyclopropyl-pyridazin-3-yl)-hydrazin-Trifluoressigsäuresalz

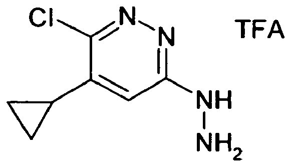

Siehe W3.010

### W3.013

### (6-Chlor-5-isopropyl-pyridazin-3-yl)-hydrazin-Trifluoressigsäuresalz

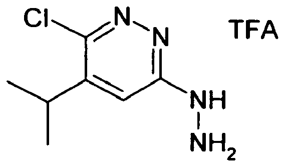

Siehe W3.009

### W3.014

### (6-Chlor-4,5-diethyl-pyridazin-3-yl)-hydrazin-Trifluoressigsäuresalz

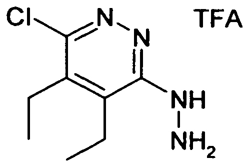

3,6-Dichlor-4,5-diethyl-pyridazin (W4.014; 3,0 g) wurde analog W3.007 umgesetzt und anschließend zur Trockne gebracht. Nach präparativer Chromatographie (Met-F) wurden 2,0 g der Titelverbindung erhalten.

| | |
|---|---|
| LCMS-rt: 0,38 min | [M+H]⁺: 201,1 (Met-b) |

Analog W3.007 wurden dargestellt:

| Nummer | | LCMS-rt | [M+H]⁺ | Bemerkung: |
|---|---|---|---|---|
| W3.015 | 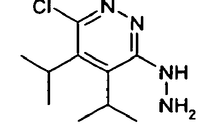 | 0,76 min | 229,2 (Met-b) | W4.015: 300 mg; 6 h MW; keine HPLC-Reinigung nötig; Produkt: 278 mg |
| W3.016 | 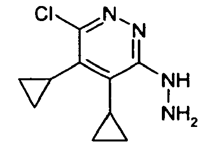 | 0,65 min | 225,1 (Met-b) | W4.016: 500 mg; HPLC (Met-B); mit Kalium-carbonat basisch gestellt und extrahiert; Produkt: 352 mg |

### W3.017

### (4-Chlor-6,7-dihydro-5H-cyclopenta[d]pyridazin-1-yl)-hydrazin

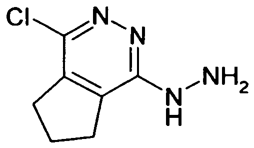

1,4-Dichlor-6,7-dihydro-5H-cyclopenta[d]pyridazin (W4.017; 3,89 g) wurde analog W3.006 umgesetzt, aufgearbeitet und isoliert. Es wurden 3,24 g der Titelverbindung erhalten. LCMS-rt: 0,20 min [M+H]⁺: 185,1 (Met-b)

### W3.018

### (4-Chlor-5,6,7,8-tetrahydro-phthalazin-1-yl)-hydrazin

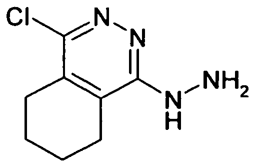

1,4-Dichlor-5,6,7,8-tetrahydro-phthalazin (W4.018; 10,3 g) wurde analog W3.006 umgesetzt. Nach Wasserzugabe wurde allerdings mit DCM extrahiert, die DCM-Phase abgetrennt, getrocknet, filtriert und eingeengt. Es wurden 9,1 g der Titelverbindung erhalten. LCMS-rt: 0,68 min [M+H]⁺: 199,1 (Met-a)

### W3.019 und W3.020

### 3-Chlor-6-hydrazino-pyridazin-4-carbonsäurediethylamid-Trifluoressigsäuresalz und 6-Chlor-3-hydrazino-pyridazin-4-carbonsäurediethylamid-Trifluoressigsäuresatz

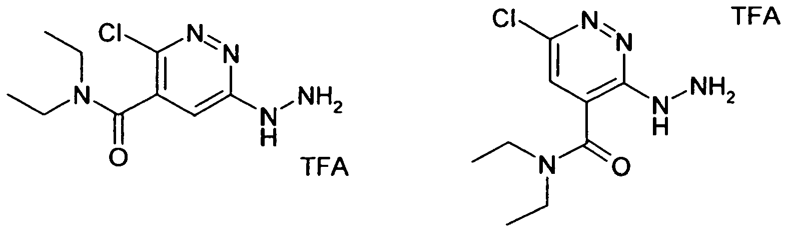

3,6-Dichlor-pyridazine-4-carbonsäurediethylamid (W4.019; 18 g) wurde in Wasser (60 ml) suspendiert und mit Hydrazin-monohydrat (2,8 ml) versetzt. Nach 1 h Rühren bei 60°C wurde 2 h auf 100°C erhitzt. Nach Abkühlen auf RT wurde mit DCM versetzt und viermal mit DCM extrahiert. Die vereinigten Extrakte wurden über Natriumsulfat getrocknet, filtriert und eingeengt. Der Rückstand wurde mittels präparativer HPLC (Met-C) aufgereinigt. Die sauberen, Produkt enthaltenden Fraktionen wurden jeweils vereinigt, vom ACN befreit und gefriergetrocknet. Es wurden 910 mg 3-Chlor-6-hydrazino-pyridazin-4-carbonsäurediethylamid-Trifluoressigsäuresalz und 560 mg 6-Chlor-3-hydrazino-pyridazin-4-carbonsäurediethylamid-Trifluoressigsäuresalz erhalten. 3-Chlor-6-hydrazino-pyridazin-4-carbonsäurediethylamid-Trifluoressigsäuresalz

| | |
|---|---|
| LCMS-rt: 0,79 min | [M+H]⁺: 244,1 (Met-a) |

### 6-Chlor-3-hydrazino-pyridazin-4-carbonsäurediethylamid-Trifluoressigsäuresalz

| | |
|---|---|
| LCMS-rt: 0,68 min | [M+H]⁺: 244,1 (Met-a) |

### W3.021

### (3-Chlor-6-hydrazino-pyridazin-4-yl)-diethyl-amin-Trifluoressigsäuresalz

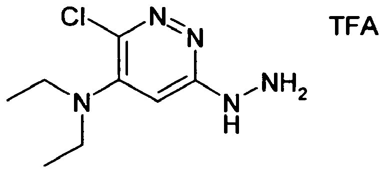

(3,6-Dichlor-pyridazin-4-yl)-diethyl-amin (W4.021; 500 mg) wurde in Dioxan (20 ml) unter Rühren vorgelegt und mit Hydrazinhydrat (0,65 ml) versetzt. Danach wurde erst 2 h bei 80°C und dann 3 h auf Rückfluss erhitzt. Nach Stehen übers Wochenende wurde weitere 48 h auf Rückfluss erhitzt und nach Erkalten das Lösungsmittel abgezogen. Der Rückstand wurde mittels präparativer HPLC (Met-A) aufgereinigt. Die sauberen, Produkt enthaltenden Fraktionen wurden jeweils vereinigt, vom ACN befreit, mit gesättigter Natriumhydrogencarbonat-Lösung basisch gestellt und dann fünfmal mit EE und fünfmal mit DCM extrahiert. Die vereinigten organischen Phasen wurden über Magnesiumsulfat getrocknet, filtriert und eingeengt. Es wurden 118 mg Edukt und 220 mg der Titelverbindung isoliert.

| | |
|---|---|
| LCMS-rt: 1,24 min | [M+H]⁺: 220,1 (Met-a) |

### W3.100

### (6-Chlor-5-trifluormethyl-pyridazin-3-yl)-hydrazin-Hydrochlorid

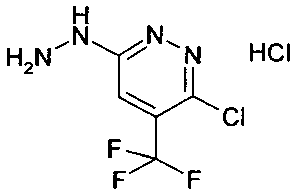

Siehe W3.004

### W3.120

### (5,6-Diethoxy-pyridazin-3-yl)-hydrazin

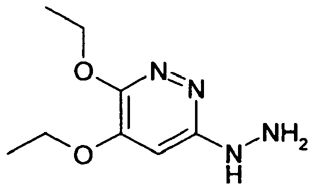

N-(5,6-Diethoxy-pyridazin-3-yl)-N-nitro-amin (W4.120; 114 mg) wurde in Essigsäure (5 ml) gelöst und unter Eiskühlung und Rühren zwischen 10 und 20°C in eine Mischung aus Zink (130 mg) in Wasser (3 ml) getropft. Danach wurde das Eisbad entfernt und 1 h bei RT gerührt. Dann wurde mit 10 N Natronlauge alkalisch gestellt, die wässrige Phase dreimal mit DCM extrahiert, die vereinigten DCM-Phasen über Natriumsulfat getrocknet, filtriert und eingeengt. Der Rückstand wurde mittels präparativer HPLC (Met-A) aufgereinigt. Die sauberen, Produkt enthaltenden Fraktionen wurden jeweils vereinigt, vom ACN befreit, mit gesättigter Kaliumcarbonat-Lösung basisch gestellt und dann dreimal mit DCM extrahiert. Die vereinigten organischen Phasen wurden über Natriumsulfat getrocknet, filtriert und eingeengt. Es wurden 50 mg der Titelverbindung erhalten.

| | |
|---|---|
| LCMS-rt: 0,37 min | [M+H]⁺: 199,2 (Met-b) |

### W3.126

### (6-Chlor-4-methansulfonyl-pyridazin-3-yl)-hydrazin

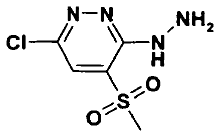

3,6-Dichlor-4-methansulfonyl-pyridazin (W4.126; 660 mg, 2,9 mmol) wurde in 1,4-Dioxan (5 ml) gelöst und Hydrazin-Hydrat (295 µl, 5,8 mmol) wurde hinzugegeben. Die Mischung wurde bei RT 4 h gerührt, dann konzentriert und der Rückstand durch Flash-Chromatographie (Dichlormethan:Essigester) gereinigt. Ausbeute: 200 mg

| | |
|---|---|
| LCMS-rt: 0,17 min | [M+H]⁺: 248,0 (Met-c) |

### W3.150

### N*-(6-Chlor-4,5-dimethyl-pyridazin-3-yl)-amino-N-(2,2,2-trifluorethyl)-harnstoff und N*-(6-Chlor-4,5-dimethyl-pyridazin-3-yl)-amino-N-(2,2,2-trifluorethyl)-harnstoff-Hydrochlorid

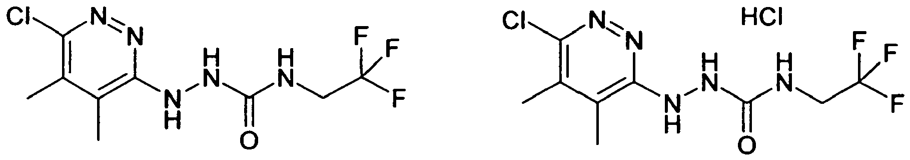

4-Nitrophenyl-chloroformat (750 mg) wurde in THF (55 ml) gelöst, unter Rühren 2,2,2-Trifluorethylamin (0,3 ml) zugegeben und bei RT 3 h gerührt. Dann wurde (6-Chlor-4,5-dimethyl-pyridazin-3-yl)-hydrazin (W3.006, 620 mg) in THF (100 ml) gelöst zugegeben, gefolgt von Triethylamin (0,7 ml) und 3 h bei RT gerührt. Nach Stehen über Nacht wurde der ausgefallene Niederschlag abgesaugt und getrocknet. Es wurden 840 mg der freien Base erhalten, die noch deutliche Mengen Triethylamin-Hydrochlorid enthielt.

Die Mutterlauge wurde zur Trockne gebracht und mittels präparativer HPLC (Met-D) aufgereinigt. Die sauberen, Produkt enthaltenden Fraktionen wurden vereinigt und zur Trockne gebracht. Es wurden weitere 400 mg der Titelverbindung als Hydrochlorid gewonnen. LCMS-rt: 0,40 min [M+H]⁺: 298,1 (Met-b)

### W3.166

### N*-(6-Chlor-4,5-dimethyl-pyridazin-3-yl)-amino-N-(cyclopropyl)-thioharnstoff

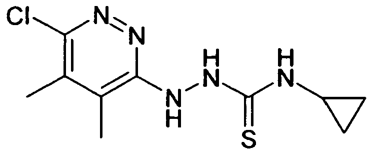

6-Chlor-4,5-dimethyl-pyridazin-3-yl)-hydrazin (W3.006, 540 mg) wurde in Methylenchlorid (50 ml) gelöst und unter Rühren wurde Cyclopropyl-isothiocyanat (290 µl) zugegeben. Es wurde 7 h bei RT gerührt und dann über Nacht stehen gelassen. Danach wurde mit Diethylether (50 ml) versetzt, 3 h gerührt und der gebildete Niederschlag abgesaugt. Der Niederschlag wurde mit Ether gewaschen und im Vakuum getrocknet. Es wurden 830 mg der Titelverbindung erhalten.

| | |
|---|---|
| LCMS-rt: 0,62 min | [M+H]⁺: 272,1 (Met-b) |

### W3.167

### N*-(6-Chlor-4,5-dimethyl-pyridazin-3-yl)-amino-N-(isopropyl)-thioharnstoff

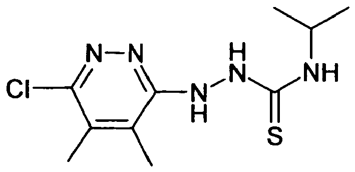

6-Chlor-4,5-dimethyl-pyridazin-3-yl)-hydrazin (W3.006, 380 mg) wurde analog W3.166 mit Isopropyl-isothiocyanat (235 µl) umgesetzt und aufgearbeitet. Als Niederschlag wurden 428 mg erhalten. Die Mutterlauge wurde zur Trockne gebracht und über Kieselgel (70 g Kartusche, DCM/Methanol-Gradient 0-30 % in 30 min) aufgereinigt. Dadurch wurden weitere 119 mg Produkt erhalten.

| | |
|---|---|
| LCMS-rt: 0,78 min | [M+H]⁺: 274,1 (Met-b) |

### W3.168

### N*-(6-Chlor-5-methyl-pyridazin-3-yl)-amino-N-(isopropyl)-thioharnstoff

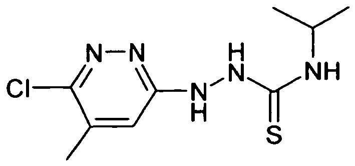

6-Chlor-5-dimethyl-pyridazin-3-yl)-hydrazin als Trifluoressigsäuresalz (W3.005, 660 mg) wurde in DCM (55 ml) gelöst, unter Rühren mit Isopropyl-isothiocyanat (258 µl) und Triethylamin (310 ml) zugegeben. Aufarbeitung und Isolierung erfolgten analog wie in W3.167 beschrieben. Dabei wurden 140 mg der Titelverbindung als Niederschlag isoliert. LCMS-rt: 0,81 min [M+H]⁺: 260,1 (Met-b)

### W3. 169

### N'-(6-Chlor-4,5-dimethyl-pyridazin-3-yl)-amino-N-methyl-thioharnstoff

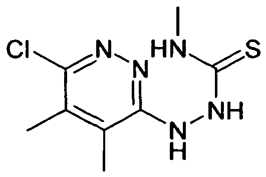

(6-Chlor-4,5-dimethyl-pyridazin-3-yl)-hydrazin (W3.006; 8,00 g) wurde in DCM (400 ml) gelöst und mit Methylisothiocyanat (3,39 g) versetzt. Anschließend wurde 24 h bei RT gerührt und übers Wochenende stehen gelassen. Der Niederschlag wurde abfiltriert, mit DCM gewaschen und im Trockenschrank bei 45°C getrocknet. Es wurden 11,25 g der Titelverbindung erhalten.

| | |
|---|---|
| LCMS-rt: 0,29 min | [M+H]⁺: 246,1 (Met-b) |

### W3.170

### N'-(6-Chlor-4-methyl-pyridazin-3-yl)-amino-N-methyl-thioharnstoff

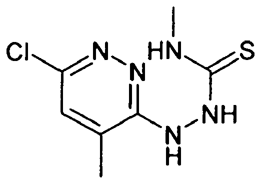

(6-Chlor-4-methyl-pyridazin-3-yl)-hydrazin (W3.002; 5,5 g) wurde analog W3.169 umgesetzt und aufgearbeitet. Allerdings wurde statt einem drei Tage gerührt. Es wurden 7,75 g der Titelverbindung erhalten.

| | |
|---|---|
| LCMS-rt: 0,20 min | [M+H]⁺: 232,1 (Met-b) |

Analog W3.169 wurden dargestellt:

| Nummer | | LCMS-rt | [M+H]⁺ | Bemerkung: |
|---|---|---|---|---|
| W3.191 | 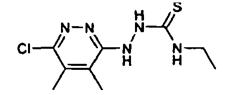 | 0,50 min | 260,0 (Met-b) | W3.006: 5 g; Produkt: 7,6 g |
| W3.192 | 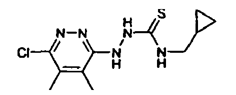 | 0,64 min | 286,0 (Met-b) | W3.006: 5,0 g; Produkt: 7,8 g |

### W4

### W4.003 und W4.014

### 3,6-Dichlor-4-ethyl-pyridazin und 3,6-Dichlor-4,5-diethyl-pyridazin

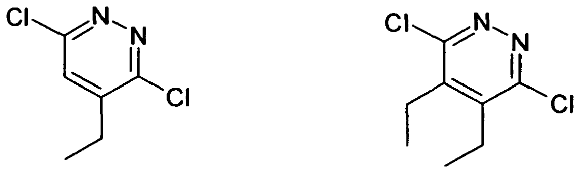

Analog: Samaritoni, Org. Prep. Proc. Int. 117 (1988)

3,6-Dichlor-pyridazin (10 g), Silbernitrat (5,7 g) und Propionsäure (7,5 ml) wurden in Wasser (125 ml) vorgelegt und bei 50°C konzentrierte Schwefelsäure (11 ml) zugetropft. Nach Zugabe wurde das Reaktionsgemisch auf 60°C erwärmt und eine Lösung aus Ammoniumpersulfat (46 g) in Wasser (125 ml) wurde innerhalb 20 min langsam zugegeben. Nach Zugabe wurde 30 min auf 70°C erhitzt. Nach Stehen über Nacht wurde das Reaktionsgemisch auf Eis/Wasser gegossen und mit 25%-iger Ammoniumhydroxid-Lösung auf pH7 gestellt. Dann wurde dreimal mit Dichlormethan extrahiert. Die vereinigten organischen Phasen wurden über Natriumsulfat getrocknet und nach Abfiltrieren des Trockenmittels im Vakuum zur Trockne gebracht. Der Rückstand wurde mittels präparativer HPLC (Met-C) gereinigt. Die jeweils sauberen Produktfraktionen wurden vereinigt, im Vakuum vom Acetonitril befreit und dreimal mit Dichlormethan extrahiert. Die vereinigten organischen Phasen wurden über Natriumsulfat getrocknet und nach Abfiltrieren des Trockenmittels im Vakuum zur Trockne gebracht. Es wurden 6,6 g 3,6-Dichlor-4-ethyl-pyridazin und 3,0 g 3,6-Dichlor-4,5-diethyl-pyridazin erhalten.

### 3,6-Dichlor-4-ethyl-pyridazin, W4.003

| | |
|---|---|
| LCMS-rt: 0,83 min | [M+H]⁺: 177,1 (Met-b) |

### 3,6-Dichlor-4,5-diethyl-pyridazin, W4.014

| | |
|---|---|
| LCMS-rt: 1,02 min | [M+H]⁺: 205,1 (Met-b) |

### W4.004

### 3,6-Dichlor-4-trifluormethyl-pyridazin

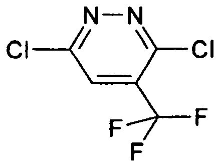

4-Trifluormethyl-1,2-dihydro-pyridazin-3,6-dion (W5.004; 125 mg) wurde in Phosphoroxiychlorid (2 ml) unter Rühren bei RT als Suspension vorgelegt. Danach erwärmte man das Reaktionsgemisch 7 h auf 80°C, gab dann Phosphorpentachlorid (100 mg) zu rührte über Nacht bei 80°C. Nach Abkühlen wurde auf Eiswasser gegeben und die wässrige Phase dreimal mit DCM extrahiert. Die vereinigten organischen Phasen wurden über Natriumsulfat getrocknet und nach Abfiltrieren des Trockenmittels im Vakuum zur Trockne gebracht. Es wurden 150 mg der Titelverbindung erhalten. LCMS-rt: 0,94 min [M+H]⁺: 217,1 (Met-b)

### W4.006

### 3,6-Dichlor-4,5-dimethyl-pyridazin

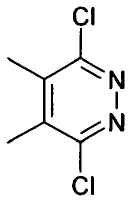

4,5-Dimethyl-1,2-dihydro-pyridazin-3,6-dion (W5.006; 69,7 g) wurde in Phosphoroxychlorid (150 ml) suspendiert und 2 h auf 80°C erwärmt. Nach Abkühlen wurde auf Eiswasser gegeben und mit 10 M NaOH unter Eiskühlung vorsichtig auf pH 10 gestellt. Der Niederschlag wurde abgesaugt, mit Wasser gewaschen und getrocknet. Es wurden 78,3 g der Titelverbindung erhalten.

| | |
|---|---|
| LCMS-rt: 0,63 min | [M+H]⁺: 177,1 (Met-b) |

### W4.007

### 3,6-Dichlor-4-ethyl-5-methyl-pyridazin

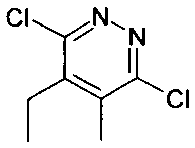

Die Verbindung wurde analog W4.003 synthetisiert. Als Edukt diente 3,6-Dichlor-4-methyl-pyridazin (4 g). Es wurden 3,6 g der Titelverbindung erhalten.

| | |
|---|---|
| LCMS-rt: 0,92 min | [M+H]⁺: 191,1 (Met-b) |

### W4.009 und W4.015

### 3,6-Dichlor-4-isopropyl-pyridazin und 3,6-Dichlor-4,5-diisopropyl-pyridazin

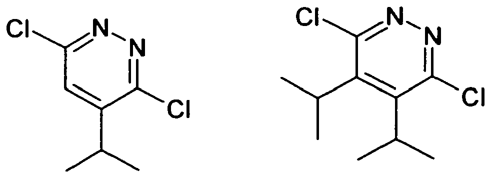

Die Verbindung wurde analog W4.003 synthetisiert. Aus 2,5 g 3,6-Dichlor-pyridazin und Isobuttersäure (2,34 ml) wurden 2,46 g 3,6-Dichlor-4-isopropyl-pyridazin und 0,33 g 3,6-Dichlor-4,5-diisopropyl-pyridazin erhalten.

### 3,6-Dichlor-4-isopropyl-pyridazin, W4.009

| | |
|---|---|
| LCMS-rt: 0,96 min | [M+H]⁺: 191,1 (Met-b) |

### 3,6-Dichlor-4,5-diisopropyl-pyridazin, W4.015

| | |
|---|---|
| LCMS-rt: 1,14 min | [M+H]⁺: 233,1 (Met-b) |

### W4.010 und W4.016

### 3,6-Dichlor-4-cyclopropyl-pyridazin und 3,6-Dichlor-4,5-dicyclopropyl-pyridazin

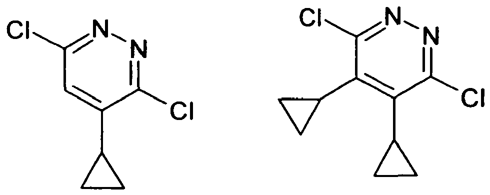

Die Verbindung wurde analog W4.003 synthetisiert. Aus 3 g 3,6-Dichlor-pyridazin und Cyclopropancarbonsäure (2,41 ml) wurden 1,6 g 3,6-Dichlor-4-cyclopropyl-pyridazin und 0,96 g 3,6-Dichlor-4,5-dicyclopropyl-pyridazin erhalten.

### 3,6-Dichlor-4-cyclopropyl-pyridazin, W4.01 0

| | |
|---|---|
| LCMS-rt: 0,87 min | [M+H]⁺: 189,1 (Met-b) |

### 3,6-Dichlor-4,5-dicyclopropyl-pyridazin, W4.016

| | |
|---|---|
| LCMS-rt: 1,05 min | [M+H]⁺: 229,1 (Met-b) |

### W4.017

### 1,4-Dichlor-6,7-dihydro-5H-cyclopenta[d]pyridazin

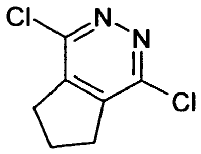

2,3,6,7-Tetrahydro-5H-cyclopenta[d]pyridazin-1,4-dion (W5.017; 11,3 g) wurde in Phosphoroxychlorid (36,7 ml) suspendiert und 2 h auf 80°C erwärmt. Nach Abkühlen wurde auf Eiswasser gegeben, mit 10 M NaOH unter Eiskühlung vorsichtig auf pH 10 gestellt und die Wasserphase dreimal mit EE extrahiert. Die vereinigten EE-Phasen wurden getrocknet, filtriert und eingeengt. Es wurden 3,89 g der Titelverbindung erhalten. LCMS-rt: 0,71 min [M+H]⁺: 189,1 (Met-b)

### W4.018

### 1,4-Dichlor-5,6,7,8-tetrahydro-phthalazin

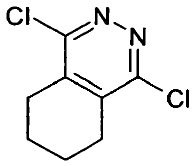

2,3,5,6,7,8-Hexahydro-phthalazin-1,4-dion (W5.018; 10,1 g) wurde analog W4.006 dargestellt. Allerdings wurde der Niederschlag anschließend über Kieselgel (300 g, n-Heptan/EE 7:3) aufgereinigt. Es wurden 10,32 g der Titelverbindung erhalten.

| | |
|---|---|
| LCMS-rt: 1,29 min | [M+H]⁺: 203,1 (Met-a) |

### W4.019

### 3,6-Dichlor-pyridazin-4-carbonsäurediethylamid

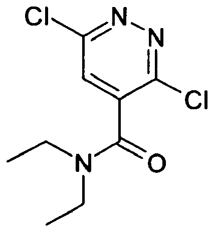

3,6-Dichlor-pyridazin-4-carbonylchlorid (2,5 g) wurde in DCM (25 ml) bei RT vorgelegt. Danach tropfte man unter Rühren Diethylamin (1,5 ml) in DCM (5 ml) vorgelöst langsam zu. Nach 3 h Rühren bei RT wurde mit Wasser versetzt und dreimal gegen DCM extrahiert. Die vereinigten DCM-Phasen trocknete man über Natriumsulfat, filtrierte und engte ein. Der Rückstand wurde über Kieselgel ( 70 g Kartusche, n-Heptan/EE-Gradient) aufgereinigt. Es wurden 1,8 g der Titelverbindung erhalten.

| | |
|---|---|
| LCMS-rt: 0,97 min | [M+H]⁺: 248,1 (Met-a) |

### W4.021

### (3,6-Dichlor-pyridazin-4-yl)-diethyl-amin

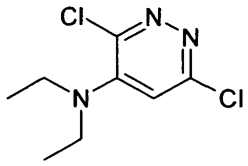

3,4,6-Trichlorpyridazin (2 g) und Diethylamin (2,4 ml) wurden in Toluol (10 ml) vorgelegt und 3 Tage bei RT stehengelassen. Dann wurde mit Wasser und EE versetzt und die EE-Phase abgetrennt. Die EE-Phase wurde dreimal mit Wasser gewaschen, über Magnesiumsulfat getrocknet, filtriert und eingeengt. Der Rückstand wurde über Kieselgel ( 70 g Kartusche, n-Heptan/EE-Gradient 0-50 % in 60 min) aufgereinigt. Es wurden 1,1 g der Titelverbindung erhalten.

| | |
|---|---|
| LCMS-rt: 1,24 min | [M+H]⁺: 248,1 (Met-a) |

### W4.120

### N-(5,6-Diethoxy-pyridazin-3-yl)-N-nitro-amin

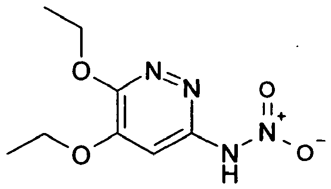

5,6-Diethoxy-pyridazin-3-ylamin (analog T. Horie in Chemical & Pharmaceutical Bulletin (1963), 11 (9), 1157-67; 160 mg) wurde in konzentrierter Schwefelsäure (4 ml) bei RT gelöst vorgelegt. Danach wurde auf 0°C gekühlt und 2 ml eines 1:1-Gemisches aus konzentrierter Salpetersäure und konzentrierter Schwefelsäure zugetropft. Nach 20 min bei 0°C wurde das Eisbad entfernt und 4 h gerührt. Dann wurde erneut auf 0°C gekühlt, weitere 0,5 ml des Säuregemisches zugegeben und noch eine Stunde bei 0°C gerührt. Dann wurde unter Eiskühlung mit Eis versetzt. Nach Zugabe von DCM wurden die Phasen getrennt und dreimal mit DCM extrahiert. Die vereinigten DCM-Phasen trocknete man über Natriumsulfat, filtrierte und engte ein. Es wurden 188 mg der Titelverbindung erhalten.

| | |
|---|---|
| LCMS-rt: 0,81 min | [M+H]⁺: 236,2 (Met-b) |

### W4.126

### 3,6-Dichlor-4-methansulfonyl-pyridazin

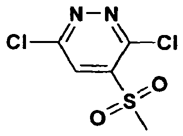

3,4,6-Trichlor-pyridazin (2,5 g; 13,6 mmol) und Methansulfinsäure-Natriumsalz (2,78 g, 27,26 mmol) wurden in 20 ml eines Lösungsmittelgemisches von Wasser/THF/DMF (5:10:5) 30 h bei RT gerührt. Das Lösungsmittel wurde im Vakuum entfernt und der Rückstand zwischen Essigester und gesättigter Ammoniumchlorid-Lösung verteilt. Die organische Phase wurde abgetrennt, konzentriert und das Rohprodukt durch Flash-Chromatographie (Dichlormethan:Methanol) gereinigt. Ausbeute: 1,4 g.
LCMS-rt: 0,44 min [M+H]⁺: 227,0 (Met-b)

### W5.004

### 4-Trifluormethyl-1,2-dihydro-pyridazin-3,6-dion

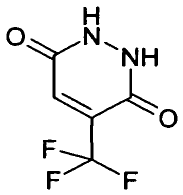

3-Trifluormethyl-furan-2,5-dion (5 g) wurde portionsweise in eine bereits siedende Lösung von Hydrazin-Dihydrochlorid (3,2 g) in Wasser (50 ml) eingetragen. Nach 4 h Rühren wurde über Nacht stehen gelassen, am nächsten Tag weitere 8 h rückflussiert und über Nacht stehen gelassen. Dann wurde zur Trockne gebracht und der Rückstand mittels präparativer Chromatographie (Met-C) gereinigt. Die sauberen Fraktionen wurden vereinigt, das ACN abgezogen und gefriergetrocknet, Es wurden 250 mg der Titelverbindung erhalten. LCMS-rt: 0,23 min [M+H]⁺: 181,1 (Met-b)

### W5

### W5.006

### 4,5-Dimethyl-1,2-dihydro-pyridazin-3,6-dion

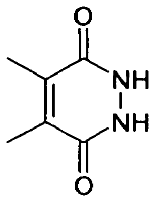

Hydrazin-dihydrochlorid (83,6 g) wurde in Wasser (20 ml) gelöst, auf 100°C erwärmt und 3,4-Dimethyl-furan-2,5-dion (100,4 g) unter Rühren eingetragen. Dann wurde 3 h auf Rückfluss erhitzt. Anschließend wurde der gebildete Niederschlag abgesaugt, mit Wasser gewaschen und getrocknet. Der Rückstand wurde in EE (2 I) suspendiert, abgesaugt und getrocknet. Es wurden 69,7 g der Titelverbindung erhalten. LCMS-rt: 0,19 min [M+H]⁺: 141,1 (Met-b)

### W5.017

### 2,3,6,7-Tetrahydro-5H-cyclopenta[d]pyridazin-1,4-dion

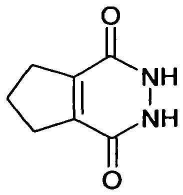

5,6-Dihydro-4H-cyclopenta[c]furan-1,3-dion (11,3 g) wurde analog W5.006 umgesetzt und aufgearbeitet. Suspendieren in EE wurde nicht durchgeführt. Es wurden 11,3 g Rohprodukt erhalten. LCMS-rt: 0,19 min [M+H]⁺: 153,1 (Met-b)

### W5.018

### 2,3,5,6,7,8-Hexahydro-phthalazin-1,4-dion

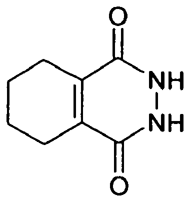

2,3,5,6,7,8-Hexahydro-phthalazin-1,4-dion (10,0 g) wurde analog W5.017 umgesetzt. Es wurden 10,14 g der Titelverbindung erhalten.

| | |
|---|---|
| LCMS-rt: 0,72 min | [M+H]⁺: 167,1 (Met-a) |

Synthese der Bausteine der Osthälfte

### O1.001

### N-[3-(2-Brom-acetyl)-5-(pentafluorsulfanyl)-phenyl]-acetamid

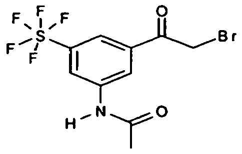

N-[3-Acetyl-5-(pentafluorsulfanyl)-phenyl]-acetamid (859 mg, Synthese siehe Beispiel 1) wurde in einem Gemisch aus Methanol (10 ml) und THF (10 ml) gelöst und unter Rühren portionsweise Phenyl-trimethyl-ammonium-tribromid (1,065 g) zugegeben. Nach 2 h Rühren bei RT wurde weitere 3 h auf 40°C erwärmt. Nach dem Erkalten wurde das Reaktionsgemisch in 2 N Schwefelsäure gegeben und die wässrige Phase 3 Mal mit Essigester extrahiert. Die vereinigten Extrakte wurden über Natriumsulfat getrocknet, filtriert und eingeengt. Das Rohprodukt wurde über Kieselgel mit Essigester/Heptan als Laufmittel aufgereinigt. Es wurden 480 mg der gewünschten Verbindung erhalten. LCMS-rt: 1,47 min [M+H]⁺: 382,0 (Met-a)

### O1.002

### 2-Brom-1-(3,5-di-tert-butyl-4-hydroxy-phenyl)-ethanon (Apollo Scientific)

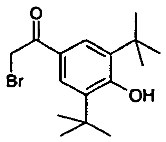

### O1.003

### 2-Brom-1-(3-tert-butyl-4-methoxy-5-morpholin-4-yl-phenyl)-ethanon

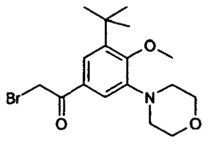

Hergestellt wie in WO 2004/078721 beschrieben.

### O1.004

### 2-Brom-1-(4-methoxy-3-morpholin-4-yl-5-trifluormethyl-phenyl)-ethanon

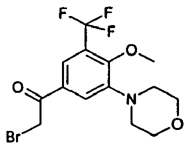

4-[5-(1,1-Dimethoxy-ethyl)-2-methoxy-3-trifluormethyl-phenyl]-morpholin (02.004; 460 mg) wurde in einem Gemisch aus Methanol (1,4 ml) und THF (4 ml) gelöst, auf 7°C gekühlt und unter Rühren portionsweise Phenyl-trimethyl-ammonium-tribromid (530 mg) zugegeben. Nach 3 h Rühren bei RT wurde über Nacht stehen gelassen. Dann wurde wässrige Thiosulfat-Lösung (0,8 ml; w = 5 %) und Wasser (4 ml) zugegeben, mit EE versetzt und dreimal mit EE extrahiert. Die vereinigten Extrakte wurden über Magnesiumsulfat getrocknet, filtriert und eingeengt. Der Rückstand wurde in einem Gemisch aus Acetonitril (20 ml) und Wasser (0,5 ml) gelöst und unter Rühren mit TFA (0,5 ml) versetzt. Nach 5 h Rühren bei RT wurde das Lösungsmittel abgezogen, der Rückstand mit Wasser versetzt, mit gesättigter Natriumhydrogencarbonat-Lösung neutralisiert und dreimal mit Essigester extrahiert. Die vereinigten Extrakte wurden über Magnesium getrocknet, filtriert und eingeengt. Das Rohprodukt wurde über Kieselgel mit Essigester/Heptan als Laufmittel aufgereinigt. Es wurden 200 mg der gewünschten Verbindung erhalten.

| | |
|---|---|
| LCMS-rt: 1,67 min | [M+H]⁺: 382,0 (Met-a) |

### O1.005

### N-[3-(2-Brom-acetyl)-5-pentafluorsulfanyl-phenyl]-N-methyl-acetamid

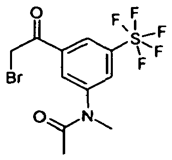

2-Brom-1-[3-methylamino-5-(pentafluorsulfanyl)-phenyl]-ethanon (O1.006; 100 mg) wurde in DCM (5 ml) gelöst und unter Rühren Acetylbromid (21 µl) zugegeben. Nach 4 h Rühren bei RT wurde weiteres Acetylbromid (21 µl) zugegeben und weiter bei RT gerührt. Nach Stehen über Nacht wurde nochmals mit Acetylbromid (21 µl) versetzt und 2 h gerührt. Dann wurde das Reaktionsgemisch eingeengt und der Rückstand mittels präparativer HPLC (Met-A) gereinigt. Die jeweils sauberen Produktfraktionen wurden vereinigt, im Vakuum vom Acetonitril befreit, mit Natriumhydrogencarbonat-Lösung auf pH 9 festellt und dreimal mit EE extrahiert. Die vereinigten organischen Phasen wurden über Magnesiumsulfat getrocknet und nach Abfiltrieren des Trockenmittels im Vakuum zur Trockne gebracht. Es wurden 50 mg der Titelverbindung und 18 mg Edukt isoliert.

| | |
|---|---|
| LCMS-rt: 1,47 min | [M+H]⁺: 396,0 (Met-a) |

### O1.006

### 2-Brom-1-[3-methylamino-5-(pentafluorsulfanyl)-phenyl]-ethanon

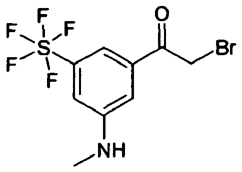

N-[3-(2-Brom-acetyl)-5-(pentafluorsulfanyl)-phenyl]-2,2,2-trifluor-N-methyl-acetamid (O1.075; 1,2 g) wurde mit Wasser (15 ml) versetzt und unter Rühren und Eiskühlung konzentrierte Schwefelsäure (15 ml) zugetropft. Es wurde auf 80°C erwärmt und 7 h bei dieser Temperatur gerührt. Nach Erkalten wurde das Reaktionsgemisch langsam in ein Gemisch aus 10 N Natriumhydroxid-Lösung und EE gegeben und die wässrige Phase fünfmal mit EE extrahiert. Die vereinigten organischen Phasen wurden über Magnesiumsulfat getrocknet und nach Abfiltrieren des Trockenmittels im Vakuum zur Trockne gebracht. Der Rückstand wurde mittels präparativer HPLC (Met-A) gereinigt. Die jeweils sauberen Produktfraktionen wurden vereinigt, im Vakuum vom Acetonitril befreit, mit Natriumhydrogencarbonat neutralisiert und dreimal mit EE extrahiert. Die vereinigten organischen Phasen wurden über Magnesiumsulfat getrocknet und nach Abfiltrieren des Trockenmittels im Vakuum zur Trockne gebracht. Es wurden 420 mg der Titelverbindung isoliert.

| | |
|---|---|
| LCMS-rt: 1,64 min | [M+H]⁺: 354,0 (Met-a) |

### O1.007

### 2-Brom-1-[3-methoxy-5-(pentafluorsulfanyl)-phenyl]-ethanon

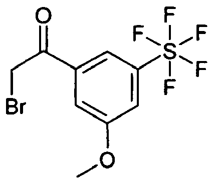

1-[3-Methoxy-5-(pentafluorsulfanyl)-phenyl]-ethanon (02.007; 1,63 g) wurde in THF (150 ml) gelöst und unter Rühren bei RT Phenyltrimethylammonium - Tribromid (2,2 g) zugegeben. Nach 2 h Rühren bei RT wurde mit Wasser versetzt, mit Natriumhydrogencarbonat-Lösung neutralisiert und dreimal mit EE extrahiert. Die alkalische Wasserphase wurde 3 x mit EE extrahiert. Die vereinigten organischen Phasen wurden über Magnesiumsulfat getrocknet und nach Abfiltrieren des Trockenmittels im Vakuum zur Trockne gebracht. Der Rückstand wurde mittels präparativer HPLC (Met-A) gereinigt. Die jeweils sauberen Produktfraktionen wurden vereinigt, im Vakuum vom Acetonitril befreit, mit Natriumhydrogencarbonat neutralisiert und dreimal mit EE extrahiert. Die vereinigten organischen Phasen wurden über Magnesiumsulfat getrocknet und nach Abfiltrieren des Trockenmittels im Vakuum zur Trockne gebracht. Es wurden 1,27 g der Titelverbindung isoliert.

| | |
|---|---|
| LCMS-rt: 1,65 min | [M+H]⁺: 354,9 (Met-b) |

### O1.008

### 2-Brom-1-(3-tert-butyl-5-ethoxymethyl-phenyl)-ethanon

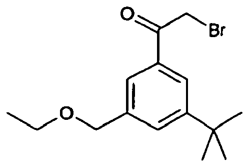

1-(3-tert-Butyl-5-ethoxymethyl-phenyl)-ethanon (02.008; 550 mg) wurde in Methanol/THF (10 ml/10 ml) gelöst, unter Rühren mit Phenyltrimethylammoniumtribromid (882 mg) versetzt und 2 h bei RT gerührt. Anschließend wurde das Reaktionsgemisch auf DCM (200 ml) gegossen und einmal mit 5%iger Natriumthiosulfat-Lösung und einmal mit Wasser gründlich gewaschen. Dann wurde die DCM-Phase getrocknet und eingeengt. Der Rückstand wurde über Kieselgel (40 g Kartusche, n-Heptan/EE-Gradient von 0-50% in 30 min) aufgereinigt. Es wurden 566 mg der Titelverbindung erhalten. LCMS-rt: 1,83 min [M+H]⁺: 313,2 (Met-a)

Analog wurden dargestellt:

| Nummer | | LCMS-rt | [M+H]⁺ | Bemerkung: |
|---|---|---|---|---|
| O1.009 | 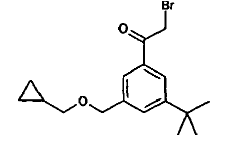 | 1,92 min | 339,2(Met-a) | O2.009; 595 mg; Produkt: 691 mg |
| O1.010 | 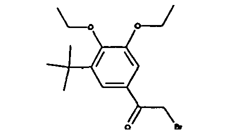 | 2,01 min | 343,2(Met-a) | O2.010; 410 mg; Produkt: 405 mg |
| O1.011 | 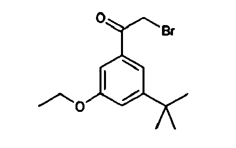 | 1,77 min | 299,2(Met-a) | O2.011; 390 mg; statt Thiosulfat- 5%ige Zitronensäure-Lösung zugegeben; Produkt: 387 mg, |
| O1.012 | 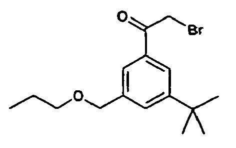 | 1,94 min | 327,2 (Met-a) | O2.012; 333 mg; 12 h bei RT gerührt; statt Thiosulfat-Lösung mit 20-%iger Zitronensäure-Lösung 1 h berührt ; Produkt: 327 mg |
| O1.013 | 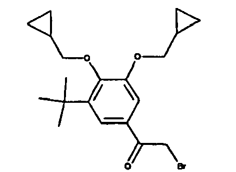 | 2,16 min | 395,3 (Met-a) | O2.013; 540 mg; nach Thiosulfat-Lösung zusätzlich mit 20-%iger Zitronensäure-Lösung gewaschen; Produkt: 600 mg |

### O1.014

### 2-Brom-1-(3-methoxy-5-trifluormethyl-phenyl)-ethanon

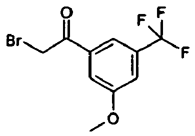

1-(3-Methoxy-5-trifluormethyl-phenyl)-ethanon (02.014, 50 mg) wurde in DCM (0,8 ml) gelöst und bei RT zu einem Gemisch aus Kupfer(II)bromid (102 mg) in EE (1,2 ml) getropft. Nach 2 h Erhitzen auf RF ließ man über Nacht stehen, saugte dann das Reaktionsgemisch über "Celite" ab, wusch gut mit EE und brachte das Filtrat zur Trockne. Der Rückstand wurde mit EE und halbgesättigter Natriumhydrogen-carbonat-Lösung aufgenommen und dann zweimal mit EE extrahiert. Die vereinigten EE-Phasen wurden mit halbgesättigter Natriumhydrogencarbonat-Lösung gewaschen, über Natriumsulfat getrocknet, filtriert und eingeengt. Das Rohprodukt wurde mittels präparativer HPLC (Met-A) gereinigt. Die jeweils sauberen Produktfraktionen wurden vereinigt, im Vakuum vom Acetonitril befreit und dreimal mit EE extrahiert. Die vereinigten organischen Phasen wurden über Natriumsulfat getrocknet und nach Abfiltrieren des Trockenmittels im Vakuum zur Trockne gebracht. Es wurden 39 mg der Titelverbindung isoliert.

| | |
|---|---|
| LCMS-rt: 1,64 min | [M+H]⁺: 297,0 (Met-a) |

Analog O1.008 wurden dargestellt:

| Nummer | | LCMS-rt | [M+H]⁺ | Bemerkung: |
|---|---|---|---|---|
| O1.015 | 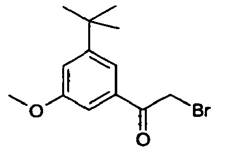 | 1,77 min | 285,1 (Met-a) | O2.015: 880 mg; statt Thiosulfat-Lösung 5-%ige Zitronensäure-Lösung zugegeben ; Produkt: 327 mg |
| O1.016 | 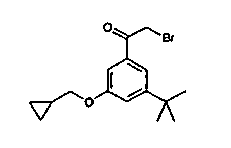 | 1,97 min | 325,1 (Met-a) | O2.016: 1,02 g; statt Thiosulfat-Lösung mit 5 -%iger Zitronensäure-Lösung 2 h gerührt ; Produkt: 1,23 g |
| O1.017 | 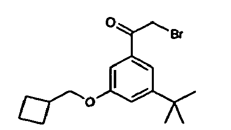 | 2,14 min | 339,2(Met-a) | O2.017: 25 mg; statt Thiosulfat-Lösung mit 5 -%iger Zitronensäure-Lösung 2 h gerührt ; Produkt: 314 mg |
| O1.018 | 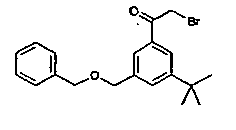 | 2,18 min | 389,3(Met-a) | O2.018: 638 mg; statt Thiosulfat-Lösung mit 5 -%iger Zitronensäure-Lösung 2 h gerührt ; Produkt: 809 mg |
| O1.019 | 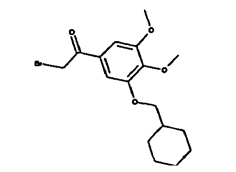 | 1,94 min | 371,3(Met-a) | O2.019: 360 mg; nach Thiosulfat-Lösung zusätzlich mit 20-%iger Zitronensäure-Lösung gewaschen; Produkt: 261 mg |
| O1.020 | 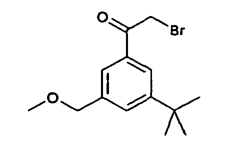 | 1,72 min | 299,1 (Met-a) | O2.020: 1,53 g; nach Thiosulfat-Lösung zusätzlich mit 20-%iger Zitronensäure-Lösung gewaschen; Produkt: 1,04 g |
| O1.021 | 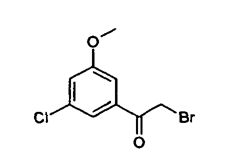 | 1,58 min | 262,9/ 265,0(Met-a) | O2.021: 41 g; nach Thiosulfat-Lösung zusätzlich mit 20-%iger Zitronensäure-Lösung gewaschen; Produkt: 572 mg |

### O1.022

### 2-Brom-1-(8-tert-butyl-4-methyl-3, 4-dihydro-2H-benzo[1,4]oxazin-6-yl)-ethanon

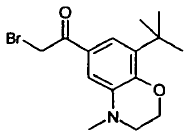

1-(8-tert-Butyl-4-methyl-3,4-dihydro-2H-benzo[1,4]oxazin-6-yl)-ethanon (250 mg, gekauft von Chembiotek, Indien) wurde in einer Mischung aus Essigsäure (4 ml) und Toluol (8 ml) auf 50 °C bis 55°C erwärmt. Bei dieser Temperatur wurde vorsichtig Brom (200 mg in Essigsäure gelöst) zugetropft. Nach 2,5 h wurde die Heizung entfernt, bei RT mit Eiswasser versetzt und drei Mal mit Toluol extrahiert. Die vereinigten organischen Phasen wurden über Natriumsulfat getrocknet, filtriert und eingeengt. Das Rohprodukt wurde über Kieselgel gereinigt, so dass 65 mg der gewünschten Verbindung erhalten wurden, neben weiteren 43 mg Produkt, die leicht verunreinigt waren und 37 mg Edukt.

| | |
|---|---|
| LCMS-rt: 1,81 min | [M+H]⁺: 326,0 (Met-a) |

### O1.030

### 2-Brom-1-(3-isopropyl-5-methoxy-phenyl)-ethanon

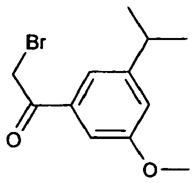

1-(3-Isopropyl-5-methoxy-phenyl)-ethanon (02.030; 425 mg) wurde in Methanol/THF (15ml/15 ml) gelöst und unter Rühren mit Phenyltrimethylammoniumtribromid (831 mg) versetzt. Nach 3 h Rühren bei RT wurde das Reaktionsgemisch auf 50 ml 20%iger Zitronensäure gegeben und 1 h gerührt. Nach Zugabe von Wasser und EE wurde die EE-Phase abgetrennt, getrocknet und eingeengt. Der Rückstand wurde in Acetonitril (50 ml) gelöst, und zur Lösung 2 N Schwefelsäure (15 ml) gegeben. Nach 2 h Stehen bei RT wurde mit Wasser versetzt und mit EE extrahiert. Die EE-Phase wurde mit gesättigter Natriumhydrogencarbonat-Lösung gewaschen, getrocknet und eingeengt. Der Rückstand wurde über Kieselgel (40 g Kartusche, n-Heptan/EE-Gradient von 0-50% in 30 min) aufgereinigt. Es wurden 520 mg der Titelverbindung erhalten. LCMS-rt: 1,65 min [M+H]⁺: 271,1 (Met-a)

### O1.031

### 2-Brom-1-(3-cyclohexylmethoxy-5-ethoxy-phenyl)-ethanon

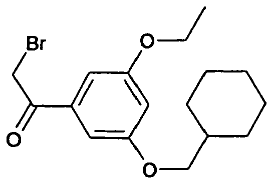

Ausgehend von 1-(3-Cyclohexylmethoxy-5-ethoxy-phenyl)-ethanon (02.031, 1,76 g) wurde die Titelverbindung analog O1.008 dargestellt. Zur weiteren Aufreinigung wurde aber nach der Kieselgel-Chromatographie eine weitere Reinigung mittels präparativer HPLC angeschlossen. Es wurden 370 mg der Titelverbindung erhalten.

| | |
|---|---|
| LCMS-rt: 2,11 min | [M+H]⁺: 355,1 (Met-a) |

### O1.032

### 2-Brom-1-(3-brom-5-methoxy-phenyl)-ethanon

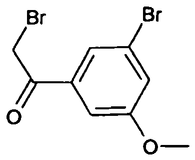

1-(3-Brom-5-methoxy-phenyl)-ethanon (02.032, 1,45 g) wurde in Methanol/THF (40 ml/40 ml) gelöst und unter Rühren mit Phenyltrimethylammoniumtribromid (2,38 g) versetzt. Nach 24 h Rühren bei RT wurden Wasser und EE zugegeben. Die EE-Phase wurde abgetrennt, getrocknet und eingeengt. Der Rückstand wurde in Acetonitril (50 ml) gelöst, und zur Lösung 2 N Schwefelsäure (15 ml) gegeben. Nach 1 h Stehen bei RT wurde mit Wasser versetzt und zweimal mit EE extrahiert. Die EE-Phase wurde mit gesättigter Natriumhydrogencarbonat-Lösung gewaschen, getrocknet und eingeengt. Der Rückstand wurde über Kieselgel (40 g Kartusche, n-Heptan/EE-Gradient von 0-50% in 30 min) aufgereinigt. Es wurden 1,39 g der Titelverbindung erhalten.
¹H-NMR (500 MHz, DMSO-d6) [ppm]: 7,71 (1 H), 7,48 (2 H), 4,97 (2 H), 3,84 (3 H)

### O1.033

### 2-Brom-1-[3-(3,3-dimethyl-butoxy)-5-methoxy-phenyl]-ethanon

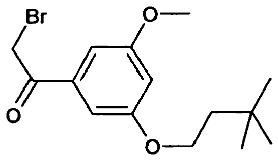

Ausgehend von 1-[3-(3,3-Dimethyl-butoxy)-5-methoxy-phenyl]-ethanon (1,11 g) wurde die Titelverbindung analog O1.031 dargestellt. Es wurden 127 mg der Titelverbindung erhalten.

| | |
|---|---|
| LCMS-rt: 1,89 min | [M+H]⁺: 329,1 (Met-a) |

### O1.034

### 2-Brom-1-[3-(3,3-dimethyl-butoxy)-5-ethoxy-phenyl]-ethanon

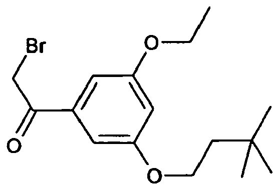

Ausgehend von 1-[3-(3,3-Dimethyl-butoxy)-5-ethoxy-phenyl]-ethanon (950 mg) wurde die Titelverbindung analog O1.031 dargestellt. Es wurden 236 mg der Titelverbindung erhalten, die immer noch etwas verunreinigt waren.

| | |
|---|---|
| LCMS-rt: 2,06 min | [M+H]⁺: 343,2 (Met-a) |

### O1.035

### 2-Brom-1-(3-cyclohexylmethoxy-5-methoxy-phenyl)-ethanon

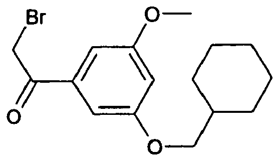

1-(3-Cyclohexylmethoxy-5-methoxy-phenyl)-ethanon (02.035; 1,33 g) wurde analog O1.032 umgesetzt. Dabei wurde allerdings statt 24 nur 3 h bei RT gerührt. Es wurden 950 mg der Titelverbindung erhalten.

| | |
|---|---|
| LCMS-rt: 2,03 min | [M+H]⁺: 341,2 (Met-a) |

### O1.040

### 2-Brom-1-(3-brom-4,5-dimethoxy-phenyl)-ethanon

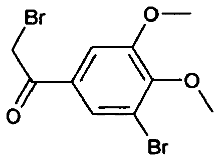

1-(3-Brom-4,5-dimethoxy-phenyl)-ethanon (02.040; 1,22 g) wurde analog O1.032 umgesetzt. Es wurden 1,05 g der Titelverbindung erhalten.
¹H-NMR (500 MHz, DMSO-d6) [ppm]: 7,86 (1 H), 7,58 (1 H), 4,96 (2 H), 3,90 (3 H), 3,82 (3 H)

### O1.041

### 2-Brom-1-(5-brom-2,3-dimethoxy-phenyl)-ethanon

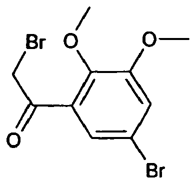

1-(5-Brom-2,3-dimethoxy-phenyl)-ethanon (02.041; 1,1 g) wurde analog O1.032 umgesetzt. Es wurden 1,21 g der Titelverbindung erhalten.

| | |
|---|---|
| LCMS-rt: 1,62 min | [M+H]⁺: 337,0 (Met-a) |

### O1.042

### 2-Brom-1-(3-chlor-4,5-dimethoxy-phenyl)-ethanon

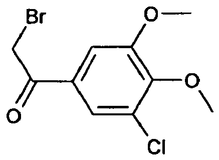

1-(3-Chlor-4,5-dimethoxy-phenyl)-ethanon (02.042; 490 mg) wurde analog O1.032 umgesetzt. Es wurden 577 mg der Titelverbindung erhalten.

| | |
|---|---|
| LCMS-rt: 1,54 min | [M+H]⁺: 293,0 (Met-a) |

### O1.043

### 2-Brom-1-[3-tert-butyl-5-(2-methoxy-ethoxy)-phenyl]-ethanon

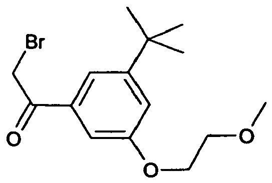

1-[3-tert-Butyl-5-(2-methoxy-ethoxy)-phenyl]-ethanon (02.043; 1 g) wurde in Methanol/THF (25 ml/25 ml) gelöst, unter Rühren mit Phenyltrimethylammoniumtribromid (882 mg) versetzt und über Nacht bei RT gerührt. Dann 50 ml 20 %ige Zitronensäure-Lösung zugegeben und 1 h gerührt. Anschließend wurde DCM (200 ml) zugegeben und dreimal mit DCM extrahiert. Dann wurden die vereinigten DCM-Phasen mit Magnesiumsulfat getrocknet, filtriert und eingeengt. Der Rückstand wurde über Kieselgel (40 g Kartusche, n-Heptan/EE-Gradient von 0-30% in 30 min) aufgereinigt. Es wurden 1,31 g der Titelverbindung erhalten.

| | |
|---|---|
| LCMS-rt: 1,72 min | [M+H]⁺: 329,2 (Met-a) |

### O1.044

### 2-Brom-1-[3-morpholin-4-yl-5-(pentafluorsulfanyl)-phenyl]-ethanon

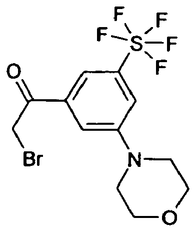

1-[3-Morpholin-4-yl-5-(pentafluorsulfanyl)-phenyl]-ethanon (02.044; 1,1 g) wurde in Methanol/THF (20/20 ml) gelöst und unter Rühren mit Phenyltrimethylammoniumtribromid (1,25 g) versetzt. Nach 27 h Rühren bei RT wurden 50 ml 20%ige Zitronensäure zugegeben und 1 h gerührt. Nach Zugabe von DCM (100 ml) wurde die DCM-Phase abgetrennt, getrocknet und eingeengt. Der Rückstand wurde in Acetonitril (100 ml) gelöst, und zur Lösung 2 N Schwefelsäure (20 ml) gegeben. Nach 24 h Rühren bei RT wurde mit Wasser versetzt und mit EE extrahiert. Die EE-Phase wurde mit gesättigter Natriumhydrogencarbonat-Lösung gewaschen, getrocknet und eingeengt. Der Rückstand wurde über Kieselgel (80 g Kartusche, n-Heptan/EE-Gradient von 0-60% in 40 min) aufgereinigt. Es wurden 866 mg der Titelverbindung erhalten.

| | |
|---|---|
| LCMS-rt: 1,69 min | [M+H]⁺: 410,0 (Met-a) |

### O1.045

### 2-Brom-1-[3-tert-butyl-5-(2-methoxy-ethoxymethyl)-phenyl]-ethanon

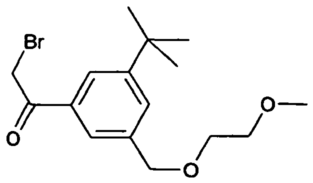

1-[3-tert-Butyl-5-(2-methoxy-ethoxymethyl)-phenyl]-ethanon (02.045, 508 mg) wurde analog O1.032 umgesetzt. Es wurden 470 mg der Titelverbindung erhalten.
¹H-NMR (500 MHz, DMSO-d6) [ppm]: 7,91 (1 H), 7,76 (1 H), 7,66 (1 H), 4,96 (2 H), 4,56 (2 H), 3,58 (2 H), 3,50 (2 H), 3,26 (3 H), 1,32 (9 H)

### O1.050

### 2-Brom-1-[3-(2-methoxy-ethoxy)-5-(pentafluorsulfanyl)-phenyl]-ethanon

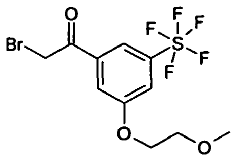

1-[3-(2-Methoxy-ethoxy)-5-(pentafluorsulfanyl)-phenyl]-ethanon (03.050, 578 mg) wurde analog O1.032 umgesetzt. Es wurden 290 mg der Titelverbindung erhalten.

| | |
|---|---|
| LCMS-rt: 1,70 min | [M+H]⁺: 399,0 (Met-a) |

### O1.051

### 2-Chlor-1-[3-(3-methoxy-propoxy)-5-(pentafluorsulfanyl)-phenyl]-ethanon

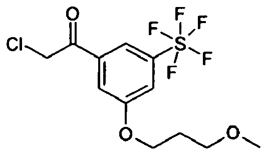

1-[3-(3-Methoxy-propoxy)-5-(pentafluorsulfanyl)-phenyl]-ethanon (02.051; 670 mg) wurde in Methanol/THF (10 ml/10 ml) gelöst und unter Rühren mit Phenyltrimethylammoniumtribromid (753 mg) versetzt. Nach 24 h Rühren bei RT wurden Wasser und EE zugegeben. Die EE-Phase wurde abgetrennt, getrocknet und eingeengt. Der Rückstand wurde in Acetonitril (50 ml) gelöst, und zur Lösung 2 N Salzsäure (15 ml) gegeben. Nach 1 h Rühren bei RT wurde mit Wasser versetzt und mit EE zweimal extrahiert. Die EE-Phase wurde getrocknet und eingeengt. Der Rückstand wurde über Kieselgel (40 g Kartusche, n-Heptan/EE-Gradient von 0-30% in 30 min) aufgereinigt. Es wurden 722 mg der Titelverbindung erhalten, die noch durch die analoge 2-Brom-Verbindung verunreinigt war.

| | |
|---|---|
| LCMS-rt: 1,11 min | [M+H]⁺: 369,0 (Met-b) |

| Nummer | LCMS-rt | LCMS-rt | [M+H]⁺ | Bemerkung: |
|---|---|---|---|---|
| O1.052 | 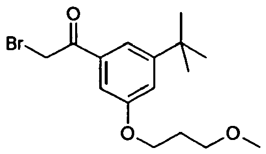 | 1,82 min | 343,1 (Met-a) | Synthese analog O1.013: O2.052: 880 mg; Chromatographie: 80 g Kartusche, n-Heptan /MTB-Ether 0-30% in 60 min;.Produkt: 629 mg |

### O1.059

### 2-Brom-1-[3-tert-butyl-5-(3-hydroxy-propoxy)-phenyl]-ethanon

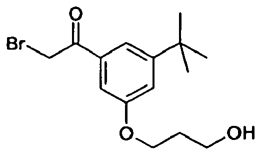

1-{3-tert-Butyl-5-[3-(tetrahydro-pyran-2-ylooy)-propoxy]-phenyl}-ethanon (02.059; 3,4 g) wurde in Methanol/THF (50/50 ml) gelöst und unter Rühren mit Phenyltrimethylammoniumtribromid (4,21 g) versetzt. Nach 3 h Rühren bei RT wurde 20%ige Zitronensäure-Lösung zugegeben und 1 h gerührt. Dann wurde EE zugegeben und die EE-Phase abgetrennt. Die EE-Phase wurde über Magnesiumsulfat getrocknet und einrotiert. Der Rückstand wurde über Kieselgel (80 g Kartusche, n-Heptan/EE-Gradient von 0-50% in 60 min) aufgereinigt. Es wurden 2,49 g der Titelverbindung erhalten.

| | |
|---|---|
| LCMS-rt: 2,57 min | [M+H]⁺: 329,1 (Met-e) |

### O1.060

### 1-[3-tert-Butyl-5-(3-hydroxy-propoxymethyl)-phenyl]-2-chlor-ethanon

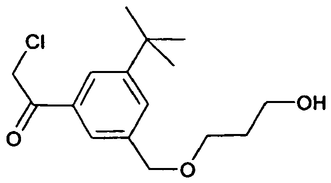

1-{3-tert-Butyl-5-[3-(tetrahydro-pyran-2-yloxy)-propoxymethyl]-phenyl}-ethanon (02.060; 1,75 g) wurde analog O1.063 umgesetzt. Es wurden 455 mg der Titelverbindung erhalten. LCMS-rt: 1,47 min [M+H]⁺: 299,1 (Met-a)

### O1.061

### 2-Brom-1-[3-tert-butyl-5-(2-hydroxy-ethoxy)-phenyl]-ethanon

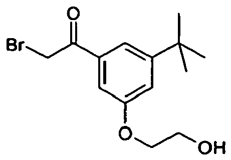

1-{3-tert-Butyl-5-[2-(tetrahydro-pyran-2-yloxy)-ethoxy]-phenyl}-ethanon (02.061; 3,9 g) wurde in Methanot/THF (60 ml/60 ml) gelöst und unter Rühren mit Phenyltrimethylammoniumtribromid (5,03 g) versetzt. Nach 3 h Rühren bei RT wurde das Reaktionsgemisch auf 20%ige Zitronensäure gegeben und 1 h gerührt. Nach Zugabe EE wurde die EE-Phase abgetrennt, getrocknet und eingeengt. Der Rückstand wurde über Kieselgel (80 g Kartusche, n-Heptan/EE-Gradient von 0-50% in 60 min) aufgereinigt. Es wurden 2,56 g der Titelverbindung erhalten.

| | |
|---|---|
| LCMS-rt: 0,90 min | [M+H]⁺: 315,0 (Met-b) |

### O1.062

### 2-Brom-1-[3-ethoxy-5-(pentafluorsulfanyl)-phenyl]-ethanon

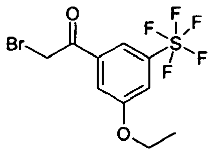

1-[3-Ethoxy-5-(pentafluorsulfanyl)-phenyl]-ethanon (02.062; 2,24 g) wurde analog O1.008 umgesetzt. Die Aufreinigung über Kieselgel wurde wie folgt durchgeführt: (120 g Kartusche, n-Heptan/MtB-Ether-Gradient von 0-20% in 60 min). Es wurden 2,07 g der Titelverbindung erhalten.

| | |
|---|---|
| LCMS-rt: 1,83 min | [M+H]⁺: 368,9 (Met-a) |

### O1.063

### 1-[3-tert-Butyl-5-(2-hydroxy-ethoxymethyl)-phenyl]-2-chlor-ethanon

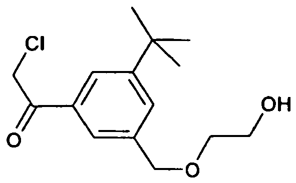

1-{3-tert-Butyl-5-[2-(tetrahydro-pyran-2-yloxy)-ethoxymethyl]-phenyl}-ethanon (02.063; 2,0 g) wurde in Methanol/THF (30 ml/30 ml) gelöst und unter Rühren mit Phenyltrimethylammoniumtribromid (2,25 g) versetzt. Nach 3 h Rühren bei RT wurde mit DCM verdünnt, einmal mit 5%iger Natriumthiosulfat-Lösung gewaschen, die DCM-Phase über Magnesiumsulfat getrocknet und einrotiert. Der Rückstand wurde in THF (200 ml) gelöst und 3 N HCl (30 ml) zugegeben. Nach 1 h Stehen wurden EE und Wasser zugegeben und die EE-Phase abgetrennt. Die EE-Phase wurde über Magnesiumsulfat getrocknet und einrotiert. Der Rückstand wurde über Kieselgel (40 g Kartusche, n-Heptan/EE-Gradient von 0-50% in 40 min) aufgereinigt. Es wurden 774 mg der Titelverbindung erhalten.
¹H-NMR (500 MHz, DMSO-d6) [ppm]: 7,87 (1 H), 7,76 (1 H), 7,67 (1 H), 5,21 (2 H), 4,66 (1 H), 4,56 (2 H), 3,56 (2 H), 3,49 (2 H), 1,32 (9 H)

### O1.064

### 2-Brom-1-[3-ethoxy-5-(pentafluorsulfanyl)-phenyl]-propan-1-on

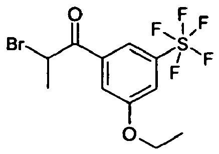

1-[3-Ethoxy-5-(pentafluorsulfanyl)-phenyl]-propan-1-on (02.064; 150 mg) wurde in THF (15 ml) gelöst und unter Rühren bei RT Phenyltrimethylammoniumtribromid (185 mg) zugegeben. Nach 5 h Rühren bei RT wurde mit Wasser und gesättigter Natriumhydrogencarbonat-Lösung versetzt und EE zugegeben. Die EE-Phase wurde abgetrennt und die alkalische Wasserphase dreimal mit EE extrahiert. Die vereinigten organischen Phasen wurden einmal mit gesättigter Kochsalz-Lösung gewaschen, über Magnesiumsulfat getrocknet, filtriert und eingeengt. Das Rohprodukt wurde mittels präparativer HPLC (Met-A) gereinigt. Die jeweils sauberen Produktfraktionen wurden vereinigt, im Vakuum vom Acetonitril befreit, mit gesättigter Natriumhydrogencarbonat-Lösung alkalisch gestellt und fünfmal mit EE extrahiert. Die vereinigten organischen Phasen wurden über Magnesiumsulfat getrocknet und nach Abfiltrieren des Trockenmittels im Vakuum zur Trockne gebracht. Es wurden 120 mg der Titelverbindung isoliert.

| | |
|---|---|
| LCMS-rt: 1,29 min | [M+H]⁺: 383,0 (Met-b) |

| Nummer | | LCMS-rt | [M+H]⁺ | Bemerkung: |
|---|---|---|---|---|
| O1.070 | 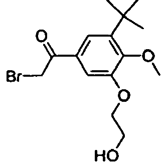 | 0,96 min | 345,1 (Met-b) | Synthese analog O1.071: O2.070: 6,68 g; Produkt: 3,3 g |

### O1.071

### 2-Brom-1-[3-tert-butyl-5-(3-hydroxy-propoxy)-4-methoxy-phenyl]-ethanon

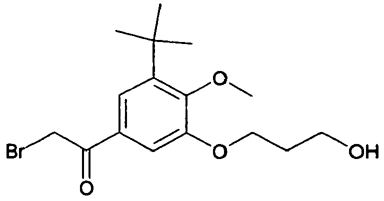

1-{3-tert-Butyl-4-methoxy-5-[3-(tetrahydro-pyran-2-yloxy)-propoxy]-phenyl}-ethanon (02.071; 12,7 g) wurde in Methanol/THF (200/200 ml) gelöst und unter Rühren mit Phenyltrimethylammoniumtribromid (13,1 g) versetzt. Bei RT wurde 1 h gerührt, dann mit DCM verdünnt und einmal mit 5%iger Natriumthiosulfat-Lösung gewaschen. Die DCM-Phase wurde über Magnesiumsulfat getrocknet, filtriert und eingeengt. Der Rückstand wurde in Acetonitril (100 ml) aufgenommen und mit 48%iger Bromwasserstoffsäure (5,91 ml) versetzt. Es wurde 1 h stehengelassen, dann mit Wasser versetzt, mit EE ausgeschüttelt, die vereinigten EE-Phasen über Magnesiumsulfat getrocknet, filtriert und eingeengt. Der Rückstand wurde über Kieselgel (120 g Kartusche, n-Heptan/MtB-Ether-Gradient von 0-100% in 60 min) aufgereinigt. Es wurden 3,29 g der Titelverbindung erhalten.

| | |
|---|---|
| LCMS-rt: 1,01 min | [M+H]⁺: 359,1 (Met-b) |

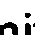

### O1.072

### 2-Brom-1-[3-tert-butyl-5-(2-methoxy-1-methoxymethyl-ethoxy)-phenyl]-ethanon

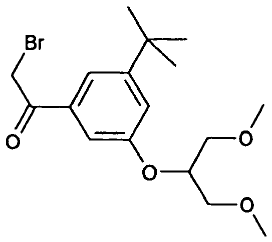

1-[3-tert-Butyl-5-(2-methoxy-1-methoxymethyl-ethoxy)-phenyl]-ethanon (02.072; 1,64 g) wurde in Methanol/THF (30/30 ml) gelöst und unter Rühren mit Phenyltrimethylammoniumtribromid (2,09 g) versetzt. Bei RT wurde 1 h gerührt, dann mit DCM verdünnt und einmal mit 5%iger Natriumthiosulfat-Lösung gewaschen. Die DCM-Phase wurde über Magnesiumsulfat getrocknet, filtriert und eingeengt. Der Rückstand wurde über Kieselgel (80 g Kartusche, n-Heptan/MtB-Ether-Gradient von 0-30% in 60 min) aufgereinigt. Es wurden 890 mg der Titelverbindung erhalten.

| | |
|---|---|
| LCMS-rt: 1,77 min | [M+H]⁺: 373,0 (Met-a) |

### O1.073

### 2-Brom-1-[3-tert-butyl-5-(4-hydroxy-butoxy)-phenyl]-ethanon

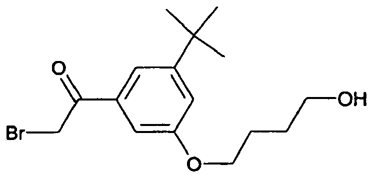

1-[3-tert-Butyl-5-(4-hydroxy-butoxy)-phenyl]-ethanon (02.073; 2,08 g) wurde analog O1.043 umgesetzt und aufgearbeitet. Die Aufreinigung über Kieselgel wurde wie folgt durchgeführt: (80 g Kartusche, n-Heptan/EE-Gradient von 0-100% in 60 min). Es wurden 1,8 g der Titelverbindung erhalten.

| | |
|---|---|
| LCMS-rt: 1,01 min | [M+H]⁺: 343,0 (Met-b) |

### O1.075

### N-[3-(2-Brom-acetyl)-5-(pentafluorsulfanyl)-phenyl]-2,2,2-trifluor-N-methyl-acetamid

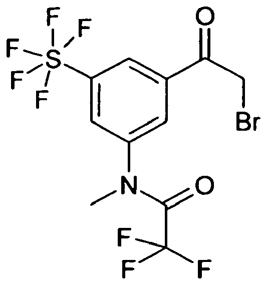

N-[3-Acetyl-5-(pentafluorsulfanyl)-phenyl]-2,2,2-trifluor-N-methyl-acetamid (02.075; 1,03 g) wurde in einem Gemisch aus Methanol (20 ml) und THF (20 ml) gelöst. Unter Rühren wurde Phenyl-trimethylammoniumtribromid (1,05 g) zugegeben. Nach 5 h Rühren bei RT wurde über Nacht stehen gelassen, dann weiteres Phenyl-trimethylammoniumtribromid (100 mg) zugegeben und 2 h auf 60°C erwärmt. Nach Erkalten wurde das Reaktionsgemisch in 2 N Schwefelsäure gegeben und 10 min gerührt. Dann wurde die wässrige Phase dreimal mit EE extrahiert. Die vereinigten organischen Phasen wurden über Magnesiumsulfat getrocknet und nach Abfiltrieren des Trockenmittels im Vakuum zur Trockne gebracht. Es wurden 1,2 g der Titelverbindung erhalten, die ausreichende Reinheit für die nächsten Umsetzungen hatte. LCMS-rt: 1,72 min [M+H]⁺: 449,9 (Met-a)

### O1.085

### 2-Brom-1-[3-tert-butyl-5-(3-hydroxy-2,2-dimethyl-propoxy)-4-methoxy-phenyl]-ethanon

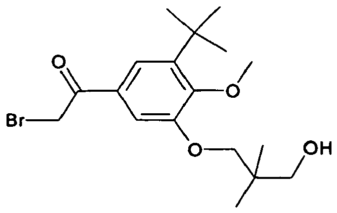

Essigsäure-3-(5-acetyl-3-tert-butyl-2-methoxy-phenoxy)-2,2-dimethyl-propylester (02.085; 1,16 g) wurde analog O1.008 umgesetzt und aufgearbeitet. Die Aufreinigung über Kieselgel wurde wie folgt durchgeführt: (80 g Kartusche, n-Heptan/MtB-Ether-Gradient von 0-70% in 60 min). Es wurden 867 mg der Titelverbindung erhalten.

| | |
|---|---|
| LCMS-rt: 1,80 min | [M+H]⁺: 387,1 (Met-a) |

### O1.086

### 2-Brom-1-[3-tert-butyl-5-(3-hydroxy-2,2-dimethyl-propoxy)-phenyl]-ethanon

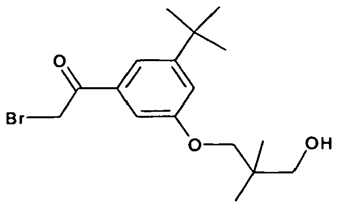

1-[3-tert-Butyl-5-(3-hydroxy-2,2-dimethyl-propoxy)-phenyl]-ethanon (02.086; 388 mg) wurde analog O1.085 umgesetzt, aufgearbeitet und gereinigt. Es wurden 311 mg der Titelverbindung erhalten. LCMS-rt: 1,78 min [M+H]⁺: 357,0 (Met-a)

### O1.090

### 2-Brom-1-[3-(2-hydroxy-ethoxy)-5-(pentafluorsulfanyl)-phenyl]-ethanon

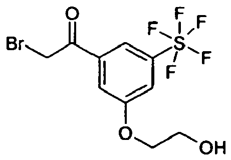

1-{3-(Pentafluorsulfanyl)-5-[2-(tetrahydro-pyran-2-yloxy)-ethoxy]-phenyl}-ethanon (02.090; 813 mg) wurde analog O1.071 umgesetzt, aufgearbeitet und gereinigt. Allerdings betrug die Reaktionszeit drei statt einer Stunde. Es wurden 174 mg der Titelverbindung erhalten. LCMS-rt: 0,94 min [M+H]⁺: 385,0 (Met-b)

### O1.095

### Essigsäure-4-[5-(2-brom-acetyl)-3-tert-butyl-2-methoxy-phenoxy]-butyl ester

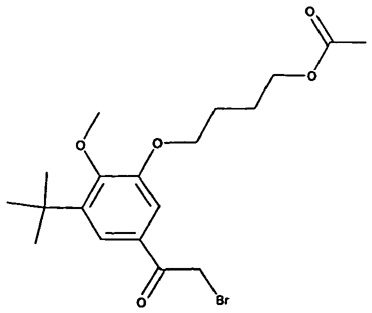

Essigsäure-4-(5-acetyl-3-tert-butyl-2-methoxy-phenoxy)-butylester (02.095; 10,51 g) wurde analog O1.071 umgesetzt, aufgearbeitet und gereinigt. Es wurden 4,81 g der Titelverbindung erhalten. LCMS-rt: 1,21 min [M+H]⁺: 415,1 (Met-b)

### 01.100

### 2-Brom-1-[3-tert-butyl-5-(1-hydroxy-1-methyl-ethyl)-phenyl]-ethanon

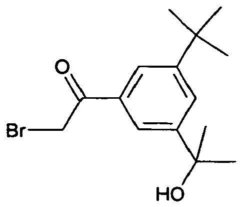

1-[3-tert-Butyl-5-(1-hydroxy-1-methyl-ethyl)-phenyl]-ethanon (02.100; 50 mg) wurde in THF (5 ml) unter Rühren bei RT vorgelegt und mit Phenyltrimethylammoniumtribromid (80 mg) versetzt. Nach 3 h Rühren bei RT ließ man über Nacht stehen und versetzte mit Wasser sowie gesättigter Natriumhydrogencarbonat-Lösung. Dann extrahierte man dreimal mit EE, trocknete die vereinigten EE-Phasen über Natriumsulfat, filtrierte und engte ein. Der Rückstand wurde über Kieselgel (10 g Kartusche, n-Heptan/EE-Gradient) aufgereinigt. Es wurden 42 mg der Titelverbindung erhalten. LCMS-rt: 1,10 min [M+H-H₂O]⁺: 295,1 (Met-b)

### 01.101

### 2-Brom-1-[3-tert-butyl-5-(1-methoxy-1-methyl-ethyl)-phenyl]-ethanon

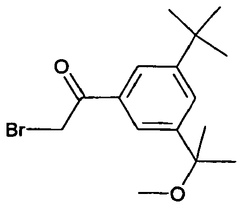

1-[3-tert-Butyl-5-(1-hydroxy-1-methyl-ethyl)-phenyl]-ethanon (02.100; 500 mg) wurde in einer Mischung aus THF und Methanol (3,5/3,5 ml) unter Rühren bei RT vorgelegt und mit Phenyltrimethylammoniumtribromid (800 mg) versetzt. Nach 7 h Rühren bei RT wurde mit Wasser und gesättigter Natriumhydrogencarbonat-Lösung versetzt. Dann extrahierte man dreimal mit EE, trocknete die vereinigten EE-Phasen über Natriumsulfat, filtrierte und engte ein. Der Rückstand wurde über Kieselgel (70 g Kartusche, n-Heptan/EE-Gradient) aufgereinigt. Es wurden 454 mg der Titelverbindung erhalten. LCMS-rt: 1,23 min [M+H-HOMe]⁺: 295,1 (Met-b)

### 01.105

### 2-Brom-1-[3-tert-butyl-5-(2-fluor-ethoxy)-phenyl]-ethanon ZSI2.063

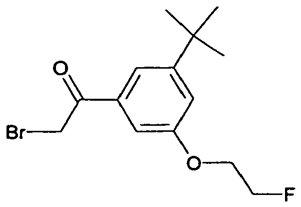

1-[3-tert-Butyl-5-(2-fluor-ethoxy)-phenyl]-ethanon (02.105; 600 mg) wurde in Methanol/THF (15/15 ml) gelöst. Unter Rühren wurde Phenyltrimethylammoniumtribromid (950 mg) zugegeben. Nach 3 h Rühren bei RT wurde Wasser (5 ml) zugegeben und zur Trockne gebracht. Der Rückstand wurde mit DCM (200 ml) und gesättigter Natriumhydrogencarbonat-Lösung (100 ml) versetzt. Die DCM-Phase wurde abgetrennt und die wässrige Phase noch dreimal mit DCM extrahiert. Die vereinigten DCM-Phasen wurden über Natriumsulfat getrocknet, filtriert und eingeengt. Der Rückstand wurde über Kieselgel (50 g Kartusche, n-Heptan/EE-Gradient von 0-60% in 40 min) aufgereinigt. Es wurden 560 mg der Titelverbindung erhalten. LCMS-rt: 1,19 min [M+H]⁺: 317,1 (Met-b)

### 01.106

### 2-Brom-1-[3-tert-butyl-5-(3-fluorpropoxy)-phenyl]-ethanon

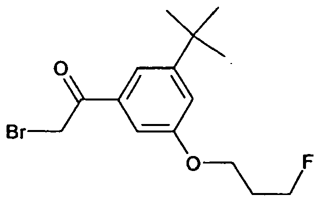

1-[3-tert-Butyl-5-(3-fluorpropoxy)-phenyl]-ethanon (630 mg) wurde analog 01.105 umgesetzt, aufgearbeitet und gereinigt. Es wurden 510 mg der Titelverbindung erhalten. LCMS-rt: 1,23 min [M+H]⁺: 331,1 (Met-b)

### 01.107

### 2-Brom-1-[3-tert-butyl-5-(3,3,3-trifluorpropoxy)-phenyl]-ethanon

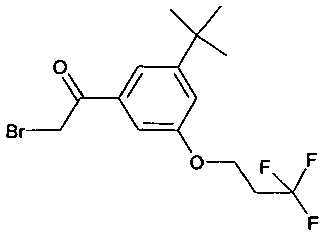

1-[3-tert-Butyl-5-(3,3,3-trifluor-propoxy)-phenyl]-ethanon (02.107; 170 mg, 0.59 mmol) wurde in einem Gemisch von THF (3 ml) und Methanol (3 ml) gelöst. Unter Rühren wurde Phenyltrimethylammoniumtribromid (222 mg, 0.59 mmol) eingetragen und über Nacht gerührt. Die Reaktionsmischung wurde mit Essigester verdünnt und mit gesättigter Natriumhydrogencarbonat-Lösung gewaschen. Nach Trocknung über Natriumsulfat wurde das Lösungsmittel entfernt und der Rückstand durch Flash-Chromatographie (n-Heptan:Essigester) gereinigt. Ausbeute: 42 mg,

| | |
|---|---|
| LCMS-rt: 1,19 min | [M+H]⁺: 367,1 (Met-b) |

### 01.110

### 2-Brom-1-[3-tert-butyl-4-methoxy-5-(2-methoxy-ethoxy)-phenyl]-ethanon

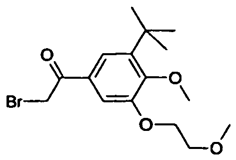

1-[3-tert-Butyl-4-methoxy-5-(2-methoxy-ethoxy)-phenyl]-ethanon (02.110; 1,17 g) wurde analog O1.008 umgesetzt, aufgearbeitet und gereinigt. Es wurden 1,18 g der Titelverbindung erhalten. LCMS-rt: 1,10 min [M+H]⁺: 359,1 (Met-b)

### 01.111

### 2-Brom-1-[3-tert-butyl-4-methoxy-5-(3-methoxy-propoxy)-phenyl]-ethanon

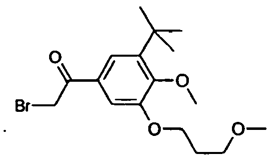

1-[3-tert-Butyl-4-methoxy-5-(3-methoxy-propoxy)-phenyl]-ethanon (02.111; 1,49 g) wurde analog O1.008 umgesetzt, aufgearbeitet und gereinigt. Es wurden 1,65 g der Titelverbindung erhalten. LCMS-rt: 1,16 min [M+H]⁺: 373,0 (Met-b)

### 02.

### 02.004

### 4-[5-(1,1-Dimethoxy-ethyl)-2-methoxy-3-trifluormethyl-phenyl]-morpholin

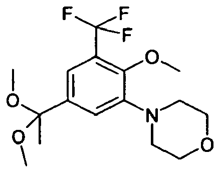

1-Brom-5-(1,1-dimethoxy-ethyl)-2-methoxy-3-trifluormethyl-benzol (03.004; 700 mg) wurde in Dioxan (7 ml) vorgelegt und nacheinander mit Pd(II)-acetat (46 mg), Cäsiumcarbonat (2 g), 9,9-Dimethyl-4,5-bis-(diphenylphosphino)-xanthen (118 mg) sowie Morpholin (0,27 ml) versetzt. Danach wurde das Reaktionsgemisch 7 h auf 90°C erwärmt und dann über Nacht stehen gelassen. Anschließend wurde filtriert und das Filtrat einrotiert. Der Rückstand wurde über Kieselgel (50 g Kartusche, n-Heptan/EE-Gradient) aufgereinigt. Es wurden 433 mg der Titelverbindung erhalten.

| | |
|---|---|
| LCMS-rt: 1,55 min | [M+H]⁺: 304,0 (Met-a) |

### 02.007

### 1-[3-Methoxy-5-(pentafluorsulfanyl)-phenyl]-ethanon

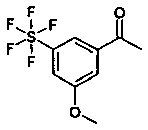

3,N-Dimethoxy-N-methyl-5-(pentafluorsulfanyl)-benzamid (03.007; 2,0 g) wurde in absolutem THF (60 ml) gelöst und bei 0°C unter Rühren Methylmagnesiumbromid (5,2 ml, 3 M in Diethylether) zugetropft. Nach Zugabe wurde das Eisbad entfernt und 2 h bei RT gerührt. Unter Kühlung wurde dann 1 N Salzsäure zugetropft, gefolgt von Wasser und Essigester. Die organische Phase wurde abgetrennt und die wässrige noch 2 Mal mit Essigester extrahiert. Die vereinigten Extrakte wurden über Magnesiumsulfat getrocknet, filtriert und eingeengt. Der Rückstand wurde mittels präparativer HPLC (Met-A) gereinigt. Die Produkt enthaltenden Fraktionen wurden vereinigt, vom Acetonitril befreit und drei Mal mit Essigester extrahiert. Die vereinigten Extrakte wurden über Magnesiumsulfat getrocknet, filtriert und eingeengt. Es wurden 1,63 g der gewünschten Verbindung erhalten.
¹H-NMR (400 MHz, DMSO-d6) [ppm]: 7,87 (1 H), 7,75 (1 H), 7,67 (1 H), 3,91 (3 H), 2,64 (3 H)

### 02.008

### 1-(3-tert-Butyl-5-ethoxymethyl-phenyl)-ethanon

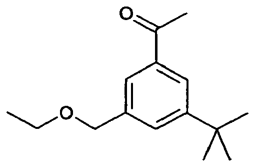

3-tert-Butyl-N-methoxy-5-methoxymethyl-N-methyl-benzamid (03.008; 854 mg) wurde analog 02.043 umgesetzt. Es wurden 550 mg der Titelverbindung erhalten.

| | |
|---|---|
| LCMS-rt: 1,70 min | [M+H]⁺: 235,3 (Met-a) |

### 02.009

### 1-(3-tert-Butyl-5-cyclopropylmethoxymethyl-phenyl)-ethanon

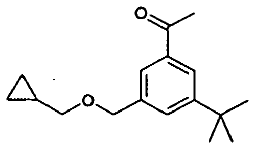

Ausgehend von 3-tert-Butyl-5-hydroxymethyl-benzoesäure-methylester und Cyclopropylmethylbromid wurde die Titelverbindung (600 mg) analog 05.008 bis 02.008 dargestellt. LCMS-rt: 1,81 min [M+H]⁺: 261,2 (Met-a)

### 02.010

### 1-(3-tert-Butyl-4,5-diethoxy-phenyl)-ethanon

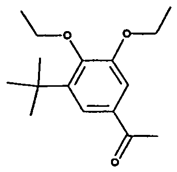

1-(3-tert-Butyl-4-ethoxy-5-hydroxy-phenyl)-ethanon (03.010; 470 mg) und Ethyliodid (193 µl) wurden in DMF (6,2 ml) gelöst und Natriumhydrid (57 mg) zugesetzt. Nach 0,5 h Rühren bei RT wurde das DMF abgezogen und der Rückstand in EE aufgenommen, mit Wasser gewaschen, getrocknet und eingeengt. Der Rückstand wurde über Kieselgel (40 g Kartusche, n-Heptan/EE-Gradient 0-30 % in 60 min) aufgereinigt. Es wurden 420 mg der Titelverbindung erhalten.

| | |
|---|---|
| LCMS-rt: 1,93 min | [M+H]⁺: 265,2 (Met-a) |

### 02.011

### 1-(3-tert-Butyl-5-ethoxy-phenyl)-ethanon

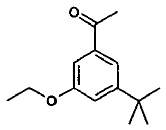

3-tert-Butyl-5-hydroxy-benzoesäuremethylester (06.043) wurde analog 05.043 mit Ethyliodid umgesetzt und analog der Sequenz 04.043 bis 02.043 in die Titelverbindung übergeführt. Es wurden 390 mg erhalten.

| | |
|---|---|
| LCMS-rt: 1,72 min | [M+H]⁺: 221,3 (Met-a) |

### 02.012

### 1-(3-tert-Butyl-5-propoxymethyl-phenyl)-ethanon

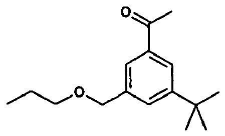

Ausgehend von 3-tert-Butyl-5-hydroxymethyl-benzoesäure-methylester und Propyliodid wurde die Titelverbindung (333 mg) analog 05.008 bis 02.008 dargestellt.

| | |
|---|---|
| LCMS-rt: 1,81 min | [M+H]⁺: 261,2 (Met-a) |

### 02.013

### 1-(3-tert-Butyl-4,5-bis-cyclopropylmethoxy-phenyl)-ethanon

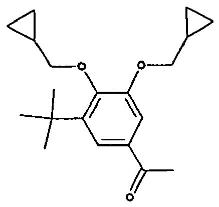

Ausgehend von 2-Brom-6-tert-butyl-4-(1,1-dimethoxy-ethyl)-phenol und Cyclopropylbromid wurde die Titelverbindung (547 mg) analog 04.010 bis 02.010 dargestellt. LCMS-rt: 2,10 min [M+H]⁺: 317,4 (Met-a)

### 02.014

### 1-(3-Methoxy-5-trifluormethyl-phenyl)-ethanon

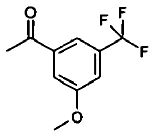

3,N-Dimethoxy-N-methyl-5-trifluormethyl-benzamid (03.014, 460 mg) wurde in THF (15 ml) vorgelegt unter bei RT Ar gelöst. Danach kühlte man auf 0°C ab und tropfte Methylmagnesiumbromid (1,5 ml; 3 M in Diethylether) zu. Anschließend wurde das Eisbad entfernt und 2 h bei RT gerührt. Dann wurde unter Eiskühlung mit 1 N Salzsäure versetzt, mit Wasser verdünnt und dreimal mit EE extrahiert. Die vereinigten EE-Phasen trocknete man über Natriumsulfat, filtrierte und engte ein. Es wurden 349 mg der Titelverbindung isoliert.
¹H-NMR (500 MHz, DMSO-d6) [ppm]: 7,80 (1 H), 7,73 (1 H), 7,53 (1 H), 3,92 (3 H), 2,66 (3 H)

### 02.015

### 1-(3-tert-Butyl-5-methoxy-phenyl)-ethanon

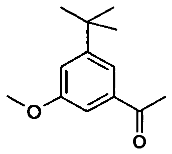

3-tert-Butyl-5-hydroxy-benzoesäuremethylester (06.043) wurde analog 05.043 mit Methyliodid umgesetzt und analog der Sequenz 04.043 bis 02.043 in die Titelverbindung übergeführt. Es wurden 880 mg erhalten.

| | |
|---|---|
| LCMS-rt: 1,65 min | [M+H]⁺: 207,1 (Met-a) |

### 02.016

### 1-(3-tert-Butyl-5-cyclopropylmethoxy-phenyl)-ethanon

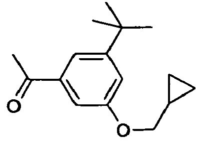

3-tert-Butyl-5-hydroxy-benzoesäuremethylester (06.043) wurde analog 05.043 mit Cyclopropylmethylbromid umgesetzt und analog der Sequenz 04.043 bis 02.043 in die Titelverbindung übergeführt. Es wurden 1,02 g erhalten.

| | |
|---|---|
| LCMS-rt: 1,86 min | [M+H]⁺: 247,1 (Met-a) |

### 02.017

### 1-(3-tert-Butyl-5-cyclobutylmethoxy-phenyl)-ethanon

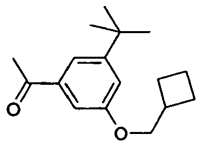

3-tert-Butyl-5-hydroxy-benzoesäuremethylester (06.043) wurde analog 05.043 mit Brommethylcyclobutan umgesetzt und analog der Sequenz 04.043 bis 02.043 in die Titelverbindung übergeführt. Es wurden 252 mg erhalten.

| | |
|---|---|
| LCMS-rt: 2,07 min | [M+H]⁺: 261,2 (Met-a) |

### 02.018

### 1-(3-Benzyloxymethyl-5-tert-butyl-phenyl)-ethanon

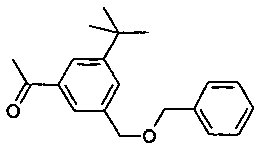

3-tert-Butyl-5-hydroxymethyl-benzoesäuremethylester wurde analog 05.043 mit Benzylbromid umgesetzt und analog der Sequenz 04.008 bis 02.008 in die Titelverbindung übergeführt. Es wurden 638 mg erhalten.

| | |
|---|---|
| LCMS-rt: 1,93 min | [M+H]⁺: 297,2 (Met-a) |

### 02.019

### 1-(3-Cyclohexylmethoxy-4,5-dimethoxy-phenyl)-ethanon

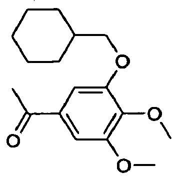

3-Cyclohexylmethoxy-4,5,N-trimethoxy-N-methyl-benzamid (420 mg) wurde analog 02.059 umgesetzt und aufgearbeitet. Eine Kieslgelreinigung wurde nicht duchgeführt. Es wurden 370 mg erhalten. LCMS-rt: 1,82 min [M+H]⁺: 293,2 (Met-a)

### 02.020

### 1-(3-tert-Butyl-5-methoxymethyl-phenyl)-ethanon

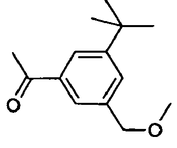

3-tert-Butyl-5-hydroxymethyl-benzoesäuremethylester wurde analog O5.043 mit Methyliodid umgesetzt und analog der Sequenz 04.008 bis 02.008 in die Titelverbindung übergeführt. Es wurden 1,54 g erhalten.

| | |
|---|---|
| LCMS-rt: 1,58 min | [M+H]⁺: 221,1 (Met-a) |

### 02.021

### 1-(3-Chlor-5-methoxy-phenyl)-ethanon

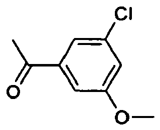

3-Chlor-5-methoxy-benzoesäure (3 g) wurde analog O3.043/O2.043 mit Thionylchlorid (23,3 ml), und N,O-Dimethylhydroxylamin-Hydrochlorid (1,57 g) und Methylmagnesiumbromid (8,91 ml) umgesetzt. Es wurden 2,42 g erhalten.
¹H-NMR (500 MHz, DMSO-d6) [ppm]: 7,54 (1 H), 7,40 (1 H), 7,30 (1 H), 3,84 (3 H), 2,58 (3 H)

### 02.030

### 1-(3-Isopropyl-5-methoxy-phenyl)-ethanon

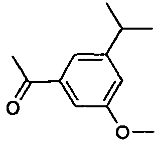

3-Hydroxy-5-isopropyl-benzoesäuremethylester wurde analog 05.043 mit Methyliodid umgesetzt und analog der Sequenz 04.043 bis 02.043 in die Titelverbindung übergeführt. Es wurden 425 mg erhalten.

| | |
|---|---|
| LCMS-rt: 1,54 min | [M+H]⁺: 193,1 (Met-a) |

### 02.031

### 1-(3-Cyclohexylmethoxy-5-ethoxy-phenyl)-ethanon

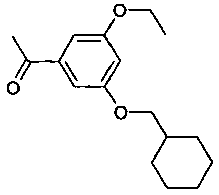

1-(3,5-Dihydroxy-phenyl)-ethanon (1,0 g) und Ethylbromid (0,531 ml) wurden bei RT in DMF (20 ml) gelöst und Natriumhydrid (189 mg) zugesetzt. Nach 2 h Rühren bei 50°C wurde Cyclohexylmethylbromid (1,36 ml) zugegeben, gefolgt von weiterem Natriumhydrid (315 mg). Nach erneuten 2 h Rühren bei 50 °C wurde das DMF abgezogen und der Rückstand in EE aufgenommen, mit Wasser gewaschen, getrocknet, filtriert und eingeengt. Der Rückstand wurde über Kieselgel (80 g Kartusche, n-Heptan/EE-Gradient von 0-20% in 60 min) aufgereinigt. Es wurden 378 mg der Titelverbindung erhalten. LCMS-rt: 2,07 min [M+H]⁺: 277,2 (Met-a)

### 02.032

### 1-(3-Brom-5-methoxy-phenyl)-ethanon

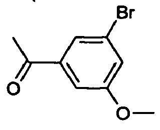

3-Brom-5-methoxy-benzoesäure-methylester (05.032; 2,50 g) wurde analog der Sequenz 04.043, 03.043 und 02.059 in die Titelverbindung übergeführt. Es wurden 1,45 g erhalten. LCMS-rt: 1,46 min [M+H]⁺: 229,0 (Met-a)

### 02.033

### 1-[3-(3,3-Dimethyl-butoxy)-5-methoxy-phenyl]-ethanon

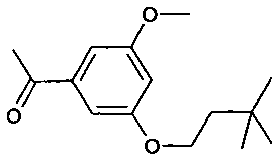

1-(3-Hydroxy-5-methoxy-phenyl)-ethanon (03.033; 1,12 g) und 1-lod-3,3-dimethylbutan (1,57 g) wurden in DMF gelöst und Natriumhydrid (194 mg) zugesetzt. Nach 2 h rühren bei RT wurde das DMF abgezogen. Der Rückstand wurde in EE aufgenommen, mit Wasser gewaschen, getrocknet, filtriert und eingeengt. Der Rückstand wurde über Kieselgel (40 g Kartusche, n-Heptan/EE 0-50% in 30 min) gereinigt. Es wurden 850 mg der Titelverbindung erhalten.

| | |
|---|---|
| LCMS-rt: 1,83 min | [M+H]⁺: 251,1 (Met-a) |

### 02.034

### 1-[3-(3,3-Dimethyl-butoxy)-5-ethoxy-phenyl]-ethanon

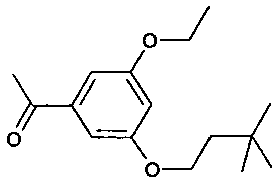

1-(3,5-Dihydroxy-phenyl)-ethanon (3,0 g) wurden analog 02.031 umgesetzt. Statt Cyclohexylbromid wurde allerdings 1-Brom-3,3-dimethyl-butan eingesetzt. Nach Chromatographie wurden 960 mg der Titelverbindung erhalten.

| | |
|---|---|
| LCMS-rt: 1,99 min | [M+H]⁺: 265,2 (Met-a) |

### 02.035

### 1-(3-Cyclohexylmethoxy-5-methoxy-phenyl)-ethanon

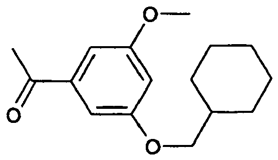

1-(3-Hydroxy-5-methoxy-phenyl)-ethanon (03.033; 1,5 g) und Brommethyl-cyclohexan (1,76 g) wurden in DMF (20 ml) gelöst und Natriumhydrid (260 mg) zugesetzt. Nach 24 h Rühren bei 50°C wurde das DMF abgezogen. Der Rückstand wurde in EE aufgenommen, mit Wasser gewaschen, getrocknet, filtriert und eingeengt. Der Rückstand wurde über Kieselgel (40 g Kartusche, n-Heptan/EE 0-50% in 30 min) gereinigt. Es wurden 1,33 g der Titelverbindung erhalten.

| | |
|---|---|
| LCMS-rt: 1,93 min | [M+H]⁺: 263,2 (Met-a) |

### 02.040

### 1-(3-Brom-4,5-dimethoxy-phenyl)-ethanon

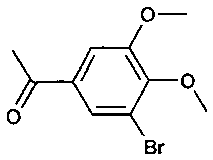

3-Brom-4,5-dimethoxy-benzoesäure (2,0 g) wurde analog 03.043 in das Benzamid-Derivat übergeführt und letzteres analog 02.059 in die Titelverbindung. Es wurden 1,22 g der Titelverbindung erhalten.
¹H-NMR (500 MHz, DMSO-d6) [ppm]: 7,78 (1 H); 7,53 (1 H); 3,90 (3 H); 3,82 (3 H); 2,57 (3 H),

### 02.041

### 1-(5-Brom-2,3-dimethoxy-phenyl)-ethanon

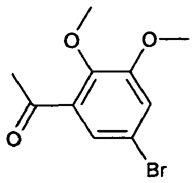

5-Brom-2,3-dimethoxy-benzoesäure (2 g) wurde analog 03.043 in das Benzamid-Derivat übergeführt und letzteres analog 02.059 in die Titelverbindung. Es wurden 1,1 g der Titelverbindung erhalten.

| | |
|---|---|
| LCMS-rt: 3,70 min | [M+H]⁺: 259,0 (Met-d) |

### 02.042

### 1-(3-Chlor-4,5-dimethoxy-phenyl)-ethanon

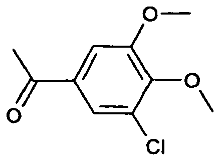

3-Chlor-4,5-dimethoxy-benzoesäure (1 g) wurde analog 03.043 in das Benzamid-Derivat übergeführt und letzteres analog 02.059 in die Titelverbindung. Es wurden 495 mg der Titelverbindung erhalten.

| | |
|---|---|
| LCMS-rt: 1,55 min | [M+H]⁺: 215,1 (Met-a) |

02.043 1-[3-tert-Butyl-5-(2-methoxy-ethoxy)-phenyl]-ethanon 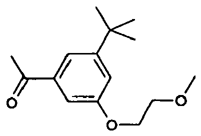 3-tert-Butyl-N-methoxy-5-(2-methoxy-ethoxy)-N-methyl-benzamid (03.043; 1,35 g) wurde in THF (40 ml) gelöst, bei 0°C Methylmagnesiumbromid (3,05 ml, 3 M in Ether) zugetropft und anschließend 2 h bei RT gerührt. Dann wurde mit 1 N Salzsäure (50 ml) versetzt, mit Wasser verdünnt und dreimal mit EE ausgeschüttelt. Dann wurden die vereinigten EE-Phasen über Magnesiumsulfat getrocknet, filtriert und eingeengt. Der Rückstand wurde über Kieselgel (40 g Kartusche, n-Heptan/EE-Gradient von 0-30% in 30 min) aufgereinigt. Es wurden 1,0 g der Titelverbindung erhalten. LCMS-rt: 1,58 min [M+H]⁺: 251,3 (Met-a) 02.044 1-[3-Morpholin-4-yl-5-(pentafluorsulfanyl)-phenyl]-ethanon 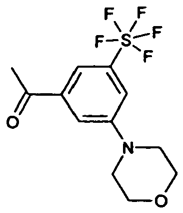 N-Methoxy-N-methyl-3-morpholin-4-yl-5-(pentafluorsulfanyl)-benzamid (03.044; 2,38 g) wurde analog 02.043 umgesetzt und aufgearbeitet. Die Aufreinigung erfolgte über Kieselgel (80 g Kartusche, n-Heptan/EE-Gradient von 0-70 % in 40 min) aufgereinigt. Es wurden 1,1 g der Titelverbindung erhalten.

| | |
|---|---|
| LCMS-rt: 1,57 min | [M+H]⁺: 332,0 (Met-a) |

### 02.045

### 1-[3-tert-Butyl-5-(2-methoxy-ethoxymethyl)-phenyl]-ethanon

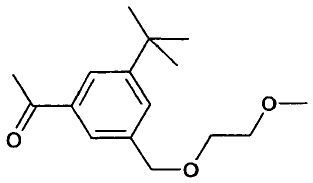

3-tert-Butyl-5-hydroxymethyl-benzoesäure-methylester (6,0 g) wurde analog 05.043 mit 1-Brom-2-methoxy-ethan zu 3-tert-Butyl-5-(2-methoxy-ethoxymethyl)-benzoesäure-methylester umgesetzt. Letzteres wurde dann analog der Sequenz 04.043 bis 02.043 in die Titelverbindung überführt. Es wurden 508 mg der Titelverbindung erhalten.
¹H-NMR (500 MHz, DMSO-d6) [ppm]: 7,85 (1 H); 7,73 (1 H); 7,63 (1 H); 4,56 (2 H); 3,58 (2 H); 3,50 (2 H); 3,26 (3 H); 2,58 (3 H); 1,32 (9 H)

### 02.050

### 1-[3-(2-Methoxy-ethoxy)-5-(pentafluorsulfanyl)-phenyl]-ethanon

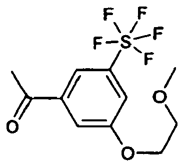

1-[3-Hydroxy-5-(pentafluorsulfanyl)-phenyl]-ethanon (03.050; 592 mg) und 1-Brom-2-methoxy-ethan (255 µl) in DMF (14,8 ml) gelöst und Natriumhydrid (65 mg) zugesetzt. Nach 2 Rühren bei RT wurde weiteres Bromid (80 µl) zugesetzt und 12 h auf 50°C erwärmt. Dann wurde das DMF abgezogen und Rückstand in EE aufgenommen, mit Wasser gewaschen, getrocknet, filtriert und eingeengt. Es wurden 578 mg der Titelverbindung erhalten. LCMS-rt: 1,58 min [M+H]⁺: 321,1 (Met-a)

### 02.051

### 1-[3-(2-Methoxy-ethoxy)-5-(pentafluorsulfanyl)-phenyl]-ethanon

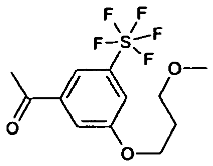

1-[3-Hydroxy-5-(pentafluorsulfanyl)-phenyl]-ethanon (03.050; 700 mg) und 1-Brom-2-methoxy-propan (490 mg) in DMF (10 ml) gelöst und Natriumhydrid (77 mg) zugesetzt. Nach 2 Rühren bei RT wurde weiteres Bromid (100 µl) zugesetzt und 6 h auf 50°C erwärmt. Dann wurde das DMF abgezogen und Rückstand in EE aufgenommen, mit Wasser gewaschen, getrocknet, filtriert und eingeengt. Es wurden 670 mg der Titelverbindung erhalten. LCMS-rt: 1,71 min [M+H]⁺: 335,1 (Met-a)

### 02.052

### 1-[3-tert-Butyl-5-(3-methoxy-propoxy)-phenyl]-ethanon

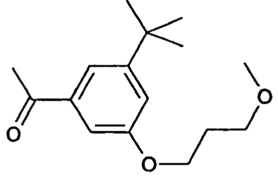

Ausgehend von 3-tert-Butyl-5-hydroxy-benzoesäuremethylester (06.043) und 1-Brom-3-methoxypropan wurde der Synthesesequenz 05.043 bis 02.043 gefolgt. Es wurden 880 mg der Titelverbindung erhalten.

| | |
|---|---|
| LCMS-rt: 1,71 min | [M+H]⁺: 265,3 (Met-a) |

### 02.059

### 1-[3-tert-butyl-5-(3-hydroxy-propoxy)-phenyl]-ethanon

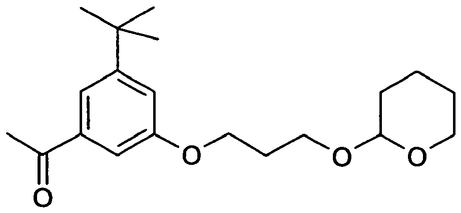

3-tert-Butyl-N-methoxy-N-methyl-5-[3-(tetrahydro-pyran-2-yloxy)-propoxy]-benzamid (03.059; 5,49 g) wurde in THF (100 ml) gelöst, auf 0°C gekühlt und mit Lithium-bis-(trimethylsilyl)-amid (14,47 ml, 1 M in MTB-Ether) versetzt. Nach 30 min Rühren bei 0°C wurde Methylmagnesiumbromid (9,65 ml, 3 M in Ether) zugetropft. Das Kältebad wurde entfernt und nach 2 h Rühren bei RT wurde mit Wasser verdünnt und gegen EE ausgeschüttelt. Die EE-Phase wurde über Magnesiumsulfat getrocknet, filtriert und einrotiert. Der Rückstand wurde über Kieselgel (200 g, n-Heptan/EE 4:1) aufgereinigt. Es wurden 3,4 g der Titelverbindung erhalten.
¹H-NMR (500 MHz, DMSO-d6) [ppm] (repräsentative Signale): 7,53 (1 H); 7,28 (1 H); 7,17 (1 H); 4,57 (1 H, -O-C(-C)H-O-); 4,11 (2 H); 2,57 (3 H)

### 02.060

### 1-{3-tert-Butyl-5-[3-(tetrahydro-pyran-2-yloxy)-propoxymethyl]-phenyl}-ethanon

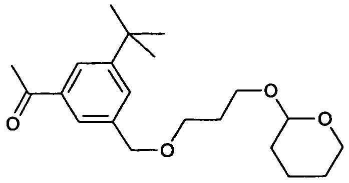

3-tert-Butyl-5-hydroxymethyl-benzoesäure-methylester (3,5 g) wurde mit 2-(3-Brompropoxy)-tetrahydro-pyran analog 05.063 zu 3-tert-Butyl-5-[3-(tetrahydro-pyran-2-yloxy)-propoxymethyl]-benzoesäure-methylester umgesetzt und letzteres anschließend entsprechend der Sequence 04.059 bis 02.059 in die Titelverbindung übergeführt. Es wurden 1,75 g der Titelverbindung erhalten.
¹H-NMR (500 MHz, DMSO-d6) [ppm] (repräsentative Signale): 7,84 (1 H); 7,73 (1 H); 7,61 (1 H); 4,53 (2 H); 4,51 (1 H, -O-C(-C)H-O-), 2,58 (3 H)

### 02.061

### 1-{3-tert-Butyl-5-[2-(tetrahydro-pyran-2-yloxy)-ethoxy]-phenyl}-ethanon

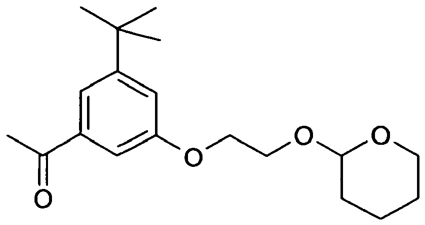

Ausgehend von 3-tert-Butyl-5-hydroxy-benzoesäuremethylester (06.043) und 2-(2-Brom-ethoxy)-tetrahydro-pyran wurde der Synthesesequenz 05.059 bis 02.059 gefolgt. Es wurden 3,9 g der Titelverbindung erhalten.
¹H-NMR (500 MHz, DMSO-d6) [ppm] (repräsentative Signale): 7,53 (1 H), 7,31 (1 H), 7,20 (1 H), 4,66 (1 H, -O-C(-C)H-O-), 4,20 (2 H), 2,58 (3 H)

### 02.062

### 1-[3-Ethoxy-5-(pentafluorsulfanyl)-phenyl]-ethanon

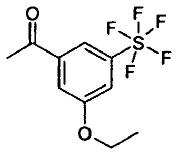

3-Ethoxy-N-methoxy-N-methyl-5-(pentafluorsulfanyl)-benzamid (03.062; 2,60 g) wurde analog 02.007 umgesetzt und aufgearbeitet. Eine Aufreinigung über Kieselgel wurde nicht durchgeführt. Es wurden 2,25 g der Titelverbindung erhalten.

| | |
|---|---|
| LCMS-rt: 1,72 min | [M+H]⁺: 291,0 (Met-a) |

### 02.063

### 1-{3-tert-Butyl-5-[2-(tetrahydro-pyran-2-yloxy)-ethoxymethyl]-phenyl}-ethanon

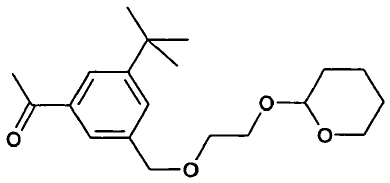

3-tert-Butyl-5-[2-(tetrahydro-pyran-2-yloxy)-ethoxymethyl]-benzoesäure-methylester (05.063, 2,28 g) wurde analog der Sequenz 04.059 bis 02.059 in die Titelverbindung übergeführt. Es wurden 2,0 g der Titelverbindung erhalten.
¹H-NMR (500 MHz, DMSO-d6) [ppm] (repräsentative Signale): 7,85 (1 H); 7,74 (1 H); 7,63 (1 H); 4,60 (1 H, -O-C(-C)H-O-), 4,58 (2 H), 2,58 (3 H)

### 02.064

### 1-[3-Ethoxy-5-(pentafluorsulfanyl)-phenyl]-propan-1-on

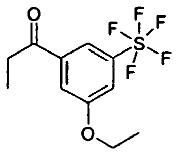

3-Ethoxy-N-methoxy-N-methyl-5-(pentafluorsulfanyl)-benzamid (03.062; 170 mg) wurde in absolutem THF (5 ml) gelöst und bei 0°C unter Rühren Ethylmagnesiumbromid (0,65 ml; 2 M in Diethylether) zugetropft. Nach Zugabe wurde das Eisbad entfernt und 2 h bei RT gerührt. Dann wurde weiteres Ethylmagnesiumbromid (0,1 ml) zugegeben und noch einmal 2 h gerührt. Unter Kühlung wurde anschließend 1 N Salzsäure zugetropft, gefolgt von Wasser und Essigester. Die organische Phase wurde abgetrennt und die wässrige noch 2 Mal mit Essigester extrahiert. Die vereinigten Extrakte wurden über Magnesiumsulfat getrocknet, filtriert und eingeengt. Es wurden 150 mg der gewünschten Verbindung erhalten.

| | |
|---|---|
| LCMS-rt: 1,26 min | [M+H]⁺: 305,1 (Met-b) |

### 02.070

### 1-{3-tert-Butyl-4-methoxy-5-[2-(tetrahydro-pyran-2-yloxy)-ethoxy]-phenyl}-ethanon

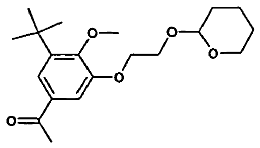

Analog 02.071 wurde 1-(3-tert-Butyl-5-hydroxy-4-methoxy-phenyl)-ethanon (03.070; 5,0 g) mit 2-(2-Brom-ethoxy)-tetrahydro-pyran (5,64 g) umgesetzt. Allerdings wurde das Rohprodukt über Kieselgel (40 g Kartusche, n-Heptan/EE-Gradient von 0-50% in 60 min) aufgereinigt. Es wurden 6,68 g der Titelverbindung erhalten.
¹H-NMR (500 MHz, DMSO-d6) [ppm] (repräsentative Signale): 7,50 (2 H, aromatisch), 4,70 (1 H, -O-C(-C)H-O-), 3,90 (3 H, -OCH₃), 2,54 (3 H, Acetyl)

### 02.071

### 1-{3-tert-Butyl-4-methoxy-5-[3-(tetrahydro-pyran-2-yloxy)-propoxy]-phenyl}-ethanon

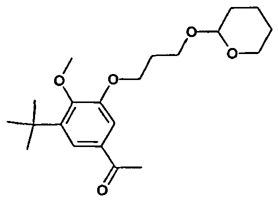

1-(3-tert-Butyl-5-hydroxy-4-methoxy-phenyl)-ethanon (03.070; 6,9 g) und 2-(3-Brompropoxy)-tetrahydro-pyran (8,31 g) wurden in DMF (80 ml) gelöst und Natriumhydrid (894 mg) zugesetzt. Nach 5 Stunden Rühren bei RT wurde das Lösungsmittel abgezogen und der Rückstand mit EE aufgenommen. Die EE-Phase wurde mit Wasser gewaschen, getrocknet und eingeengt. Es wurden 12,7 g der Titelverbindung als Rohprodukt in ausreichender Reinheit erhalten.
¹H-NMR (400 MHz, DMSO-d6) [ppm] (repräsentative Signale): 4,57 (1 H, -O-C(-C)H-O-), 2,54 (3 H, Acetyl)

### 02.072

### 1-[3-tert-Butyl-5-(2-methoxy-1-methoxymethyl-ethoxy)-phenyl]-ethanon

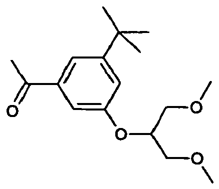

3-tert-Butyl-5-(2-methoxy-1-methoxymethyl-ethoxy)-benzoesäure-methylester (05.072) wurde analog 04.043, 03.059 und 02.043 in die Titelverbindung (1,65 g) übergeführt. Eine Chromatogrphie wurde auf der letzten Stufe nicht durchgeführt.

| | |
|---|---|
| LCMS-rt: 1,63 min | [M+H]⁺: 295,1 (Met-a) |

### 02.073

### 1-[3-tert-Butyl-5-(4-hydroxy-butoxy)-phenyl]-ethanon

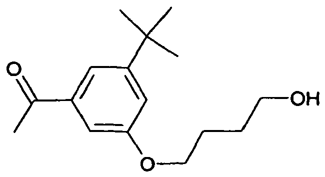

3-tert-Butyl-5-(4-hydroxy-butoxy)-N-methoxy-N-methyl-benzamid (03.073; 3,47 g) wurde analog 02.043 umgesetzt, aufgearbeitet und gereinigt. Die Reinigung über Kieselgel wurde wie folgt durchgeführt: 40 g Kartusche, n-Heptan/EE-Gradient von 0-100% in 60 min. Es wurden 2,08 g der Titelverbindung erhalten.

| | |
|---|---|
| LCMS-rt: 0,92 min | [M+H]⁺: 265,1 (Met-b) |

### 02.075

### N-[3-Acetyl-5-(pentafluorsulfanyl)-phenyl]-2,2,2-trifluor-N-methyl-acetamid

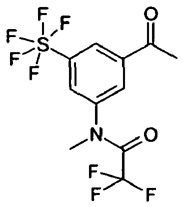

In einem Mikrowelleneinsatz wurde N-(3-Acetyl-5-pentafluorsulfanyl-phenyl)-2,2,2-trifluor-acetamid (03.075; 0,25 g) in absolutem Dimethoxyethan (7,5 ml) gelöst, gepulvertes Kaliumcarbonat zugegeben und mit Jodmethan (80 µl) versetzt. Anschließend wurde in der Mikrowelle 40 min auf 100°C erhitzt. Nachdem weiteres N-(3-Acetyl-5-pentafluorsulfanyl-phenyl)-2,2,2-trifluor-acetamid (4 x 250 mg) in beschriebener Weise umgesetzt worden war, wurden die fünf Ansätze gemeinsam aufgearbeitet, wobei vom Kaliumcarbonat unter Eiskühlung in 1 N Salzsäure dekantiert wurde. Nach mehrmaligem Waschen des Kaliumcarbonat-Rückstands mit Dimethoxyethan wurde die wässrige Phase fünfmal mit Essigester extrahiert. Die vereinigten Extrakte wurden über Magnesiumsulfat getrocknet, filtriert und eingeengt. Der Rückstand wurde mittels präparativer HPLC (Met-A) gereinigt. Die Produkt enthaltenden Fraktionen wurden vereinigt, vom Acetonitril befreit und fünfmal mit Essigester extrahiert. Die vereinigten Extrakte wurden über Magnesiumsulfat getrocknet, filtriert und eingeengt. Es wurden 1,03 g der gewünschten Verbindung erhalten. LCMS-rt: 1,62 min [M+H]⁺: 372,0 (Met-a)

### 02.085

### 1-[3-tert-Butyl-5-(3-hydroxy-2,2-dimethyl-propoxy)-4-methoxy-phenyl]-ethanon

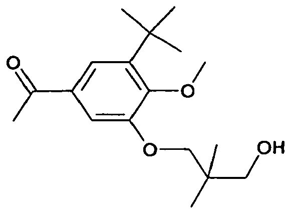

1-(3-tert-Butyl-5-hydroxy-4-methoxy-phenyl)-ethanon (03.070; 2,00 g) und Essigsäure-3-brom-2,2-dimethyl-propyl-ester (2,54 g) wurden in DMF (15 ml) gelöst und Cäsiumcarbonat (3,69 g) zugesetzt. Nach 2,5 h Rühren bei 150°C und maximal 15 bar in der Mikrowelle wurden weitere 0,5 eq. des Bromids nachgegeben und weitere 2 h bei 150°C in die Mikrowelle gestellt. Dann wurde der Ansatz einrotiert und der Rückstand zwischen DCM und Wasser verteilt. Die DCM-Phase wurde abgetrennt, über Magnesiumsulfat getrocknet, filtriert und eingeengt. Der Rückstand wurde über Kieselgel (120 g Kartusche, n-Heptan/MtB-Ether-Gradient von 0-50% in 60 min) aufgereinigt. Es wurden 1,17 g der Titelverbindung erhalten.

| | |
|---|---|
| LCMS-rt: 1,92 min | [M+H]⁺: 351,1 (Met-a) |

### 02.086

### 1-[3-tert-Butyl-5-(3-hydroxy-2,2-dimethyl-propoxy)-phenyl]-ethanon

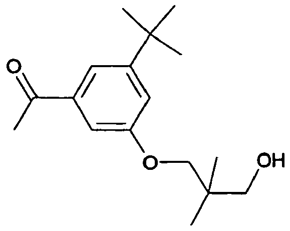

3-tert-Butyl-5-(3-hydroxy-2,2-dimethyl-propoxy)-N-methoxy-N-methyl-benzamid (03.086; 910 mg) wurde analog 02.059 umgesetzt, aufgearbeitet und gereinigt. Es wurden 388 mg der Titelverbindung erhalten.

| | |
|---|---|
| LCMS-rt: 1,65 min | [M+H]⁺: 279,1 (Met-a) |

### 02.090

### 1-{3-(Pentafluorsulfanyl)-5-[2-(tetrahydro-pyran-2-yloxy)-ethoxy]-phenyl}-ethanon

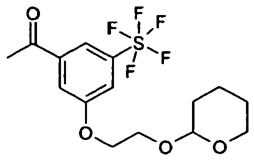

1-[3-Hydroxy-5-(pentafluorsulfanyl)-phenyl]-ethanon (03.050; 700 mg) und 2-(2-Bromethoxy)-tetrahydro-pyran (670 mg) wurde in DMF (15 ml) gelöst und Natriumhydrid (77 mg) zugesetzt. Nach 2 h Rühren bei RT wurde 4 h auf 50°C erwärmt und übers Wochenende stehengelassen. Dann wurde das DMF abgezogen und der Rückstand in EE aufgenommen. Die EE-Phase wurde mit Wasser gewaschen, getrocknet, filtriert und eingeengt. Das Rohprodukt wurde über Kiesegel (100 g, n-Heptan/ EE 4:1) aufgereinigt. Es wurden 813 mg der Titelverbindung erhalten.
¹H-NMR (500 MHz, DMSO-d6) [ppm] (repräsentative Signale): 7,87 (1 H), 7,80 (1 H), 7,71 (1 H), 4,66 (1 H, -O-C(-C)H-O-), 4,35 ( 2 H), 2,66 (3 H)

### 02.095

### Essigsäure-4-(5-acetyl-3-tert-butyl-2-methoxy-phenoxy)-butylester

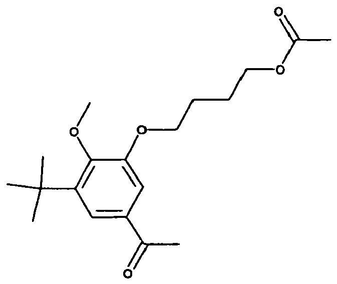

1-(3-tert-Butyl-5-hydroxy-4-methoxy-phenyl)-ethanon (03.070; 6,9 g) wurde analog 02.071 umgesetzt und aufgearbeitet. Als Bromid wurde Essigsäure-4-brom-butylester (7,27 g) eingesetzt. Es wurden 10,5 g der Titelverbindung erhalten.

| | |
|---|---|
| LCMS-rt: 1,14 min | [M+H]⁺: 337,1 (Met-b) |

### 02.100

### 3-Acetyl-5-tert-butyl-benzoesäure-methylester und 1-[3-tert-Butyl-5-(1-hydroxy-1-methyl-ethyl)-phenyl]-ethanon

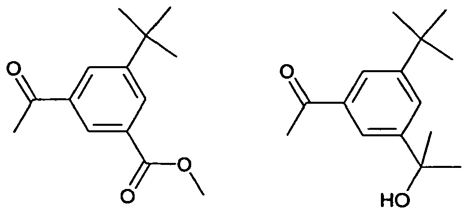

5-tert-Butyl-N-methoxy-N-methyl-isophthalamidsäure-methylester (03.100; 10,4 g) wurde in THF (150 ml) unter Argon bei RT gelöst. Danach tropfte man unter Rühren Methylmagnesiumbromid (19 ml; 3 M in Diethylether) bei 0°C zu. Nach 10 min wurde das Eisbad entfernt und 3,5 h bei RT gerührt. Dann wurde unter Eiskühlung mit 1 N Salzsäure und Waser versetzt. Die wässrige Phase wurde dreimal mit EE extrahiert und die vereinigten EE-Phasen wurden über Natriumsulfat getrocknet, filtriert und eingeengt. Der Rückstand wurde über Kieselgel gereinigt. Es wurden 7,0 g 3-Acetyl-5-tert-butyl-benzoesäure-methylester und 550 mg 1-[3-tert-Butyl-5-(1-hydroxy-1-methylethyl)-phenyl]-ethanon erhalten.

### 3-Acetyl-5-tert-butyl-benzoesäure-methylester

| | |
|---|---|
| LCMS-rt: 1,13 min | [M+H]⁺: 235,2 (Met-b) |

### 1-[3-tert-Butyl-5-(1-hydroxy-1-methyl-ethyl)-phenyl]-ethanon

| | |
|---|---|
| LCMS-rt: 1,03 min | [M+H]⁺-OH: 217,2 (Met-b) |

### O2.105

### 1-[3-tert-Butyl-5-(2-fluorethoxy)-phenyl]-ethanon ZSI2.060

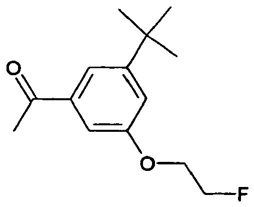

1-(3-tert-Butyl-5-hydroxy-phenyl)-ethanon (03.106; 500 mg) wurde in DMF (10 ml) gelöst und 1-Brom-2-fluorethan (195 µl) unter Rühren zugegeben. Bei RT wurde Natriumhydrid (80 mg) zugegeben. Nach 3 h Rühren bei RT wurde über Nacht stehen gelassen und dann weiteres Natriumhydrd (20 mg) zugegeben und weitere 2 h gerührt. Dann wurde Wasser zugegeben und zur Trockne gebracht. Der Rückstand wurde in EE aufgenommen und dreimal mit Wasser gewaschen. Die EE-Phase wurde über Magnesiumsulfat getrocknet, filtriert und eingeengt. Es wurden 600 mg der Titelverbindung erhalten. LCMS-rt: 1,13 min [M+H]⁺: 239,1 (Met-b)

### 02.106

### 1-[3-tert-Butyl-5-(3-fluorpropoxy)-phenyl]-ethanon

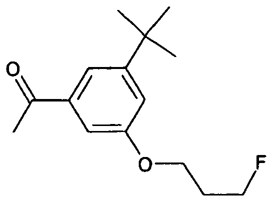

1-(3-tert-Butyl-5-hydroxy-phenyl)-ethanon (03.106; 500 mg) wurde analog 02.105 mit 1-Brom-3-fluorpropan (239 µl) umgesetzt und aufgearbeitet. Es wurden 630 mg der Titelverbindung erhalten. LCMS-rt: 1,16 min [M+H]⁺: 253,2 (Met-b)

### 02.107

### 1-[3-tert-Butyl-5-(3,3,3-trifluoropoxy)-phenyl]-ethanon

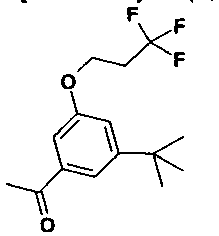

Zu einer Lösung von 1-(3-tert-Butyl-5-hydroxy-phenyl)-ethanon (03.106; 300 mg, 1,56 mmol), 3,3,3-Trifluorpropanol (178 mg, 1,56 mmol) und Triphenylphosphin (409 mg, 1,56 mmol) in Dichlormethan (5 ml) bei RT wurde eine Lösung von Di-(4-chlorbenzyl)azodicarboxylat (573 mg, 1,56 mmol) in Dichlormethan (1 ml) gegeben. Die Mischung wurde 5 Tage gerührt und dann filtriert. Das Lösungsmittel wurde entfernt und der Rückstand durch Flash-Chromatographie (n-Heptan:Essigester) gereinigt. Ausbeute: 170 mg, 38%.

| | |
|---|---|
| LCMS-rt: 1,14 min | [M+H]⁺: 289,2 (Met-b) |

### 02.110

### 1-[3-tert-Butyl-4-methoxy-5-(2-methoxy-ethoxy)-phenyl]-ethanon

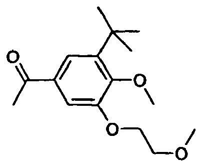

1-(3-tert-Butyl-5-hydroxy-4-methoxy-phenyl)-ethanon (03.070; 2,00 g) und 1-Brom-2-methoxy-ethan (1,5 g) wurden in DMF (20 ml) gelöst und Natriumhydrid (259 mg) zugesetzt. Nach 3 h Rühren bei RT ließ man über Nacht stehen. Dann wurde das DMF abgezogen und der Rückstand in EE aufgenommen. Die EE-Phase wurde mit Wasser gewaschen, getrocknet, filtriert und eingeengt. Der Rückstand wurde über Kieselgel (80 g Kartusche, n-Heptan/EE 0-40% in 60 min) gereinigt. Es wurden 1,17 g der Titelverbindung erhalten.

| | |
|---|---|
| LCMS-rt: 1,03 min | [M+H]⁺: 281,1 (Met-b) |

### 02.111

### 1-[3-tert-Butyl-4-methoxy-5-(3-methoxy-propoxy)-phenyl]-ethanon

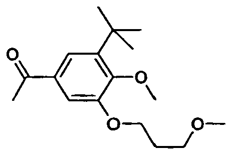

1-(3-tert-Butyl-5-hydroxy-4-methoxy-phenyl)-ethanon (03.070; 2,00 g) und 1-Brom-3-methoxypropan (1,65 g) wurden analog 02.110 umgesetzt, aufgearbeitet und gereinigt. Es wurden 1,50 g der Titelverbindung erhalten.

| | |
|---|---|
| LCMS-rt: 1,09 min | [M+H]⁺: 295,2 (Met-b) |

### 03

### 03.004

### 1-Brom-5-(1,1-dimethoxy-ethyl)-2-methoxy-3-trifluormethyl-benzol

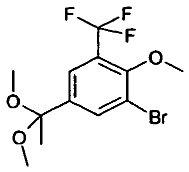

1-(3-Brom-4-hydroxy-5-trifluormethyl-phenyl)-ethanon (04.004; 6,8 g) wurden in Methanol (50 ml) gelöst und nacheinander mit DL-10-Camphersulfonsäure (111 mg) und Trimethylorthoformiat (8 ml) versetzt. Nach 2 h Rühren bei RT wurden DMF (75 ml), Kaliumcarbonat (4,98 g) und dann langsam unter Eiskühlung lodmethan (3 ml) zugesetzt. Nach 4 h Rühren bei RT ließ man über Nacht stehen und versetzte dann das Reaktionsgemisch mit n-Heptan / Wasser und trennte die organische Phase ab. Die wässrige Phase wurde noch einmal mit n-Heptan ausgeschüttelt und die vereinigten organischen Phasen dann über Magnesiumsulfat getrocknet, filtriert und eingeengt. Es wurden 7 g der Titelverbindung in ausreichender Reinheit erhalten. ¹H-NMR (500 MHz, DMSO-d6) [ppm]: 7,90 (1 H), 7,62 (1 H), 3,89 (3 H), 3,10 (6 H), 1,49 (3 H)

### 03.007

### 3,N-Dimethoxy-N-methyl-5-(pentafluorsulfanyl)-benzamid

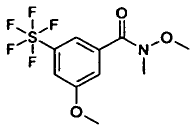

3-Methoxy-5-(pentafluorsulfanyl)-benzoesäure-methylester (04.007; 2,5 g) wurde in absolutem THF (65 ml) gelöst und N,O-Dimethylhydroxylamin-Hydrochlorid (1,2 g) zugegeben. Dann wurde auf -15°C abgekühlt und Isopropylmagnesiumbromid-Lösung (13,59 ml, 2 M in THF) zugetropft. Nach 20 min wurde das Kältebad entfernt und 1 h bei RT gerührt. Dann wurde Ammoniumchlorid-Lösung zugegeben und die wässrige Phase dreimal mit Essigester extrahiert. Die vereinigten Extrakte wurden über Magnesiumsulfat getrocknet, filtriert und eingeengt. Das so erhaltene Rohprodukt enthielt noch deutlich Edukt, daher wurde es erneut wie oben beschrieben umgesetzt und aufgearbeitet. Im dann erhaltenen Rückstand war kein Edukt mehr enthalten. Aufreinigung erfolgte mittels präparativer HPLC (Met-A). Die Produkt enthaltenden Fraktionen wurden vereinigt, vom Acetonitril befreit und die wässrige Phase dreimal mit EE extrahiert. Die vereinigten Extrakte wurden über Magnesiumsulfat getrocknet, filtriert und eingeengt. 1,78 g der gewünschten Verbindung wurden erhalten. ¹H-NMR (400 MHz, CDCl₃) [ppm]: 7,70 (1 H), 7,37 (1 H), 7,24 (1 H + CDCl₃), 3,88 (3 H), 3,56 (3 H)

### 03.008

### 3-tert-Butyl-5-ethoxymethyl-N-methoxy-N-methyl-benzamid

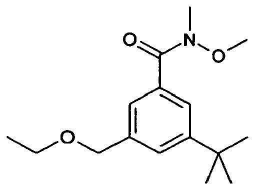

Analog 03.043 wurde 3-tert-Butyl-5-ethoxymethyl-benzoesäure (04.008; 1,15 g) erst in das Säurechlorid (1,24 g) übergeführt und dann das erhaltene 3-tert-Butyl-5-ethoxymethyl-benzoylchlorid weiter umgesetzt. Es wurden 854 mg der Titelverbindung erhalten. LCMS-rt: 3,32 min [M+H]⁺: 280,2 (Met-d)

### 03.010

### 1-(3-tert-Butyl-4-ethoxy-5-hydroxy-phenyl)-ethanon

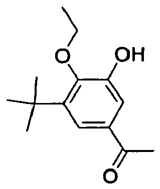

1-Brom-3-tert-butyl-5-(1,1-dimethoxy-ethyl)-2-ethoxy-benzol (04.010; 19,68 g) wurde analog 03.070 umgesetzt. Es wurden 5,15 g der Titelverbindung erhalten.

| | |
|---|---|
| LCMS-rt: 0,972 min | [M+H]⁺: 237,1 (Met-b) |

### 03.014

### 3,N-Dimethoxy-N-methyl-5-trifluormethyl-benzamid

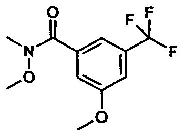

3-Methoxy-5-trifluormethyl-benzoesäuremethylester (04.014, 1 g) und N,O-Dimethylhydroxylamin (416 mg) wurden in THF (30 ml) vorgelegt. Danach kühlte man auf -15°C und tropfte innerhalb von 10 min Isopropylmagnesiumchlorid (3,2 ml; 2 M in THF) zu. Es wurde noch 20 min bei -15°C gerührt bevor das Kältebad entfernt wurde. Nach 3 h wurde erneut auf -15°C abgekühlt und weiteres Isopropylmagnesiumchlorid (3,2 ml) zugegeben. Nach Entfernen des Kältebads wurde noch eine Stunde bei RT gerührt, dann mit 20%-iger Ammoniumchlorid-Lösung versetzt und dreimal mit EE extrahiert. Die vereinigten EE-Phasen trocknete man über Natriumsulfat, filtrierte und engte ein. Der Rückstand wurde über Kieselgel (50 g Kartusche, DCM als Laufmittel) aufgereinigt. Es wurden 465 mg der Titelverbindung neben 427 mg Edukt erhalten.

| | |
|---|---|
| LCMS-rt: 1,36 min | [M+H]⁺: 264,0 (Met-a) |

### 03.033

### 1-(3-Hydroxy-5-methoxy-phenyl)-ethanon

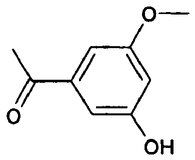

1-(3,5-Dihydroxy-phenyl)-ethanon (3 g) und Methyliodid (2,80 g) wurden in DMF (40 ml) gelöst und Natriumhydrid (568 mg) zugesetzt. Nach 2 h Rühren bei RT wurde das DMF abgezogen. Der Rückstand wurde in EE aufgenommen und mit Wasser gewaschen, getrocknet, filtriert und eingeengt. Der Rückstand wurde über Kieselgel (89 g Kartusche, n-Heptan/EE 0-50% in 30 min) gereinigt. Es wurden 1,12 g der Titelverbindung erhalten.
¹H-NMR (500 MHz, DMSO-d6) [ppm]: 9,8 (1 H); 6,93 (2 H); 6,58 (1 H); 3,76 (3 H); 2,50 (3 H + DMSO)

### 03.043

### 3-tert-Butyl-N-methoxy-5-(2-methoxy-ethoxy)-N-methyl-benzamid

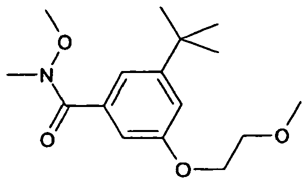

3-tert-Butyl-5-(2-methoxy-ethoxy)-benzoesäure (04.043; 1,9 g) wurde in Thionylchlorid (10,9 ml) gelöst, 2 h unter Rückfluss gehalten und dann eingeengt. Das erhaltene 3-tert-Butyl-5-(2-methoxy-ethoxy)-benzoyl-chlorid (2,04 g) wurde in DCM (20 ml) gelöst, mit Dimethylhydroxylamin (734 mg) versetzt, anschließend Hünigbase (1,37 ml) zugegeben und 1 h bei RT gerührt. Dann wurde zur Trockne gebracht, der Rückstand in EE aufgenommen, das Gemisch viermal mit Wassser gewaschen, über Magnesiumsulfat getrocknet, filtriert und einrotiert. Der Rückstand wurde über Kieselgel (40 g Kartusche, n-Heptan/EE-Gradient von 0-50% in 40 min) aufgereinigt. Es wurden 1,36 g der Titelverbindung erhalten.

| | |
|---|---|
| LCMS-rt: 1,43 min | [M+H]⁺: 296,3 (Met-a) |

### 03.044

### N-Methoxy-N-methyl-3-morpholin-4-yl-5-(pentafluorsulfanyl)-benzamid

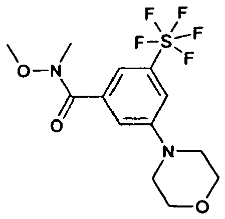

3-Amino-N-methoxy-N-methyl-5-(pentafluorsulfanyl)-benzamid (05.075; 6,3 g) wurde in DMF (80 ml) gelöst und Cäsiumcarbonat (10,1 g), Natriumiodid (0,62 g) und Bis-(2-bromethyl)ether (19,37 g) zugegeben. Das Gemisch wurde auf 10 Mikrowellengefäße verteilt, die jeweils 3 h auf 130 °C erhitzt wurden. Anschließend wurden die Ansätze vereinigt und von Lösungsmittel befreit. Der Rückstand wurde in EE aufgenommen und mit Wasser gewaschen. Die EE-Phase wurde getrocknet und eingeengt. Der Rückstand wurde über Kieselgel (120 g Kartusche, n-Heptan/EE-Gradient von 0-100 % in 30 min) aufgereinigt. Es wurden 2,48 g der Titelverbindung erhalten. LCMS-rt: 1,41 min [M+H]⁺: 377,0 (Met-a)

### 03.050

### 1-[3-Hydroxy-5-(pentafluorsulfanyl)-phenyl]-ethanon

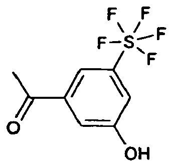

1-[3-Amino-5-(pentafluorsulfanyl)-phenyl]-ethanon (04.050; 3,00 g) wurde in ein 35%iger wässriger Schwefelsäure (25 ml) in der Wärme gelöst. Die Lösung wurde auf -5°C abgekühlt und eine Lösung von Natriumnitrit (780 mg) in 15 ml Wasser innerhalb von 10 min zugetropft. Nach 40 min bei -5°C wurde das Kältebad entfernt und 2 h auf 100°C erhitzt. Nach Abkühlen wurde mit EE zweimal extrahiert. Die vereinigten EE-Phasen wurden mit gesättigter Natriumhydrogencarbonat-Lösung gewaschen, getrocknet, filtriert und eingeengt. Der Rückstand wurde über Kieselgel (80 g Kartusche, n-Heptan/MtB-Ether-Gradient von 0-100 % in 40 min) aufgereinigt. Es wurden 1,62 g der Titelverbindung erhalten.
¹H-NMR (500 MHz, DMSO-d6) [ppm]: 10,71 (1 H), 7,73 (1 H), 7,59 (1 H), 7,47 (1 H), 2,61 (3 H)

### 03.059

### 3-tert-Butyl-N-methoxy-N-methyl-5-[3-(tetrahydro-pyran-2-yloxy)-propoxy]-benzamid

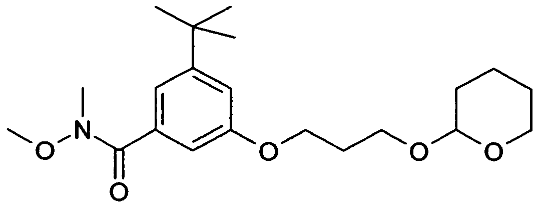

3-tert-Butyl-5-[3-(tetrahydro-pyran-2-yloxy)-propoxyl-benzoesäure (04.059; 4,90 g) und N,O-Dimethylhydroxylamin-Hydrochlorid (1,42 g) wurden in DMF (80 ml) gelöst und mit Hünig-Base (4,81 ml) und TOTU (4,78 g) versetzt. Nach 2 h Rühren wurde über Nacht stehen gelassen. Dann wurde das DMF abgezogen, zwischen EE und gesättigter Natriumhydrogencarbonat-Lösung verteilt, die EE-Phase abgetrennt, über Magnesiumsulfat getrocknet, filtriert und eingeengt. Es wurden 5,49 g der Titelverbindung erhalten.
¹H-NMR (500 MHz, DMSO-d6) [ppm] (repräsentative Signale): 7,12 (1 H), 7,01 (1 H), 6,90 (1 H), 4,56 (1 H, -O-C(-C)H-O-), 4,06 (2 H), 3,57 (3 H), 3,22 (3 H)

### 03.062

### 3-Ethoxy-N-methoxy-N-methyl-5-(pentafluorsulfanyl)-benzamid

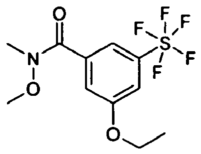

3-Ethoxy-5-(pentafluorsulfanyl)-benzoesäureethylester (04.062; 4,80 g) wurde analog 03.007 umgesetzt und aufgearbeitet. Der Rückstand wurde über Kieselgel (120 g Kartusche, n-Heptan/MtB-Ether-Gradient 20-100 % in 60 min) aufgereinigt. Es wurden 2,80 g der Titelverbindung erhalten.

| | |
|---|---|
| LCMS-rt: 1,60 min | [M+H]⁺: 336,0 (Met-a) |

### 03.070

### 1-(3-tert-Butyl-5-hydroxy-4-methoxy-phenyl)-ethanon)

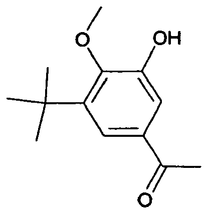

1-Brom-3-tert-butyl-5-(1,1-dimethoxy-ethyl)-2-methoxy-benzol (04.070; 36,3 g) wurde in THF (1 I) gelöst, unter Argon bei -75°C n-Butyllithium (52,6 ml; 2,5 M in Hexan) zugetropft und 30 min nachgerührt. Dann wurde Trimethylborat (37,3 ml) zugetropft und innerhalb von 2 h auf RT kommen gelassen. Anschließend wurden Natriumhydroxid (4,4 g, gelöst in 10 ml Wasser) und Wasserstoffperoxid-Lösung (62,3 ml; 35-%ig in Wasser) nacheinander zugegeben. Nach 2 h Rühren bei RT wurde über Nacht stehengelassen. Dann wurden Wasser und EE zugesetzt und mit Salzsäure sauer gestellt. Nach Abtrennen der EE-Phase wurde diese über Magnesiumsulfat getrocknet und eingeengt. Der Rückstand wurde über Kieselgel (330 g Kartusche, n-Heptan/EE-Gradient von 0-50% in 60 min) aufgereinigt. Es wurden 14 g der Titelverbindung erhalten.

| | |
|---|---|
| LCMS-rt: 0,90 min | [M+H]⁺: 223,1 (Met-b) |

### 03.073

### 3-tert-Butyl-5-(4-hydroxy-butoxy)-N-methoxy-N-methyl-benzamid

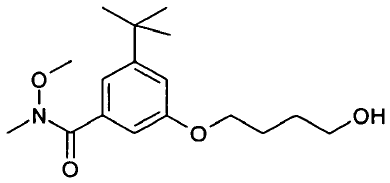

3-tert-Butyl-5-(4-hydroxy-butoxy)-benzoesäure (04.073; 4,13 g) wurde analog 03.059 umgesetzt und aufgearbeitet. Zur weiteren Reinigung wurde über Kieselgel wie folgt chromatographiert: 80 g Kartusche, n-Heptan/EE-Gradient von 0-100% in 60 min. Es wurden 3,47 g der Titelverbindung erhalten.

| | |
|---|---|
| LCMS-rt: 0,86 min | [M+H]⁺: 310,3 (Met-b) |

### 03.075

### N-(3-Acetyl-5-pentafluorsulfanyl-phenyl)-2,2,2-trifluor-acetamid

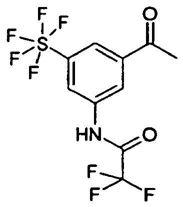

N-Methoxy-N-methyl-3-(pentafluorsulfanyl)-5-(2,2,2-trifluor-acetyfamino)-benzamid (04.075; 1,65 g) wurde in THF (25 ml) gelöst. Bei 0°C wurde unter Rühren Lithium-bis(trimethylsilyl)-amid (0,9 ml) zugegeben. Nach 30 min wurde Methylmagnesiumbromid (3,5 ml, 3 M in Diethylether) zugetropft. Nach beendeter Zugabe wurde das Eisbad entfernt und 2 h bei RT gerührt. Unter Kühlung wurden dann 1 N Salzsäure, Wasser und EE zugegeben. Nach Abtrennen der organischen Phase wurde die wässrige Phase noch zweimal mit EE extrahiert. Die vereinigten EE-Phasen wurden mit Magnesiumsulfat getrocknet, filtriert und eingeengt. Das Rohprodukt ist ein Gemisch aus N-(3-Acetyl-5-pentafluorsulfanyl-phenyl)-2,2,2-trifluor-acetamid und 1-[3-Amino-5-(pentafluorsulfanyl)-phenyl]-ethanon, so dass das Rohprodukt (1,3 g) in Methylenchlorid (60 ml) aufgenommen und mit Triethylamin (155 µl) versetzt wurde. Danach wurde unter Rühren Trifluoressigsäureanhydrid (160 µl) zugegeben. Nach 3 h Rühren bei RT wurden Wasser und gesättigte Natriumhydrogencarbonat-Lösung zugegeben, die Phasen getrennt und die DCM-Phase noch dreimal mit Wasser gewaschen. Die DCM-Phase wurde mit Magnesiumsulfat getrocknet, filtriert und eingeengt. Es wurden 1,3 g der Titelverbindung erhalten.

| | |
|---|---|
| LCMS-rt: 1,61 min | [M+H]⁺: 358,0 (Met-a) |

### 03.086

### 3-tert-Butyl-5-(3-hydroxy-2,2-dimethyl-propoxy)-N-methoxy-N-methyl-benzamid

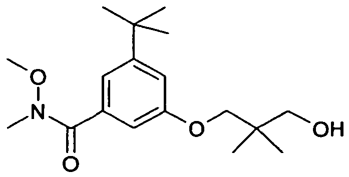

3-tert-Butyl-5-(3-hydroxy-2,2-dimethyl-propoxy)-benzoesäure (04.086; 940 mg) wurde analog 03.059 umgesetzt und aufgearbeitet. Es wurden 910 mg der Titelverbindung erhalten. LCMS-rt: 1,50 min [M+H]⁺: 324,1 (Met-a)

### 03.100

### 5-tert-Butyl-N-methoxy-N-methyl-isophthalamid-säure-methylester

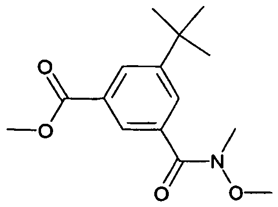

5-tert-Butyl-isophthalsäure-monomethylester (10,0 g) wurde analog 03.043 umgesetzt, aufgearbeitet und gereinigt. Es wurden 10,6 g der Titelverbindung erhalten. LCMS-rt: 1,06 min [M+H]⁺: 280,2 (Met-b)

### 03.106

### 1-(3-tert-Butyl-5-hydroxy-phenyl)-ethanon

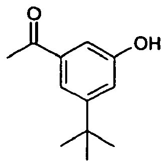

*m*-Chlorperbenzoesäure (382 g, 2,21 mol) wurde zu einer Lösung von 1,3-Bisacetyl-5-*tert*-butyl-benzol (04.106; 169 g, 776 mmol) in Dichlormethan (1,7 I) gegeben. Die resultierende Suspension wurde 24 h auf Rückflußtemperatur erhitzt. Nachdem die Suspension abgekühlt war wurde der Feststoff abfiltriert und das Filtrat wurde mit gesättigter wässriger NaHSO₃ Lösung versetzt (500 ml). Die Phasen wurden getrennt, die organische Phase wurde getrocknet (MgSO₄) und das Lösungsmittel wurde bei vermindertem Druck entfernt. Der Rückstand wurde in THF (500 ml) gelöst und mit Lithiumhydroxid (34,9 g, 1,46 mol) versetzt. Nach 4 h bei RT wurde wässrige Natriumhydroxid Lösung (1 M, 500 ml) zugegeben und die Mischung wurde mit MtB-Ether (3 500 m I) gewaschen. Die wässrige Phase wurde mit HCl (1 M) angesäuert (pH 3) und mit EE (3 500 m I) extrahiert. Die vereinigten organischen Phasen wurden getrocknet (MgSO₄) und das Lösungsmittel wurde bei vermindertem Druck entfernt. Das Rohprodukt wurde per präparativer HPLC (Met.-C) gereinigt. Das Produkt wurde als farbloses Öl, das langsam kristallisierte, erhalten (36,5 g).

| | |
|---|---|
| LCMS-rt: 1,30 min | [M+H]⁺: 193 (Met-a) |

### 04

### 04.004

### 1-(3-Brom-4-hydroxy-5-trifluormethyl-phenyl)-ethanon

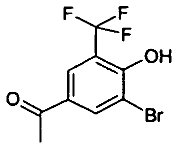

1-(4-Hydroxy-3-trifluormethyl-phenyl)-ethanon (5 g) wurde in Acetonitril (150 ml) unter Rühren bei RT vorgelegt und auf -10°C gekühlt. Bei dieser Temperatur wurde N-Bromsuccinimid (4,5 g, gelöst in 100 ml Acetonitril) zugetropft. Dann wurde das Kältebad entfernt und 5 h weiter gerührt. Nach Stehen über Nacht wurde ¾ des Lösungsmittels abgezogen und der Rückstand mit n-Heptan / Wasser versetzt. Die organische Phase wurde abgetrennt und einmal mit 5%iger Natriumthiosulfat-Lösung und einmal mit Wasser gewaschen. Der gebildete Niederschlag wurde abgesaugt, gewaschen und getrocknet. Es wurden 6,9 g der Titelverbindung in ausreichender Reinheit erhalten. LCMS-rt: 1,35 min [M+H]⁺: 283,0 (Met-a)

### 04.007

### 3-Methoxy-5-(pentafluorsulfanyl)-benzoesäure-methylester

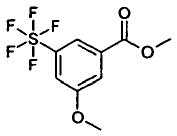

3-Hydroxy-5-(pentafluorsulfanyl)-benzoesäure (05.007; 3,0 g) wurde in absolutem DMF (75 ml) gelöst. Unter Rühren wurde dann Jodmethan (3,6 ml) zugegeben, gefolgt von feingepulvertem Kaliumcarbonat (6,3 g). Nach 5 Stunden Rühren bei 40°C wurde das Gemisch abgekühlt und mit Wasser versetzt (250 ml). Das Gemisch wurde dann viermal mit Ether (100 ml) extrahiert. Die vereinigten Extrakte wurden je ein Mal mit 1 N Natronlauge (75 ml) und Wasser (100 ml) gewaschen, über Magnesiumsulfat getrocknet, filtriert und eingeengt. Es wurden 2,9 g der gewünschten Verbindung erhalten.
¹H-NMR (400 MHz, CDCl₃) [ppm]: 8,00 (1 H), 7,70 (1 H), 7,47 (1 H), 3,96 (3 H), 3,90 (3 H)

### 04.008

### 3-tert-Butyl-5-ethoxymethyl-benzoesäure

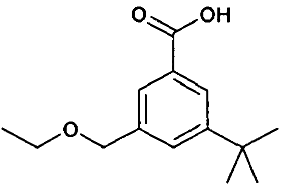

3-tert-Butyl-5-ethoxymethyl-benzoesäure-methylester (05.008; 1,18 g) wurde analog 04.043 umgesetzt. Das erhaltene Rohprodukt wurde aber anschließend über Kieselgel (50 g Kartusche, n-Heptan/EE-Gradient von 0-50% in 30 min) aufgereinigt. Es wurden 1,15 g der Titelverbindung erhalten.
¹H-NMR (400 MHz, DMSO-d₆) [ppm]: 12,90 (1 H), 7,86 (1 H), 7,73 (1 H), 7,57 (1 H), 4,50 (2 H), 3,50 (1 H), 1,30 (9 H), 1,16 (3 H)

### 04.010

### 1-Brom-3-tert-butyl-5-(1,1-dimethoxy-ethyl)-2-ethoxy-benzol

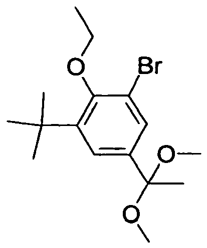

2-Brom-6-tert-butyl-4-(1,1-dimethoxy-ethyl)-phenol (20 g; Synthese siehe CA02515715) wurde mit Ethyliodid anlog der Bedingungen von 04.070 alkyliert. Es wurden 19,67 g der Titelverbindung erhalten.
¹H-NMR (500 MHz, DMSO-d6) [ppm]: 7,46 (1 H), 7,33 (1 H), 4,03 (2 H), 3,06 (6 H), 1,43 (3 H), 1,38 (3 H), 1,35 (9 H)

### 04.014

### 3-Methoxy-5-trifluormethyl-benzoesäuremethylester

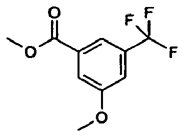

3-Hydroxy-5-trifluormethyl-benzoesäure (2 g) wurde bei RT in DMF (15 ml) unter Rühren vorgelegt und mit Methyliodid (3,0 ml) tropfenweise versetzt. Nach Zugabe von Kaliumcarbonat (5,6 g) wurde 5 h gerührt und über Nacht stehen gelassen. Dann wurde mit Wasser versetzt und dreimal mit MtB-Ether extrahiert. Die vereinigten MTB-Ether-Phasen trocknete man über Natriumsulfat, filtrierte und engte ein. Es wurdem 2,29 g der Titelverbindung erhalten.
¹H-NMR (500 MHz, DMSO-d6) [ppm]: 7,76 (1 H), 7,70 (1 H), 7,55 (1 H), 3,91 (3 H), 3,89 (3 H)

### 04.043

### 3-tert-Butyl-5-(2-methoxy-ethoxy)-benzoesäure

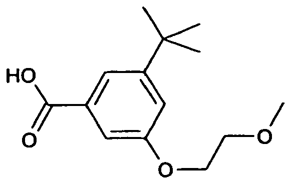

3-tert-Butyl-5-(2-methoxy-ethoxy)-benzoesäuremethylester (05.043; 2,0 g) wurde in Methanol (30 ml) und THF (60 ml) gelöst und Lithiumhydroxid-Lösung (30 ml, 1 M in Wasser) zugegeben. Es wurde auf 40°C erwärmt und 3 h gerührt. Dann wurden die organischen Lösungsmittel abgezogen und die wässrige Phase mit 1 N Salzsäure auf pH 3 gestellt. Es wurde mit EE extrahiert, getrocknet, filtriert und eingeengt. Es wurden 1,9 g der Titelverbindung erhalten.

| | |
|---|---|
| LCMS-rt: 1,40 min | [M+H-H₂O]⁺: 235,3 (Met-a) |

### 04.050

### 1-[3-Amino-5-(pentafluorsulfanyl)-phenyl]-ethanon

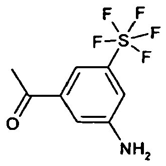

N-(3-Acetyl-5-pentafluorsulfanyl-phenyl)-2,2,2-trifluor-acetamid (03.075; 9,4 g) wurde mit halbkonzentrierter Schwefelsäure versetzt (200 ml) und DCM (15 ml) zugegeben. Nach 7 h Rühren bei 100°C wurde über Nacht stehen gelassen. Dann wurde der Ansatz auf Eiswasser gegeben und mit EE extrahiert. Die vereinigten EE-Phasen wurden mit gesättigter Natriumhydrogencarbonat-Lösung gewaschen, über Magnesiumsulfat getrocknet, filtriert und einrotiert. Es wurden 6,36 g der Titelverbindung erhalten. LCMS-rt: 1,38 min [M+H]⁺: 262,0 (Met-a)

### 04.059

### 3-tert-Butyl-5-[3-(tetrahydro-pyran-2-yloxy)-propoxy]-benzoesäure

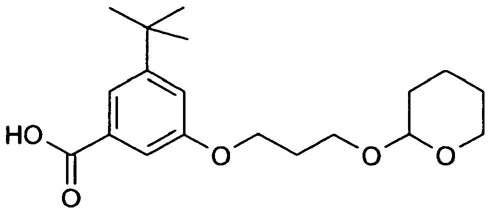

3-tert-Butyl-5-[3-(tetrahydro-pyran-2-yloxy)-propoxy]-benzoesäuremethylester (05.059; 5,37 g) wurde in Methanol (80 ml) und THF (160 ml) gelöst und Lithiumhydroxid-Lösung (61,28 ml, 1 M in Wasser) zugegeben. Nach 2 h Rühren bei 40°C wurde zur Trockne gebracht, der Rückstand mit Wasser aufgenommen und gefriergetrocknet. Das erhaltene Produkt wurde mit DCM verrührt, filtriert und zur Trockne gebracht. Es wurden 4,9 g Produkt erhalten.
¹H-NMR (500 MHz, DMSO-d6) [ppm] (repräsentative Signale): 7,52 (1 H), 7,26 (1 H), 6,80 (1 H), 4,57 (1 H, -O-C(-C)H-O-), 4,02 (2 H)

### 04.062

### 3-Ethoxy-5-(pentafluorsulfanyl)-benzoesäureethylester

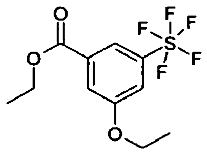

3-Hydroxy-5-(pentafluorsulfanyl)-benzoesäure (05.007; 4,76 g) wurde analog 04.007 mit Ethyliodid (7,27 ml) umgesetzt und aufgearbeitet. Es wurden 4,80 g der Titelverbindung erhalten.

| | |
|---|---|
| LCMS-rt: 1,97 min | [M+H]⁺: 321,0 (Met-a) |

### 04.070

### 1-Brom-3-tert-butyl-5-(1,1-dimethoxy-ethyl)-2-methoxy-benzol

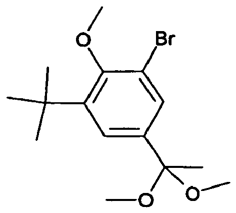

1-Brom-3-tert-butyl-5-(1,1-dimethoxy-ethyl)-2-methoxy-benzol wurde analog Patentanmeldung CA 02515715 synthetisiert.
¹H-NMR (500 MHz, DMSO-d6) [ppm]: 7,47 (1 H), 7,33 (1 H), 3,85 (3 H), 3,07 (6 H), 1,43 (3 H), 1,35 (9 H)

### 04.073

### 3-tert-Butyl-5-(4-hydroxy-butoxy)-benzoesäure

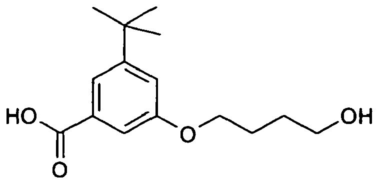

3-tert-Butyl-5-hydroxy-benzoesäure-methylester (06.043; 3,3 g) wurde analog 05.043 mit Essigsäure-4-brombutylester (3,71 g) umgesetzt und aufgearbeitet. Das Rohprodukt wurde direkt analog 04.086 mit Lithiumhydroxid zur Titelverbindung (4,13 g) verseift. LCMS-rt: 0,84 min [M+H]⁺: 267,1 (Met-b)

### 04.075

### N-Methoxy-N-methyl-3-(pentafluorsulfanyl)-5-(2,2,2-trifluor-acetylamino)-benzamid

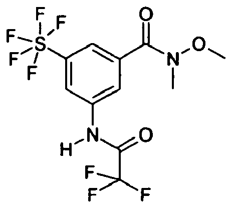

3-Amino-N-methoxy-N-methyl-5-pentafluorsulfanyl-benzamid (05.075; 1,45 g) wurde in Methylenchlorid (15 ml) gelöst und unter Rühren Triethylamin (0,8 ml) gefolgt von Trifluoressigsäureanhydrid (0,85 ml) unter Feuchtigkeitsausschluss zugesetzt. Nach 3 h Rühren bei RT und Stehen über Nacht wurden Wasser und gesättigte Natriumhydrogencarbonat-Lösung zugegeben, die Phasen getrennt und die Methylenchlorid-Phase noch drei Mal mit Wasser gewaschen, über Magnesiumsulfat getrocknet, filtriert und eingeengt. Das erhaltene Produkt (1,75 g) wurde ohne weitere Reinigung in der nächsten Stufe eingesetzt.

| | |
|---|---|
| LCMS-rt: 1,53 min | [M+H]⁺: 403,0 (Met-a) |

### 04.086

### 3-tert-Butyl-5-(3-hydroxy-2,2-dimethyl-propoxy)-benzoesäure

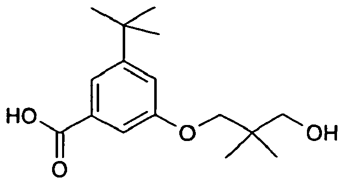

3-(3-Acetoxy-2,2-dimethyl-propoxy)-5-tert-butyl-benzoesäure-methylester (05.086; 1 g) wurde in Methanol/THF (15/30 ml) gelöst und Lithiumhydroxid-Lösung (11,89 ml, 1 M in Wasser) zugegeben. Nach 2 h Rühren bei 40°C wurde das organische Lösungsmittel abgezogen, der Rückstand mit Wasser verdünnt und mit 1 N Salzsäure auf pH 3 gestellt. Dann wurde mit EE extrahiert, die vereinigten organischen Phasen getrocknet, filtriert und eingeengt. Es wurden 940 mg der Titelverbindung erhalten.

| | |
|---|---|
| LCMS-rt: 1,45 min | [M+H]⁺: 281,1 (Met-a) |

### 04.106

### 1,3-Bisacetyl-5-tert-butyl-benzol

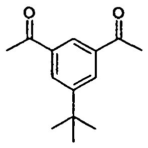

In einem 4-I-Dreihalskolben mit mechanischem Rührer wurde eine Lösung von 5-tert-Butyl-N,N'-dimethoxy-N,N'-dimethyl-isophthalamid (05.106; 254 g, 560 mmol) in THF (700 ml) innerhalb 2 h langsam zu Methylmagnesiumbromid (3 M in Et₂O, 1.49 l, 4.48 mol) getropft. Die Lösung wurde während der Zugabe gekühlt, so dass die Temperatur zwischen -10°C und +5°C gehalten wurde. Nach vollständiger Zugabe wurde die Kühlung entfernt und die Reaktionslösung wurde 16 h bei RT gerührt. Die Reaktionslösung wurde dann langsam in eine gekühlte Mischung aus HCl (1 M, 500 ml) und Eis gegeben. Während dieser Hydrolyse wurde der pH Wert durch die Zugabe von konzentrierter HCl zwischen 3 und 6 gehalten. Nach vollständiger Zugabe wurde MtB-Ether (1 l) zugegeben. Die Phasen wurden getrennt und die wässrige Phase wurde mit MtB-Ether (1 500 m l) extrahiert. Die vereinigten organischen Phasen wurden getrocknet (MgSO₄) und das Lösungsmittel wurde unter vermindertem Druck entfernt. Das Produkt wurde als farbloses Öl erhalten (123 g). LCMS-rt: 1,50 min [M+H]⁺: 219 (Met-a)

### 05

### 05.007

### 3-Hydroxy-5-(pentafluorsulfanyl)-benzoesäure

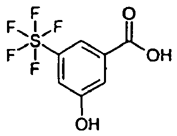

3-Amino-5-pentafluorsulfanyl-benzoesäure (06.007; 3,9 g) wurde in 35%iger Schwefelsäure (120 ml) gelöst, auf -5°C abgekühlt und innerhalb von 10 min eine Lösung aus Natriumnitrit (1,1 g) in Wasser (100 ml) zugetropft. Nach 40 min wurde weitere Nitrit-Lösung zugegeben (2 ml), nach jeweils weiteren 20 min noch einmal 2 ml und 1 ml. Dann wurde das Kältebad entfernt, und das Gemisch auf 100°C erhitzt. Nach 5 h wurde abgekühlt und die Lösung dekantiert. Die klare, saure Lösung wurde fünfmal mit Essigester extrahiert. Die vereinigten Extrakte wurden über Magnesiumsulfat getrocknet, filtriert und eingeengt. Das Rohprodukt wurde aus Essigester/Heptan umkristallisiert. Es wurden 3,6 g der gewünschten Verbindung erhalten.
¹H-NMR (400 MHz, DMSO-d₆) [ppm]: 10,72 (1 H); 7,71 (1 H); 7,57 (1 H); 7,46 (1H);

### 05.008

### 3-tert-Butyl-5-ethoxymethyl-benzoesäure-methylester

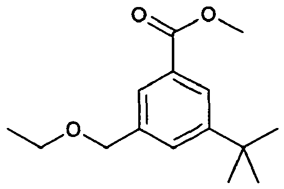

3-tert-Butyl-5-hydroxymethyl-benzoesäure-methylester (2,0 g) wurde analog der Bedingungen von 05.043 mit Ethyliodid alkyliert. Es wurden 1,18 g der Titelverbindung erhalten. LCMS-rt: 3,81 min [M+H]⁺: 250,2 (Met-d)

### 05.032

### 3-Brom-5-methoxy-benzoesäure-methylester

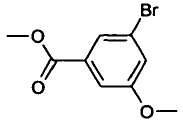

3-Brom-5-hydroxy-benzoesäure-methylester (06.032; 2,52 g) wurde analog der Bedingungen von 05.043 mit Methyliodid alkyliert und aufgearbeitet. Es wurden 2,5 g der Titelverbindung erhalten. LCMS-rt: 1,58 min [M+H]⁺: 245,0 (Met-a)

### 05.043

### 3-tert-Butyl-5-(2-methoxy-ethoxy)-benzoesäuremethylester

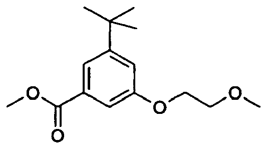

3-tert-Butyl-5-hydroxy-benzoesäuremethylester (06.043; 2,18 g) und 1-Brom-2-methoxy-ethan (1,18 ml) wurden in DMF (30 ml) gelöst und unter Rühren Natriumhydrid (301 mg) zugesetzt. Nach 2 h Rühren bei RT wurde das Lösungsmittel abgezogen. Der Rückstand wurde in EE aufgenommen, das Gemisch mit Wasser gewaschen, getrocknet und eingeengt. Der Rückstand wurde über Kieselgel (80 g Kartusche, n-Heptan/MtB-Ether-Gradient von 0-30% in 60 min) aufgereinigt. Es wurden 2,0 g der Titelverbindung erhalten.

| | |
|---|---|
| LCMS-rt: 1,69 min | [M+H-HOCH₃]⁺: 235,2 (Met-a) |

### 05.059

### 3-tert-Butyl-5-[3-(tetrahydro-pyran-2-yloxy)-propoxy]-benzoesäuremethylester

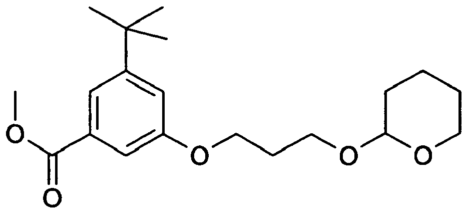

3-tert-Butyl-5-hydroxy-benzoesäuremethylester (06.043; 3,22 g) und 2-(3-Brom-propoxy)-tetrahydro-pyran (4,14 g) wurden in DMF (30 ml) gelöst und Natriumhydrid (445 mg) zugesetzt. Nach 3 h Rühren bei RT wurde über Nacht stehen gelassen. Dann wurde das DMF abgezogen und der Rückstand in EE aufgenommen, mit Wasser gewaschen, getrocknet, filtriert und eingeengt. Es wurden 5,37 g Produkt erhalten.
¹H-NMR (500 MHz, DMSO-d6) [ppm] (repräsentative Signale): 7,56 (1 H), 7,28 (1 H), 7,19 (1 H), 4,57 (1 H, -O-C(-C)H-O-), 4,10 (2 H)

### 05.063

### 3-tert-Butyl-5-[2-(tetrahydro-pyran-2-yloxy)-ethoxymethyl]-benzoesäure-methylester

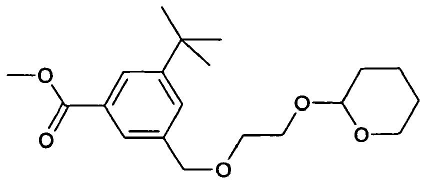

3-tert-Butyl-5-hydroxymethyl-benzoesäure-methylester (3,5 g) wurde mit 2-(2-Brom-ethoxy)-tetrahydro-pyran (3,95 g) analog 05.059 alkyliert. Allerdings wurde zur Reinigung eine Kieselgel-Chromatographie (n-Hept/EE 4:1) durchgeführt. Es wurden 2,28 g der Titelverbindung erhalten.
¹H-NMR (500 MHz, DMSO-d6) [ppm] (repräsentative Signale): 7,87 (1 H); 7,76 (1 H); 7,63 (1 H); 4,60 (1 H, -O-C(-C)H-O-); 4,57 (2 H)

### 05.072

### 3-tert-Butyl-5-(2-methoxy-1-methoxymethyl-ethoxy)-benzoesäure-methylester

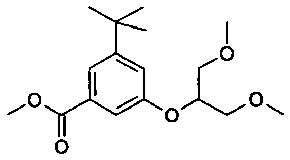

3-tert-Butyl-5-hydroxy-benzoesäure-methylester (06.043; 2,28 g) und Methansulfonsäure-2-methoxy-1-methoxymethyl-ethylester (2,93 g) wurden in DMF (50 ml) gelöst und Cäsiumcarbonat (4,82 g) zugesetzt. Nach 6 Rühren bei 50°C wurde über Nacht stehen gelassen. Nach 4 weiteren Stunden Rühren bei 50°C wurde ein weiteres eq. Cäsiumcarbonat und ein weiteres eq. des Mesylats zugegeben, weitere 4 h bei 50°C gerührt und über Nacht stehen gelassen. Dann wurde weitere 8 h bei 50°C gerührt, 6 Tage stehen gelassen und anschließend weitere 0,5 eq Cäsiumcarbonat und Mesylat zugegeben. Nach weiteren 8 h Rühren bei 50°C ließ man 5 Tage stehen, zog dann das DMF ab und nahm den Rückstand mit DCM/Wasser auf. Die DCM-Phase wurde abgetrennt und eingeengt. Der Rückstand wurde über Kieselgel (120 g Kartusche, n-Hept/MTB-Ether-Gradient 0-50% in 60min) gereinigt. Es wurden 2,05 g der Titelverbindung erhalten.

| | |
|---|---|
| LCMS-rt: 1,72 min | [M+H-HOCH₃]⁺: 279,1 (Met-a) |

### 05.075

### 3-Amino-N-methoxy-N-methyl-5-pentafluorsulfanyl-benzamid

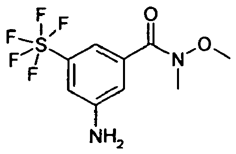

N-Methoxy-N-methyl-5-nitro-3-pentafluorsulfanyl-benzamid (06.075; 4,2 g) wurde in Methanol (120 ml) gelöst und Raney-Nickel (ca. 700 mg) zugegeben. Mit aufgesetztem Wasserstoffballon wurde auf einem Magnetrührer hydriert. Nach 5 h wurde der Katalysator abfiltriert und mit Methanol gewaschen. Das Filtrat wurde im Vakuum eingeengt und der Rückstand mittels präparativer Chromatographie aufgereinigt. Die Produkt enthaltenden Fraktionen wurden vereinigt, vom Acetonitril befreit, mit Natriumhydrogencarbonat-Lösung basisch gestellt und mit Essigester drei Mal extrahiert. Die vereinigten Extrakte wurden über Magnesiumsulfat getrocknet, filtriert und eingeengt. Es wurden 1,73 g der gewünschten Verbindung erhalten. LCMS-rt: 1,27 min [M+H]⁺: 307,0 (Met-a)

### 05.086

### 3-(3-Acetoxy-2,2-dimethyl-propoxy)-5-tert-butyl-benzoesäure-methylester

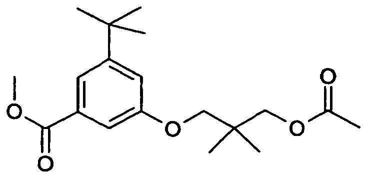

3-tert-Butyl-5-hydroxy-benzoesäuremethylester (06.043; 1,79g)wurde analog 02.85 mit Essigsäure-3-brom-2,2-dimethyl-propylester umgesetzt aufgearbeitet und gereinigt. Es wurden 1,01 g der Titelverbindung erhalten.

| | |
|---|---|
| LCMS-rt: 2,01 min | [M+H]⁺: 337,1 (Met-a) |

### 05.106

### 5-tert-Butyt-N,N'-dimethoxy-N,N'-dimethyl-isophthalamid

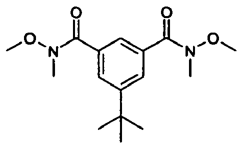

In einem 4-I-Dreihalskolben mit Rückflusskühler und mechanischem Rührer wurde 5-*tert*-Butyl-isophthalsäure (300 g, 1,35 mol) zu Thionylchlorid (1,60 l, 2,62 kg, 22,0 mol) gegeben. Die resultierende Suspension wurde bei 80°C gerührt. Nach 4 h war eine klare Lösung entstanden und das Lösungsmittel wurde abdestilliert. Der Rückstand wurde daraufhin im Vakuum getrocknet und anschließend in Dichlormethan (1,5 l) suspendiert. Diese Suspension wurde auf 0°C gekühlt und N,O-Dimethylhydroxylamin-Hydrochlorid (395 g, 4,05 mol) wurde zugegeben. Anschließend wurde Triethylamin (1,50 l, 1,09 kg, 10,8 mol) langsam zugetropft, so dass die Temperatur 15°C nicht überstieg. Nachdem 16 h bei RT gerührt worden war, wurde Wasser zugegeben (1 l). Die Phasen wurden getrennt und die organische Phase wurde mit gesättigter NH₄Cl Lösung (2 500 ml), gesättigter NaHCO₃ Lösung (2 500 ml ) und gesättigter NaCl Lösung (1 500 ml) gewaschen. Die organische Phase wurde getrocknet (MgSO₄) und das Lösungsmittel wurde bei vermindertem Druck entfernt. Das Produkt wurde als weißer Feststoff erhalten (405 g).

| | |
|---|---|
| LCMS-rt: 1,22 min | [M+H]⁺: 309 (Met-a) |

### 06

### 06.007

### 3-Amino-5-pentafluorsulfanyl-benzoesäure

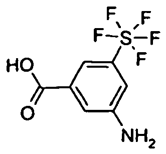

3-Pentafluorsulfanyl-benzoesäure (15 g) wurde in rauchender Salpetersäure (120 ml) gelöst und bei RT unter Feuchtigkeitsausschluss gerührt. Dann wurde konzentrierte Schwefelsäure (7,5 ml) zugegeben und bei 75 °C gerührt. Nach 8 h Rühren bei 75°C wurde über Nacht stehen gelassen, dann weitere Schwefelsäure (1,5 ml) zugegeben und 8 h unter Rühren auf 75°C erhitzt. Nach Stehenlassen über Nacht wurde auf Eiswasser gegeben und 2 h gerührt. Dann wurde der Niederschlag abgesaugt und am Hochvakuum getrocknet. Es wurden 13,7 g 3-Pentafluorsulfanyl-5-nitro-benzoesäure erhalten.

Anschließend wurde die 3-Pentafluorsulfanyl-5-nitro-benzoesäure (5 g) in Methanol (300 ml) gelöst, Raney-Nickel (etwa 750 mg) zugegeben und unter Wasserstoffatmosphäre (Wasserstoffballon) hydriert. Nach 3 h wurde der Katalysator abfiltriert und der Filterrückstand gut mit Methanol gewaschen. Das Filtrat wurde eingeengt und getrocknet. Der Rückstand wurde über Kieselgel (2 x 50 g Kartusche, n-Heptan/EE-Gradient von 0-100% in 60 min) aufgereinigt. Es wurden 3,9 g der Titelverbindung erhalten.
¹H-NMR (400 MHz, DMSO-d₆) [ppm]: 13,30 (1 H); 7,37 (2 H); 7,23 (1 H); 5,98 (2 H)

### 06.032

### 3-Brom-5-hydroxy-benzoesäure-methylester

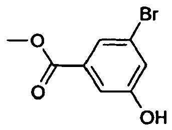

3-Brom-5-hydroxy-benzoesäure (3 g) wurde analog 06.043 in die Titelverbindung übergeführt. Es wurden 2,52 g erhalten.
¹H-NMR (500 MHz, DMSO-d₆) [ppm]: 10,40 (1 H); 7,47 (1 H); 7,33 (1 H); 7,22 (1 H); 3,84 (3 H);

### 06.043

### 3-tert-Butyl-5-hydroxy-benzoesäuremethylester

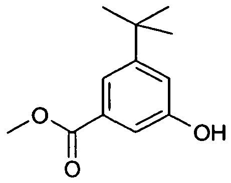

3-tert-Butyl-5-hydroxy-benzoesäure (07.043; 1,93 g) wurde in Methanol (20 ml) gelöst und unter Rühren langsam Thionylchlorid (0,937 ml) zugetropft. Nach 1 h Rühren bei 65°C gerührt wurde zur Trockne gebracht, der Rückstand in DCM aufgenommen, die Lösung mit gesättigter Natriumhydrogencarbonat-Lösung gewaschen, über MgSO4 getrocknet, filtriert und einrotiert. Es wurden 2,19 g der Titelverbindung erhalten.

| | |
|---|---|
| LCMS-rt: 1,44 min | [M+H]⁺: 209,2 (Met-a) |

### 06.075

### N-Methoxy-N-methyl-5-nitro-3-pentafluorsulfanyl-benzamid

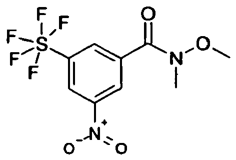

3-Pentafluorsulfanyl-benzoesäure (5,0 g) wurde in rauchender Salpetersäure (20 ml) gelöst und bei RT unter Feuchtigkeitsausschluss gerührt. Dann wurde konzentrierte Schwefelsäure (3 ml) zugegeben und bei 75 °C gerührt. Nach 5 h Rühren bei 75°C wurde weitere Schwefelsäure (2 ml) zugegeben und weitere 2 h bei 75°C gerührt. Nach Stehenlassen über Nacht wurde auf Eiswasser gegeben und 2 h gerührt. Dann wurde der Niederschlag abgesaugt und am Hochvakuum getrocknet. Es wurden 4,2 g 3-Pentafluorsulfanyl-5-nitro-benzoesäure erhalten. Weitere 900 mg konnten aus der Mutterlauge nach dreimaligem Extrahieren mit Methylenchlorid, Trockenen der vereinigten Methylenchlorid-Phasen über Magnesiumsulfat und Einengen des Lösungsmittels erhalten werden. Anschließend wurden 4,0 g der 3-Pentafluorsulfanyl-5-nitro-benzoesäure unter Rühren in Thionylchlorid (25 ml) gelöst und 10 h unter Feuchtigkeitsausschluss unter Rückfluss gehalten. Nach Stehen über Nacht bei RT wurde überschüssiges Thionylchlorid im Vakuum entfernt, der erhaltene Rückstand in Dichlormethan (50 ml) gelöst und unter Rühren mit N,O-Dimethylhydroxylamin x HCl (1,25 g) und Diethylisopropylamin (1,66 g) versetzt. Nach 1 h Rühren bei RT wurde das Gemisch im Vakuum eingeengt, der Rückstand in Essigester gelöst und fünfmal mit Wasser gewaschen. Die organische Phase wurde über Magnesiumsulfat getrocknet, filtriert und eingeengt. Die erhaltenen 4,2 g Rohprodukt wurden direkt in der nächsten Stufe eingesetzt.

| | |
|---|---|
| LCMS-rt: 1,50 min | [M+H]⁺: 337,0 (Met-a) |

### O7

### O7.043

### 3-tert-Butyl-5-hydroxy-benzoesäure

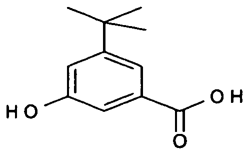

3-Brom-5-tert-butyl-benzoesäure (5 g) wurde in THF (180 ml) gelöst und unter Argon und bei -75°C n-Butyllithium (18,7 ml, 2,5 M in Hexan) zugetropft und 30 min nachgerührt. Dann wurde Trimethylborat (6,63 ml) zugetropft und innerhalb 1 h auf RT kommen gelassen. Danach wurde Natriumhydroxid (0,778 g), gelöst in 2 ml Wasser, und Wasserstoffperoxid (12,89 ml, 30 %) nacheinander zugegeben. Nach 3 h Rühren bei RT wurde übers Wochenende stehen gelassen. Dann wurden Wasser und EE zugegeben, mit 1 N Salzsäure auf pH 3 gestellt und die EE-Pase abgetrennt. Diese wurde dreimal mit Wasser gewaschen, getrocknet, filtriert und eingeengt. Der Rückstand wurde über Kieselgel (120 g Kartusche, n-Heptan/MtB-Ether-Gradient von 0-50% in 60 min) aufgereinigt. Es wurden 1,94 g der Titelverbindung erhalten.

| | |
|---|---|
| LCMS-Rt: 1,18 min | [M+H]⁺: 195,1 (Met-a) |

### Beispiel 1

### N-[3-[2-(6-Ethoxy-3-imino-[1,2,4]triazolo[4,3-b]pyridazin-2-yl)-acetyl]-5-(pentafluorsulfanyl)-phenyl]-acetamid als Trifluoressigsäuresalz

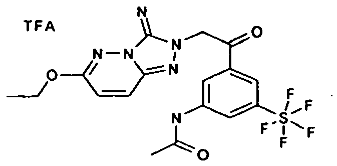

### a) 3-Nitro-5-pentafluorsulfanyl-benzoesäure

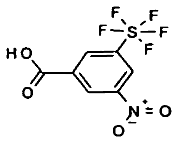

3-Pentafluorsulfanyl-benzoesäure (5,0 g) wurde in rauchender Salpetersäure (20 ml) gelöst und bei RT unter Feuchtigkeitsausschluss gerührt. Dann wurde konzentrierte Schwefelsäure (3 ml) zugegeben und bei 75 °C gerührt. Nach 5 h Rühren bei 75°C wurde weitere Schwefelsäure (1,5 ml) zugegeben und nach 2 h Rühren bei 75°C über Nacht stehen gelassen. Dann wurde auf Eiswasser gegeben und 2 h gerührt. Der gebildete Niederschlag wurde abgesaugt und am Hochvakuum getrocknet. Es wurden 4,2 g 3-Pentafluorsulfanyl-5-nitro-benzoesäure erhalten. Weitere 900 mg konnten aus der Mutterlauge nach dreimaligem Extrahieren mit Methylenchlorid, Trockenen der vereinigten Methylenchlorid-Phasen über Magnesiumsulfat und Einengen des Lösungsmittels erhalten werden. Der Niederschlag wurde ohne weitere Reinigung in der nächsten Stufe eingesetzt.
¹H-NMR (400 MHz, DMSO-d₆) [ppm]: 8,82 (1 H); 8,80 (1 H); 8,62 (1 H)

### b) N-Methoxy-N-methyl-5-nitro-3-pentafluorsulfanyl-benzamid

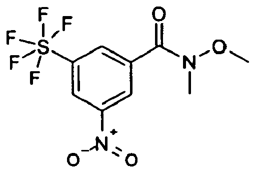

3-Nitro-5-pentafluorsulfanyl-benzoesäure (4,0 g) wurde unter Rühren in Thionylchlorid (25ml) gelöst und 10 h unter Feuchtigkeitsausschluss unter Rückfluss gehalten. Nach Stehen über Nacht bei RT wurde überschüssiges Thionylchlorid im Vakuum entfernt, der erhaltene Rückstand in Dichlormethan (50 ml) gelöst und unter Rühren mit N,O-Dimethylhydroxylamin-Hydrochlorid (1,25 g) und Diethylisopropylamin (1,66 g) versetzt. Nach 1 h Rühren bei RT wurde das Gemisch im Vakuum eingeengt, der Rückstand in Essigester gelöst und 5 Mal mit Wasser gewaschen. Die organische Phase wurde über Magnesiumsulfat getrocknet, filtriert und eingeengt. Das erhaltene Rohprodukt (4,2 g) wurde direkt in der nächsten Stufe eingesetzt. LCMS-rt: 1,50 min [M+H]⁺: 337,0 (Met-a)

### c) 3-Amino-N-methoxy-N-methyl-5-pentafluorsulfanyl-benzamid

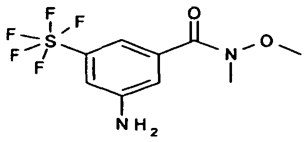

N-Methoxy-N-methyl-5-nitro-3-pentafluorsulfanyl-benzamid (4,2 g) wurde in Methanol (120 ml) gelöst und Raney - Nickel (ca. 700 mg) zugegeben. Mit aufgesetztem Wasserstoffballon wurde auf einem Magnetrührer hydriert. Nach 5 h wurde der Katalysator abfiltriert und mit Methanol gewaschen. Das Filtrat wurde im Vakuum eingeengt und der Rückstand mittels präparativer Chromatographie aufgereinigt. Die produktenthaltenden Fraktionen wurden vereinigt, vom Acetonitril befreit, mit Natriumhydrogencarbonat-Lösung basisch gestellt und mit Essigester drei Mal extrahiert. Die vereinigten Extrakte wurden über Magnesiumsulfat getrocknet, filtriert und eingeengt. Es wurden 1,73 g der gewünschten Verbindung erhalten.

| | |
|---|---|
| LCMS-rt: 1,27 min | [M+H]⁺: 307,0 (Met-a) |

### d) 3-Acetylamino-N-methoxy-N-methyl-5-(pentafluorsulfanyl)-benzamid

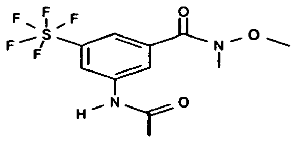

3-Amino-N-methoxy-N-methyl-5-pentafluorsulfanyl-benzamid (1,2 g) wurde in Methylenchlorid (15 ml) gelöst und unter Rühren Triethylamin (0,7 ml) gefolgt von Essigsäureanhydrid (1,75 ml) unter Feuchtigkeitsausschluss zugesetzt. Nach 3 h Rühren bei RT wurden Wasser und gesättigte Natriumhydrogencarbonat-Lösung zugegeben, die Phasen getrennt und die Methylenchlorid-Phase noch drei Mal mit Wasser gewaschen, über Magnesiumsulfat getrocknet, filtriert und eingeengt. Das erhaltene Produkt (1,3 g) wurde ohne weitere Reinigung in der nächsten Stufe eingesetzt. LCMS-rt: 1,26 min [M+H]⁺: 349,0 (Met-a)

### e) N-[3-Acetyl-5-(pentafluorsulfanyl)-phenyl]-acetamid

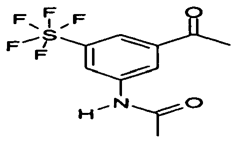

3-Acetylamino-N-methoxy-N-methyl-5-(pentafluorsulfanyl)-benzamid (1,2 g) wurde in absolutem THF (30 ml) gelöst und bei 0 °C mit Lithiumhexmethyldisilazan (721 µl; Dichte: 0,8 g/l; 23%-ig in tert.-Butylmethylether) 30 min gerührt. Bei 0°C wurde dann unter Rühren Methylmagnesiumbromid (2,87 ml, 3 M in Diethylether) zugetropft. Nach 2,5 h Rühren bei RT wurde weiteres Methylmagnesiumbromid (1 ml, 3 M in Diethylether) zugegeben und erneut 2,5 h gerührt. Zur Aufarbeitung wurde unter Eiskühlung 1 N Salzsäure zugetropft, gefolgt von Wasser und Essigester. Die organische Phase wurde abgetrennt und die Wasserphase noch zwei Mal mit Essigester extrahiert. Die vereinigten Essigesterphasen wurden über Natriumsulfat getrocknet, filtriert und eingeengt. Das Rohprodukt (1,03 g) wurde mit einem in gleicher Weise hergestellten Rohprodukt (75 mg) vereinigt und über Kieselgel mit Dichlormethan-Methanol als Laufmittel aufgereinigt. Es wurden 860 mg der gewünschten Verbindung erhalten. LCMS-t: 1,34 min [M+H]⁺: 304,0 (Met-a)

### f) N-[3-(2-Brom-acetyl)-5-(pentafluorsulfanyl)-phenyl]-acetamid (O1.001)

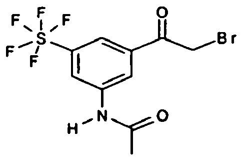

N-[3-Acetyl-5-(pentafluorsulfanyl)-phenyl]-acetamid (859 mg) wurde in einem Gemisch aus Methanol (10 ml) und THF (10 ml) gelöst und unter Rühren portionsweise Phenyl-trimethyl-ammonium-tribromid (1,065 g) zugegeben. Nach 2 h Rühren bei RT wurde weitere 3 h auf 40°C erwärmt. Nach dem Erkalten wurde das Reaktionsgemisch in 2 N Schwefelsäure gegeben und die wässrige Phase 3 Mal mit Essigester extrahiert. Die vereinigten Extrakte wurden über Natriumsulfat getrocknet, filtriert und eingeengt. Das Rohprodukt wurde über Kieselgel mit Essigester/Heptan als Laufmittel aufgereinigt. Es wurden 480 mg der gewünschten Verbindung erhalten. LCMS-rt: 1,47 min [M+H]⁺: 382,0 (Met-a)

### g) 6-Chlor-[1,2,4]triazolo[4,3-b]pyridazin-3-ylamin als Hydrobromid und 6-Chlor-[1,2,4]triazolo[4,3-b]pyridazin-3-ylamin

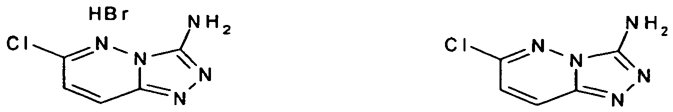

(6-Chlor-pyridazin-3-yl)-hydrazin (1 g) wurde in einer Mischung aus Ethanol (22,5 ml) und Wasser (9 ml) bei RT unter Rühren gelöst. Danach tropfte man Bromcyan (2,8 ml, 5 M in Acetonitril) zu. Nach 6 h Rühren und Stehenlassen über Nacht wurde der Niederschlag abgesaugt und getrocknet. Auf diese Weise wurden 1,14 g des gewünschten Produkts als Hydrobromid erhalten.

| | |
|---|---|
| LCMS-rt: 0,24 min | [M+H]⁺: 170,1 (Met-a) |

Weiteres Produkt in Form der freien Base wurde erhalten, indem die Mutterlauge mit gesättigter Kaliumcarbonat-Lösung basisch gestellt wurde. Der dabei gebildete Niederschlag wurde abgesaugt und getrocknet (326 mg).

| | |
|---|---|
| LCMS-t: 0,24 min | [M+H]⁺: 170,1 (Met-a) |

### h) 6-Ethoxy-[1,2,4]triazolo[4,3-b]pyridazin-3-ylamin

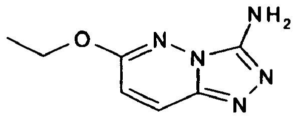

6-Chloro-[1,2,4]triazolo[4,3-b]pyridazin-3-ylamin-Hydrobromid (1,1 g) wurde in viel Wasser aufgenommen und mit gesättigter Kaliumcarbonat-Lösung alkalisch gestellt. Der dabei ausgefallene Niederschlag wurde abgesaugt und getrocknet (388 mg). Mehrmaliges Extrahieren der Mutterlauge mit Dichlormethan, Trockenen der vereinigten organischen Phasen über Natriumsulfat, Filtrieren und Einengen lieferte insgesamt weitere 228 mg Produkt.

Die erhaltene freie Base (616 mg) wurde in absolutem Ethanol (40 ml) gelöst und protionsweise mit festem Natriumethylat (990 mg) versetzt. Nach 2 h Rühren bei 55°C wurde Wasser zugegeben und die wässrige Phase dreimal mit Dichlormethan extrahiert. Die vereinigten Extrakte wurden über Natriumsulfat getrocknet, filtriert und eingeengt. Es wurden 709 mg der gewünschten Verbindung erhalten.

| | |
|---|---|
| LCMS-rt: 0,51 min | [M+H]⁺: 180,1 (Met-a) |

### i) N-[3-[2-(6-Ethoxy-3-imino-[1,2,4]triazolo[4,3-b]pyridazin-2-yl)-acetyl]-5-(pentafluorsulfanyl)-phenyl]-acetamid als Trifluoressigsäuresalz

6-Ethoxy-[1,2,4]triazolo[4,3-b]pyridazin-3-ylamin (W1.001; 40 mg) wurde in absolutem DMF (3,5 ml) unter Rühren vorgelegt und tropfenweise N-[3-(2-Brom-acetyl)-5-(pentafluorsulfanyl)-phenyl]-acetamid (O1.001; 94 mg), gelöst in absolutem DMF (1,5 ml), zugegeben. Nach 5 h Rühren bei RT und Stehenlassen über Nacht wurde das Lösungsmittel abgezogen und der Rückstand mittels präparativer Chromatographie (Met-A) aufgereinigt. Die produktenthaltenden Fraktionen wurden vereinigt, vom Acetonitril befreit und gefriergetrocknet (90 mg). Zur Abtrennung des kationischen Isomers wurde eine weitere Reinigung über Kieselgel mit einem

Dichlormethan/Methanol-Gradienten durchgeführt. Die Fraktionen des gesuchten, zuerst eluierenden Produkts wurden vereinigt und zur Trockne gebracht. Der Rückstand wurde dann mittels präparativer Chromatographie (Met-A) weiter aufgereinigt. Die produktenthaltenden Fraktionen wurden vereinigt, vom Acetonitril befreit und gefriergetrocknet. Es wurden 42 mg der gewünschten Verbindung erhalten.

| | |
|---|---|
| LCMS-rt: 1,12 min | [M+H]⁺: 481,0 (Met-a) |

### Beispiel 2

### 2-(6-Chlor-3-imino-[1,2,4]triazolo[4,3-b]pyridazin-2-yl)-1-(3,5-di-tert-butyl-4-hydroxyphenyl)-ethanon

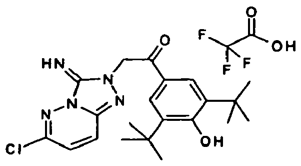

6-Chlor-[1,2,4]triazolo[4,3-b]pyridazin-3-ylamin (W2.001a; 30 mg) wurde in DMF (3,5 ml) bei RT unter Rühren vorgelegt und 2-Brom-1-(3,5-di-tert-butyl-4-hydroxyphenyl)-ethanon (O1.002; 58 mg) in DMF (0,5 ml) vorgelöst zugetropft. Das Reaktionsgemisch rührte 30 min. bei RT und wurde dann 5 h auf 60°C erwärmt. Nach Stehen über Nacht wurde das Lösungsmittel im Vakuum entfernt und der Rückstand mittels präparativer HPLC (Met-A) aufgereinigt. Die sauberen Produktfraktionen wurden vereinigt, im Vakuum vom Acetonitril befreit und gefriergetrocknet. Es wurden 28 mg der Titelverbindung erhalten. LCMS-rt: 1,28 min [M+H]⁺: 416,1 (Met-a)

Analog Beispiel 2 wurden erhalten:

| | | | | | | |
|---|---|---|---|---|---|---|
| Bsp 3 | 1-(3-tert-Butyl-4-methoxy-5-morpholin-4-yl-phenyl)-2-(3-imino-6-isopropoxy-[1,2,4]triazolo[4,3-b]pyridazin-2-yl)-ethanon-Trifluoressigsäuresalz (16 mg) | | | | | |
| | 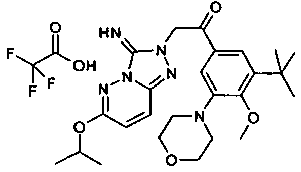 | W | O | LCMS- | [M+H]⁺ | |
| | | 1.003 | 1.003 | rt [min] | | |
| | | | | | 483,3 | |
| | | 15 mg | 29 mg | 1,35 | (Met-a) | |
| Bsp 4 | 1-(3-tert-Butyl-4-methoxy-5-morpholin-4-yl-phenyl)-2-(3-imino-6-methoxy-[1,2,4]triazolo[4,3-b]pyridazin-2-yl)-ethanon ethanon- Trifluoressigsäuresalz (34 mg) | | | | | |
| | 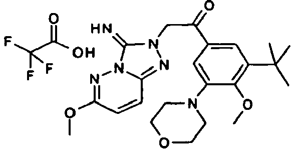 | W | O | LCMS- | [M+H]⁺ | |
| | | 1.002 | 1.003 | rt [min] | | |
| | | | | | 455,1 | |
| | | 30 mg | 74 mg | 1,24 | (Met-a) | |

### Beispiel 5

### 2-(6-Ethoxy-3-imino-[1,2,4]triazolo[4,3-b]pyridazin-2-yl)-1-(4-methoxy-3-morpholin-4-yl-5-trifluormethyl-phenyl)-ethanon-Trifluoressigsäuresalz

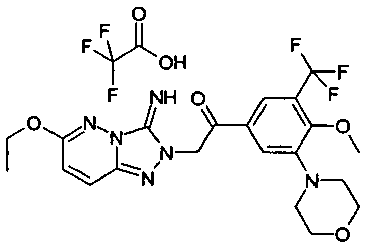

6-Ethoxy-[1,2,4)triazolo[4,3-b)pyridazin-3-ylamin (W1.001; 10 mg) wurde in DMF (3,5 ml) bei RT unter Rühren vorgelegt und 2-Brom-1-(4-methoxy-3-morpholin-4-yl-5-trifluoromethyl-phenyl)-ethanon (O1.004; 21 mg) in DMF (1,5 ml) vorgelöst zugetropft. Das Reaktionsgemisch rührte 2 h bei RT. Nach Stehen über Nacht wurde das Lösungsmittel im Vakuum entfernt und der Rückstand mittels präparativer HPLC (Met-A) aufgereinigt. Die sauberen Produktfraktionen wurden vereinigt, im Vakuum vom Acetonitril befreit und gefriergetrocknet. Die mit 1-Substitutionsprodukt verunreinigte Titelverbindung wurde über Kieselgel mit Dichlormethan/Methanol-Gradienten gereinigt. Die sauberen Produktfraktionen wurden zur Trockne gebracht, mit wenig Acetonitril/Wasser+0,05 % TFA aufgenommen und gefriergetrocknet. Es wurden 7 mg der Titelverbindung erhalten.

| | |
|---|---|
| LCMS-rt: 1,20 min | [M+H]⁺: 481,1 (Met-a) |

Analog obiger Beispiele wurden erhalten:

| | | | | | | |
|---|---|---|---|---|---|---|
| Bsp 6 | N-[3-[2-(6-Ethoxy-3-imino-[1,2,4]triazolo[4,3-b]pyridazin-2-yl)-acetyl]-5-(pentafluorsulfanyl)-phenyl]-N-methyl-acetamid-Trifluoressigsäuresalz (6 mg) | | | | | |
| | 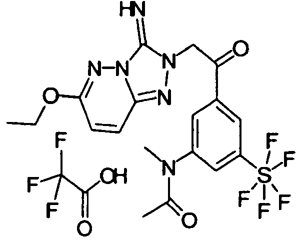 | W | O | LCMS- | [M+H]⁺ | |
| | | 1.001 | 1.005 | rt [min] | | analog Bsp. |
| | | | | | 495,0 | 5 |
| | | 15 mg | 29 mg | 1,10 | (Met-a) | |
| Bsp 7 | 1-(3-tert-Butyl-4-methoxy-5-morpholin-4-yl-phenyl)-2-(6-ethoxy-3-imino-[1,2,4]triazolo[4,3-bJpyridazin-2-yl)-ethanon-Trifluoressigsäuresalz (15 mg) | | | | | |
| | 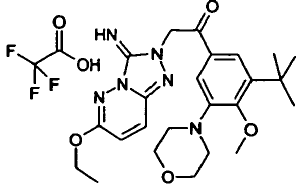 | W | O | LCMS- | [M+H]⁺ | |
| | | 1.001 | 1.003 | rt [min] | | analog Bsp. |
| | | | | | 469,2 | 2 |
| | | 30 mg | 62 mg | 1,34 | (Met-a) | |
| Bsp 8 | 1-(3-tert-Butyl-4-methoxy-5-morpholin-4-yl-phenyl)-2-(6-cyclopentyloxy-3-imino-[1,2,4]triazolo[4,3-b]pyridazin-2-yl)-ethanon-Trifluoressigsäuresalz (4 mg) | | | | | |
| | 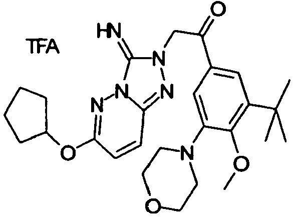 | W | O | | [M+H]⁺ | analog Bsp. 5, |
| | | 1.007 | 1.003 | LCMS-rt [min] | | aber in Gegenwart von 7,3 µl |
| | | | | | 509,2 | |
| | | 15 mg | 18 mg | 1,44 | (Met-a) | TEA |
| Bsp 9 | 1-(3-tert-Butyl-4-methoxy-5-morpholin-4-yl-phenyl)-2-(6-cyclobutoxy-3-imino-[1,2,4]triazolo[4,3-b]pyridazin-2-yl)-ethanon-Trifluoressigsäuresalz (41 mg) | | | | | |
| | 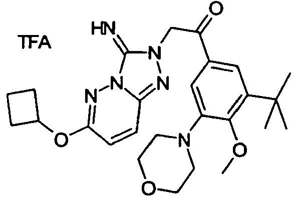 | W | O | LCMS- | [M+H]⁺ | |
| | | 1.004 | 1.003 | rt [min] | | analog Bsp. |
| | | | | | 495,2 | 5 |
| | | 15 mg | 18 mg | 1,40 | (Met-a) | |
| Bsp 10 | 1-(3-tert-B utyl-4-methoxy-5-morp holin-4-yl-phenyl)-2-(3-imino-6-p henoxy-[1,2,4]triazolo[4,3-b]pyridazin-2-yl)-ethanon-Trifluoressigsäuresalz (29 mg) | | | | | |
| | 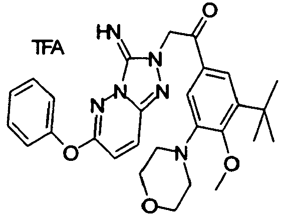 | W | W | LCMS- | [M+H]⁺ | |
| | | 1.013 | 1.003 | rt [min] | | analog Bsp 5 |
| | | | | | 517,3 | |
| | | 18 mg | 32 mg | 1,35 | (Met-a) | |
| Bsp 11 | 2-(6-Benzyloxy-3-imino-[1,2,4]triazolo[4,3-b]pyridazin-2-yl)-1 -(3-tert-butyl-4-methoxy-5-morpholin-4-yl-phenyl)-ethanon-Trifluoressigsäuresalz (27 mg) | | | | | |
| | 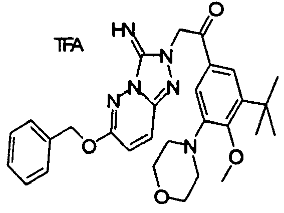 | W | O | LCMS- | [M+H]⁺ | |
| | | 1.005 | 1.003 | rt [min] | | analog Bsp. |
| | | | | | 531,2 | 5 |
| | | 20 mg | 34 mg | 1,40 | (Met-a) | |
| Bsp 12 | 1-(3-tert-Butyl-4-methoxy-5-morphotin-4-yl-phenyl)-2-(6-cyclohexyloxy-3-imino-[1,2,4]triazolo[4,3-b]pyridazin-2-yl)-ethanon-Trifluoressigsäuresalz (5 mg) | | | | | |
| | 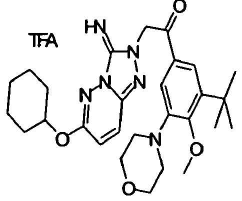 | W | O | LCMS | [M+H]⁺ | analog Bsp. 5, |
| | | 1.006 | 1.003 | -rt [min] | 523,3 | aber in Gegen-wart von 7,2 µl |
| | | 20 mg | 18 mg | 1,54 | (Met-a) | TEA |
| Bsp 13 | 1-(3-tert-Butyl-4-methoxy-5-morpholin-4-yl-phenyl)-2-[3-imino-6-(2,2,2-trifluor-ethoxy)-[1,2,4]triazolo[4,3-b]pyridazin-2-yl]-ethanon-Trifluoressigsäuresalz (25 mg) | | | | | |
| | 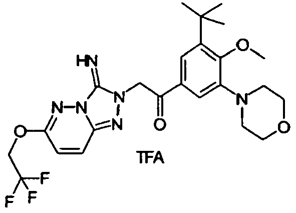 | W | O | LCMS- | [M+H]⁺ | |
| | | 1.014 | 1.003 | rt [min] | | analog Bsp. |
| | | | | | 523,2 | 5 |
| | | 23 mg | 39 mg | 1,33 | (Met-a) | |
| Bsp 14 | 1-(3-tert-Butyl-4-methoxy-5-morpholin-4-yl-phenyl)-2-[6-(1-ethyl-propoxy)-3-imino-[1,2,4]triazolo[4,3-b]pyridazin-2-yl]-ethanon-Trifluoressigsäuresalz (14 mg) | | | | | |
| | 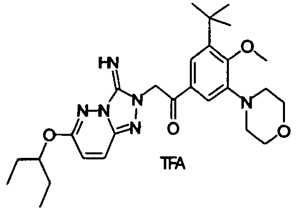 | W | O | LCMS- | [M+H]⁺ | |
| | | 1.008 | 1.003 | rt [min] | | analog Bsp. |
| | | | | | 511,2 | 5 |
| | | 20 mg | 37 mg | 1,48 | (Met-a) | |

### Beispiel 15

### 1-(3-tert-Butyl-4-methoxy-5-morpholin-4-yl-phenyl)-2-(6-cyclopropylmethoxy-3-imino-[1,2,4]triazolo[4,3-b]pyridazin-2-yl)-ethanon-Hydrochlorid

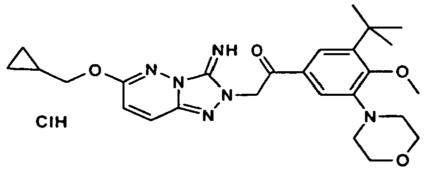

6-Cyclopropylmethoxy-[1,2,4]triazolo[4,3-b]pyridazin-3-ylamin (W1.009; 350 mg) wurde in DMF (5 ml) bei RT unter Rühren vorgelegt und 2-Brom-1-(3-tert-butyl-4-methoxy-5-morpholin-4-yl-phenyl)-ethanon (O1.003; 631 mg) in DMF (5 ml) vorgelöst zugetropft. Das Reaktionsgemisch rührte 14 h bei RT. Dann wurde das Lösungsmittel im Vakuum entfernt und der Rückstand wurde über Kieselgel (40 g Kartusche, Dichlormethan/- Ethanol-Gradient von 0-40% in 60 min) aufgereinigt. Das Produkt wurde mit wenig Acetonitril aufgenommen, mit Wasser verdünnt und nach Zugabe von 2 eq. HCl gefriergetrocknet. Es wurden 500 mg der Titelverbindung erhalten.

| | |
|---|---|
| LCMS-rt: 1,37 min | [M+H⁺]⁺: 495,2 (Met-a) |

Analog obiger Beispiele wurden erhalten:

| | | | | | | |
|---|---|---|---|---|---|---|
| Bsp 16 | 2-[6-(1-Ethyl-propoxy)-3-imino-[1,2,4]triazolo[4,3-b]pyridazin-2-yl]-1-[3-methyl-amino-5-(pentafluorsulfanyl)-phenyl]-ethanon-Trifluoressigsäuresalz (11 mg) | | | | | |
| | 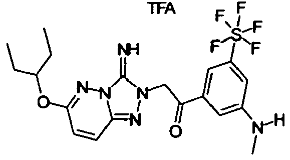 | W | O | LCMS- | [M+H]⁺ | |
| | | 1.008 | 1.006 | rt [min] | | analog Bsp. |
| | | | | | 495,1 | 5 |
| | | 19 mg | 30 mg | 1,35 | (Met-a) | |
| Bsp 17 | 2-[6-(1-Ethyl-propoxy)-3-imino-[1,2,4]triazolo[4,3-b]pyridazin-2-yl]-1-[3-meth-oxy-5-(pentafluorsulfanyl)-phenyl]-ethanon-Trifluoressigsäuresalz (23 mg) | | | | | |
| | 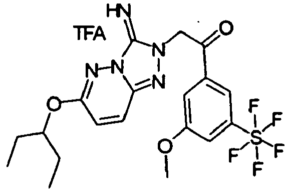 | W | O | LCMS- | [M+H]⁺ | |
| | | 1.008 | 1.007 | rt [min] | | analog Bsp. |
| | | | | | 496,1 | 5 |
| | | 25 mg | 40 mg | 1,35 | (Met-a) | |

### Beispiel 18

### 1-(3-tert-Butyl-5-ethoxymethyl-phenyl)-2-[6-(1-ethyl-propoxy)-3-imino-[1,2,4]triazolo[4,3-b]pyridazin-2-yl]-ethanon-Hydrochlorid

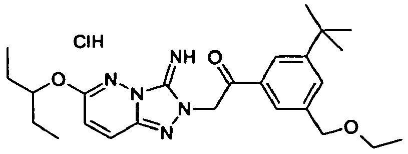

6-(1-Ethyl-propoxy)-[1,2,4]triazolo[4,3-b]pyridazin-3-ylamin (W1.008; 205 mg) wurde in DMF (5 ml) bei RT unter Rühren vorgelegt und 2-Brom-1-(3-tert-butyl-5-ethoxymethylphenyl)-ethanon (O1.008; 290 mg) in DMF (5 ml) vorgelöst zugetropft. Das Reaktionsgemisch rührte 14 h bei RT. Dann wurde das Lösungsmittel im Vakuum entfernt und der Rückstand wurde über Kieselgel (40 g Kartusche, Dichlormethanl-Ethanol-Gradient von 0-40% in 60 min) aufgereinigt. Das noch verunreinigte Produkt wurde anschließend mittels präparativer HPLC gereinigt. Die sauberen Produktfraktionen wurden vereinigt, im Vakuum vom Acetonitril befreit und gefriergetrocknet. Der Rückstand wurde in Wasser und Acetonitril gelöst und nach Zugabe von 3 eq. HCl gefriergetrocknet. Es wurden 72 mg der Titelverbindung erhalten. LCMS-rt: 1,44 min [M+H]⁺: 454,5 (Met-a)

Analog obiger Beispiele wurden dargestellt:

| | | | | | | |
|---|---|---|---|---|---|---|
| Bsp 19 | 1-(3-tert-Butyl-5-cyclopropylmethoxymethyl-phenyl)-2-[6-(1-ethyl-propoxy)-3-imino-[1,2,4]triazolo[4,3-b]pyridazin-2-ylJ-ethanon-Hydrochlorid (155 mg) | | | | | |
| | 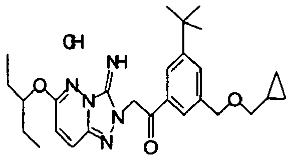 | W | O | | | |
| | | | | LCMS-rt | [M+H]⁺ | |
| | | 1.008 | 1.009 | | | analog |
| | | | | [min] | 480,2 | Bsp. 18 |
| | | | | 3,36 | (Met-d) | |
| | | 198 mg | 304 mg | | | |
| Bsp 20 | 1-(3-tert-Butyl-4,5-diethoxy-phenyl)-2-[6-(1-ethyl-propoxy)-3-imino-[1,2,4]triazolo[4,3-b]pyridazin-2-yl]-ethanon-Hydrochlorid (96 mg) | | | | | |
| | 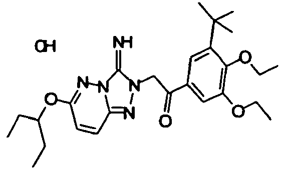 | W | O | LCMS-rt | [M+H]⁺ | |
| | | 1.008 | 1.010 | [min] | | analog |
| | | | | | 484,1 | Bsp. 18 |
| | | 130 mg | 202 mg | 3,47 | (Met-d) | |
| Bsp 21 | 1-(3-tert-Butyl-5-ethoxy-phenyl)-2-[6-(1-ethyl-propoxy)-3-imino-[1,2,4]triazolo[4,3-b]pyridazin-2-yl]-ethanon-Hydrochlorid (114 mg) | | | | | |
| | 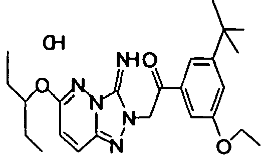 | W | O | | [M+H]⁺ | |
| | | | | LCMS-rt | | |
| | | 1.008 | 1.011 | | | analog Bsp |
| | | | | [min] | | |
| | | | | | 440,5 | 18 |
| | | | | 1,46 | | |
| | | 140 mg | 189 mg | | (Met-a) | |
| Bsp 22 | 1-(3-tert-Butyl-5-propoxymethyl-phenyl)-2-[6-(1-ethyl-propoxy)-3-imino-[1,2,4]triazolo[4,3-b]pyridazin-2-yl]-ethanon-Hydrochlorid (92 mg) | | | | | |
| | 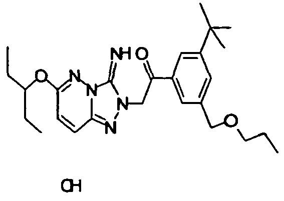 | W | O | LCMS-rt | [M+H]⁺ | |
| | | 1.008 | 1.012 | [min] | | analog |
| | | | | | 468,1 | Bsp. 18 |
| | | 180 mg | 266 mg | 3,00 | (Met-d) | |
| Bsp 23 | 1-(3-tert-Butyl-4,5-bis-cyclopropylmethoxy-phenyl)-2-[6-(1-ethyl-propoxy)-3-imino-[1,2,4]triazolo[4,3-b]pyridazin-2-yl]-ethanon-Hydrochlorid (112 mg) | | | | | |
| | 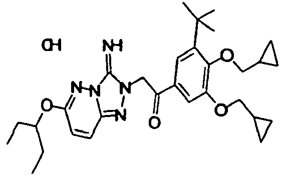 | W | O | | [M+H]⁺ | |
| | | | | LCMS-rt | | |
| | | 1.008 | 1.013 | | | analog |
| | | | | [min] | | |
| | | | | | 536,5 | Bsp. 18 |
| | | | | 1,662 | | |
| | | 165 mg | 295 mg | | (Met-a) | |
| Bsp 24 | 2-[6-(1-Ethyl-propoxy)-3-imino-[1,2,4]triazolo[4,3-b]pyridazin-2-yl]-1-(3-methoxy-5-trifluormethyl-phenyl)-ethanon-Trifluoressigsäuresalz (31 mg) | | | | | |
| | 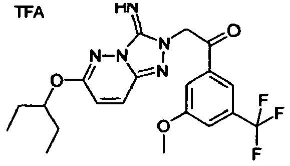 | W | O | | [M+H]⁺ | |
| | | | | LCMS-rt | | |
| | | 1.008 | 1.014 | | | analog |
| | | | | [min] | | |
| | | | | | 438,1 | Bsp. 2 |
| | | | | 1,33 | | |
| | | 25 mg | 34 mg | | (Met-a) | |
| Bsp 25 | 1-(3-tert-Butyl-5-methoxy-phenyl)-2-[6-(1-ethyl-propoxy)-3-imino-[1,2,4]triazolo[4,3-b]pyridazin-2-yl]-ethanon-Hydrochlorid (176 mg) | | | | | |
| | 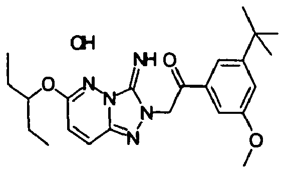 | W | O | | [M+H]⁺ | |
| | | | | LCMS-rt | | |
| | | 1.008 | 1.015 | | | analog |
| | | | | [min] | | |
| | | | | | 426,1 | Bsp. 18 |
| | | | | 3,18 | | |
| | | 200 mg | 258 mg | | (Met-d) | |
| Bsp 26 | 1-(3-tert-Butyl-5-cyclopropylmethoxy-phenyl)-2-[6-(1-ethyl-propoxy)-3-imino-[1,2,4]triazolo[4,3-b]pyridazin-2-yl]-ethanon-Hydrochlorid (152 mg) | | | | | |
| | 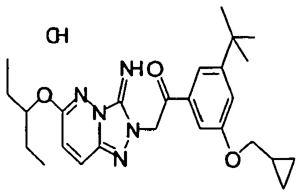 | W | O | | [M+H]⁺ | |
| | | | | LCMS-rt | | |
| | | 1.008 | 1.016 | | | analog |
| | | | | [min] | | |
| | | | | | 466,3 | Bsp. 18 |
| | | | | 3,44 | | |
| | | 180 mg | 265 mg | | (Met-d) | |
| Bsp 27 | 1-(3-tert-Butyl-5-cyclobutylmethoxy-phenyl)-2-[6-(1-ethyl-propoxy)-3-imino-[1,2,4]triazolo[4,3-b]pyridazin-2-yl]-ethanon-Hydrochlorid (137 mg) | | | | | |
| | 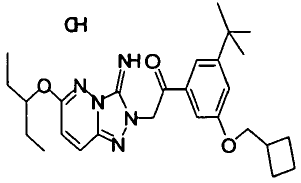 | W | O | | [M+H]⁺ | |
| | | | | LCMS-rt | | |
| | | 1.008 | 1.017 | | | analog |
| | | | | [min] | | |
| | | | | | 480,5 | Bsp. 18 |
| | | | | 1,58 | | |
| | | 180 mg | 276 mg | | (Met-a) | |
| Bsp 28 | 1-(3-Benzyloxymethyl-5-tert-butyl-phenyl)-2-[6-(1-ethyl-propoxy)-3-imino-[1,2,4]triazolo[4,3-b]pyridazin-2-yl]-ethanon-Hydrochlorid (183 mg) | | | | | |
| | 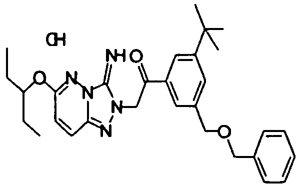 | W | O | | [M+H]⁺ | |
| | | | | LCMS-rt | | |
| | | 1.008 | 1.018 | | | analog |
| | | | | [min] | | |
| | | | | | 516,5 | Bsp. 18 |
| | | | | 1,53 | | |
| | | 200 mg | 339 mg | | (Met-a) | |
| Bsp 29 | 1-(3-Cyclohexylmethoxy-4,5-dimethoxy-phenyl)-2-[6-(1-ethyl-propoxy)-3-imino-[1,2,4]triazolo[4,3-b]pyridazin-2-yl]-ethanon-Hydrochlorid (76 mg) | | | | | |
| | 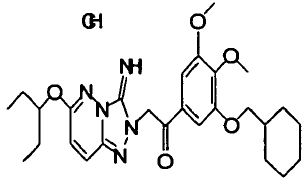 | W | O | | [M+H]⁺ | |
| | | | | LCMS-rt | | |
| | | 1.008 | 1.019 | | | analog |
| | | | | [min] | | |
| | | | | | 512,5 | Bsp. 18 |
| | | | | 1,50 | | |
| | | 75 mg | 126 mg | | (Met-a) | |
| Bsp 30 | 2-(6-Butoxy-3-imino-[1,2,4]triazolo[4,3-b]pyridazin-2-yl)-1-(3-tert-butyl-5-methoxymethyl-phenyl)-ethanon-Hydrochlorid (83 mg) | | | | | |
| | 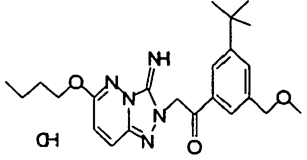 | W | O | LCMS-rt | [M+H]⁺ | |
| | | 1.010 | 1.020 | [min] | | analog |
| | | | | | 426,3 | Bsp. 18 |
| | | 240 mg | 347 mg | 1,33 | (Met-a) | |
| Bsp 31 | 1-(3-Chlor-5-methoxy-phenyl)-2-[6-(1-ethyl-propoxy)-3-imino-[1,2,4]triazolo[4,3-b]pyridazin-2-yl]-ethanon-Hydrochlorid (169 mg) | | | | | |
| | 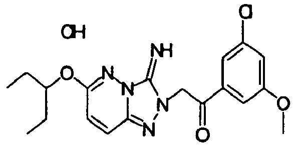 | W | O | | [M+H]⁺ | |
| | | | | LCMS-rt | | |
| | | 1.008 | 1.021 | | | analog |
| | | | | [min] | | |
| | | | | | 404,2 | Bsp. 18 |
| | | | | 1,28 | | |
| | | 250 mg | 298 mg | | (Met-a) | |
| Bsp 32 | 1-(8-tert-Butyl-4-methyl-3,4-dihydro-2H-benzo[1,4]oxazin-6-yl)-2-[6-(1-ethyl-propoxy)-3-imino-[1,2,4]triazolo[4,3-b]pyridazin-2-yl]-ethanon-Trifluoressig-säuresalz (13 mg) | | | | | |
| | 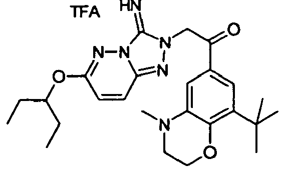 | W | O | | [M+H]⁺ | |
| | | | | LCMS-rt | | |
| | | 1.008 | 1.022 | | | analog |
| | | | | [min] | | |
| | | | | | 467,3 | Bsp. 5 |
| | | | | 1,43 | | |
| | | 25 mg | 37 mg | | (Met-a) | |
| Bsp 33 | 1-(3-tert-Butyl-4-methoxy-5-morpholin-4-yl-phenyl)-2-(3-imino-6-piperidin-1-yl-(1,2,4]triazolo[4,3-b]pyridazin-2-yl)-ethanon-Trifluoressigsäuresalz (30 mg) | | | | | |
| | 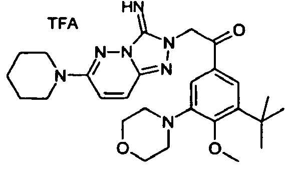 | W | O | | [M+H]⁺ | |
| | | | | LCMS-rt | | |
| | | 1.020 | 1.003 | | | analog |
| | | | | [min] | 508,3 | Bsp. 5 |
| | | | | 1,37 | | |
| | | 25 mg | 42 mg | | (Met-a) | |

Analog obiger Methoden wurden mit den W1- und O1-Grundkörpem folgende Beispiele dargestellt und charakterisiert:

| | | Name | LCMS-rt [min] | [M+H⁺]⁺ |
|---|---|---|---|---|
| Bsp.: 034 | 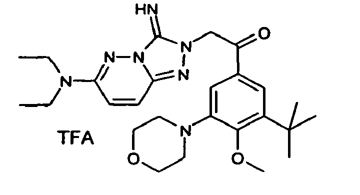 | 1-(3-tert-Butyl-4-methoxy-5-morpholin-4-yl-phenyl)-2-(6-diethylamino-3-imino-[1,2,4]triazolo[4,3-b]pyridazin-2-yl)-ethanon-Trifluoressigsäuresalz | 1,37 | 496,3 (Met-a) |
| Bsp.: 035 | 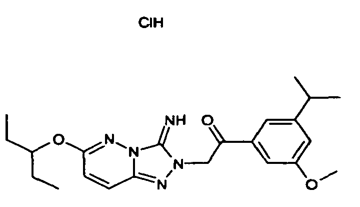 | 2-[6-(1-Ethyl-propoxy)-3-imino-[1,2,4]triazolo[4,3-b]pyridazin-2-yl]-1-(3-isopropyl-5-methoxy phenyl)-ethanon-Hydrochlorid | 1,36 | 412,3 (Met-a) |
| Bsp.: 036 | 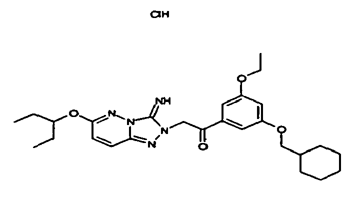 | 1-(3-Cyclohexylmethoxy-5-ethoxy-phenyl)-2-[6-(1-ethyl-propoxy)-3-imino-[1,2,4]triazolo[4,3-b]pyridazin-2-yl]-ethanon-Hydrochlorid | 1,56 | 496,4 (Met-a) |
| Bsp.: 037 | 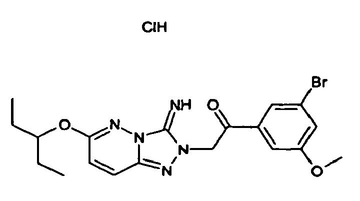 | 1-(3-Brom-5-methoxy-phenyl)-2-[6-(1-ethyl-propoxy)-3-imino-[1,2,4]triazolo[4,3-b]pyridazin-2-yl]-ethanon-Hydrochlorid | 1,3 | 448,1 (Met-a) |
| Bsp.: 038 | 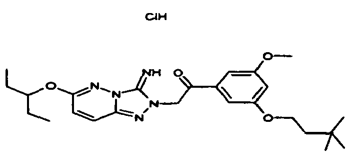 | 1-[3-(3,3-Dimethyl-butoxy)-5-methoxy-phenyl]-2-[6-(1-ethyl-propoxy)-3-imino-[1,2,4]triazolo[4,3-b]pyridazin-2-yl]-ethanon-Hydrochlorid | 1,52 | 470,4 (Met-a) |
| Bsp.: 039 | 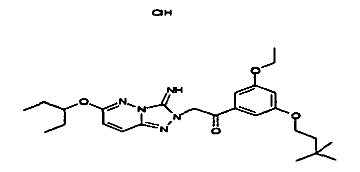 | 1-[3-(3,3-Dimethyl-butoxy)-5-ethoxy-phenyl]-2-[6-(1-ethyl-propoxy)-3-imino-[1,2,4]triazolo[4,3-b]pyridazin-2-yl]-ethanon-Hydrochloride | 1,56 | 484,4 (Met-a) |
| Bsp.: 040 | 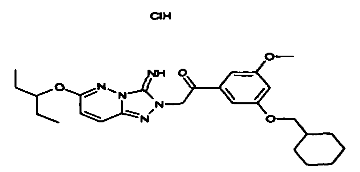 | 1-(3-Cyclohexylmethoxy-5-methoxy-phenyl)-2-[6-(1-ethyl-propoxy)-3-imino-[1,2,4]triazolo[4,3-b]pyridazin-2-yl]-ethanon-Hydrochlorid | 1,55 | 482,4 (Met-a) |
| Bsp.: 041 | 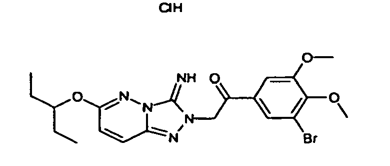 | 1-(3-Brom-4,5-dimethoxy-phenyl)-2-[6-(1-ethyl-propoxy)-3-imino-[1,2,4]triazolo[4,3-b]pyridazin-2-yl]-ethanon-Hydrochlorid | 1,29 | 478,1 (Met-a) |
| Bsp.: 042 | 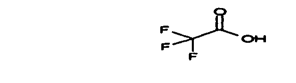 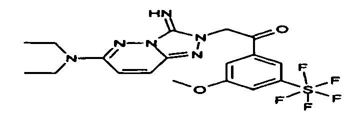 | 2-(6-Diethylamino-3-imino-[1,2,4]triazolo[4,3-b]pyridazin-2-yl)-1-[3-methoxy-5-(pentafluor-sulfanyl)-phenyl]-ethanon-Trifluoressigsäuresalz | 1,29 | 481,1 (Met-a) |
| Bsp.: 043 | 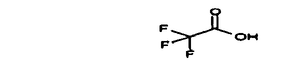 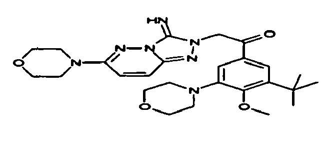 | 1-(3-tert-Butyl-4-methoxy-5-morpholin-4-yl-phenyl)-2-(3-imino-6-morpholin-yl-[1,2,4]triazolo[4,3-b]pyridazin-2-yl)-ethanon-Trifluoressigsäuresalz | 1,24 | 510,2 (Met-a) |
| Bsp.: 044 | 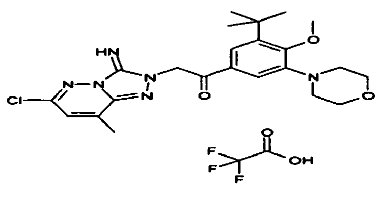 | 1-(3-tert-Butyl-4-methoxy-5-morpholin-4-yl-phenyl)-2-(6-chlor-3-imino-8-methyl-[1,2,4]triazolo[4,3-b]pyridazin-2-yl)-ethanon-Trifluoressigsäuresalz | 1,27 | 473,1 (Met-a) |
| Bsp.: 045 | 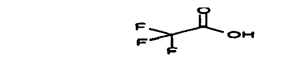 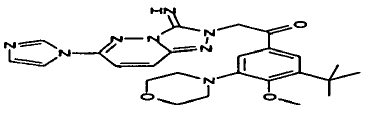 | 1-(3-tert-Butyl-4-methoxy-5-morpholin-4-yl-phenyl)-2-(6-imidazol-1-yl-3-imino-[1,2,4]triazolo[4,3-b]pyridazin-2-yl)-ethanon-Trifluoressigsäuresalz | 1,12 | 491,1 (Met-a) |
| Bsp.: 046 | 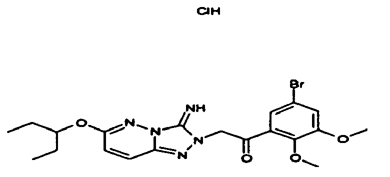 | 1-(5-Bromo-2,3-dimethoxy-phenyl)-2-[6-(1-ethyl-propoxy)-3-imino-[1,2,4]triazolo[4,3-b]pyridazin-2-yl]-ethanon-Hydrochlorid | 1,35 | 479,1 (Met-a) |
| Bsp.: 047 | 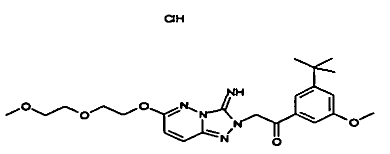 | 1-(3-tert-Butyl-5-methoxy-phenyl)-2-{3-imino-6-[2-(2-methoxy-ethoxy)-ethoxy]-[1,2,4]triazolo[4,3-b]pyridazin-2-yl}-ethanon-Hydrochlorid | 1,22 | 458,3 (Met-a) |
| Bsp.: 048 | 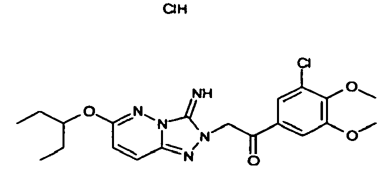 | 1-(3-Chlor-4,5-dimethoxy-phenyl)-2-[6-(1-ethyl-propoxy)-3 imino-[1,2,4]triazolo[4,3-b]pyridazin-2-yl]-ethanon | 1,3 | 434,1 (Met-a) |
| Bsp.: 049 | 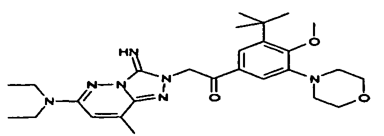 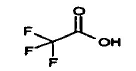 | 1-(3-tert-Butyl-4-methoxy-5-morpholin-4-yl-phenyl)-2-(6-diethylamino-3-imino-8-methyl-[1,2,4]triazolo[4,3-b]pyridazin-2-yl)-ethanon-Trifluoressigsäuresalz | 1,42 | 510,4 (Met-a) |
| Bsp.: 050 | 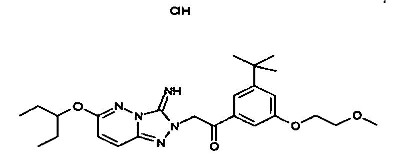 | 1-[3-tert-Butyl-5-(2-methoxy-ethoxy)-phenyl]-2-[6-(1-ethyl-propoxy)-3-imino-[1,2,4]triazolo[4,3-b]pyridazin-2-yl]-ethanon-Hydrochlorid | 1,42 | 470,3 (Met-a) |
| Bsp.: 051 | 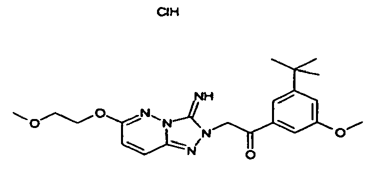 | 1-(3-tert-Butyl-5-methoxy-phenyl)-2-[3-imino-6-(2-methoxy-ethoxy)-[1,2,4]triazolo[4,3-b]pyridazin-2-yl]-ethanon-Hydrochlorid | 1,21 | 414,2 (Met-a) |
| Bsp.: 052 | 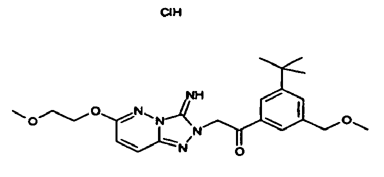 | 1-(3-tert-Buty)-5-methoxymethy) phenyl)-2-[3-imino-6-(2-methoxy-ethoxy)-[1,2,4]triazolo[4,3-b]pyridazin-2-yl]-ethanon-Hydrochlorid | 1,21 | 428,3 (Met-a) |
| Bsp.: 053 | 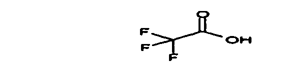 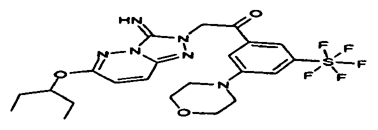 | 2-[6-(1-Ethyl-propoxy)-3-imino-[1,2,4]triazolo[4,3-b]pyridazin-2-yl]-1-[3-morpholin-4-yl-5-(pentafluor-sulfanyl)-phenyl]-ethanon-Trifluoressigsäuresalz | 1,34 | 551,2 (Met-a) |
| Bsp.: 054 | 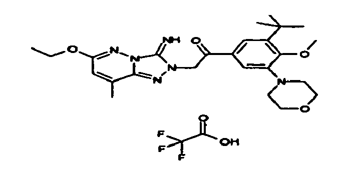 | 1-(3-tert-Butyl-4-methoxy-5-morpholin-4-yl-phenyl)-2-(6-ethoxy-3-imino-8-methyl-[1,2,4]triazolo[4,3-b]pyridazin-2-yl)-ethanon-Trifluoressigsäuresalz | 1,35 | 483,3 (Met-a) |
| Bsp.: 055 | 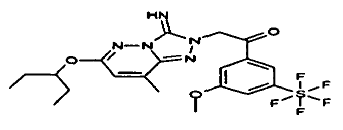 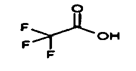 | 2-[6-(1-Ethyl-propoxy)-3-imino-8-methyl-[1,2,4]triazolo[4,3-b]pyridazin-2-yl]-1-[3-methoxy-5-(pentafluoro-sulfanyl)-phenyl]-ethanon-Trifluoressigsäuresalz | 1,41 | 510,1 (Met-a) |
| Bsp.: 056 | 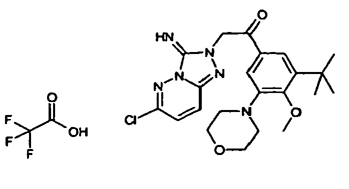 | 1-(3-tert-Butyl-4-methoxy-5-morpholin-4-yl-phenyl)-2-(6-chloro-3-imino-[1,2,4]triazolo[4,3-b]pyridazin-2-yl)-ethanon-Trifluoressigsäuresalz | 1,23 | 459,1 (Met-a) |
| Bsp.: 057 | 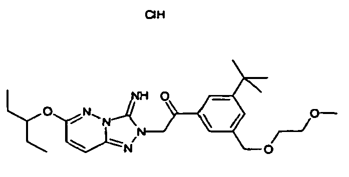 | 1-[3-tert-Butyl-5-(2-methoxy-ethoxymethyl)-phenyl]-2-[6-(1-ethyl-propoxy)-3-imino-[1,2,4]triazolo[4,3-b]pyridazin-2-yl]-ethanon-Hydrochlorid | 1,38 | 484,4 (Met-a) |
| Bsp.: 058 | 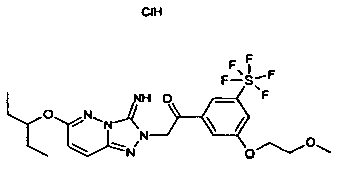 | 2-[6-(1-Ethyl-propoxy)-3-imino-[1,2,4]triazolo[4,3-b]pyridazin-2-yl]-1-[3-(2-methoxy-ethoxy)-5-(pentafluorsulfanyl)-phenyl]-ethanon-Hydrochlorid | 1,38 | 484,4 (Met-a) |
| Bsp.: 059 | 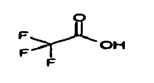 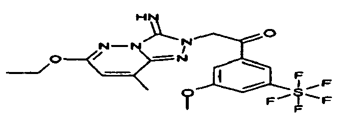 | 2-(6-Ethoxy-3-imino-8-methyl-[1,2,4]triazolo[4,3-b]pyridazin-2-yl)-1-[3-methoxy-5-(pentafluorsulfanyl)-phenyl]-ethanon-Trifluoressigsäuresalz | 1,28 | 468,0 (Met-a) |
| Bsp.: 060 | 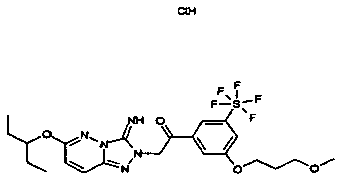 | 2-[6-(1-Ethyl-propoxy)-3-imino-[1,2,4]triazolo[4,3-b]pyridazin-2-yl]-1-[3-(3-methoxy-propoxy) 5-(pentafluoro-sulfanyl)-phenyl]-ethanon-Hydrochlorid | 0,95 | 554.2 (Met-b) |
| Bsp.: 061 | 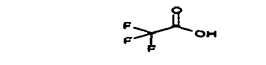 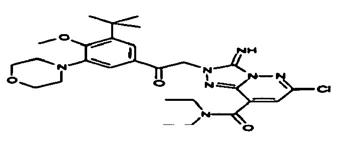 | 2-[2-(3-tert-Butyl-4-methoxy-5-morpholin-4-yl-phenyl)-2-oxo-ethyl]-6-chloro-3-imino-2,3-dihydro-[1,2,4]triazolo[4,3-b]pyridazin-8-carbonsäure-diethylamid-Trifluoressigsäuresalz | 1,29 | 558,3 (Met-a) |
| Bsp.: 062 | 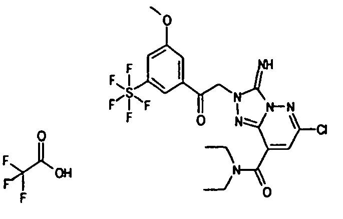 | 6-Chloro-3-imino-2-{2-[3-methoxy-5-(pentafluor-sulfanyl)-phenyl]-2-oxo-ethyl}-2,3-dihydro-[1,2,4]triazolo[4,3-b]pyridazine-8-carbonsäure-diethylamid-Trifluoressigsäuresalz | 1,25 | 543,0 (Met-a) |
| Bsp.: 063 | 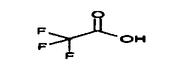 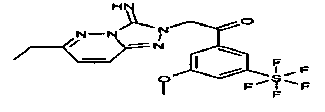 | 2-(6-Ethyl-3-imino-[1,2,4]triazolo[4,3-b]pyridazin-2-yl)-1-[3-methoxy-5-(pentafluoro-sulfanyl)-phenyl]-ethanon-Trifluoressigsäuresalz | 1,18 | 438,0 (Met-a) |
| Bsp.: 064 | 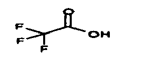 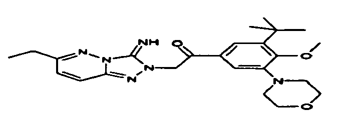 | 1-(3-tert-Butyl-4-methoxy-5-morpholin-4-yl-phenyl)-2-(6-ethy)-3-imino-[1,2,4]triazolo[4,3-b]pyridazin-2-yl)-ethanon-Trifluoressigsäuresalz | 1,27 | 453,3 (Met-a) |
| Bsp.: 065 | 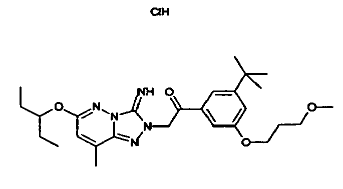 | 1-[3-tert-Butyl-5-(3-methoxy-propoxy)-phenyl]-2-[6-(1 -ethyl-propoxy)-3-imino-8-methyl-[1,2,4]triazolo[4,3-b]pyridazin-2-yl]-ethanon-Hydrochlorid | 1,52 | 498,5 (Met-a) |
| Bsp.: 066 | 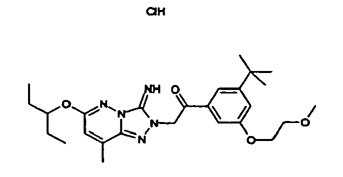 | 1-[3-tert-Butyl-5-(2-methoxy-ethoxy)-phenyl]-2-[6-(1-ethyl-propoxyl-3-imino-8-methyl-[1,2,4]triazolo[4,3-b]pyridazin-2-yl]-ethanon-Hydrochlorid | 1,48 | 484,4 (Met-a) |
| Bsp.: 067 | 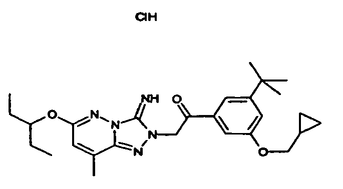 | 1-(3-tert-Butyl-5-cyclopropylmethoxy-phenyl)-2-[6-(1-ethyl-propoxy)-3-imino-8-methyl-[1,2,4]triazolo[4,3-b]pyridazin-2-yl]-ethanon-Hydrochlorid | 1,63 | 480,5 (Met-a) |
| Bsp.: 068 | 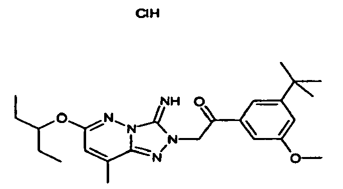 | 1-(3-tert-Butyl-5-methoxy-phenyl) 2-[6-(1-ethyl-propoxy)-3-imino-8-methyl-[1,2,4]triazolo[4,3-b]pyridazin-2-yl]-ethanon-Hydrochlorid | 1,52 | 440,4 (Met-a) |
| Bsp.: 069 | 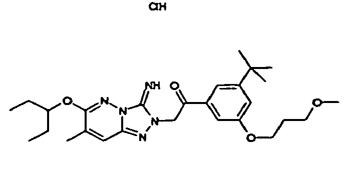 | 1-[3-tert-Butyl-5-(3-methoxy-propoxy)-phenyl]-2-[6-(1-ethyl-propoxy)-3-imino-7-methyl-[1,2,4]triazolo[4,3-b]pyridazin-2-yl]-ethanon-Hydrochlorid | 1,55 | 498,5 (Met-a) |
| Bsp.: 070 | 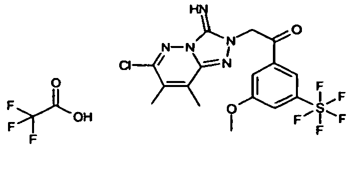 | 2-(6-Chlor-3-imino-7,8-dimethyl [1,2,4]triazolo[4,3-b]pyridazin-2-yl)-1-[3-methoxy-5-(pentafluoro-sulfanyl)-phenyl]-ethanon-Trifluoressigsäuresalz | 1,47 | 472,1 (Met-a) |
| Bsp.: 071 | 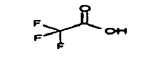 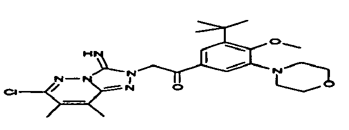 | 1-(3-tert-Butyl-4-methoxy-5-morpholin-4-yl-phenyl)-2-(6-chloro-3-imino-7,8-dimethyl-[1,2,4]triazolo[4,3-b]pyridazin-2-yl)-ethanon-Trifluoressigsäuresalz | 1,57 | 487,2 (Met-a) |
| Bsp.: 072 | 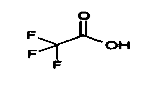 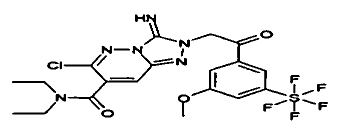 | 6-Chlor-3-imino-2-{2-[3-methoxy-5-(pentafluoro-sulfanyl)-phenyl]-2-oxo-ethyl}-2,3-dihydro-[1,2,4]triazolo[4.3-b]pyridazine-7-carbonsäure-diethylamid-Trifluoressigsäuresalz | 1,47 | 543,1 (Met-a) |
| Bsp.: 073 | 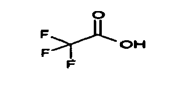 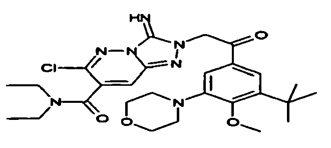 | 2-[2-(3-tert-Butyl-4-methoxy-5-morpholin-4-yl-phenyl)-2-oxo-ethyl]-6-chlor-3-imino-2,3-dihydro-[1,2,4]triazolo[4,3-b]pyridazine-7-carbonsäure-diethylamid-Trifluoressigsäuresalz | 1,55 | 558,3 (Met-a) |
| Bsp.: 074 | 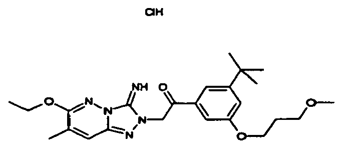 | 1-[3-tert-Butyl-5-(3-methoxy-propoxy)-phenyl]-2-(6-ethoxy-3-imino-7-methyl-[1,2,4]triazolo[4,3-b]pyridazin-2-yl)-ethanon-Hydrochlorid | 0,89 | 456,2 (Met-b) |
| Bsp.: 075 | 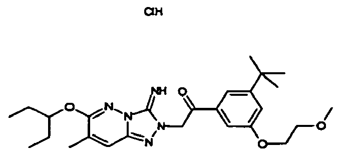 | 1-[3-tert-Butyl-5-(2-methoxy-ethoxy)-phenyl]-2-[6-(1-ethy)-propoxy)-3-imino-7-methyl-[1,2,4]triazolo[4,3-b]pyridazin-2-yl]-ethanon-Hydrochlorid | 1,48 | 484,5 (Met-a) |
| Bsp.: 076 | 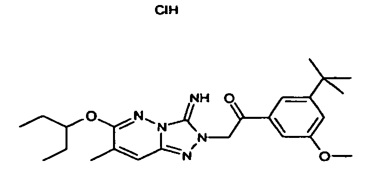 | 1-(3-tert-Butyl-5-methoxy-phenyl)-2-[6-(1-ethyl-propoxy)-3-imino-7-methyl-[1,2,4]triazolo[4,3-b]pyridazin-2-yl]-ethanon-Hydrochlorid | 0,83 | 472,2 (Met-b) |
| Bsp.: 077 | 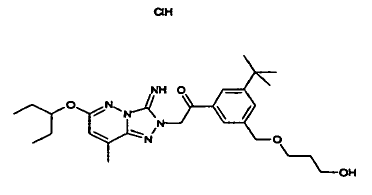 | 1-[3-tert-Butyl-5-(3-hydroxy-propoxymethyl)-phenyl]-2-[6-(1-ethyl-propoxy)-3-im ino-8-methyl-[1,2,4]triazolo[4,3-b]pyridazin-2-yl]-ethanon-Hydrochlorid | 1,38 | 498,2 (Met-a) |
| Bsp.: 078 | 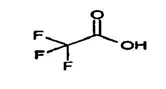 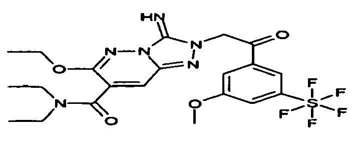 | 6-Ethoxy-3-imino-2-{2-[3-methoxy-5-(pentafluor-sulfanyl)-phenyl]-2-oxo-ethyl}-2,3-dihydro-[1,2,4]triazolo[4,3-b]pyridazine-7-carbonsäure-diethylamid-Trifluoressigsäuresalz | 1,25 | 553,2 (Met-a) |
| Bsp.: 079 | 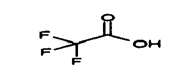 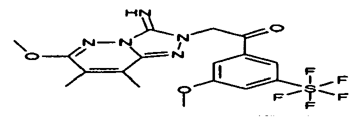 | 2-(3-Imino-6-methoxy-7,8-dimethyl-[1,2,4]triazolo[4,3-b]pyridazin-2-yl)-1-[3-methoxy-5-(pentafluorsulfanyl)-phenyl]-ethanon-Trifluoressigsäuresalz | 1,24 | 468,1 (Met-a) |
| Bsp.: 080 | 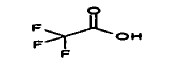 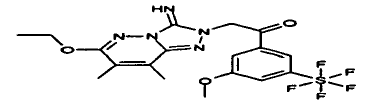 | 2-(6-Ethoxy-3-imino-7,8-dimethyl-[1,2,4]triazolo[4,3-b]pyridazin-2-yl)-1-[3-methoxy-5-(pentafluorsulfanyl)-phenyl]-ethanon-Trifluoressigsäuresalz | 1,32 | 482,1 (Met-a) |
| Bsp.: 081 | 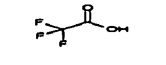 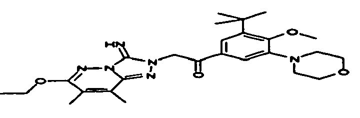 | 1-(3-tert-Butyl-4-methoxy-5-morpholin-4-yl-phenyl)-2-(6-ethoxy-3-imino-7,8-dimethyl-[1,2,4]triazolo[4,3-b]pyridazin-2-yl)-ethanon-Trifluoressigsäuresalz | 1,38 | 497,2 (Met-a) |
| Bsp.: 082 | 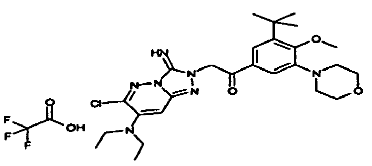 | 1-(3-tert-Butyl-4-methoxy-5-morphotin-4-yl-phenyl)-2-(6-chlor-7-diethylamino-3-imino-[1,2,4]triazolo[4,3-b]pyridazin-2-yl)-ethanon-Trifluoressigsäuresalz | 1,40 | 530,2 (Met-a) |
| Bsp.: 083 | 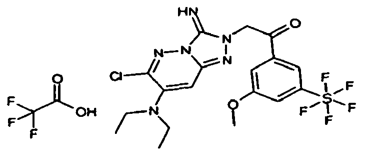 | 2-(6-Chlor-7-diethylamino-3-imino-[1,2,4]triazolo[4,3-b]pyridazin-2-yl)-1-[3-methoxy-5-(pentafluorsulfanyl)-phenyl]-ethanon-Trifluoressigsäuresalz | 1,32 | 515,1 (Met-a) |
| Bsp.: 084 | 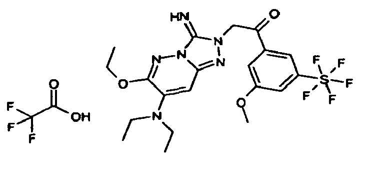 | 2-(7-Diethylamino-6-ethoxy-3-imino-[1,2,4]triazolo[4,3-b]pyridazin-2-yl)-1-[3-methoxy-5-(pentafluorsulfanyl)-phenyl]-ethanon-Trifluoressigsäuresalz | 1,65 | 525,2 (Met-a) |
| Bsp.: 085 | 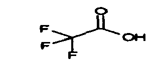 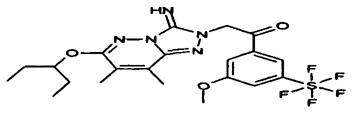 | 2-[6-(1-Ethyl-propoxy)-3-imino-7,8-dimethyl-[1,2,4]triazolo[4,3-b]pyridazin-2-yl]-1-[3-methoxy-5-(pentafluorsulfanyl)-phenyl]-ethanon-Trifluoressigsäuresalz | 1,47 | 524,2 (Met-a) |
| Bsp.: 086 | 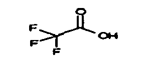 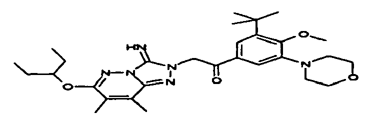 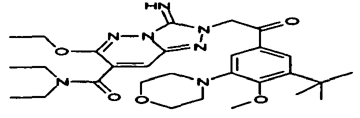 | 1-(3-tert-Butyl-4-methoxy-5-morpholin-4-yl-phenyl)-2-[6-(1 -ethyl-propoxy)-3-imino-7,8-dimethyl-[1,2,4]triazolo[4,3-b]pyridazin-2-yl]-ethanon-Trifluoressigsäuresalz | 1,52 | 539,3 (Met-a) |
| Bsp.: 087 | 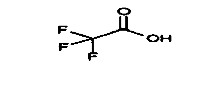 | 2-[2-(3-tert-Butyl-4-methoxy-5-morpholin-4-yl-phenyl)-2-oxo-ethyl]-6-ethoxy-3-imino-2,3-dihydro-[1,2,4]triazolo[4,3-b]pyridazine-7-carbonsäure-diethylamid-Trifluoressigsäuresalz | 1,32 | 568,3 (Met-a) |
| Bsp.: 088 | 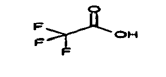 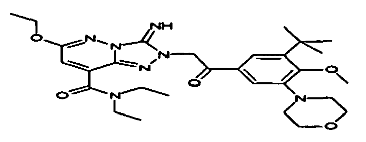 | 2-[2-(3-tert-Butyl-4-methoxy-5-morpholin-4-yl-phenyl)-2-oxo-ethyl]-6-ethoxy-3-imino-2,3-dihydro-[1,2,4]triazolo[4,3-b]pyridazine-8-carbonsäure-diethylamid-Trifluoressigsäuresalz | 1,35 | 568,2 (Met-a) |
| Bsp.: 089 | 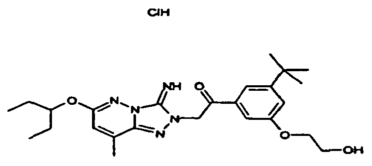 | 1-[3-tert-Butyl-5-(2-hydroxy-ethoxy)-phenyl]-2-[6-(1-ethyl-propoxy)-3-imino-8-methyl-[1,2,4]triazolo[4,3-b]pyridazin-2-yl]-ethanon-Hydrochlorid | 1,35 | 470,2 (Met-a) |
| Bsp.: 090 | 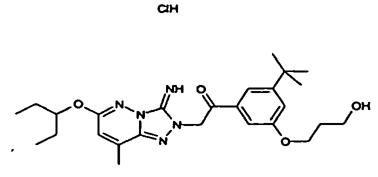 | 1-[3-tert-Butyl-5-(3-hydroxy-propoxy)-phenyl]-2-[6-(1-ethyl-propoxy)-3-imino-8-methyl-[1,2,4]triazolo[4,3-b]pyridazin-2-yl]-ethanon-Hydrochlorid | 1,38 | 484,2 (Met-a) |
| Bsp.: 091 | 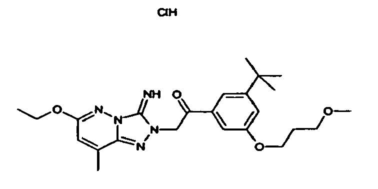 | 1-[3-tert-Butyl-5-(3-methoxy-propoxy)-phenyl]-2-(6-ethoxy-3-imino-8-methyl-[1,2,4]triazolo[4,3-b]pyridazin-2-yl)-ethanon-Hydrochlorid | 1,34 | 456,2 (Met-a) |
| Bsp.: 092 | 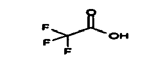 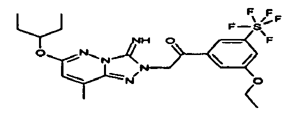 | 1-[3-Ethoxy-5-(pentafluoro-sulfanyl)-phenyl]-2-[6-(1-ethyl-propoxy)-3-imino-8-methyl-[1,2,4]triazolo[4,3-b]pyridazin-2-yl]-ethanon-Trifluoressigsäuresalz | 1,47 | 524,2 (Met-a) |
| Bsp.: 093 | 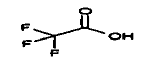 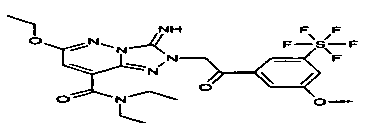 | 6-Ethoxy-3-imino-2-{2-[3-methoxy-5-(pentafluoro-sulfanyl)-phenyl]-2-oxo-ethyl}-2,3-dihydro-[1,2,4]triazolo[4,3-bjpyridazine-8-carbonsäure-diethylamid-Trifluoressigsäuresalz | 1,28 | 553,2 (Met-a) |
| Bsp.: 094 | 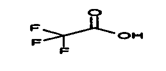 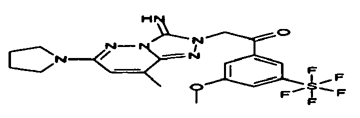 | 2-(3-Imino-8-methyl-6-pyrrolidin-1-yl-[1,2,4]triazolo[4,3-b]pyridazin-2-yl)-1-[3-methoxy-5-(pentafluorsulfanyl)-phenyl]-ethanon-Trifluoressigsäuresalz | 1,27 | 493,1 (Met-a) |
| Bsp.: 095 | 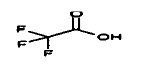 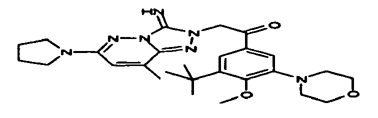 | 1-(3-tert-Butyl-4-methoxy-5-morpholin-4-yl-phenyl)-2-(3-imino-8-methyl-6-pyrrolidin-1-yl-[1,2,4]triazolo[4,3-b]pyridazin-2-yl)-ethanon-Trifluoressigsäuresalz | 1,36 | 508,3 (Met-a) |
| Bsp.: 096 | 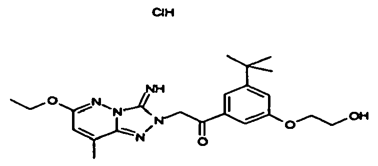 | 1-[3-tert-Butyl-5-(2-hydroxy-ethoxy)-phenyl]-2-(6-ethoxy-3-imino-8-methyl-[1,2,4]triazolo[4,3-bjpyridazin-2-yl)-ethanon-Hydrochlorid | 1,16 | 428,3 (Met-a) |
| Bsp.: 097 | 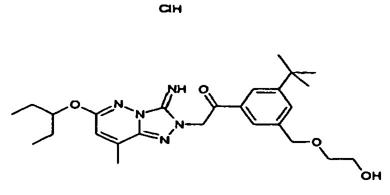 | 1-[3-tert-Butyl-5-(2-hydroxy-ethoxymethyl)-phenyl]-2-[6-(1-ethyl-propoxy)-3-imino-8-methyl [1,2,4]triazolo[4,3-b]pyridazin-2-yl]-ethanon-Hydrochlorid | 1,34 | 484,2 (Met-a) |
| Bsp.: 098 | 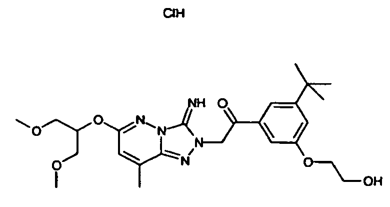 | 1-[3-tert-Butyl-5-(2-hydroxy-ethoxy)-phenyl]-2-[3-imino-6-(2-methoxy-1-methoxymethyl-ethoxy)-8-methyl-[1,2,4]triazolo[4, 3-b]pyridazin-2-yl]-ethanon-Hydrochlorid | 1,15 | 502,2 (Met-a) |
| Bsp.: 099 | 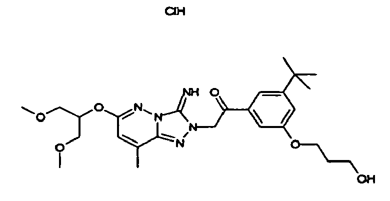 | 1-[3-tert-Butyl-5-(3-hydroxy-propoxy)-phenyl]-2-[3-imino-6-(2-methoxy-1-methoxymethyl-ethoxy)-8-methyl-[1,2,4]triazolo[4,3-b]pyridazin-2-yl]-ethanon-Hydrochlorid | 1,21 | 516,3 (Met-a) |
| Bsp.: 100 | 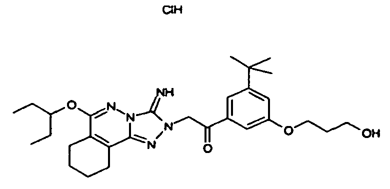 | 1-[3-tert-Butyl-5-(3-hydroxy-propoxy)-phenyl]-2-[6-(1-ethyl-propoxy)-3-imino-7,8,9,10-tetrahydro-[1,2,4]triazolo[3,4-a]phthalazin-2-yl]-ethanon-Hydrochlorid | 0,99 | 524,3 (Met-b) |
| Bsp.: 101 | 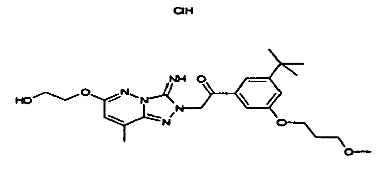 | 1-[3-tert-Butyl-5-(3-methoxy-propoxy)-phenyl]-2-[6-(2-hydroxy-ethoxy)-3-imino-8-methyl-[1,2,4]triazolo[4,3-b]pyridazin-2-yl]-ethanon-Hydrochlorid | 1,24 | 472,2 (Met-a) |
| Bsp.: 102 | 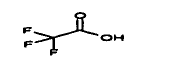 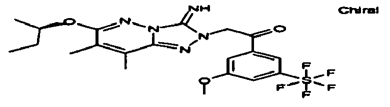 | 2-[6-((R)-sec-Butoxy)-3-imino-7,8-dimethyl-[1,2,4]triazolo[4,3-b]pyridazin-2-yl]-1-[3-methoxy-5-(pentafluorsulfanyl)-phenyl]-Trifluoressigsäuresalz | 1,05 | 510,2 (Met-b) |
| Bsp.: 103 | 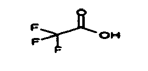 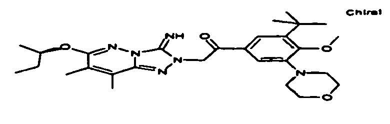 | 2-[6-((R)-sec-Butoxy)-3-imino-7,8-dimethyl-[1,2,4]triazolo[4,3-b]pyridazin-2-yl]-1-(3-tert-butyl-4-methoxy-5-morpholin-4-yl-phenyl)-ethanon-Trifluoressigsäuresalz | 1,08 | 525,4 (Met-b) |
| Bsp.: 104 | 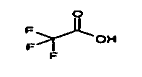 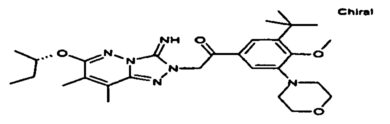 | 2-[6-((S)-sec-Butoxy)-3-imino-7,8-dimethyl-[1,2,4]triazolo(4,3-b]pyridazin-2-yl]-1-(3-tert-butyl-4-methoxy-5-morpholin-4-yl-phenyl)-ethanon-Trifluoressig säuresalz | 1,08 | 525,4 (Met-b) |
| Bsp.: 105 | 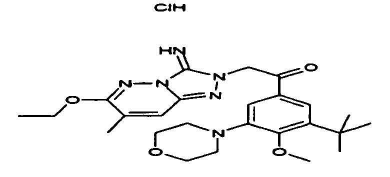 | 1-(3-tert-Butyl-4-methoxy-5-morpholin-4-yl-phenyl)-2-(6-ethoxy-3-imino-7-methyl-[1,2,4]triazolo[4,3-b]pyridazin-2-yl)-ethanon-Hydrochlorid | 1,38 | 483,3 (Met-a) |
| Bsp.: 106 | 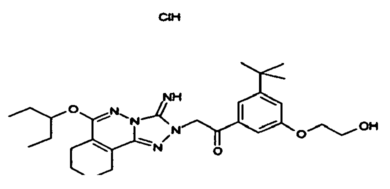 | 1-[3-tert-Butyl-5-(2-hydroxy-ethoxy)-phenyl]-2-[6-(1-ethyl-propoxy)-3-imino-7,8,9,10-tetrahydro-[1,2,4]triazolo[3,4-a]phthalazin-2-yl]-ethanon-Hydrochlorid | 1,48 | 510,3 (Met-a) |
| Bsp.: 107 | 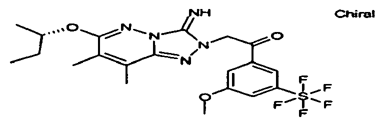 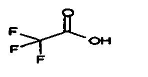 | 2-[6-((S)-sec-Butoxy)-3-imino-7,8-dimethyl-[1,2,4]triazolo[4,3-b]pyridazin-2-yl]-1-[3-methoxy-5 (pentafluorsulfanyl)-phenyl]-ethanon-Hydrochlorid | 1,05 | 510,2 (Met-b) |
| Bsp.: 108 | 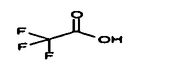 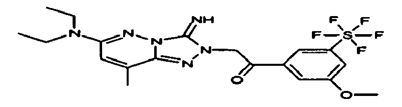 | 2-(6-Diethylamino-3-imino-8-methyl-[1,2,4]triazolo[4,3-b]pyridazin-2-yl)-1-[3-methoxy-5 (pentafluorsulfanyl)-phenyl]-ethanon-Trifluoressigsäuresalz | 1,01 | 495,2 (Met-b) |
| Bsp.: 109 | 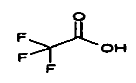 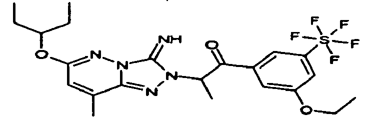 | 1-[3-Ethoxy-5-(pentafluoro-sulfanyl)-phenyl]-2-[6-(1-ethyl-propoxy)-3-imino-8-methyl-[1,2,4]triazolo[4,3-b]pyridazin-2-yl]-propan-1-on-Trifluoressig säuresalz | 1,07 | 538,2 (Met-b) |
| Bsp.: 110 | 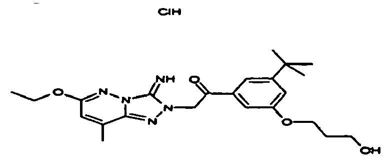 | 1-[3-tert-Butyl-5-(3-hydroxy-propoxy)-phenyl]-2-(6-ethoxy-3-imino-8-methyl-[1,2,4]triazolo[4,3-b]pyridazin-2-yl)-ethanon-Hydrochlorid | 1,23 | 442,3 (Met-a) |
| Bsp.: 111 | 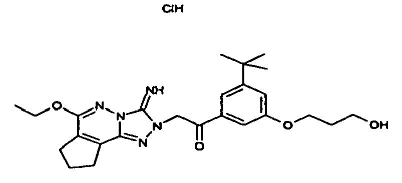 | 1-[3-tert-Butyl-5-(3-hydroxy-propoxy)-phenyl]-2-(6-ethoxy-3-imino-8,9-dihydro-7H-cyclopenta[d][1,2,4]triazolo[4,3-b]pyridazin-2-yl)-ethanon-Hydrochlorid | 1,3 | 468,2 (Met-a) |
| Bsp.: 112 | 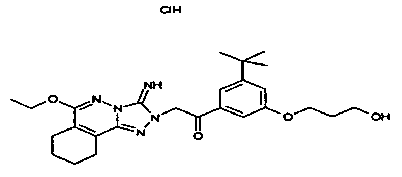 | 1-[3-tert-Butyl-5-(3-hydroxy-propoxy)-phenyl]-2-(6-ethoxy-3-imino-7,8,9,10-tetrahydro-[1,2,4]triazolo[3,4-a]phthalazin-2-yl)-ethanon-Hydrochlorid | 1,36 | 482,2 (Met-a) |
| Bsp.: 113 | 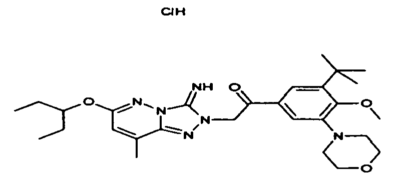 | 1-(3-tert-Butyl-4-methoxy-5-morpholin-4-yl-phenyl)-2-[6-(1 -ethyl-propoxy)-3-imino-8-methyl-[1,2,4]triazolo[4,3-b]pyridazin-2-yl]-ethanon-Hydrochlorid | 1,53 | 525,3 (Met-a) |
| Bsp.: 114 | 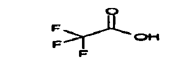 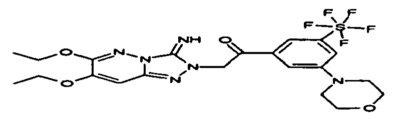 | 2-(6,7-Diethoxy-3-imino-[1,2,4]triazolo[4,3-b]pyridazin-2-yl)-1-[3-morpholin-4-yl-5-(pentafluorsulfanyl)-phenyl]-ethanon-Trifluoressigsäuresalz | 0,91 | 553,2 (Met-b) |
| Bsp.: 115 | 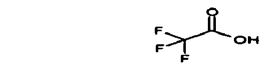 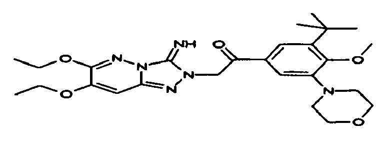 | 1-(3-tert-Butyl-4-methoxy-5-morpholin-4-yl-phenyl)-2-(6,7-diethoxy-3-imino-[1,2,4]triazolo[4,3-b]pyridazin-2-yl)-ethanon-Trifluoressigsäuresalz | 1,01 ' | 513,4 (Met-b) |
| Bsp.: 116 | 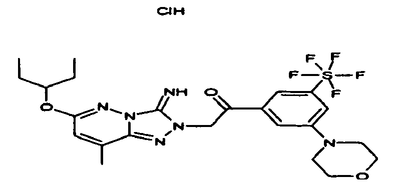 | 2-[6-(1-Ethyl-propoxy)-3-imino-8-methyl-[1,2,4]triazolo[4,3-b]pyridazin-2-yl]-1-[3-morpholin-4-yl-5-(pentafluorsulfanyl)-phenyl]-ethanon-Hydrochlorid | 1,05 | 565,3 (Met-b) |
| Bsp.: 117 | 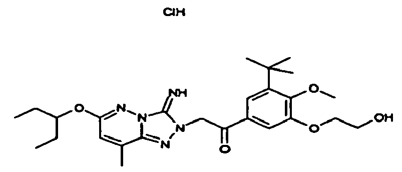 | 1-[3-tert-Butyl-5-(2-hydroxy-ethoxy)-4-methoxy-phenyl]-2-[6. (1-ethyl-propoxy)-3-imino-8-methyl-[1,2,4]triazolo[4,3-b]pyridazin-2-yl]-ethanon-Hydrochlorid | 1,38 | 500,3 (Met-a) |
| Bsp.: 118 | 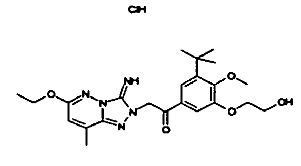 | 1-[3-tert-Butyl-5-(2-hydroxy-ethoxy)-4-methoxy-phenyl]-2-(6-ethoxy-3-imino-8-methyl-[1,2,4]triazolo[4,3-b]pyridazin-2-yl)-ethanon-Hydrochlorid | 1,19 | 458,3 (Met-a) |
| Bsp.: 119 | 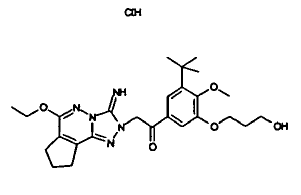 | 1-[3-tert-Butyl-5-(3-hydroxy-propoxy)-4-methoxy-phenyl]-2-(6-ethoxy-3-imino-8,9-dihydro-7H-cyclopenta[d][1,2,4]triazolo [4,3-b]pyridazin-2-yl)-ethanon-Hydrochlorid | 1,32 | 498,2 (Met-a) |
| Bsp.: 120 | 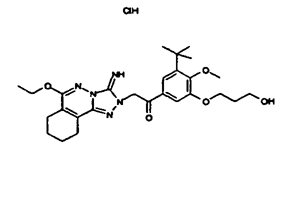 | 1-[3-tert-Butyl-5-(3-hydroxy-propoxy)-4-methoxy-phenyl]-2-(6-ethoxy-3-imino-7,8,9,10-tetrahydro-[1,2,4]triazolo[3,4-a]phthalazin-2-yl)-ethanon-Hydrochlorid | 1,38 | 512,3 (Met-a) |
| Bsp.: 121 | 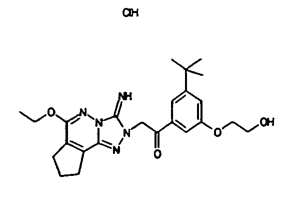 | 1-[3-tert-Butyl-5-(2-hydroxy-ethoxy)-phenyl]-2-(6-ethoxy-3-imino-8,9-dihydro-7H-cyclopenta[d][1,2,4]triazolo[4,3-b]pyridazin-2-yl)-ethanon-Hydrochlorid | 1,24 | 454,2 (Met-a) |
| Bsp.: 122 | 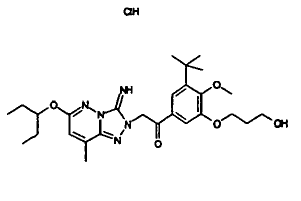 | 1-[3-tert-Butyl-5-(3-hydroxy-propoxy)-4-methoxy-phenyl]-2-[6-(1-ethyl-propoxy)-3-imino-8-methyl-[1,2,4]triazolo[4,3-b]pyridazin-2-yl]-ethanon-Hydrochlorid | 1,41 | 514,2 (Met-a) |
| Bsp.: 123 | 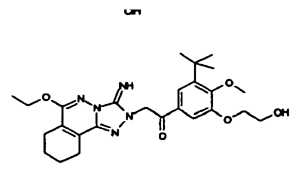 | 1-[3-tert-Butyl-5-(2-hydroxy-ethoxy)-4-methoxy-phenyl]-2-(6-ethoxy-3-imino-7,8,9,10-tetrahydro-[1,2,4]triazolo[3,4-a]phthalazin-2-yl)-ethanon-Hydrochlorid | 1,27 | 498,2 (Met-a) |
| Bsp.: 124 | 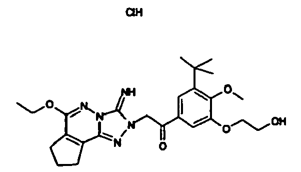 | 1-[3-tert-Butyl-5-(2-hydroxy-ethoxy)-4-methoxy-phenyl]-2-(6-ethoxy-3-imino-8,9-dihydro-7H-cyclopenta[d][1,2,4]triazolo[4,3-b]pyridazin-2-yl)-ethanon-Hydrochlorid | 1,21 | 484,2 (Met-a) |
| Bsp.: 125 | 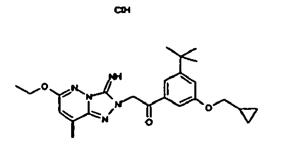 | 1-(3-tert-Butyl-5-cyclopropylmethoxy-phenyl)-2-(6-ethoxy-3-imino-8-methyl-[1,2,4]triazolo[4,3-b]pyridazin-2-yl)-ethanon-Hydrochlorid | 1,4 | 438,3 (Met-a) |
| Bsp.: 126 | 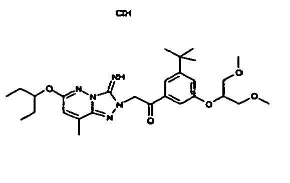 | 1-[3-tert-Butyl-5-(2-methoxy-1-methoxymethyl-ethoxy)-phenyl]-2-[6-(1-ethyl-propoxy)-3-imino-8-methyl-[1,2,4]triazolo[4,3-b]pyridazin-2-yl]-ethanon-Hydrochlorid | 1,46 | 528,3 (Met-a) |
| Bsp.: 127 | 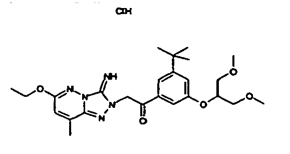 | 1-[3-tert-Butyl-5-(2-methoxy-1-methoxymethyl-ethoxy)-phenyl]-2-(6-ethoxy-3-imino-8-methyl-[1,2,4]triazolo[4,3-b]pyridazin-2-yl)-ethanon-Hydrochlorid | 1,31 | 486,2 (Met-a) |
| Bsp.: 128 | 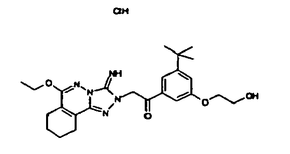 | 1-[3-tert-Butyl-5-(2-hydroxy-ethoxy)-phenyl]-2-(6-ethoxy-3-imino-7,8,9,10-tetrahydro-[1,2,4]triazolo[3,4-a]phthalazin-2-yl)-ethanon-Hydrochlorid | 1,3 | 468,2 (Met-a) |
| Bsp.: 129 | 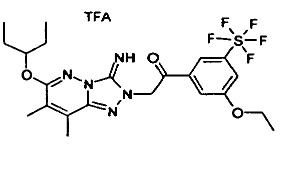 | 1-[3-Ethoxy-5-(pentafluorsulfanyl)-phenyl]-2-[6-(1-ethyl-propoxy)-3-imino-7,8-dimethyl-[1,2,4]triazolo[4,3-b]pyridazin-2-yl]-ethanon-Trifluoressigsäuresalz | 1,1 | 538,2 (Met-b) |
| Bsp.: 130 | 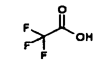 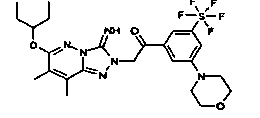 | 2-[6-(1-Ethyl-propoxy)-3-imino-7,8-dimethyl-[1,2,4]triazolo[4,3-b]pyridazin-2-yl]-1-[3-morpholin-4 yl-5-(pentafluorsulfanyl)-phenyl]-ethanon-Trifluoressigsäuresalz | 1,05 | 565,3 (Met-b) |
| Bsp.: 131 | 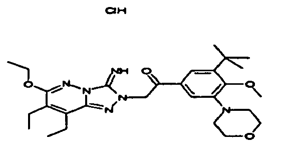 | 1-(3-tert-B utyl-4-methoxy-5-morpholin-4-yl-phenyl)-2-(6-ethoxy-7,8-diethyl-3-imino-[1,2,4]triazolo[4,3-b]pyridazin-2-yl)-ethanon-Hydrochlorid | 1,09 | 525,4 (Met-b) |
| Bsp.: 132 | 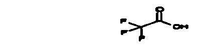 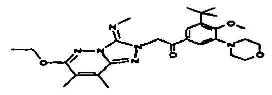 | 1-(3-tert-Buty!-4-methoxy-5-morpholin-4-yl-phenyl)-2-{6-ethoxy-7,8-dimethyl-3-[(E)-methylimino]-[1,2,4]triazolo[4,3-b]pyridazin-2-yl}-ethanon-Trifluoressigsäuresalz | 1,06 | 511,4 (Met-b) |
| Bsp.: 133 | 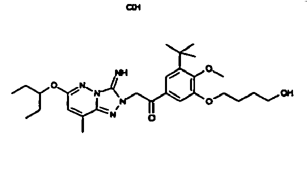 | 1-[3-tert-Butyl-5-(4-hydroxy-butoxy)-4-methoxy-phenyl]-2-[6-(1-ethyl-propoxy)-3-imino-8-methyl-[1,2,4]triazolo[4,3-b]pyridazin-2-yl]-ethanon-Hydrochlorid | 1,42 | 528,3 (Met-a) |
| Bsp.: 134 | 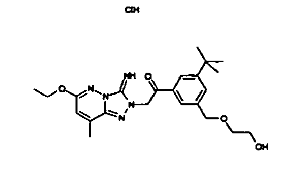 | 1-[3-tert-Butyl-5-(2-hydroxy-ethoxymethyl)-phenyl]-2-(6-ethoxy-3-imino-8-methyl-[1,2,4]triazolo[4,3-b]pyridazin-2-yl)-ethanon-Hydrochlorid | 1,16 | 442,3 (Met-a) |
| Bsp.: 135 | 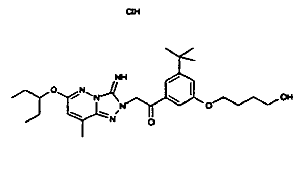 | 1-[3-tert-Butyl-5-(4-hydroxy-butoxy)-phenyl]-2-[6-(1-ethyl-propoxy)-3-imino-8-methyl-[1,2,4]triazolo[4,3-b]pyridazin-2-yl]-ethanon-Hydrochlorid | 1,4 | 498,2 (Met-a) |
| Bsp.: 136 | 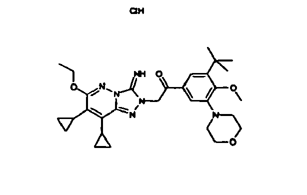 | 1-(3-tert-Butyl-4-methoxy-5-morpholin-4-yl-phenyl)-2-(7,8-dicyclopropyl-6-ethoxy-3-imino-[1,2,4]triazolo[4,3-b]pyridazin-2-yl)-ethanon-Hydrochlorid | 1,11 | 549,4 (Met-b) |
| Bsp.: 137 | 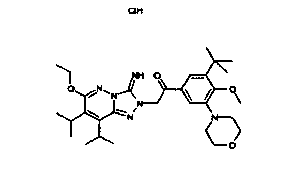 | 1-(3-tert-Butyl-4-methoxy-5-morpholin-4-yl-phenyl)-2-(6-ethoxy-3-imino-7,8-diisopropyl-[1,2,4]triazolo[4,3-b]pyridazin-2-yl)-ethanon-Hydrochlorid | 1,15 | 553,4 (Met-b) |
| Bsp.: 138 | 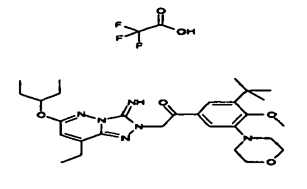 | 1-(3-tert-Butyl-4-methoxy-5-morpholin-4-yl-phenyl)-2-[8-ethyl-6-(1-ethyl-propoxy)-3-imino-[1,2,4]triazolo[4,3-b]pyridazin-2-yl]-ethanon-Trifluoressigsäuresalz | 1,11 | 539,4 (Met-b) |
| Bsp.: 139 | 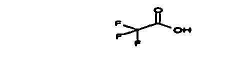 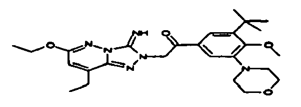 | 1-(3-tert-Butyl-4-methoxy-5-morpholin-4-yl-phenyl)-2-(6-ethoxy-8-ethyl-3-imino-[1,2,4] triazolo[4,3-b]pyridazin-2-yl)-ethanon-Trifluoressigsäuresalz | 1,04 | 497,3 (Met-b) |
| Bsp.: 140 | 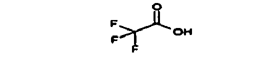 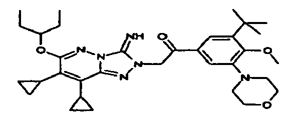 | 1-(3-tert-Butyl-4-methoxy-5-morpholin-4-yl-phenyl)-2-[7,8-dicyclopropyl-6-(1-ethyl-propoxy)-3-imin-[1,2,4] triazolo[4,3-b]pyridazin-2-yl]-ethanon-Trifluoressigsäuresalz | 1,16 | 591,4 (Met-b) |
| Bsp.: 141 | 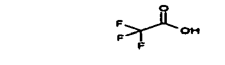 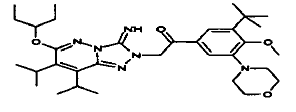 | 1-(3-tert-Butyl-4-methoxy-5-morpholin-4-yl-phenyl)-2-[6-(1-ethyl-propoxy)-3-imino-7,8-diisopropyl-[1,2,4]triazolo[4,3-b]pyridazin-2-yl]-ethanon-Trifluoressigsäuresalz | 1,19 | 595,5 (Met-b) |
| Bsp.: 142 | 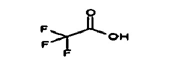 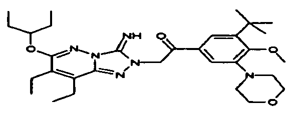 | 1-(3-tert-Butyl-4-methoxy-5-morpholin-4-yl-phenyl)-2-[7,8-diethyl-6-(1-ethyl-propoxy)-3-imino-[1,2,4]triazolo[4,3-b]pyridazin-2-yl]-ethanon-Trifluoressigsäuresalz | 1,15 | 567,4 (Met-b) |
| Bsp.: 143 | 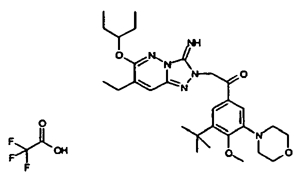 | 1-(3-tert-Butyl-4-methoxy-5-morpholin-4-yl-phenyl)-2-[7-ethyl-6-(1-ethyl-propoxy)-3-imino-[1,2,4]triazolo[4,3-b]pyridazin-2-yl]-ethanon-Trifluoressigsäuresalz | 1,11 | 539,4 (Met-b) |
| Bsp.: 144 | 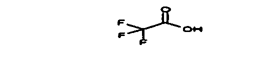 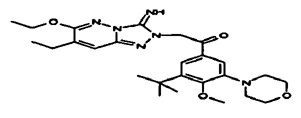 | 1-(3-tert-Butyl-4-methoxy-5-morpholin-4-yl-phenyl)-2-(6-ethoxy-7-ethyl-3-imino-[1,2,4]triazolo[4,3-b]pyridazin-2-yl)-ethanon-Trifluoressigsäuresalz | 1,04 | 497,3 (Met-b) |
| Bsp.: 145 | 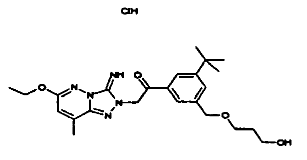 | 1-[3-tert-Butyl-5-(3-hydroxy-propoxymethyl)-phenyl]-2-(6-ethoxy-3-imino-8-methyl-[1,2,4]triazolo[4,3-b]pyridazin-2-yl)-ethanon-Hydrochlorid | 1,19 | 456,2 (Met-a) |
| Bsp.: 146 | 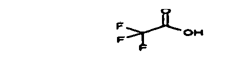 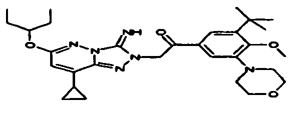 | 1-(3-tert-Butyl-4-methoxy-5-morpholin-4-yl-phenyl)-2-[8-cyclopropyl-6-(1-ethyl-propoxy)-3, imino-[1,2,4]triazolo[4,3-b]pyridazin-2-yl]-ethanon-Trifluoressigsäuresalz | 1,11 | 551,4 (Met-b) |
| Bsp.: 147 | 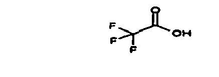 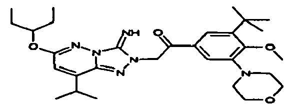 | 1-(3-tert-Butyl-4-methoxy-5-morpholin-4-yl-phenyl)-2-[6-(1-ethyl-propoxy)-3-imino-8-isopropyl-[1,2,4]triazolo[4,3-b]pyridazin-2-yl]-ethanon-Trifluoressigsäuresalz | 1,13 | 553,4 (Met-b) |
| Bsp.: 148 | 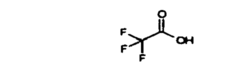 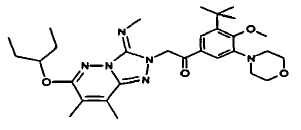 | 1-(3-tert-Butyl-4-methoxy-5-morpholin-4-yl-phenyl)-2-{6-(1-ethyl-propoxy)-7,8-dimethyl-3-[(E)-methylimino]-[1,2,4]triazolo[4,3-b]pyridazin-2-yl}-ethanon-Trifluoressigsäuresalz | 1,12 | 553,4 (Met-b) |
| Bsp.: 149 | 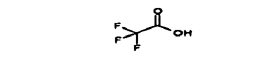 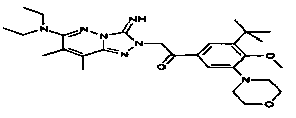 | 1-(3-tert-Butyl-4-methoxy-5-morpholin-4-yl-phenyl)-2-(6-diethylamino-3-imino-7,8-dimethyl-[1,2,4]triazolo[4,3-b]pyridazin-2-yl)-ethanon-Trifluoressigsäuresalz | 1,08 | 524,4 (Met-b) |
| Bsp.: 150 | 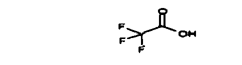 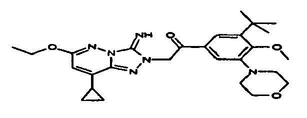 | 1-(3-tert-Butyl-4-methoxy-5-morpholin-4-yl-phenyl)-2-(8-cyclopropyl-6-ethoxy-3-imino-[1,2,4]triazolo[4,3-b]pyridazin-2-yl)-ethanon-Trifluoressigsäuresalz | 1,05 | 509,3 (Met-b) |
| Bsp.: 151 | 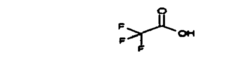 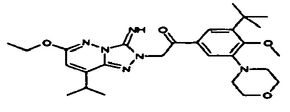 | 1-(3-tert-Butyl-4-methoxy-5-morpholin-4-yl-phenyl)-2-(6-ethoxy-3-imino-8-isopropyl-[1,2,4]triazolo[4,3-b]pyridazin-2-yl)-ethanon-Trifluoressigsäuresalz | 1,07 | 511,4 (Met-b) |
| Bsp.: 152 | 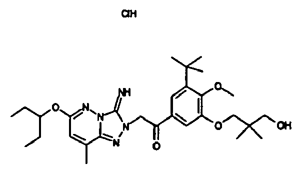 | 1-[3-tert-Butyl-5-(3-hydroxy-2,2-dimethyl-propoxy)-4-methoxy-phenyl]-2-[6-(1-ethyl-propoxy)-3-imino-8-methyl-[1,2,4]triazolo[4,3-b]pyridazin-2-yl]-ethanon-Hydrochlorid | 1,5 | 542,3 (Met-a) |
| Bsp.: 153 | 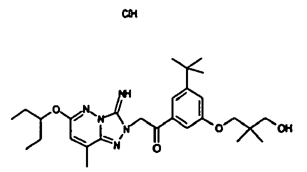 | 1-[3-tert-Butyl-5-(3-hydroxy-2,2-dimethyl-propoxy)-phenyl]-2-[6-(1-ethyl-propoxy)-3-imino-8-methyl-[1,2,4]triazolo[4,3-b]pyridazin-2-yl]-ethanon-Hydrochlorid | 1,48 | 512,3 (Met-a) |
| Bsp.: 154 | 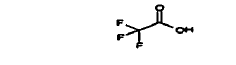 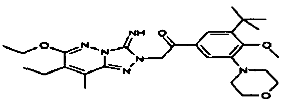 | 1-(3-tert-Butyl-4-methoxy-5-morpholin-4-yl-phenyl)-2-(6-ethoxy-7-ethyl-3-imino-8-methyl-[1,2,4]triazolo[4,3-b]pyridazin-2-yl)-ethanon-Trifluoressigsäuresalz | 1,07 | 511,4 (Met-b) |
| Bsp.: 155 | 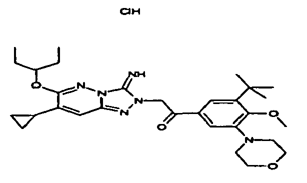 | 1-(3-tert-Butyl-4-methoxy-5-morpholin-4-yl-phenyl)-2-[7-cyclopropyl-6-(1-ethyl-propoxy)-3-imino-[1,2,4]triazolo[4,3-b]pyridazin-2-yl]-ethanon-Hydrochlorid | 1,12 | 551,4 (Met-b) |
| Bsp.: 156 | 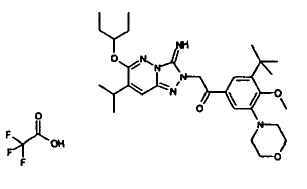 | 1-(3-tert-Butyl-4-methoxy-5-morpholin-4-yl-phenyl)-2-[6-(1-ethyl-propoxy)-3-imino-7-isopropyl-[1,2,4]triazolo[4,3-b]pyridazin-2-yl]-ethanon-Trifluoressigsäuresalz | 1,13 | 553,4 (Met-b) |
| Bsp.: 157 | 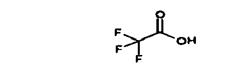 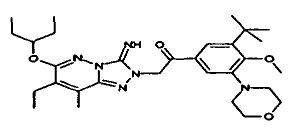 | 1-(3-tert-Butyl-4-methoxy-5-morpholin-4-yl-phenyl)-2-[7-ethyl-6-(1-ethyl-propoxy)-3-imino-8-methyl-[1,2,4]triazolo[4,3-b]pyridazin-2-yl]-ethanon-Trifluoressigsäuresalz | 1,14 | 553,4 (Met-b) |
| Bsp.: 158 | 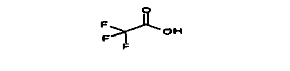 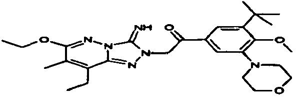 | 1-(3-tert-Butyl-4-methoxy-5-morpholin-4-yl-phenyl)-2-(6-ethoxy-8-ethyl-3-imino-7-methyl-[1,2,4]triazolo[4,3-b]pyridazin-2-yl)-ethanon-Trifluoressigsäuresalz | 1,07 | 511,4 (Met-b) |
| Bsp.: 159 | 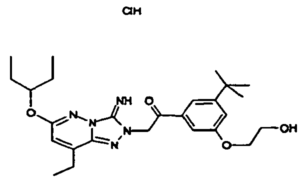 | 1-[3-tert-Butyl-5-(2-hydroxy-ethoxy)-pheny)]-2-[8-ethy)-6-(1-ethyl-propoxy)-3-imino-[1,2,4]triazolo[4,3-b]pyridazin-2-yl]-ethanon-Hydrochlorid | 1,03 | 484,4 (Met-b) |
| Bsp.: 160 | 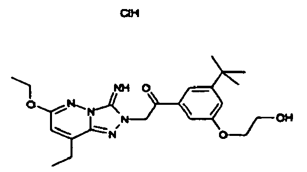 | 1-[3-tert-Butyl-5-(2-hydroxy-ethoxy)-phenyl]-2-(6-ethoxy-8-ethyl-3-imino-[1,2,4]triazolo[4,3-b]pyridazin-2-yl)-ethanon-Hydrochlorid | 0,95 | 442,3 (Met-b) |
| Bsp.: 161 | 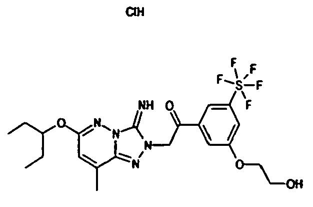 | 2-[6-(1-Ethyl-propoxy)-3-imino-8-methyl-[1,2,4]triazolo[4,3-b]pyridazin-2-yl]-1-[3-(2-hydroxy-ethoxy)-5-(pentafluorsulfanyl)-phenyl]-ethanon-Trifluoressigsäuresalz | 0,89 | 540,2 (Met-b) |
| Bsp.: 162 | 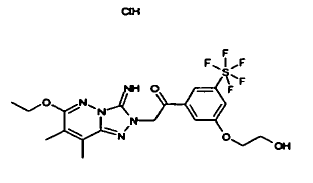 | 2-(6-Ethoxy-3-imino-7,8-dimethyl-[1,2,4]triazolo[4,3-b]pyridazin-2-yl)-1-[3-(2-hydroxy-ethoxy)-5-(pentafluorsulfanyl)-phenyl]-ethanon-Hydrochlorid | 1,23 | 512,2 (Met-a) |
| Bsp.: 163 | 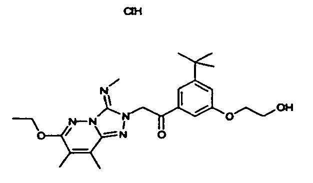 | 1-[3-tert-Butyl-5-(2-hydroxy-ethoxy)-phenyl]-2-(6-ethoxy-7,8-dimethyl-3-[(E)-methylimino]-[1,2,4]triazolo[4,3-b]pyridazin-2-yl}-ethanon-Hydrochlorid | 0,97 | 456,3 (Met-b) |
| Bsp.: 164 | 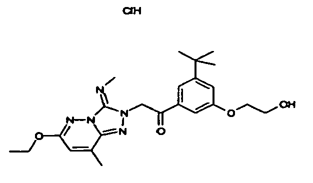 | 1-[3-tert-Butyl-5-(2-hydroxy-ethoxy)-phenyl]-2-{6-ethoxy-8-methyl-3-[(E)-methylimino]-[1,2,4]triazolo[4,3-b]pyridazin-2-yl)-ethanon-Hydrochlorid | 0,94 | 442,3 (Met-b) |
| Bsp.: 165 | 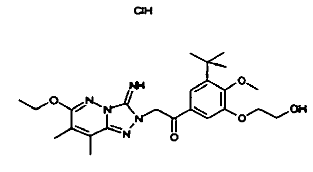 | 1-[3-tert-Butyl-5-(2-hydroxy-ethoxy)-4-methoxy-phenyl]-2-(6-ethoxy-3-imino-7,8-dimethyl-[1,2,4]triazolo[4,3-b]pyridazin-2-yl)-ethanon-Hydrochlorid | 0,83 | 472,2 (Met-b) |
| Bsp.: 166 | 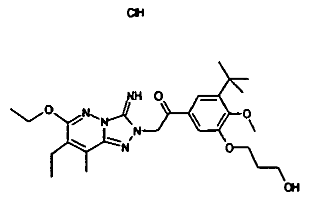 | 1-[3-tert-Butyl-5-(3-hydroxy-propoxy)-4-methoxy-phenyl]-2-(6-ethoxy-7-ethyl-3-imino-8-methyl-[1,2,4]triazolo[4,3-b]pyridazin-2-yl)-ethanon-Hydrochlorid | 1,03 | 500,3 (Met-b) |
| Bsp.: 167 | 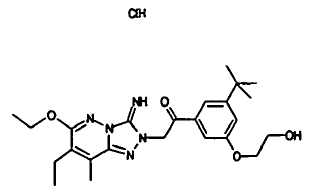 | 1-[3-tert-Butyl-5-(2-hydroxy-ethoxy)-phenyl]-2-(6-ethoxy-7-ethyl-3-imino-8-methyl-[1,2,4]triazolo[4,3-b]pyridazin-2-yl)-ethanon-Hydrochlorid | 0,98 | 456,3 (Met-b) |
| Bsp.: 168 | 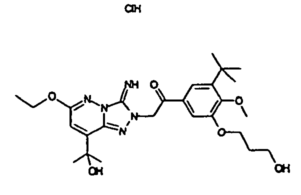 | 1-[3-tert-Butyl-5-(3-hydroxy-propoxy)-4-methoxy-phenyl]-2-[6-ethoxy-8-(1-hydroxy-1-methyl-ethyl)-3-imino-[1,2,4]triazolo[4,3-b]pyridazin-2-yl]-ethanon-Hydrochlorid | 0,96 | 516,3 (Met-b) |
| Bsp.: 169 | 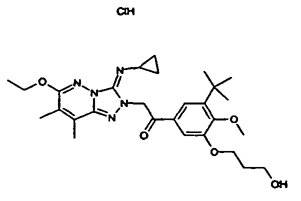 | 1-[3-tert-Butyl-5-(3-hydroxy-propoxy)-4-methoxy-phenyl]-2-{3-[(E)-cyclopropylimino]-6-ethoxy-7,8-dimethyl-[1,2,4]triazolo[4,3-b]pyridazin-2-yl}-ethanon-Hydrochlorid | 1,04 | 526,4 (Met-b) |
| Bsp.: 170 | 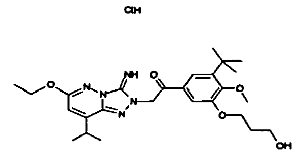 | 1-[3-tert-Butyl-5-(3-hydroxy-propoxy)-4-methoxy-phenyl]-2-(6-ethoxy-3-imino-8-isopropyl-[1,2,4]triazolo[4,3-b]pyridazin-2-yl)-ethanon-Hydrochlorid | 1,03 | 500,3 (Met-b) |
| Bsp.: 171 | 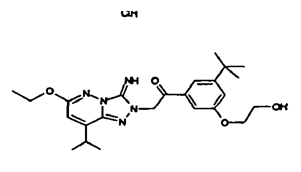 | 1-[3-tert-Butyl-5-(2-hydroxy-ethoxy)-phenyl]-2-(6-ethoxy-3-imino-8-isopropyl-[1,2,4]triazolo[4,3-b]pyridazin-2-yl)-ethanon-Hydrochlorid | 1,00 | 456,3 (Met-b) |
| Bsp.: 172 | 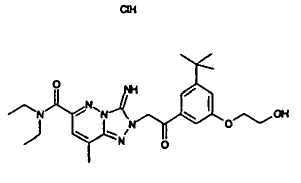 | 2-{2-[3-tert-Butyl-5-(2-hydroxy-ethoxy)-phenyl]-2-oxo-ethyl}-3-imino-8-methyl-2,3-dihydro-[1,2,4]triazolo[4,3-b]pyridazine-6-carbonsäure-diethylamide-Hydrochlorid | 0,74 | 483,3 (Met-b) |
| Bsp.: 173 | 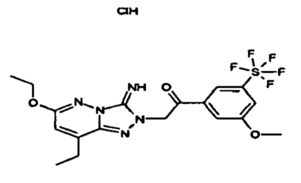 | 2-(6-Ethoxy-8-ethyl-3-imino-[1,2,4]triazolo[4,3-b]pyridazin-2-yl)-1-[3-methoxy-5-(pentafluorsulfanyl)-phenyl]-ethanon-Hydrochlorid | 1,02 | 482,2 (Met-b) |
| Bsp.: 174 | 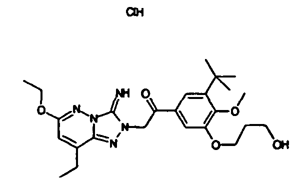 | 1-[3-tert-Butyl-5-(3-hydroxy-propoxy)-4-methoxy-phenyl]-2-(6-ethoxy-8-ethyl-3-imino-[1,2,4]triazolo[4,3-b]pyridazin-2-yl)-ethanon-Hydrochlorid | 1,00 | 486,3 (Met-b) |
| Bsp.: 175 | 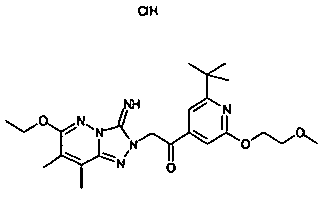 | 1-[2-tert-Butyl-6-(2-methoxy-ethoxy)-pyridin-4-yl]-2-(6-ethoxy-3-imino-7,8-dimethyl-[1,2,4]triazolo[4,3-b]pyridazin-2-yl)-ethanon-Hydrochlorid | 0,83 | 472,2 (Met-b) |
| Bsp.: 176 | 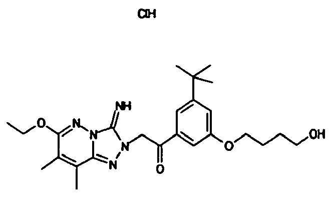 | 1-[3-tert-Butyl-5-(4-hydroxy-butoxy)-phenyl]-2-(6-ethoxy-3-imino-7,8-dimethyl-[1,2,4]triazolo[4,3-b]pyridazin-2-yl)-ethanon-Hydrochlorid | 0,82 | 470,2 (Met-b) |
| Bsp.: 178 | 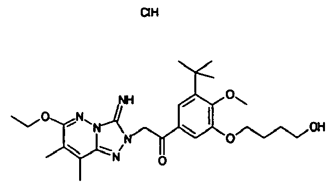 | 1-[3-tert-Butyl-5-(4-hydroxy-butoxy)-4-methoxy-phenyl]-2-(6-ethoxy-3-imino-7,8-dimethyl-[1,2,4]triazolo[4,3-b]pyridazin-2-yl)-ethanon-Hydrochlorid | 0,86 | 500,3 (Met-b) |
| Bsp.: 179 | 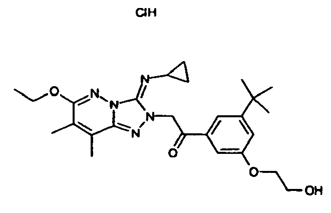 | 1-[3-tert-Butyl-5-(2-hydroxy-ethoxy)-phenyl]-2-{3-[(E)-cyclopropylimino]-6-ethoxy-7,8-dimethyl-[1,2,4]triazolo[4,3-b]pyridazin-2-yl}-ethanon-Hydrochlorid | 0,99 | 482,3 (Met-b) |
| Bsp.: 180 | 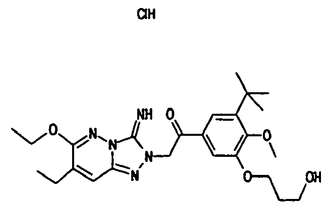 | 1-[3-tert-Butyl-5-(3-hydroxy-propoxy)-4-methoxy-phenyl]-2-(6-ethoxy-7-ethyl-3-imino-[1,2,4]triazolo[4,3-b]pyridazin-2-yl)-ethanon-Hydrochlorid | 0,99 | 486,3 (Met-b) |
| Bsp.: 181 | 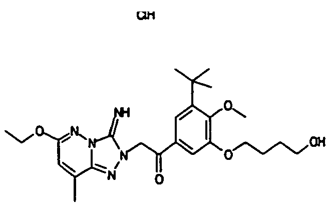 | 1-[3-tert-Butyl-5-(4-hydroxy-butoxy)-4-methoxy-phenyl]-2-(6-ethoxy-3-imino-8-methyl-[1,2,4]triazolo[4,3-b]pyridazin-2-yl)-ethanon-Hydrochlorid | 0,86 | 482,2 (Met-b) |
| Bsp.: 182 | 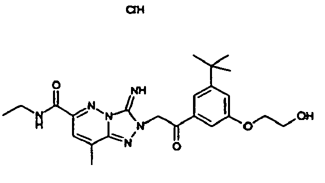 | 2-{2-[3-tert-Butyl-5-(2-hydroxy-ethoxy)-phenyl]-2-oxo-ethyl}-3-imino-8-methyl-2,3-dihydro-[1,2,4]triazolo[4,3-b]pyridazine-6-carbonsäure-ethylamid-Hydrochlorid | 0,74 | 455,2 (Met-b) |
| Bsp.: 183 | 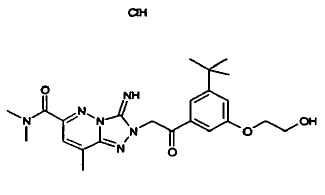 | 2-{2-[3-tert-Butyl-5-(2-hydroxy-ethoxy)-phenyl]-2-oxo-ethyl}-3-imino-8-methyl-2,3-dihydro-[1,2,4]triazolo[4,3-b]pyridazine-6-carbonsäure-dimethylamid-Hydrochlorid | 0,17 | 455,3 (Met-b) |
| Bsp.: 184 | 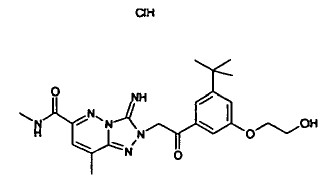 | 2-{2-[3-tert-Butyl-5-(2-hydroxy-ethoxy)-phenyl]-2-oxo-ethyl}-3-imino-8-methyl-2,3-dihydro-[1,2,4]triazolo[4,3-b]pyridazine-6-carbonsäure-methylamid-Hydrochlorid | 0,7 | 441,2 (Met-b) |
| Bsp.: 186 | 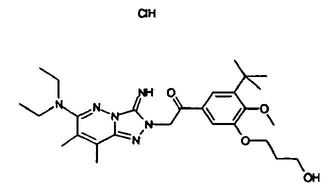 | 1-[3-tert-Butyl-5-(3-hydroxy-propoxy)-4-methoxy-phenyl]-2-(6-diethylamino-3-imino-7,8-dimethyl-[1,2,4]triazolo[4,3-b]pyridazin-2-yl)-ethanon-Hydrochlorid | 1,02 | 513,4 (Met-b) |
| Bsp.: 187 | 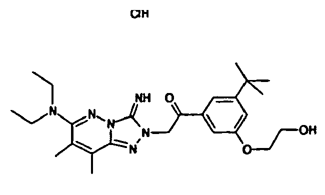 | 1-[3-tert-Butyl-5-(2-hydroxy-ethoxy)-phenyl]-2-(6-diethylamino-3-imino-7,8-dimethyl-[1,2,4]triazolo[4,3-b]pyridazin-2-yl)-ethanon-Hydrochlorid | 0,98 | 469,4 (Met-b) |
| Bsp.: 188 | 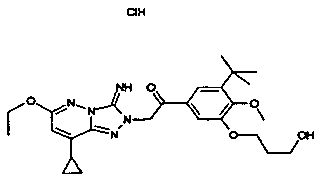 | 1-[3-tert-Butyl-5-(3-hydroxy-propoxy)-4-methoxy-phenyl]-2-(8-cyclopropyl-6-ethoxy-3-imino-[1,2,4]triazolo[4,3-b]pyridazin-2-yl)-ethanon-Hydrochlorid | 1,00 | 498,3 (Met-b) |
| Bsp.: 189 | 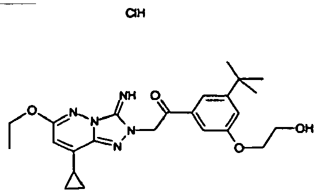 | 1-[3-tert-Butyl-5-(2-hydroxy-ethoxy)-phenyl]-2-(8-cyclopropyl-6-ethoxy-3-imino-[1,2,4]triazolo[4,3-b]pyridazin-2-yl)-ethanon-Hydrochlorid | 0,96 | 454,3 (Met-b) |
| Bsp.: 190 | 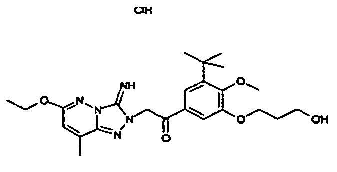 | 1-[3-tert-Butyl-5-(3-hydroxy-propoxy)-4-methoxy-phenyl]-2-(6-ethoxy-3-imino-8-methyl-[1,2,4]triazolo[4,3-b]pyridazin-2-yl)-ethanon-Hydrochlorid | 0,82 | 472,2 (Met-b) |
| Bsp.: 191 | 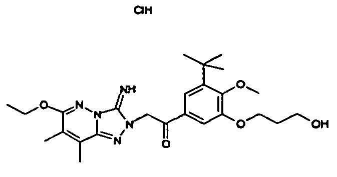 | 1-[3-tert-Butyl-5-(3-hydroxy-propoxy)-4-methoxy-phenyl]-2-(6-ethoxy-3-imino-7,8-dimethyl-[1,2,4]triazolo[4,3-b]pyridazin-2-yl)-ethanon-Hydrochlo rid | 0,85 | 486,2 (Met-b) |
| Bsp.: 192 | 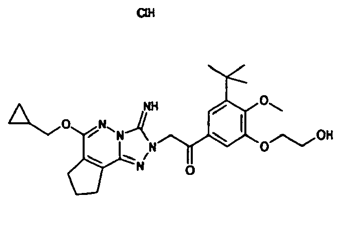 | 1-[3-tert-Butyl-5-(2-hydroxy-ethoxy)-4-methoxy-phenyl]-2-(6-cyclopropylmethoxy-3-imino-8,9-dihydro-7H-cyclopenta[d][1,2,4]triazolo [4,3-b]pyridazin-2-yl)-ethanon-Hydrochlorid | 0,89 | 510,2 (Met-b) |
| Bsp.: 193 | 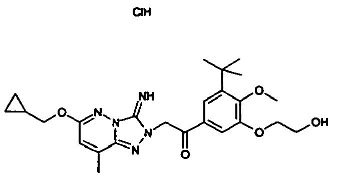 | 1-[3-tert-Butyl-5-(2-hydroxy-ethoxy)-4-methoxy-phenyl]-2-(6-cyclopropylmethoxy-3-imino-8-methyl-[1,2,4]triazolo[4,3-b]pyridazin-2-yl)-ethanon-Hydrochlorid | 0,83 | 484,2 (Met-b) |
| Bsp.: 194 | 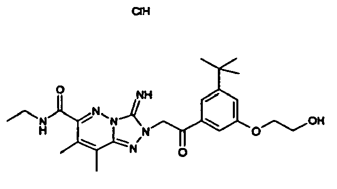 | 2-{2-[3-tert-Butyl-5-(2-hydroxy-ethoxy)-phenyl]-2-oxo-ethyl}-3-imino-7,8-dimethyl-2,3-dihydro-[1,2,4]triazolo[4,3-b]pyridazine-6-carbonsäure-ethylamid-Hydrochlorid | 0,73 | 469,2 (Met-b) |
| Bsp.: 195 | 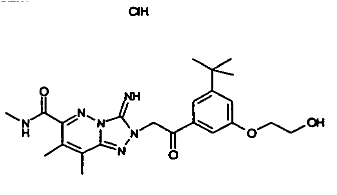 | 2-{2-[3-tert-Butyl-5-(2-hydroxy-ethoxy)-phenyl]-2-oxo-ethyl}-3-imino-7,8-dimethyl-2,3-dihydro-[1,2,4]triazolo[4,3-b]pyridazine-6-carbonsäuremethylamid-Hydrochlorid | 0,71 | 455,3 (Met-b) |
| Bsp.: 196 | 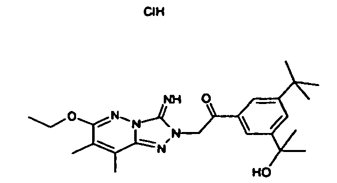 | 1-[3-tert-Butyl-5-(1-hydroxy-1-methyl-ethyl)-phenyl]-2-(6-ethoxy-3-imino-7,8-dimethyl-[1,2,4]triazolo[4,3-b]pyridazin-2-yl)-ethanon-Hydrochlorid | 0,98 | 440,3 (Met-b) |
| Bsp.: 197 | 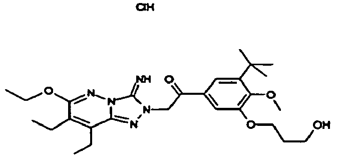 | 1-[3-tert-Butyl-5-(3-hydroxy-propoxy)-4-methoxy-phenyl]-2-(6-ethoxy-7,8-diethyl-3-imino-[1,2,4]triazolo[4,3-b]pyridazin-2-yl)-ethanon-Hydrochlorid | 1,06 | 514,4 (Met-b) |
| Bsp.: 198 | 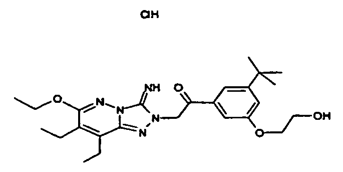 | 1-[3-tert-Butyl-5-(2-hydroxy-ethoxy)-phenyl]-2-(6-ethoxy-7,8-diethyl-3-imino-[1,2,4]triazolo[4,3-b]pyridazin-2-yl)-ethanon-Hydrochlorid | 1,02 | 470,3 (Met-b) |
| Bsp.: 199 | 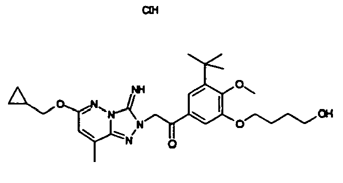 | 1-[3-tert-Butyl-5-(4-hydroxy-butoxy)-4-methoxy-phenyl]-2-(6-cyclopropylmethoxy-3-imino-8-methyl-[1,2,4]triazolo[4,3-b]pyridazin-2-yl)-ethanon-Hydrochlorid | 0,87 | 512,3 (Met-b) |
| Bsp.: 200 | 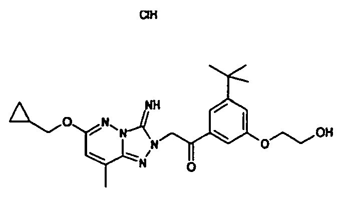 | 1-[3-tert-Butyl-5-(2-hydroxy-ethoxy)-phenyl]-2-(6-cyclopropylmethoxy-3-imino-8-methyl-[1,2,4]triazolo[4,3-b]pyridazin-2-yl)-ethanon-Hydrochlorid | 0,81 | 454,2 (Met-b) |
| Bsp.: 201 | 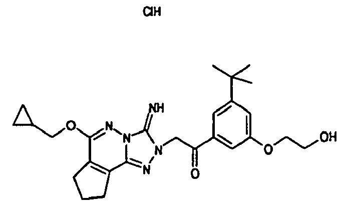 | 1-[3-tert-Butyl-5-(2-hydroxy-ethoxy)-phenyl]-2-(6-cyclopropylmethoxy-3-imino-8,9-dihydro-7H-cyclopenta[d][1,2,4]triazolo(4, 3-b]pyridazin-2-yl)-ethanon-Hydrochlorid | 0,85 | 480,2 (Met-b) |
| Bsp.: 202 | 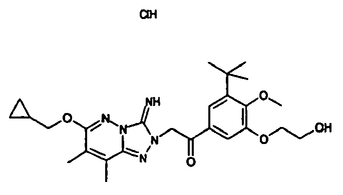 | 1-[3-tert-Butyl-5-(2-hydroxy-ethoxy)-4-methoxy-phenyl]-2-(6-cyclopropylmethoxy-3-imino-7,8-dimethyl-[1,2,4]triazolo[4,3-b]pyridazin-2-yl)-ethanon-Hydrochlorid | 0,87 | 498,2 (Met-b) |
| Bsp.: 203 | 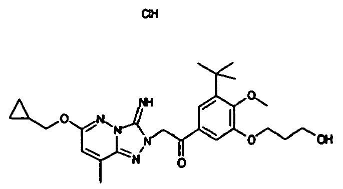 | 1-[3-tert-Butyl-5-(3-hydroxy-propoxy)-4-methoxy-phenyl]-2-(6-cyclopropylmethoxy-3-imino-8-methyl-[1,2,4]triazolo[4,3-b]pyridazin-2-yl)-ethanon-Hydrochlorid | 0,86 | 498,2 (Met-b) |
| Bsp.: 204 | 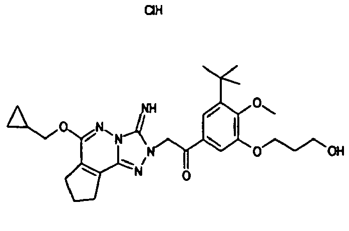 | 1-[3-tert-Butyl-5-(3-hydroxy-propoxy)-4-methoxy-phenyl]-2-(6-cyclopropylmethoxy-3-imino-8,9-dihydro-7H-cyclopenta[d][1,2,4]triazolo[4,3-b]pyridazin-2-yl)-ethanon-Hydrochlorid | 0,88 | 524,3 (Met-b) |
| Bsp.: 205 | 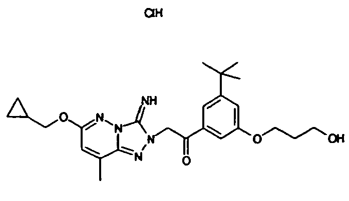 | 1-[3-tert-Butyl-5-(3-hydroxy-propoxy)-phenyl]-2-(6-cyclopropylmethoxy-3-imino-8-methyl-[1,2,4]triazolo[4,3-b]pyridazin-2-yl)-ethanon-Hydrochlorid | 0,83 | 468,2 (Met-b) |
| Bsp.: 206 | 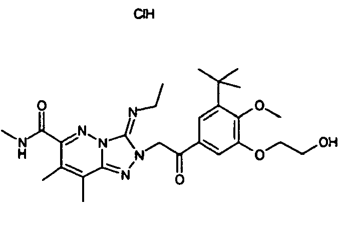 | 2-{2-[3-tert-Butyl-5-(2-hydroxy-ethoxy)-4-methoxy-phenyl]-2-oxo-ethyl}-3-[(E)-ethylimino]-7,8-dimethyl-2,3-dihydro-[1,2,4]triazolo[4,3-b]pyridazine-6-carbonsäure-methylamid-Hydrochlorid | 0,76 | 513,3 (Met-b) |
| Bsp.: 207 | 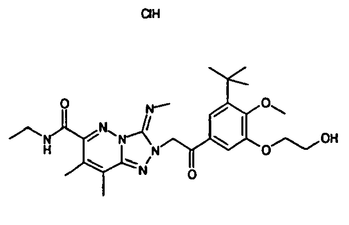 | 2-{2-[3-tert-Butyl-5-(2-hydroxy-ethoxy)-4-methoxy-phenyl]-2-oxo-ethyl}-7,8-dimethyl-3-[(E)-methylimino]-2,3-dihydro-[1,2,4]triazolo[4,3-b]pyridazine-6-carbonsäure-ethylamid-Hydrochlorid | 0,77 | 513,3 (Met-b) |
| Bsp.: 208 | 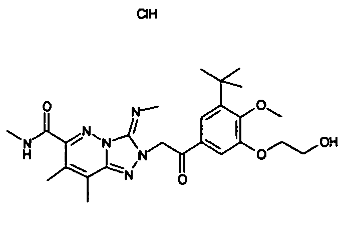 | 2-{2-[3-tert-Butyl-5-(2-hydroxy-ethoxy)-4-methoxy-phenyl]-2-oxo-ethyl}-7,8-dimethyl-3-[(E)-methylimino]-2,3-dihydro-[1,2,4]triazolo[4,3-b]pyridazine-6-carbonsäure-methylamid-Hydrochlorid | 0,75 | 499,2 (Met-b) |
| Bsp.: 209 | 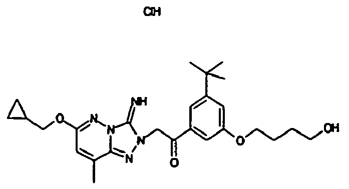 | 1-[3-tert-Butyl-5-(4-hydroxy-butoxy)-phenyl]-2-(6-cyclopropylmethoxy-3-imino-8-methyl-[1,2,4]triazolo[4,3-b]pyridazin-2-yl)-ethanon-Hydrochlorid | 0,85 | 482,2 (Met-b) |
| Bsp.: 210 | 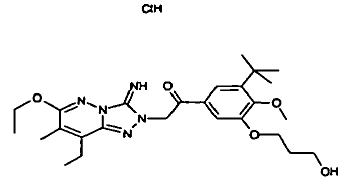 | 1-[3-tert-Butyl-5-(3-hydroxy-propoxy)-4-methoxy-phenyl]-2-(6-ethoxy-8-ethyl-3-imino-7-methyl-[1,2,4]triazolo[4,3-b]pyridazin-2-yl)-ethanon-Hydrochlorid | 1,02 | 500,4 (Met-b) |
| Bsp.: 211 | 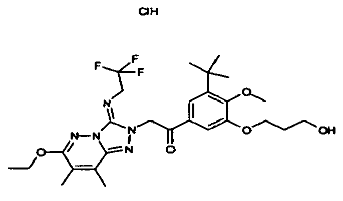 | 1-[3-tert-Butyl-5-(3-hydroxy-propoxy)-4-methoxy-phenyl]-2-{6-ethoxy-7,8-dimethyl-3-[(E)-2,2,2-trifluoro-ethylimino]-[1,2,4]triazolo[4,3-b]pyridazin-2-yl}-ethanon-Hydrochlorid | 1,04 | 568,3 (Met-b) |
| Bsp.: 212 | 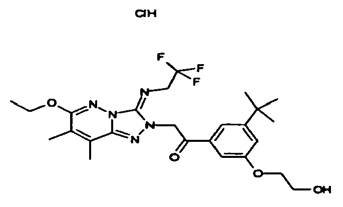 | 1-[3-tert-Butyl-5-(2-hydroxy-ethoxy)-phenyl]-2-{6-ethoxy-7,8-dimethyl-3-[(E)-2,2,2-trifluoro-ethylimino]-[1,2,4]triazolo[4,3-b]pyridazin-2-yl}-ethanon-Hydrochlorid | 1,01 | 524,3 (Met-b) |
| Bsp.: 213 | 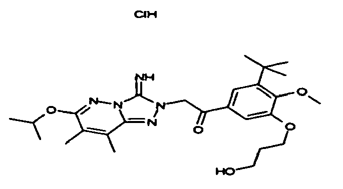 | 1-[3-tert-Butyl-5-(2-hydroxy-ethoxy)-phenyl]-2-{6-ethoxy-7,8-dimethyl-3-[(E)-2,2,2-trifluoro-ethylimino]-[1,2,4]triazolo[4,3-b]pyridazin-2-yl}-ethanon-Hydrochlorid | 1,02 | 500,4 (Met-b) |
| Bsp.: 214 | 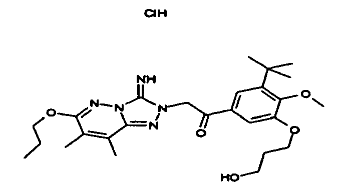 | 1-[3-tert-Butyl-5-(3-hydroxy-propoxy)-4-methoxy-phenyl]-2-(3-imino-7,8-dimethyl-6-propoxy-[1,2,4]triazolo[4,3-b]pyridazin-2-yl)-ethanon-Hydrochlorid | 1,02 | 500,3 (Met-b) |
| Bsp.: 215 | 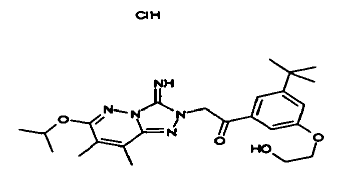 | 1-[3-tert-Butyl-5-(2-hydroxy-ethoxy)-phenyl]-2-(3-imino-6-isopropoxy-7,8-dimethyl-[1,2,4]triazofo[4,3-b]pyridazin-2-yl)-ethanon-Hydrochlorid | 0,98 | 456,3 (Met-b) |
| Bsp.: 216 | 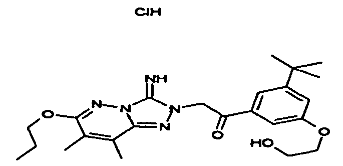 | 1-[3-tert-Butyl-5-(2-hydroxy-ethoxy)-phenyl]-2-(3-imino-7,8-dimethyl-6-propoxy-[1,2,4]triazolo[4,3-b]pyridazin-2-yl)-ethanon-Hydrochlorid | 0,98 | 456,3 (Met-b) |
| Bsp.: 217 | 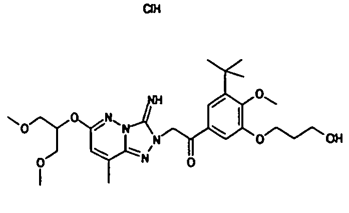 | 1-[3-tert-Butyl-5-(3-hydroxy-propoxy)-4-methoxy-phenyl]-2-[3-imino-6-(2-methoxy-1-methoxymethyl-ethoxy)-8-methyl-[1,2,4]triazolo[4,3-b]pyridazin-2-yl]-ethanon-Hydrochlorid | 0,8 | 546,3 (Met-b) |
| Bsp.: 218 | 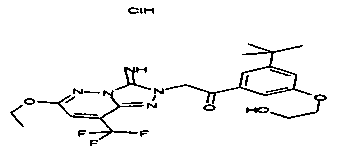 | 1-[3-tert-Butyl-5-(2-hydroxy-ethoxy)-phenyl]-2-(6-ethoxy-3-imino-8-trifluoromethyl-[1,2,4]triazolo[4,3-b]pyridazin-2-yl)-ethanon-Hydrochlorid | 0,97 | 482,3 (Met-b) |
| Bsp.: 219 | 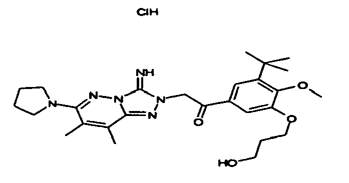 | 1-[3-tert-Butyl-5-(3-hydroxy-propoxy)-4-methoxy-phenyl]-2-(3-imino-7,8-dimethyl-6-pyrrolidin-1-yl-[1,2,4]triazolo[4,3-b]pyridazin-2-yl)-ethanon-Hydrochlorid | 1,01 | 511,4 (Met-b) |
| Bsp.: 220 | 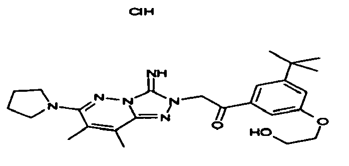 | 1-[3-tert-Butyl-5-(2-hydroxy-ethoxy)-phenyl]-2-(3-imino-7,8-dimethyl-6-pyrrolidin-1-yl-[1,2,4]triazolo[4,3-b]pyridazin-2-yl)-ethanon-Hydrochlorid | 0,97 | 467,3 (Met-b) |
| Bsp.: 221 | 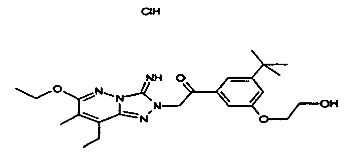 | 1-[3-tert-Butyl-5-(2-hydroxy-ethoxy)-phenyl]-2-(6-ethoxy-8-ethyl-3-imino-7-methyl-[1,2,4]triazolo[4,3-b]pyridazin-2-yl)-ethanon-Hydrochlorid | 0,97 | 456,3 (Met-b) |
| Bsp.: 222 | 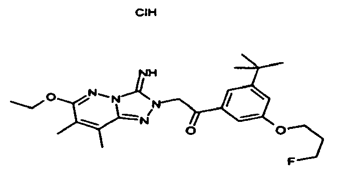 | 1-[3-tert-B utyl-5-(3-fluoro-propoxy)-phenyl]-2-(6-ethoxy-3-imino-7,8-dimethyl-[1,2,4]triazolo[4,3-b]pyridazin-2-yl)-ethanon-Hydrochlorid | 1,04 | 458,3 (Met-b) |
| Bsp.: 223 | 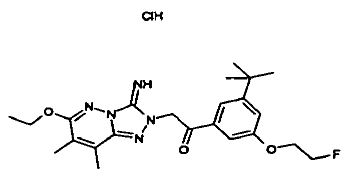 | 1-[3-tert-Butyl-5-(2-fluoro-ethoxy)-phenyl]-2-(6-ethoxy-3-imino-7,8-dimethyl-[1,2,4]triazolo[4,3-b]pyridazin-2-yl)-ethanon-Hydrochlorid | 1,02 | 444,3 (Met-b) |
| Bsp.: 224 | 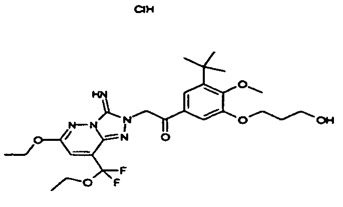 | 1-[3-tert-Butyl-5-(3-hydroxy-propoxy)-4-methoxy-phenyl]-2-[6-ethoxy-8-(ethoxy-difluoro-methyl)-3-imino-[1,2,4]triazolo[4,3-b]pyridazin-2-yl]-ethanon-Hydrochlorid | 1,02 | 552,3 (Met-b) |
| Bsp.: 225 | 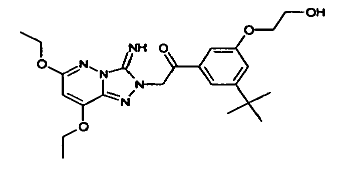 | 1-[3-tert-Butyl-5-(2-hydroxy-ethoxy)-phenyl]-2-(6,8-diethoxy-3-imino-[1,2,4]triazolo[4,3-b]pyridazin-2-yl)-ethanon-Hydrochlorid | 0,82 | 458,2 (Met-b) |
| Bsp.: 226 | 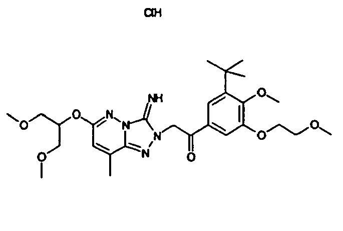 | 1-[3-tert-Butyl-4-methoxy-5-(2methoxy-ethoxy)-phenyl]-2-[3-imino-6-(2-methoxy-1-methoxymethyl-ethoxy)-8-methyl-[1,2,4]triazolo[4,3-b]pyridazin-2-yl]-ethanon-Hydrochlorid | 0,85 | 546,3 (Met-b) |
| Bsp.: 227 | 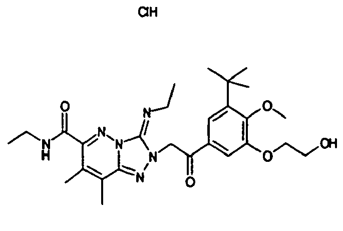 | 2-{2-[3-tert-Butyl-5-(2-hydroxy-ethoxy)-4-methoxy-phenyl]-2-oxo-ethyl}-3-[(E)-ethylimino]-7,8-dimethyl-2,3-dihydro-[1,2,4]triazolo[4,3-b]pyridazine-6-carbonsäure-ethylamid-Hydrochlorid | 0,78 | 527,2 (Met-b) |
| Bsp.: 228 | 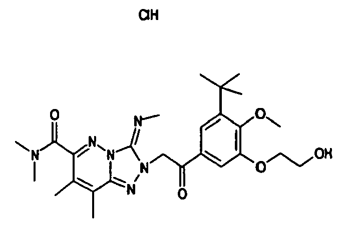 | 2-{2-[3-tert-Butyl-5-(2-hydroxy-ethoxy)-4-methoxy-phenyl]-2-oxo ethyl}-7,8-dimethyl-3-[(E)-methylimino]-2,3-dihydro-[1,2,4]triazolo[4,3-b]pyridazine-6-carbonsäure-dimethylamid-Hydrochlorid | 0,75 | 513,3 (Met-b) |
| Bsp.: 229 | 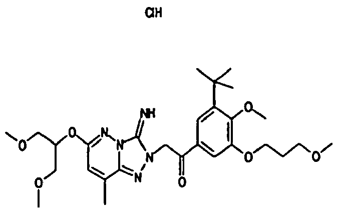 | 1-[3-tert-Butyl-4-methoxy-5-(3-methoxy-propoxy)-phenyl]-2-[3-imino-6-(2-methoxy-1-methoxymethyl-ethoxy)-8-methyl-[1,2,4]triazolo[4,3-b]pyridazin-2-yl]-ethanon-Hydrochlorid | 0,88 | 560,3 (Met-b) |
| Bsp.: 230 | 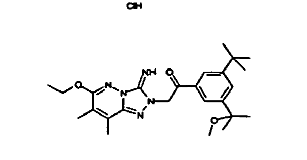 | 1-[3-tert-Butyl-5-(1-methoxy-1-methyl-ethyl)-phenyl]-2-(6-ethoxy-3-imino-7,8-dimethyl-[1,2,4]triazolo[4,3-b]pyridazin-2-yl)-ethanon-Hydrochlorid | 0,99 | 454,3 (Met-b) |
| Bsp.: 231 | 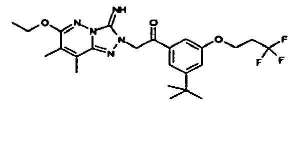 | 1-[3-tert-Butyl-5-(3,3,3-trifluoro-propoxy)-phenyl]-2-(6-ethoxy-3-imino-7,8-dimethyl-[1,2,4]triazolo[4,3-b]pyridazin-2-yl)-ethanon-Hydrochlorid | 0,98 | 494,2 (Met-b) |
| Bsp.: 232 | 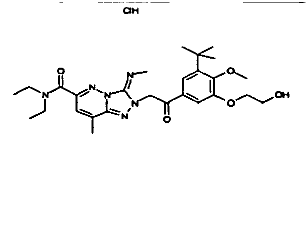 | 2-{2-[3-tert-Butyl-5-(2-hydroxy-ethoxy)-4-methoxy-phenyl]-2-oxo ethyl}-8-methyl-3-[(E)-methylimino]-2,3-dihydro-[1,2,4]triazolo[4,3-b]pyridazine-6-carbonsäure-diethylamid-Hydrochlorid | 0,78 | 527,2 (Met-b) |
| Bsp.: 233 | 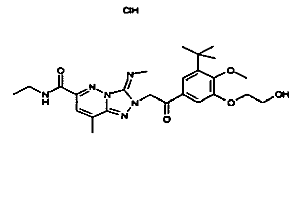 | 2-{2-[3-tert-Butyl-5-(2-hydroxy-ethoxy)-4-methoxy-phenyl]-2-oxo-ethyl}-8-methyl-3-[(E)-methylimino]-2,3-dihydro-[1,2,4]triazolo[4,3-b]pyridazine-6-carbonsäure-ethylamid-Hydrochlorid | 0,77 | 499,2 (Met-b) |
| Bsp.: 234 | 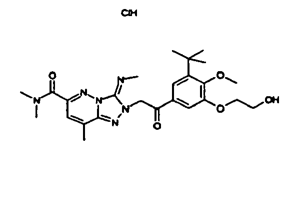 | 2-{2-[3-tert-Butyl-5-(2-hydroxy-ethoxy)-4-methoxy-phenyl]-2-oxo-ethyl}-8-methyl-3-[(E)-methylimino]-2,3-dihydro-[1,2,4]triazolo[4,3-b]pyridazine-6-carboxylic acid dimethylamid-Hydrochlorid | 0,74 | 499,2 (Met-b) |
| Bsp.: 235 | 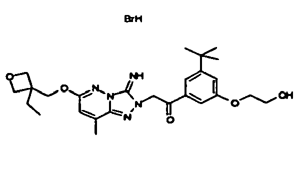 | 1-[3-tert-Butyl-5-(2-hydroxy-ethoxy)-phenyl]-2-[6-(3-ethyl-oxetan-3-ylmethoxy)-3-imino-8-methyl-[1,2,4]triazolo[4,3-b]pyridazin-2-yl]-ethanon-Hydrobromid | 0,78 | 498,2 (Met-b) |
| Bsp.: 236 | 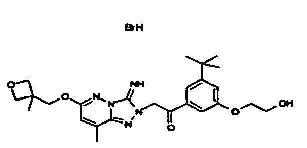 | 1-[3-tert-Butyl-5-(2-hydroxy-ethoxy)-phenyl]-2-[3-imino-8-methyl-6-(3-methyl-oxetan-3-ylmethoxy)-[1,2,4]triazolo[4,3-b]pyridazin-2-yl]-ethanon-Hydrobromid | 0,75 | 484,2 (Met-b) |
| Bsp.: 237 | 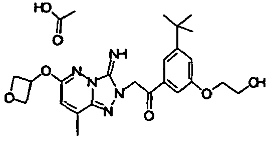 | 1-[3-tert-Butyl-5-(2-hydroxy-ethoxy)-phenyl]-2-[3-imino-8-methyl-6-(oxetan-3-yloxy)-[1,2,4]triazolo[4,3-b]pyridazin-2-yl]-ethanon | 0,73 | 456,2 (Met-b) |
| Bsp.: 238 | 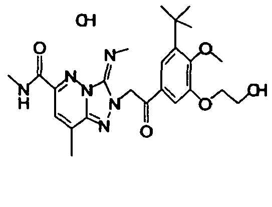 | 2-{2-[3-tert-Butyl-5-(2-hydroxy-ethoxy)-4-methoxy-phenyl]-2-oxo-ethyl}-8-methyl-3-[(E)-methylimino]-2,3-dihydro-[1,2,4]triazolo[4,3-b]pyridazin-6-carbonsäure-methylamid-Hydrochlorid | 0,76 | 485,2 (Met-b) |
| Bsp.: 239 | 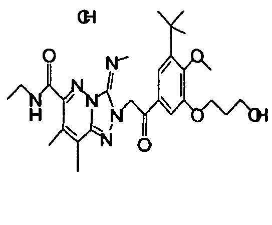 | 2-{2-[3-tert-Butyl-5-(3-hydroxy-propoxy)-4-methoxy-phenyl]-2-oxo-ethyl}-7,8-dimethyl-3-[(E)-methylimino]-2,3-dihydro-[1,2,4]triazolo[4,3-b]pyridazin-6-carbonsäure-ethylamid-Hydrochlorid | 0,80 | 527,2 (Met-b) |
| Bsp.: 240 | 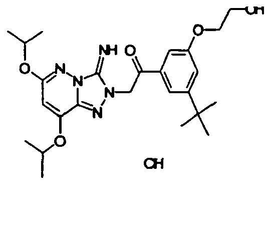 | 1-[3-tert-Butyl-5-(2-hydroxy-ethoxy)-phenyl]-2-(3-imino-6,8-diisopropoxy-[1,2,4]triazolo[4,3-b]pyridazin-2-yl)-ethanon-Hydrochlorid | 0,90 | 486,3 (Met-b) |
| Bsp.: 241 | 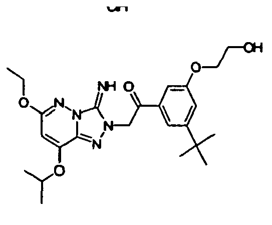 | 1-[3-tert-Butyl-5-(2-hydroxy-ethoxy)-phenyl]-2-(6-ethoxy-3-imino-8-isopropoxy-[1,2,4]triazolo[4,3-b]pyridazin-2-yl)-ethanon-Hydrochlorid | 0,86 | 472,2 (Met-b) |
| Bsp.: 242 | 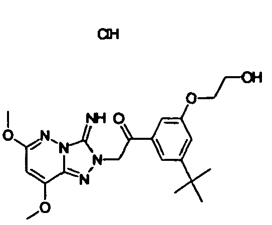 | 1-[3-tert-Butyl-5-(2-hydroxy-ethoxy)-phenyl]-2-(3-imino-6,8-dimethoxy-[1,2,4]triazolo[4,3-b]pyridazin-2-yl)-ethanon-Hydrochlorid | 0,76 | 430,2 (Met-b) |

### Pharmakologische Beispiele

### PAR 1 Bestimmungsmethode: Hemmung der PAR1 mediierten

### Thrombozytenaggregation

Die pharmakologische Testung der Substanzen erfolgte in der durch TRAP (Thrombinrezeptor aktivierendes Peptid) induzierten Thrombozytenaggregation im 96-well Format. Dazu wurde von gesunden freiwilligen Spendern Blut in 20 ml Spritzen abgenommen, in denen 2 ml 3,13 %-ige Natriumcitratlösung vorgelegt war. Nach einer 20-minütigen Zentrifugation bei 150 x g wurde das Plättchenreiche Plasma (PRP) abgetrennt und mit 1 µl PGE1-Lösung (500 µg/ml in Ethanol) / ml PRP versetzt. Nach 5 Minuten Inkubation bei RT wurde 15 Minuten bei 120 x g zentrifugiert um die Leukozyten zu entfernen. Das Leukozytenfreie PRP wurde in 5 ml Portionen in 15 ml PP Röhrchen überführt und 15 Minuten bei 360xg abzentrifugiert, um die Plättchen zu pelletieren. Anschließend wurde das Plasma dekantiert und das Plättchensediment aus 5 ml PRP in 1 ml Tyrode (120 mM NaCl, 2,6 mM KCl, 12 mM NaHCO₃, 0,39 mM NaH₂PO₄ x H₂O, 10 mM HEPES, 0,35% BSA, 5,5 mM Glukose, pH 7,4) resuspendiert und mit Tyrode auf eine Plättchenzahl von 3x10⁵ / Mikroliter (µl) eingestellt. 13 ml dieser Zellsuspension wurde dann mit 866 µL 10 mM CaCl₂-Lösung versetzt und 120 µL davon pro well einer 96-well-Platte pipettiert, in dem 15 µL der zu testenden Substanz vorgelegt waren. Nach 30 Minuten Inkubation bei Raumtemperatur im Dunklen wurden 15 µL einer TRAP-Lösung (70-100 µM) als Agonist zugegeben und in einem SpectraMax 340 bei 650 nm über 20 Minuten bei 37° C unter Schütteln eine Kinetik aufgezeichnet. Die Flächen unter den Kurven von Negativkontrolle (Tyrode/ DMSO) und Positivkontrolle (15 µl Agonist /DMSO) wurden berechnet und die Differenz als 100 % Wert festgelegt. Die zu testenden Substanzen wurden in Doppelbestimmung als Verdünnungsreihen pipettiert, ebenfalls die AUC jeder Substanzkonzentration bestimmt und die % Hemmung der AUC gegen die Kontrolle errechnet. Anhand der % Hemmung wurde mit Hilfe nichtlinearer Regressionsanalyse gemäß der 4 Parameter-Gleichung die IC₅₀ berechnet.

Tabelle 1 zeigt die Ergebnisse.

**Tabelle 1:**

| Verbindung aus Beispiel | Hemmung der Thrombozytenaggregation IC₅₀ [mikro M] | Verbindung aus Beispiel | Hemmung der Thrombozytenaggregation IC₅₀ [mikro M] |
|---|---|---|---|
| 1 | 0,65 | 2 | 1,6 |
| 5 | 8,5 | 7 | 0,158 |
| 16 | 0,127 | 66 | 0,025 |
| 77 | 0,009 | 85 | 0,059 |
| 90 | 0,012 | 98 | 0,334 |
| 108 | 0,016 | | |

### PAR1-Bindungstest

Die synthetisierten Substanzen wurden in einem PAR1-Bindungstest untersucht. Hierbei wurde geprüft, ob die Substanzen die Bindung eines radioaktiv markierten, literaturbekannten PAR1-Agonisten an den PAR1-Rezeptor inhibieren können (Ho-Sam Ahn, Mol Pharm, 51:350-356, 1997).

Der humane PAR1-Rezeptor wurde transient in High Five Insektzenzellen exprimiert. Aus diesen wurde gemäß Standardmethoden nach 48 Stunden eine Membranpräparation hergestellt, in 10 mM Tris-HCl; 0,3 mM EDTA; 1 mM EGTA; 250 mM Surose pH 7,5 aliquotiert und bei -80°C gelagert.

Die Substanzen wurden 15 Minuten bei Raumtemperatur mit der Membran vorinkubiert, dann erfolgte die Zugabe des Radioliganden (ALA-(para-F-Phe)-Arg-ChA-homoArg-(3,4-³H-Tyr)-NH₂; ca. 40 Ci/mMol). Die Endkonzentration des Radioliganden im Testpuffer (50 mM Tris-HCl; 10 mM MgCl₂; 1 mM EGTA; 0,1 % BSA; 2% DMSO) betrug 20 nM, die der Membran 1mg/ml. Nach einer Inkubationszeit von 60 Minuten wurden 25 µL des Ansatzes in eine 96-well MultiScreenHTS FB Mikrotiterfiltrationsplatte (Fa. Millipore) übertragen, die zuvor 5 Stunden bei Raumtemperatur mit einer 0,75%-igen wässrigen Polyethyleniminlsg. vorbehandelt worden war. Danach wurde unter Vakuumabsaugung jedes well viermal mit 300 µL Puffer (50 mM Tris-HCl; 10 mM MgCl₂; 1 mM EGTA) gewaschen. Die Platte wurde dann über Nacht getrocknet, 100 µl Szintillator pro well zugegeben und nach 6 Stunden in einem Wallac MicroBeta (Fa. PerkinElmer) Flüssigszintillationzähler vermessen. Die unspezifische Bindung wurde in Gegenwart von 100 µM SCH79797 (PAR-1 Antagonist; Fa. Tocris Cat. No 1592) bestimmt und von allen Messwerten subtratiert. Als 100% Wert diente eine Kontrolle ohne Inhibitor. Aus den % Hemmungswerten einer Substanzverdünnungsreihe wurde mit Hilfe nichtlinearer Regressionsanalyse gemäß der 4 Parameter-Gleichung die IC₅₀ berechnet. Tabelle 2 zeigt die Ergebnisse.

**Tabelle 2:**

| Verbindung aus Beispiel | Hemmung der Bindung IC₅₀ [mikro M] | Verbindung aus Beispiel | Hemmung der Bindung IC₅₀ [mikro M] |
|---|---|---|---|
| 14 | 0,279 | 178 | 0,126 |
| 26 | 1,1 | 186 | 0,119 |
| 50 | 0,631 | 197 | 0,149 |
| 56 | 24 | 199 | 0,206 |
| 76 | 0,951 | 207 | 0,174 |
| 84 | 9,8 | 215 | 0,219 |
| 89 | 0,107 | 221 | 0,155 |
| 101 | 1,4 | 222 | 0,416 |
| 117 | 0,098 | 230 | 0,234 |
| 127 | 2,5 | 236 | 1,4 |
| 144 | 0,827 | 239 | 0,113 |
| 161 | 0,365 | 240 | 0,484 |
| 163 | 0,364 | | |
| 168 | 0,257 | | |
| 174 | 0,183 | | |

## Patentansprüche

1. Verbindung der Formel I, 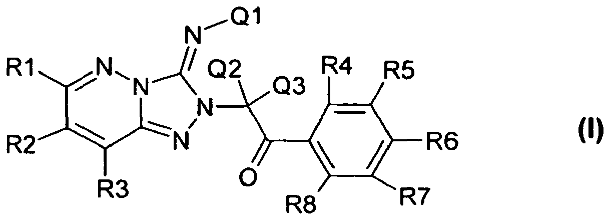 und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei Q1 für Wasserstoffatom, -(C₁-C₆)-Alkyl, -(C₃-C₆)-Cycloalkyl, -OH, -O-(C₁-C₆)-Alkyl oder -O-(C₃-C₆)-Cycloalkyl steht, wobei Alkyl und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können,
Q2 und Q3 gleich oder verschieden sind und unabhängig voneinander für Wasserstoffatom, -(C₁-C₆)-Alkyl oder -(C₃-C₆)-Cycloalkyl stehen, wobei in Alkyl oder Cycloalkyl die Wasserstoffatome ganz oder teilweise durch Fluor ersetzt sein können,
R1, R2 und R3 gleich oder verschieden sind und unabhängig voneinander für Wasserstoffatom, -(C₁-C₆)-Alkyl, -(C₃-C₆)-Cycloalkyl, -O-(C₁-C₈)-Alkyl, -O-(C₃-C₆)-Cycloalkyl, -(C₀-C₄)-Alkylen-C(O)-N(R11)-R12, -(C₀-C₄)-A)kylen-C(O)-O-R11, -(C₀-C₄)-Alkylen-C(O)-R11, -(C₀-C₄)-Alkylen-N(R11)-R12, -(C₀-C₄)-Alkylen-N(R11)-C(O)-R12, Halogen, OH, -CN, -NO₂, -SO₂CH₃, -SO₂CF₃, -SF₅, -Si[-(C₁-C₄)-Alkyl]₃, -(C₁-C₆)-Alkylen-O-(C₁-C₆)-Alkyl, -O-(C₁-C₆)-Alkylen-O-(C₁-C₆)-Alkyl, -O-(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -O-(C₁-C₄)-Alkylen-(C₃-C₆)-Cycloalkyl, -(C₄-C₁₅)-Het,
wobei Het unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₁-C₆)-Alkylen-O-(C₁-C₆)-Alkylen-O-(C₁-C₆)-Alkyl, stehen,
wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₃-C₆)-Cycloalkyl, substituiert sind, oder
wobei in Alkyl, Alkylen oder Cycloalkyl die Wasserstoffatome ganz oder teilweise durch Fluor ersetzt sein können, oder
R1 und R2 oder R2 und R3 bilden zusammen mit den Ringatomen, an die sie jeweils gebunden sind, 5- bis 8-gliedrigen Ring, wobei der Ring nur aus Kohlenstoffatomen besteht oder 1, 2 oder 3 dieser Atome durch N-, O- oder S-Atome ersetzt sind, wobei der Ring unsubstituiert oder ein- oder zweifach unabhängig voneinander durch -(C₁-C₄)-Alkyl, -(C₃-C₆)-Cycloalkyl, OH, -O-(C₁-Cg)-Alkyl oder -O-(C₃-C₆)-Cycloalkyl substituiert ist, wobei im 5- bis 8-gliedrigen gebildeten Ring, in Alkyl oder Cycloalkyl die Wasserstoffatome ganz oder teilweise durch Fluor ersetzt sein können,
R11 und R12 unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -(C₀-C₄)-Alkylen-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, -SO₂CH₃ oder -SO₂CF₃ stehen,
wobei in Alkyl, Alkylen oder Cycloalkyl die Wasserstoffatome ganz oder teilweise durch Fluor ersetzt sein können, oder
R11 und R12 in den Fragmenten "N(R11)-R12" und "N(R11)-C(O)-R12" für einen 5- bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei im 5- bis 8-gliedrigen gebildeten Ring, in Alkyl oder Cycloalkyl die Wasserstoffatome ganz oder teilweise durch Fluor ersetzt sein können,
R4, R5, R6, R7 und R8 gleich oder verschieden sind und unabhängig voneinander für Wasserstoffatom, -(C₁-C₆)-Alkyl, -(C₃-C₆)-Cycloalkyl, OH, -CN, -NO₂, -O-(C₁-C₈)-Alkyl, -O-(C₃-C₆)-Cycloalkyl, -(C₀-C₄)-Alkylen-(CO)-N(R21)-R22, -SO₂CH₃, -SO₂CF₃, -(C₀-C₄)-Alkylen-C(O)-O-R21, Halogen, -SF₅, -(C₀-C₄)-Alkylen-C(O)-R21, -(C₀-C₄)-Alkylen-N(R21)-R22, -(C₀-C₄)-Alkylen-N(R21)-C(O)-R22, -(C₁-C₆)-Alkylen-O-(C₁-C₆)-Alkyl, -(C₀-C₆)-Alkylen-O-(C₁-C₆)-Alkylen-O-(C₁-C₆)-Alkyl, -Si[-(C₁-C₄)-Alkyl]₃, -(C₀-C₆)-Alkylen-O-(C₁-C₄)-Alkylen-(C₃-C₆)-Cycloalkyl, -(C₀-C₆)-Alkylen-O-(C₀-C₆)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, oder -(C₄-C₁₅)-Het stehen, wobei Het unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist,
wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₃-C₆)-Cycloalkyl, substituiert sind,
wobei in Alkyl, Alkylen oder Cycloalkyl die Wasserstoffatome ganz oder teilweise durch Fluor ersetzt sein können, oder
R4 und R5, R5 und R6, R6 und R7 oder R7 und R8 bilden zusammen mit den Ringatomen, an die sie jeweils gebunden sind, einen 5- bis 8-gliedrigen Ring, wobei der Ring nur aus Kohlenstoffatomen besteht oder 1,2 oder 3 dieser Atome durch N-, O- oder S-Atome ersetzt sind, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei im 5- bis 8-gliedrigen gebildeten Ring, in Alkyl oder Cycloalkyl die Wasserstoffatome ganz oder teilweise durch Fluor ersetzt sein können,
R21 und R22 unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -(C₀-C₄)-Alkylen-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, -SO₂CH₃ oder -SO₂CF₃ stehen,
wobei in Alkyl, Alkylen oder Cycloalkyl die Wasserstoffatome ganz oder teilweise durch Fluor ersetzt sein können, oder
R21 und R22 in den Fragmenten "N(R21)-R22" und "N(R21)-C(O)-R22" für einen 5- bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder-O-(C₃-C₆)-Cycloalkyl, substituiert ist, wobei im 5- bis 8-gliedrigen gebildeten Ring, in Alkyl oder Cycloalkyl die Wasserstoffatome ganz oder teilweise durch Fluor ersetzt sein können.

2. Verbindung der Formel 1 gemäß Anspruch 1, wobei Q1, Q2 und Q3 gleich oder verschieden sind und unabhängig voneinander für Wasserstoffatom, -(C₁-C₆)-Alkyl oder -(C₃-C₆)-Cycloalkyl stehen, wobei in Alkyl oder Cycloalkyl die Wasserstoffatome ganz oder teilweise durch Fluor ersetzt sein können,
R1, R2 und R3 gleich oder verschieden sind und unabhängig voneinander für Wasserstoffatom, -(C₁-C₆)-Alkyl, -(C₃-C₆)-Cycloalkyl, -O-(C₁-C₈)-Alkyl, -O-(C₃-C₆)-Cycloalkyl, -(C₀-C₄)-Alkylen-C(O)-N(R11)-R12, -(C₀-C₄)-Alkylen-C(O)-O-R11, -(C₀-C₄)-Alkylen-C(O)-R11, -(C₀-C₄)-Alkylen-N(R11)-R12, -(C₀-C₄)-Alkylen-N(R11)-C(O)-R12, Halogen, OH, -CN, -NO₂, -SO₂CH₃, -SO₂CF₃, -SF₅, -Si[-(C₁-C₄)-Alkyl]₃, -(C₁-C₆)-Alkylen-O-(C₁-C₆)-Alkyl, -O-(C₁-C₆)-Alkylen-O-(C₁-C₆)-Alkyl, -O-(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl substituiert ist, -O-(C₁-C₄)-Alkylen-(C₃-C₆)-Cycloalkyl, -(C₄-C₁₅)-Het,
wobei Het unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₁-C₆)-Alkylen-O-(C₁-C₆)-Alkylen-O-(C₁-C₆)-Alkyl, stehen, wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₃-C₆)-Cycloalkyl, substituiert sind, oder
wobei in Alkyl, Alkylen oder Cycloalkyl die Wasserstoffatome ganz oder teilweise durch Fluor ersetzt sein können,
mit der Maßgabe, das mindestens ein R1, R2 oder R3 nicht Wasserstoffatom ist oder
R1 und R2 oder R2 und R3 bilden zusammen mit den Ringatomen, an die sie jeweils gebunden sind, einen 5- bis 8-gliedrigen Ring, wobei der Ring nur aus Kohlenstoffatomen besteht oder 1, 2 oder 3 dieser Atome durch N-, O- oder S-Atome ersetzt sind, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, wobei im 5- bis 8-gliedrigen Ring, in Alkyl oder Cycloalkyl die Wasserstoffatome ganz oder teilweise durch Fluor ersetzt sein können,
R11 und R12 unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -(C₀-C₄)-Alkylen-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalky substituiert ist, -SO₂CH₃ oder -SO₂CF₃ stehen,
wobei in Alkyl, Alkylen oder Cycloalkyl die Wasserstoffatome ganz oder teilweise durch Fluor ersetzt sein können, oder
R11 und R12 in den Fragmenten "N(R11)-R12" und "N(R11)-C(O)-R12" für einen 5- bis 8-gliedrigen Ring stehen, der gemeinsam mit dem Stickstoffatom "N" oder der Gruppe "N-(CO)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, wobei in Alkyl oder Cycloalkyl die Wasserstoffatome ganz oder teilweise durch Fluor ersetzt sein können,
R4, R5, R6, R7 und R8 gleich oder verschieden sind und unabhängig voneinander für Wasserstoffatom, -(C₁-C₆)-Alkyl, -(C₃-C₆)-Cycloalkyl, OH, -CN, -NO₂, -O-(C₁-C₈)-Alkyl, -O-(C₃-C₆)-Cycloalkyl, -(C₀-C₄)-Alylen-(CO)-N(R21)-R22, -SO₂CH₃, -SO₂CF₃, -(C₀-C₄)-Alkylen-C(O)-O-R21, Halogen, -SF₅, -(C₀-C₄)-Alkylen-C(O)-R21, -(C₀-C₄)-Alkylen-N(R21)-R22, -(C₀-C₄)-Alkylen-N(R21)-C(O)-R22, -(C₁-C₆)-Alkylen-O-(C₁-C₆)-Alkyl, -(C₀-C₆)-Alkylen-O-(C₁-C₆)-Alkylen-O-(C₁-C₆)-Alkyl, -Si[-(C₁-C₄)-Alkyl]₃, -(C₀)-C₆)-Alkylen-O-(C₁-C₄)-Alkylen-(C₃-C₆)-Cycloalkyl, -(C₀-C₆)-Alkylen-O-(C₀-C₆)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, oder -(C₄-C₁₅)-Het stehen, wobei Het unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist,
wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₃-C₆)-Cycloalkyl, substituiert sindt, oder
wobei in Alkyl, Alkylen oder Cycloalkyl die Wasserstoffatome ganz oder teilweise durch Fluor ersetzt sein können,
mit der Maßgabe, das mindestens ein R4, R5, R6, R7 oder R8 nicht Wasserstoffatom ist, oder
R4 und R5, R5 und R6, R6 und R7 oder R7 und R8 bilden zusammen mit den Ringatomen, an die sie jeweils gebunden sind, einen 5- bis 8-gliedrigen Ring, wobei der Ring nur aus Kohlenstoffatomen besteht oder 1, 2 oder 3 dieser Atome durch N-, O- oder S-Atome ersetzt sind, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei im 5- bis 8-gliedrigen gebildeten Ring, in Alkyl oder Cycloalkyl die Wasserstoffatome ganz oder teilweise durch Fluor ersetzt sein können,
R21 und R22 unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -(C₀-C₄)-Alkylen-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, -SO₂CH₃ oder -SO₂CF₃ stehen,
wobei in Alkyl, Alkylen oder Cycloalkyl die Wasserstoffatome ganz oder teilweise durch Fluor ersetzt sein können, oder
R21 und R22 in den Fragmenten "N(R21)-R22" und "N(R21)-C(O)-R22" für einen 5- bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei im 5- bis 8-gliedrigen gebildeten Ring, in Alkyl oder Cycloalkyl die Wasserstoffatome ganz oder teilweise durch Fluor ersetzt sein können.

3. Verbindung der Formel 1 gemäß den Ansprüchen 1 oder 2, wobei
Q1, Q2 und Q3 gleich oder verschieden sind und unabhängig voneinander für Wasserstoffatom, -(C₁-C₆)-Alkyl oder -(C₃-C₆)-Cycloalkyl stehen, wobei in Alkyl oder Cycloalkyl die Wasserstoffatome ganz oder teilweise durch Fluor ersetzt sein können,
R1, R2 und R3 gleich oder verschieden sind und unabhängig voneinander für Wasserstoffatom, -(C₁-C₆)-Alkyl, -(C₃-C₆)-Cycloalkyl, -O-(C₁-C₈)-Alkyl -O-(C₃-C₆)-Cycloalkyl, -(C₀-C₄)-Alkylen-C(O)-N(R11)-R12, -(C₀-C₄)-Alkylen-C(O)-O-R11, -(C₀-C₄)-Alkylen-C(O)-R11, -(C₀-C₄)-Alkylen-N(R11)-R12, -(C₀-C₄)-Alkylen-N(R11)-C(O)-R12,
Halogen, OH, -CN, -NO₂, -SO₂CH₃, -Si[-(C₁-C₄)-Alkyl]₃, -(C₁-C₆)-Alkylen-O-(C₁-C₆)-Alkyl, -O-(C₁-C₆)-Alkylen-O-(C₁-C₆)-Alkyl, -O-(C₀-C₄)-Alkylen-(C₆-C₁₄)-Aryl, -O-(C₁-C₄)-Alkylen-(C₃-C₆)-Cycloalkyl, -(C₄-C₁₅)-Het oder -O-(C₁-C₆)-Alkylen-O-(C₁-C₆)-Alkylen-O-(C₁-C₆)-Alkyl, stehen,
wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert sind, oder
wobei in Alkyl, Alkylen oder Cycloalkyl die Wasserstoffatome ganz oder teilweise durch Fluor ersetzt sein können,
mit der Maßgabe, das mindestens ein R1, R2 oder R3 nicht Wasserstoffatom ist oder
R1 und R2 oder R2 und R3 bilden zusammen mit den Ringatomen, an die sie jeweils gebunden sind, einen Ring ausgewählt aus der Gruppe 2,3,5,6,7,8-hexa-hydro-1,2,3a,4,5,8-hexaaza-cyclopenta[b]naphthalin; 2,6,7,8-tetrahydro-3H-5-oxa-1,2,3a,4,8-pentaaza-cyclopenta[b]naphthalin; 2,3,6,7-tetrahydro-5,8-dioxa-1,2,3a,4-tetraaza-cyclopenta[b]naphthalin; 2,3,6,7-tetrahydro-5H-8-oxa-1,2,3a,4,5-pentaaza-cyclopenta[b]naphthalin; 2,6,7,8-tetrahydro-3H-5-thia-1,2,3a,4,8-pentaaza-cyclopenta[b]naphthalin; 2,3,6,7,8,9-hexahydro-1,2,3a,4,6,9-hexaaza-cyclopenta[a]naphthalin; 2,3-dihydro-5,7-dioxa-1,2,3a,4-tetraaza-s-indacin; 2,6,7,8-tetrahydro-3H-cyclopenta[e][1,2,4]triazolo[4,3-b]pyridazin; 2,7,8,9-tetrahydro-3H-cyclopenta[d][1,2,4]triazolo[4,3-b]pyridazin und 2,3,6a,9a-tetrahydro-[1,3]dioxolo[4,5-d][1,2,4]triazolo[4,3-b]pyridazin, wobei der Ring unsubstituiert oder ein- oder zweifach unabhängig voneinander durch - (C₁-C₄)-Alkyl, -(C₃-C₆)-Cycloalkyl, OH, -O-(C₁-C₆)-Alkyl oder -O-(C₃-C₆)-Cycloalkyl substituiert ist, wobei in Alkyl oder Cycloalkyl die Wasserstoffatome ganz oder teilweise durch Fluor ersetzt sein können,
R11 und R12 unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, -(C₀-C₄)-Alkylen-(C₄-C₁₅)-Het, -SO₂CH₃ oder -SO₂CF₃ stehen,
wobei in Alkyl, Alkylen oder Cycloalkyl die Wasserstoffatome ganz oder teilweise durch Fluor ersetzt sein können, oder
R11 und R12 in den Fragmenten "N(R11)-R12" und "N(R11)-C(O)-R12" für einen 5- bis 8-gliedrigen Ring stehen, ausgewählt aus der Gruppe Azetidinyl, Pyrrolidinyl, Piperidinyl, Piperazinyl, Azepinyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, wobei in Alkyl oder Cycloalkyl die Wasserstoffatome ganz oder teilweise durch Fluor ersetzt sein können,
R4, R5, R6, R7 und R8 gleich oder verschieden sind und unabhängig voneinander für Wasserstoffatom, -(C₁-C₆)-Alkyl, -(C₃-C₆)-Cycloalkyl, OH, -CN, -NO₂, -O-(C₁-C₈)-Alkyl, -O-(C₃-C₆)-Cycloalkyl, -(C₀-C₄)-Alkylen-(CO)-N(R21)-R22, -SO₂CH₃, -SO₂CF₃, -(C₀-C₄)-Alkylen-C(O)-O-R21, Halogen, -SF₅, -(C₀-C₄)-Alkylen-C(O)-R21, -(C₀-C₄)-Alkylen-N(R21)-R22, -(C₀-C₄)-Alkylen-N(R21)-C(O)-R22, -(C₁-C₆)-Alkylen-O-(C₁-C₆)-Alkyl, -(C₀-C₆)-Alkylen-O-(C₁-C₆)-Alkylen-O-(C₁-C₆)-Alkyl, -Si[-(C₁-C₄)-Alkyl]₃, -(C₀-C₆)-Alkylen-O-(C₁-C₄)-Alkylen-(C₃-C₆)-Cycloalkyl, -(C₀-C₆)-Alkylen-O-(C₀-C₆)-Alkylen-(C₆-C₁₄)-Aryl oder -(C₄-C₁₅)-Het stehen, wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₃-C₆)-Cycloalkyl, substituiert ist, oder
wobei in Alkyl, Alkylen oder Cycloalkyl die Wasserstoffatome ganz oder teilweise durch Fluor ersetzt sein können,
mit der Maßgabe, das mindestens ein R4, R5, R6, R7 oder R8 nicht Wasserstoffatom ist, oder
R4 und R5, R5 und R6, R6 und R7 oder R7 und R8 bilden zusammen mit den Ringatomen, an die sie jeweils gebunden sind, einen 5- bis 8-gliedrigen Ring ausgewählt aus der Gruppe 2,3-Dihydro-benzo[1,4]dioxin; 3,4-Dihydro-2H-benzo[1,4]oxazin; 1,2,3,4-Tetrahydro-quinoxalin; Benzo[1,3]dioxol; 3,4-Dihydro-2H-benzo[1,4]thiazin und 2,3,4,5-Tetrahydro-1H-benzo[b][1,4]diazepin,
wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können,
R21 und R22 unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, -(C₀-C₄)-Alkylen-(C₄-C₁₅)-Het, -SO₂CH₃ oder -SO₂CF₃ stehen,
wobei in Alkyl, Alkylen oder Cycloalkyl die Wasserstoffatome ganz oder teilweise durch Fluor ersetzt sein können, oder
R21 und R22 in den Fragmenten "N(R21)-R22" und "N(R21)-C(O)-R22" für einen 5- bis 8-gliedrigen Ring stehen, ausgewählt aus der Gruppe Azetidinyl, Pyrrolidinyl, Piperidinyl, Piperazinyl, Azepinyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können.

4. Verbindung der Formel I gemäß den Ansprüchen 1 bis 3, wobei
Q1, Q2 und Q3 gleich sind und für jeweils ein Wasserstoffatom stehen,
R1, R2 und R3 gleich oder verschieden sind und unabhängig voneinander für Wasserstoffatom, -(C₁-C₆)-Alkyl, -O-(C₁-C₈)-Alkyl, -O-(C₃-C₆)-Cycloalkyl, -(C₀-C₄)-Alkylen-C(O)-N(R11)-R12, -(C₀-C₄)Alkylen-N(R11)-R12, Halogen, -O-(C₁-C₆)-Alkylen-O-(C₁-C₆)-Alkyl, -O-(C₀-C₄)-Alkylen-Phenyl oder -O-(C₁-C₆)-Alkylen-O-(C₁-C₆)-Alkylen-O-(C₁-C₆)-Alkyl, stehen,
wobei Alkyl oder Alkylen jeweils unsubstituiert oder ein- oder zweifach durch, - O-(C₁-C₆)-Alkyl substituiert ist,
wobei in Alkyl oder Alkylen die Wasserstoffatome ganz oder teilweise durch Fluor ersetzt sein können,
mit der Maßgabe, das mindestens ein R1, R2 oder R3 nicht Wasserstoffatom ist,
R11 und R12 unabhängig voneinander für Wasserstoffatom oder -(C₁-C₆)-Alkyl stehen, oder
R11 und R12 in dem Fragment "N(R11)-R12" für einen 5- bis 8-gliedrigen Ring stehen, ausgewählt aus der Gruppe Azetidinyl, Pyrrolidinyl, Piperidinyl, Piperazinyl, Azepinyl, Imidazolyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl, R4, R5, R6, R7 und R8 gleich oder verschieden sind und unabhängig voneinander für
Wasserstoffatom, -(C₁-C₆)-Alkyl, OH, -O-(C₁-C₈)-Alkyl, Halogen, -SF₅, -(C₀-C₄)-Alkylen-N(R21)-R22, -(C₀-C₄)-Alkylen-N(R21)-C(O)-R22, -CF₃, -(C₀-C₆)-Alkylen-O-(C₁-C₆)-Alkylen-O-(C₁-C₆)-Alkyl, -(C₀-C₆)-Alkylen-O-(C₁-C₄)-Alkylen-(C₃-C₆)-Cycloalkyl, oder -(C₀-C₆)-Alkylen-O-(C₀-C₆)-Alkylen-Phenyl stehen,
wobei Alkyl oder Alkylen jeweils unsubstituiert oder ein- oder zweifach durch -O-(C₁-C₆)-Alkyl substituiert ist,
mit der Maßgabe, das mindestens ein R4, R5, R6, R7 oder R8 nicht Wasserstoffatom ist, oder
R4 und R5, R5 und R6, R6 oder R7 oder R7 und R8 bilden zusammen mit den Ringatomen, an die sie jeweils gebunden sind, einen fünf- bis achtgliedrigen Ring ausgewählt aus der Gruppe 2,3-Dihydro-benzo[1,4]dioxin; 3,4-Dihydro-2H-benzo[1,4]oxazin; 1,2,3,4-Tetrahydro-quinoxalin; Benzo[1,3]dioxol; 3,4-Dihydro-2H-benzo[1,4]thiazin und 2,3,4,5-Tetrahydro-1H-benzo[b][1,4]diazepin,
wobei der Ring unsubstituiert oder ein- oder zweifach durch -(C₁-C₄)-Alkyl substituiert ist,
R21 und R22 unabhängig voneinander für Wasserstoffatom oder -(C₁-C₆)-Alkyl stehen, oder
R21 und R22 in dem Fragment "N(R21)-R22" für einen 5- bis 8-gliedrigen Ring stehen, ausgewählt aus der Gruppe Azetidinyl, Pyrrolidinyl, Piperidinyl, Piperazinyl, Azepinyl, Imidazolyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl.

5. Verbindung der Formel I gemäß den Ansprüchen 1 bis 4, wobei die Verbindung der Formel I ausgewählt is aus der Gruppe 2-(6-Chlor-3-imino-[1,2,4]triazolo[4,3-b]pyridazin-2-yl)-1-(3,5-di-tert-butyl-4-hydroxy-phenyl)-ethanon, 1-(3-tert-Butyl-4-methoxy-5-morpholin-4-yl-phenyl)-2-(3-imino-6-isopropoxy-[1,2,4]triazolo[4,3-b]pyridazin-2-yl)-ethanon, 1-(3-tert-Butyl-4-methoxy-5-morpholin-4-yl-phenyl)-2-(3-imino-6-methoxy-[1,2,4]triazolo[4,3-b]pyridazin-2-yl)-ethanon, 2-(6-Ethoxy-3-imino-[1,2,4]triazolo[4,3-b]pyridazin-2-yl)-1-(4-methoxy-3-morpholin-4-yl-5-trifluoromethyl-phenyl)-ethanon, N-[3-[2-(6-Ethoxy-3-imino-[1,2,4]triazolo[4,3-b]pyridazin-2-yl)-acetyl]-5-(pentafluorsulfanyl)-phenyl]-N-methyl-acetamid, N-[3-[2-(6-Ethoxy-3-imino-[1,2,4]triazolo[4,3-b]pyridazin-2-yl)-acetyl]-5-(pentafluor-sulfanyl)-phenyl]-acetamid, 1-(3-tert-Butyl-4-methoxy-5-morpholin-4-yl-phenyl)-2-(6-ethoxy-3-imino-[1,2,4]triazolo[4,3-b]pyridazin-2-yl)-ethanon, 1-(3-tert-Butyl-4-methoxy-5-morpholin-4-yl-phenyl)-2-(6-cyclopentyloxy-3-imino-[1,2,4]triazolo[4,3-b]pyridazin-2-yl)-ethanon, 1-(3-tert-Butyl-4-methoxy-5-morpholin-4-yl-phenyl)-2-(6-cyclobutoxy-3-imino-[1,2,4]triazolo[4,3-b]pyridazin-2-yl)-ethanon, 1-(3-tert-Butyl-4-methoxy-5-morpholin-4-yl-phenyl)-2-(3-imino-6-phenoxy-[1,2,4]triazolo[4,3-b]pyridazin-2-yl)-ethanon, 2-(6-Benzyloxy-3-imino-[1,2,4]triazolo[4,3-b]pyridazin-2-yl)-1-(3-tert-butyl-4-methoxy-5-morpholin-4-yl-phenyl)-ethanon, 1-(3-tert-Butyl-4-methoxy-5-morpholin-4-yl-phenyl)-2-(6-cyclohexyloxy-3-imino-[1,2,4]triazolo[4,3-b]pyridazin-2-yl)-ethanon, 1-(3-tert-Butyl-4-methoxy-5-morpholin-4-yl-phenyl)-2-[3-imino-6-(2,2,2-trifluoro-ethoxy)-[1.2,4]triazolo[4,3-b]pyridazin-2-yl]-ethanon, 1-(3-tert-Butyl-4-methoxy-5-morpholin-4-yl-phenyl)-2-[6-(1-ethyl-propoxy)-3-imino-[1,2,4]triazolo[4,3-b]pyridazin-2-yl]-ethanon, 1-(3-tert-Buty)-4-methoxy-5-morpholin-4-yl-phenyl)-2-(6-cyclopropylmethoxy-3-imino-[1,2,4]triazolo[4,3-b]pyridazin-2-yl)-ethanon, 2-[6-(1-Ethyl-propoxy)-3-imino-[1,2,4]triazolo[4,3-b]pyridazin-2-yl]-1-[3-methylamino-5-(pentafluor-sulfanyl)-phenyl]-ethanon, 2-[6-(1-Ethyl-propoxy)-3-imino-[1,2,4]triazolo[4,3-b]pyridazin-2-yl]-1-[3-methoxy-5-(pentafluor-sulfanyl)-phenyl]-ethanon, 1-(3-tert-Butyl-5-ethoxymethyl-phenyl)-2-[6-(1-ethyl-propoxy)-3-imino-[1,2,4]triazolo[4,3-b]pyridazin-2-yl]-ethanon, 1-(3-tert-Butyl-5-cyclopropylmethoxymethyl-phenyl)-2-[6-(1-ethyl-propoxy)-3-imino-[1,2,4]triazolo[4,3-b]pyridazin-2-yl]-ethanon, 1-(3-tert-Butyl-4,5-diethoxy-phenyl)-2-[6-(1-ethyl-propoxy)-3-imino-[1,2,4]triazolo[4,3-b]pyridazin-2-yl]-ethanon, 1-(3-tert-Butyl-5-ethoxy-phenyl)-2-[6-(1-ethyl-propoxy)-3-imino-[1,2,4]triazolo[4,3-b]pyridazin-2-yl]-ethanon, 1-(3-tert-Butyl-5-propoxymethyl-phenyl)-2-[6-(1-ethyl-propoxy)-3-imino-[1,2,4]triazolo[4,3-b]pyridazin-2-yl]-ethanon, 1-(3-tert-Butyl-4,5-bis-cyclopropylmethoxy-phenyl)-2-[6-(1-ethyl-propoxy)-3-imino-[1,2,4]triazolo[4,3-b]pyridazin-2-yl]-ethanon, 2-[6-(1-Ethyl-propoxy)-3-imino-[1,2,4]triazolo[4,3-b]pyridazin-2-yl]-1-(3-methoxy-5-trifluoromethyl-phenyl)-ethanon, 1-(3-tert-Butyl-5-methoxy-phenyl)-2-[6-(1-ethyl-propoxy)-3-imino-[1,2,4]triazolo[4,3-b]pyridazin-2-yl]-ethanon, 1-(3-tert-Butyl-5-cyclopropylmethoxy-phenyl)-2-[6-(1-ethyl-propoxy)-3-imino-[1,2,4]triazolo[4,3-b]pyridazin-2-yl]-ethanon, 1-(3-tert-Butyl-5-cyclobutyl-methoxy-phenyl)-2-[6-(1-ethyl-propoxy)-3-imino-[1,2,4]triazolo[4,3-b]pyridazin-2-yl]-ethanon, 1-(3-Benzyloxymethyl-5-tert-butyl-phenyl)-2-[6-(1-ethyl-propoxy)-3-imino-[1,2,4]triazolo[4,3-b]pyridazin-2-yl]-ethanon, 1-(3-Cyclo-hexylmethoxy-4,5-dimethoxy-phenyl)-2-[6-(1-ethyl-propoxy)-3-imino-[1,2,4]triazolo[4,3-b]pyridazin-2-yl]-ethanon, 2-(6-Butoxy-3-imino-[1,2,4]triazolo[4,3-b]pyridazin-2-yl)-1-(3-tert-butyl-5-methoxymethyl-phenyl)-ethanon, 1-(3-Chlor-5-methoxy-phenyl)-2-[6-(1-ethyl-propoxy)-3-imino-[1,2,4]triazolo[4,3-b]pyridazin-2-yl]-ethanon, 1-(8-tert-Butyl-4-methyl-3,4-dihydro-2H-benzo[1,4]oxazin-6-yl)-2-[6-(1-ethyl-propoxy)-3-imino-[1,2,4]triazolo[4,3-b]pyridazin-2-yl]-ethanon, 1-(3-tert-Butyl-4-methoxy-5-morpholin-4-yl-phenyl)-2-(3-imino-6-piperidin-1-yl-[1,2,4]triazolo[4,3-b]pyridazin-2-yl)-ethanon, 1-(3-tert-Butyl-4-methoxy-5-morpholin-4-yl-phenyl)-2-(6-diethylamino-3-imino-[1,2,4]triazolo[4,3-b]pyridazin-2-yl)-ethanon, 2-[6-(1-Ethyl-propoxy)-3-imino-[1,2,4]triazolo[4,3-b]pyridazin-2-yl]-1-(3-isopropyl-5-methoxyphenyl)-ethanon, 1-(3-Cyclohexylmethoxy-5-ethoxy-phenyl)-2-[6-(1-ethyl-propoxy)-3-imino-[1,2,4]triazolo[4,3-b]pyridazin-2-yl]-ethanon, 1-(3-Brom-5-methoxy-phenyl)-2-[6-(1-ethyl-propoxy)-3-imino-[1,2,4]triazolo[4,3-b]pyridazin-2-yl]-ethanon, 1-[3-(3,3-Dimethyl-butoxy)-5-methoxy-phenyl]-2-[6-(1-ethyl-propoxy)-3-imino-[1,2,4]triazolo[4,3-b]pyridazin-2-yl]-ethanon, 1-[3-(3,3-Dimethyl-butoxy)-5-ethoxy-phenyl]-2-[6-(1-ethyl-propoxy)-3-imino-[1,2,4]triazolo[4,3-b]pyridazin-2-yl]-ethanon, 1-(3-Cyclohexylmethoxy-5-methoxy-phenyl)-2-[6-(1-ethyl-propoxy)-3-imino-[1,2,4]triazolo[4,3-b]pyridazin-2-yl]-ethanon, 1-(3-Brom-4,5-dimethoxy-phenyl)-2-[6-(1-ethyl-propoxy)-3-imino-[1,2,4]triazolo[4,3-b]pyridazin-2-yl]-ethanon, 2-(6-Diethylamino-3-imino-[1,2,4]triazolo[4,3-b]pyridazin-2-yl)-1-[3-methoxy-5-(pentafluor-sulfanyl)-phenyl]-ethanon, 1-(3-tert-Butyl-4-methoxy-5-morpholin-4-yl-phenyl)-2-(3-imino-6-morpholin-4-yl-[1,2,4]triazolo[4,3-b]pyridazin-2-yl)-ethanon, 1-(3-tert-Butyl-4-methoxy-5-morpholin-4-yl-phenyl)-2-(6-chloro-3-imino-8-methyl-[1,2,4]triazolo[4,3-b]pyridazin-2-yl)-ethanon, 1-(3-tert-Butyl-4-methoxy-5-morpholin-4-yl-phenyl)-2-(6-imidazol-1-yl-3-imino-[1,2,4]triazolo[4,3-b]pyridazin-2-yl)-ethanon, 1-(5-Brom-2,3-dimethoxy-phenyl)-2-[6-(1-ethyl-propoxy)-3-imino-[1,2,4]triazolo[4,3-b]pyridazin-2-yl]-ethanon, 1-(3-tert-Butyl-5-methoxy-phenyl)-2-{3-imino-6-[2-(2-methoxy-ethoxy)-ethoxy]-[1,2,4]tri-azolo[4,3-b]pyridazin-2-yl}-ethanon, 1-(3-Chlor-4,5-dimethoxy-phenyl)-2-[6-(1-ethyl-propoxy)-3-imino-[1,2,4]triazolo[4,3-b]pyridazin-2-yl]-ethanon, 1-(3-tert-Butyl-4-methoxy-5-morpholin-4-yl-phenyl)-2-(6-diethylamino-3-imino-8-methyl-[1,2,4]triazolo[4,3-b]pyridazin-2-yl)-ethanon, 1-[3-tert-Butyl-5-(2-methoxy-ethoxy)-phenyl]-2-[6-(1-ethyl-propoxy)-3-imino-[1,2,4]triazolo[4,3-b]pyridazin-2-yl]-ethanon, 1-(3-tert-Butyl-5-methoxy-phenyl)-2-[3-imino-6-(2-methoxy-ethoxy)-[1,2,4]triazolo[4,3-b]pyridazin-2-yl]-ethanon, 1-(3-tert-Butyl-5-methoxymethyl-phenyl)-2-[3-imino-6-(2-methoxy-ethoxy)-[1,2,4]triazolo[4,3-b]pyridazin-2-yl]-ethanon, 2-[6-(1-Ethyl-propoxy)-3-imino-[1,2,4]triazolo[4,3-b]pyridazin-2-yl]-1-[3-morpholin-4-yl-5-(pentafluor-sulfanyl)-phenyl]-ethanon, 1-(3-tert-Butyl-4-methoxy-5-morpholin-4-yl-phenyl)-2-(6-ethoxy-3-imino-8-methyl-[1,2,4]triazolo[4,3-b]pyridazin-2-yl)-ethanon, 2-[6-(1-Ethyl-propoxy)-3-imino-8-methyl-[1,2,4]triazolo[4,3-b]pyridazin-2-yl]-1-(5-methoxy-3-pentafluor-sulfanyl-phenyl)-ethanon, 1-(3-tert-Butyl-4-methoxy-5-morpholin-4-yl-phenyl)-2-(6-chlor-3-imino-[1,2,4]triazolo[4,3-b]pyridazin-2-yl)-ethanon, 1-[3-tert-Butyl-5-(2-methoxy-ethoxymethyl)-phenyl]-2-[6-(1-ethyl-propoxy)-3-imino-[1,2,4]triazolo[4,3-b]pyridazin-2-yl]-ethanon, 2-[6-(1-Ethyl-propoxy)-3-imino-[1,2,4]triazolo[4, 3-b]pyridazin-2-yl]-1-[3-(2-methoxy-ethoxy)-5-(pentafluoro-sulfanyl)-phenyl]-ethanon, 2-(6-Ethoxy-3-imino-8-methyl-[1,2,4]triazolo[4,3-b]pyridazin-2-yl)-1-[3-methoxy-5-(pentafluor-sulfanyl)-phenyl]-ethanon, 2-[6-(1-Ethyl-propoxy)-3-imino-[1,2,4]triazolo[4,3-b]pyridazin-2-yl]-1-[3-(3-methoxy-propoxy)-5-(pentafluoro-sulfanyl)-phenyl]-ethanon, 2-[2-(3-tert-Butyl-4-methoxy-5-morpholin-4-yl-phenyl)-2-oxo-ethyl]-6-chlor-3-imino-2,3-dihydro-[1,2,4]triazolo[4,3-b]pyridazin-8-carbonsäure-diethylamid, 6-Chlor-3-imino-2-{2-[3-methoxy-5-(pentafluorsulfanyl)-phenyl]-2-oxo-ethyl}-2,3-dihydro-[1,2,4]triazolo[4,3-b]pyridazin-8-carbonsäure-diethylamid, 2-(6-Ethyl-3-imino-[1,2,4]triazolo[4,3-b]pyridazin-2-yl)-1-[3-methoxy-5-(pentafluor-sulfanyl)-phenyl]-ethanon, 1-(3-tert-Butyl-4-methoxy-5-morpholin-4-yl-phenyl)-2-(6-ethyl-3-imino-[1,2,4]triazolo[4,3-b]pyridazin-2-yl)-ethanon, 1-[3-tert-Butyl-5-(3-methoxy-propoxy)-phenyl]-2-[6-(1-ethyl-propoxy)-3-imino-8-methyl-[1,2,4]triazolo[4,3-b]pyridazin-2-yl]-ethanon, 1-[3-tert-Butyl-5-(2-methoxy-ethoxy)-phenyl]-2-[6-(1-ethyl-propoxy)-3-imino-8-methyl-[1,2,4]triazolo[4,3-b]pyridazin-2-yl]-ethanon, 1-(3-tert-Butyl-5-cyclopropylmethoxy-phenyl)-2-[6-(1-ethyl-propoxy)-3-imino-8-methyl-[1,2,4]triazolo[4,3-b]pyridazin-2-yl]-ethanon, 1-(3-tert-Butyl-5-methoxyphenyl)-2-[6-(1-ethyl-propoxy)-3-imino-8-methyl-[1,2,4]triazolo[4,3-b]pyridazin-2-yl]-ethanon, 1-[3-tert-Butyl-5-(3-methoxy-propoxy)-phenyl]-2-[6-(1-ethyl-propoxy)-3-imino-7-methyl-[1,2,4]triazolo[4,3-b]pyridazin-2-yl]-ethanon, 2-(6-Chlor-3-imino-7,8-dimethyl-[1,2,4]triazolo[4,3-b]pyridazin-2-yl)-1-[3-methoxy-5-(pentafluorsulfanyl)-phenyl]-ethanon, 1-(3-tert-Butyl-4-methoxy-5-morpholin-4-yl-phenyl)-2-(6-chlor-3-imino-7,8-dimethyl-[1,2,4]triazolo[4,3-b]pyridazin-2-yl)-ethanon, 6-Chlor-3-imino-2-{2-[3-methoxy-5-(pentafluor-sulfanyl)-phenyl]-2-oxo-ethyl}-2,3-dihydro-[1,2,4]triazolo[4,3-b]pyridazin-7-carbonsäure-diethylamid, 2-[2-(3-tert-Butyl-4-methoxy-5-morpholin-4-yl-phenyl)-2-oxo-ethyl]-6-chlor-3-imino-2,3-dihydro-[1,2,4]triazolo[4,3-b]pyridazine-7-carbonsäure-diethylamid, 1-[3-tert-Butyl-5-(3-methoxy-propoxy)-phenyl]-2-(6-ethoxy-3-imino-7-methyl-[1,2,4]triazolo[4,3-b]pyridazin-2-yl)-ethanon oder 1-[3-tert-Butyl-5-(2-methoxy-ethoxy)-phenyl]-2-[6-(1-ethyl-propoxy)-3-imino-7-methyl-[1,2,4]triazolo[4,3-b]pyridazin-2-yl]-ethanon.

6. Arzneimittel, **gekennzeichnet durch** einen wirksamen Gehalt an mindestens einer Verbindung der Formel I gemäß einem oder mehreren der Ansprüche 1 bis 5 zusammen mit einem pharmazeutisch geeigneten und physiologisch verträglichen Trägerstoff, Zusatzstoff und/oder anderen Wirk- und Hilfsstoffen.

7. Verbindung der Formel I gemäß einem oder mehreren der Ansprüche 1 bis 5 zur Anwendung in der Prophylaxe, Sekundärprevention und Therapie all solcher Erkrankungen, die mit Thrombosen, Embolien, Hyperkoagulabilität, fibrotischen Veränderungen oder entzündlichen Erkrankungen einhergehen.

8. Verbindung gemäß Anspruch 7, **dadurch gekennzeichnet, dass** es sich bei Erkrankungen um Myokardinfarkt, Angina pectoris und andere Formen des akuten Koronarsyndroms, den Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen handelt, oder die Reduktion der Thrombosegefahr nach chirurgischen Eingriffen wie bei Knie- und Hüftgelenksoperationen, oder Eingriffen, die zu einem Kontakt des Blutes mit Fremdoberflächen führen wie bei Dialysepatienten und Patienten mit Verweilkathetern oder um die disseminierte intravaskuläre Koagulation, Sepsis und anderen intravaskulären Ereignissen, die mit einer Entzündung einhergehen, Atherosklerose, Diabetes und dem metabolischen Syndrom und deren Folgen, Tumorwachstum und Tumormetastasierung, entzündliche und degenerative Gelenkserkrankungen wie der rheumatoiden Arthritis und der Arthrose, Störungen des hämostatischen Systems wie Fibrinablagerungen, fibrotische Veränderungen der Lunge wie die chronische obstruktive Lungenerkrankung, das adult respiratory distress syndrom oder Fibrinablagerungen des Auges nach Augenoperationen oder Verhinderung und/oder Behandlung von Narbenbildung.

9. Verfahren zur Herstellung der Verbindung der Formel I gemäß einem oder mehreren der Ansprüche 1 bis 5, **dadurch gekennzeichnet, dass** man
a) eine Verbindung der Formel II, 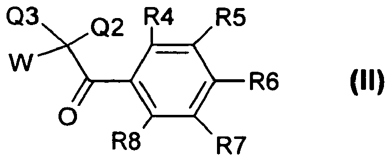 wobei R4 bis R8, Q2 und Q3 wie in Formel I definiert sind und W für Chlorid, Bromid, Mesylat oder Tosylat steht, mit einer Verbindung der Formel III, 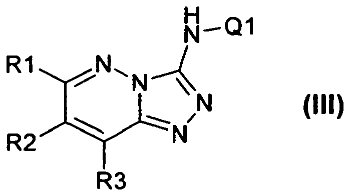 wobei R1, R2, R3 und Q1 wie in Formel I definiert sind, mit oder ohne Basenzugabe in einem Lösungsmittel zu einer Verbindung der Formel I umsetzt, oder
b) eine Verbindung der Formel VII, 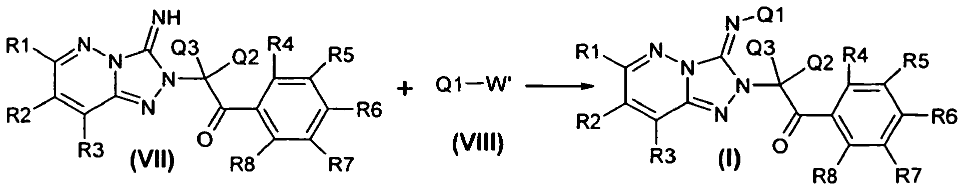 wobei R1, R2, R3, R4, R5, R6, R7, R8, Q1, Q2 und Q3 wie in Formel I definiert sind, mit einer Verbindung Q-W', wobei W' Chlorid, Bromid, Mesylat, Tosylat, Methylsulfat oder eine ähnliche gute Fluchtgruppe bedeutet, mit oder ohne Basenzugabe zu einer Verbindung der Formel I umsetzt, oder
c) die nach den Verfahren a) oder b) hergestellte Verbindung der Formel I entweder in freier Form isoliert oder aus physiologisch unverträglichen Salzen freisetzt oder im Falle des Vorliegens von sauren oder basischen Gruppen in physiologisch verträgliche Salze umwandelt, oder
d) eine nach den Verfahren a) oder b) hergestellte Verbindung der Formel I, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt.

## Claims

1. Compound of the formula I 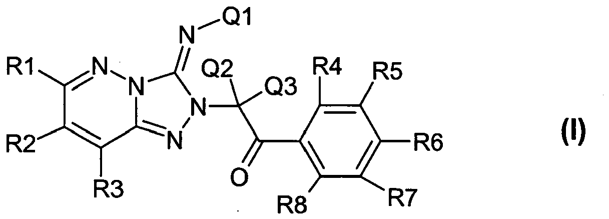 and/or any stereoisomeric or tautomeric forms of the compound of the formula I and/or mixtures of these forms in any ratio, and/or a physiologically compatible salt of the compound of the formula I, where Q1 is a hydrogen atom, -(C₁-C₆)-alkyl, -(C₃-C₆)-cycloalkyl, -OH, -O-(C₁-C₆)-alkyl or -O-(C₃-C₆)-cycloalkyl, where alkyl and cycloalkyl are each unsubstituted or mono-, di- or trisubstituted independently by - (C₁-C₄)-alkyl, -(C₃-C₆)-cycloalkyl, OH, -O-(C₁-C₆)-alkyl or -O-(C₃-C₆)-cycloalkyl, where some or all of the hydrogen atoms in alkyl or cycloalkyl may be replaced by fluorine,
Q2 and Q3 are the same or different and are each independently a hydrogen atom, -(C₁-C₆)-alkyl or - (C₃-C₆)-cycloalkyl, where some or all of the hydrogen atoms in alkyl or cycloalkyl may be replaced by fluorine,
R1, R2 and R3 are the same or different and are each independently a hydrogen atom, -(C₁-C₆)-alkyl, -(C₃-C₆)-cycloalkyl, -O-(C₁-C₈)-alkyl, -O-(C₃-C₆)-cycloalkyl, -(C₀-C₄)-alkylene-C(O)-N(R11)-R12, -(C₀-C₄)-alkylene-C(O)-O-R11, -(C₀-C₄)-alkylene-C(O)-R11, -(C₀-C₄)-alkylene-N(R11)-R12, -(C₀-C₄)-alkylene-N(R11)-C(O)-R12, halogen, OH, -CN, -NO₂, -SO₂CH₃, -SO₂CF₃, -SF₅, -Si[-(C₁-C₄)-alkyl]₃, -(C₁-C₆)-alkylene-O-(C₁-C₆)-alkyl, -O-(C₁-C₆)-alkylene-O-(C₁-C₆)-alkyl, -O-(C₀-C₄)-alkylene-(C₆-C₁₄)-aryl where aryl is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl, -O-(C₁-C₄)-alkylene-(C₃-C₆)-cycloalkyl, -(C₄-C₁₅)-Het,
where Het is unsubstituted or mono-, di- or trisubstituted independently by -(C₁-C₄)-alkyl, - (C₃-C₆)-cycloalkyl, OH, -O-(C₁-C₆)-alkyl or -0-(C₃-C₆)-cycloalkyl or -O-(C₁-C₆)-alkylene-O-(C₁-C₆)-alkylene-O-(C₁-C₆)-alkyl, where alkyl, alkylene and cycloalkyl are each unsubstituted or mono-, di- or trisubstituted independently by -(C₁-C₄)-alkyl, -(C₃-C₆)-cycloalkyl, OH, -O-(C₁-C₆)-alkyl, -(C₆-C₁₄)-aryl where aryl is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, -(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -0-(C₃-C₆)-cycloalkyl, or -O-(C₃-C₆)-cycloalkyl, or
where some or all of the hydrogen atoms in alkyl, alkylene or cycloalkyl may be replaced by fluorine, or
R1 and R2 or R2 and R3, together with the ring atoms to which they are each bonded, form a 5- to 8-membered ring, where the ring consists only of carbon atoms or 1, 2 or 3 of these atoms are replaced by nitrogen, oxygen or sulfur atoms, where the ring is unsubstituted or mono- or disubstituted independently by -(C₁-C₄)-alkyl, - (C₃-C₆)-cycloalkyl, OH, -O-(C₁-C₆)-alkyl or -O-(C₃-C₆)-cycloalkyl, where some or all of the hydrogen atoms in the 5- to 8-membered ring formed, and in alkyl or cycloalkyl,
may be replaced by fluorine,
R11 and R12 are each independently a hydrogen atom, -(C₁-C₆)-alkyl, -(C₃-C₆)-cycloalkyl, -(C₀-C₄)-alkylene-(C₆-C₁₄)-aryl where aryl is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl, -(C₀-C₄)-alkylene-(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, -SO₂CH₃ or -SO₂CF₃,
where some or all of the hydrogen atoms in alkyl, alkylene or cycloalkyl may be replaced by fluorine, or
R11 and R12 in the "N(R11)-R12" and "N(R11)-C(O)-R12" fragments represent a 5- to 8-membered ring which is formed together with the nitrogen atom "N" or the "N-C(O)" group to form cyclic amines, imides or lactams which contain up to 2 further heteroatoms from the group of N, O and S, where the ring is unsubstituted or mono- or disubstituted independently by -(C₁-C₄)-alkyl, - (C₃-C₆)-cycloalkyl, OH, -O-(C₁-C₆)-alkyl or -O-(C₃-C₆)-cycloalkyl, where some or all of the hydrogen atoms in the 5- to 8-membered ring formed, and in alkyl or cycloalkyl, may be replaced by fluorine,
R4, R5, R6, R7 and R8 are the same or different and are each independently a hydrogen atom, -(C₁-C₆)-alkyl, -(C₃-C₆)-cycloalkyl, OH, -CN, -NO₂, -O-(C₁-C₈)-alkyl, -O-(C₃-C₆)-cycloalkyl, -(C₀-C₄)-alkylene-(CO)-N(R21)-R22, -SO₂CH₃, - SO₂CF₃, -(C₀-C₄)-alkylene-C(O)-O-R21, halogen, -SF₅, -(C₀-C₄)-alkylene-C(O)-R21, -(C₀-C₄)-alkylene-N(R21)-R22, -(C₀-C₄)-alkylene-N(R21)-C(O)-R22, -(C₁-C₆)-alkylene-O-(C₁-C₆)-alkyl, -(C₀-C₆)-alkylene-O-(C₁-C₆)-alkylene-O-(C₁-C₆)-alkyl, -Si[-(C₁-C₄)-alkyl]₃, -(C₀-C₆)-alkylene-O-(C₁-C₄)-alkylene-(C₃-C₆)-cycloalkyl, -(C₀-C₆)-alkylene-O-(C₀-C₆)-alkylene-(C₆-C₁₄)-aryl where aryl is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl, or -(C₄-C₁₅)-Het where Het is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl,
where alkyl, alkylene and cycloalkyl are each unsubstituted or mono-, di- or trisubstituted independently by -(C₁-C₄)-alkyl, -(C₃-C₆)-cycloalkyl, OH, -O-(C₁-C₆)-alkyl, -(C₆-C₁₄)-aryl where aryl is unsubstituted or mono-, di-, tri-, tetra- or pentasubstituted independently by halogen, -(C₁-C₄)-alkyl, -(C₃-C₆₎-cycloalkyl, OH, -O-(C₁-C₆)-alkyl or -O-(C₃-C₆)-cycloalkyl, -(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, or -O-(C₃-C₆)-cycloalkyl,
where some or all of the hydrogen atoms in alkyl, alkylene or cycloalkyl may be replaced by fluorine, or
R4 and R5, R5 and R6, R6 and R7 or R7 and R8, together with the ring atoms to which they are each bonded, form a 5- to 8-membered ring, where the ring consists only of carbon atoms or 1, 2 or 3 of these atoms are replaced by nitrogen, oxygen or sulfur atoms, where the ring is unsubstituted or mono- or disubstituted independently by -(C₁-C₄)-alkyl, -(C₃-C₆)-cycloalkyl, OH, -O-(C₁-C₆)-alkyl or -O-(C₃-C₆)-cycloalkyl, where some or all of the hydrogen atoms in the 5- to 8-membered ring formed, and in alkyl or cycloalkyl, may be replaced by fluorine,
R21 and R22 are each independently a hydrogen atom, -(C₁-C₆)-alkyl, -(C₃-C₆)-cycloalkyl, -(C₀-C₄)-alkylene-(C₆-C₁₄)-aryl where aryl is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl, -(C₀-C₄)-alkylene-(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, -SO₂CH₃ or -SO₂CF₃,
where some or all of the hydrogen atoms in alkyl, alkylene or cycloalkyl may be replaced by fluorine, or
R21 and R22 in the "N(R21)-R22" and "N(R21)-C(O)-R22" fragments represent a 5- to 8-membered ring which is formed together with the nitrogen atom "N" or the "N-C(O)" group to form cyclic amines, imides or lactams which contain up to 2 further heteroatoms from the group of N, O and S, where the ring is unsubstituted or mono- or disubstituted independently by -(C₁-C₄)-alkyl, - (C₃-C₆)-cycloalkyl, OH, -O-(C₁-C₆)-alkyl or -O-(C₃-C₆)-cycloalkyl, where some or all of the hydrogen atoms in the 5- to 8-membered ring formed, and in alkyl or cycloalkyl, may be replaced by fluorine.

2. Compound of the formula I according to Claim 1, wherein
Q1, Q2 and Q3 are the same or different and are each independently a hydrogen atom, -(C₁-C₆)-alkyl or -(C₃-C₆)-cycloalkyl, where some or all of the hydrogen atoms in alkyl or cycloalkyl may be replaced by fluorine,
R1, R2 and R3 are the same or different and are each independently
a hydrogen atom, -(C₁-C₆)-alkyl, -(C₃-C₆)-cycloalkyl, -O-(C₁-C₈)-alkyl, -O-(C₃-C₆)-cycloalkyl, -(C₀-C₄)-alkylene-C(O)-N(R11)-R12, -(C₀-C₄)-alkylene-C(O)-O-R11, -(C₀-C₄)-alkylene-C(O)-R11, -(C₀-C₄)-alkylene-N(R11)-R12, -(C₀-C₄)-alkylene-N(R11)-C(O)-R12, halogen, OH, -CN, -NO₂, -SO₂CH₃, -SO₂CF₃, -SF₅, -Si[-(C₁-C₄)-alkyl]₃, -(C₁-C₆)-alkylene-O-(C₁-C₆)-alkyl, -O-(C₁-C₆)-alkylene-O-(C₁-C₆)-alkyl, -O-(C₀-C₄)-alkylene-(C₆-C₁₄)-aryl where aryl is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl, -O-(C₁-C₄)-alkylene-(C₃-C₆)-cycloalkyl, -(C₄-C₁₅)-Het,
where Het is unsubstituted or mono-, di- or trisubstituted independently by -(C₁-C₄)-alkyl, - (C₃-C₆)-cycloalkyl, OH, -O-(C₁-C₆)-alkyl or -0-(C₃-C₆)-cycloalkyl, or -O-(C₁-C₆)-alkylene-O-(C₁-C₆)-alkylene-O-(C₁-C₆)-alkyl,
where alkyl, alkylene and cycloalkyl are each unsubstituted or mono-, di- or trisubstituted independently by -(C₁-C₄)-alkyl, -(C₃-C₆)-cycloalkyl, OH, -O-(C₁-C₆)-alkyl, -(C₆-C₁₄)-aryl where aryl is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, -(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -0-(C₃-C₆)-cycloalkyl, or -O-(C₃-C₆)-cycloalkyl, or
where some or all of the hydrogen atoms in alkyl, alkylene or cycloalkyl may be replaced by fluorine,
with the proviso that at least one R1, R2 or R3 is not a hydrogen atom or
R1 and R2 or R2 and R3, together with the ring atoms to which they are each bonded, form a 5- to 8-membered ring, where the ring consists only of carbon atoms or -1, 2 or 3 of these atoms are replaced by nitrogen, oxygen or sulfur atoms, where the ring is unsubstituted or mono- or disubstituted independently by -(C₁-C₄)-alkyl, - (C₃-C₆)-cycloalkyl, OH, -O-(C₁-C₆)-alkyl or -O-(C₃-C₆)-cycloalkyl, where some or all of the hydrogen atoms in the 5- to 8-membered ring, in alkyl or cycloalkyl may be replaced by fluorine,
R11 and R12 are each independently a hydrogen atom, -(C₁-C₆)-alkyl, -(C₃-C₆)-cycloalkyl, -(C₀-C₄)-alkylene-(C₆-C₁₄)-aryl where aryl is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl, -(C₀-C₄)-alkylene-(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl -SO₂CH₃ or -SO₂CF₃,
where some or all of the hydrogen atoms in alkyl, alkylene or cycloalkyl may be replaced by fluorine, or
R11 and R12 in the "N(R11)-R12" and "N(R11)-C(O)-R12" fragments represent a 5- to 8-membered ring which is formed together with the nitrogen atom "N" or the "N-(CO)" group to form cyclic amines, imides or lactams which contain up to 2 further heteroatoms from the group of N, O and S, where the ring is unsubstituted or mono- or disubstituted independently by -(C₁-C₄)-alkyl, - (C₃-C₆)-cycloalkyl, OH, -O-(C₁-C₆)-alkyl or -O-(C₃-C₆)-cycloalkyl, where some or all of the hydrogen atoms in alkyl or cycloalkyl may be replaced by fluorine,
R4, R5, R6, R7 and R8 are the same or different and are each independently a hydrogen atom, -(C₁-C₆)-alkyl, -(C₃-C₆)-cycloalkyl, OH, -CN, -NO₂, -O-(C₁-C₈)-alkyl, -O-(C₃-C₆)-cycloalkyl, -(C₀-C₄)-alkylene-(CO)-N(R21)-R22, -SO₂CH₃, - SO₂CF₃, -(C₀-C₄)-alkylene-C(O)-O-R21, halogen, -SF₅, -(C₀-C₄)-alkylene-C(O)-R21, -(C₀-C₄)-alkylene-N(R21)-R22, -(C₀-C₄)-alkylene-N(R21)-C(O)-R22, -(C₁-C₆)-alkylene-O-(C₁-C₆)-alkyl, -(C₀-C₆)-alkylene-O-(C₁-C₆)-alkylene-O-(C₁-C₆)-alkyl, -Si[-(C₁-C₄)-alkyl]₃, -(C₀-C₆)-alkylene-O-(C₁-C₄)-alkylene-(C₃-C₆)-cycloalkyl, -(C₀-C₆)-alkylene-O-(C₀-C₆)-alkylene-(C₆-C₁₄)-aryl where aryl is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl, or -(C₄-C₁₅)-Het where Het is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl,
where alkyl, alkylene and cycloalkyl are each unsubstituted or mono-, di- or trisubstituted independently by -(C₁-C₄)-alkyl, -(C₃-C₆)-cycloalkyl, OH, -O-(C₁-C₆)-alkyl, -(C₆-C₁₄)-aryl where aryl is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, -(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, or -O-(C₃-C₆)-cycloalkyl, or
where some or all of the hydrogen atoms in alkyl, alkylene or cycloalkyl may be replaced by fluorine,
with the proviso that at least one R4, R5, R6, R7 or R8 is not a hydrogen atom, or
R4 and R5, R5 and R6, R6 and R7 or R7 and R8, together with the ring atoms to which they are each bonded, form a 5- to 8-membered ring, where the ring consists only of carbon atoms or 1, 2 or 3 of these atoms are replaced by nitrogen, oxygen or sulfur atoms, where the ring is unsubstituted or mono- or disubstituted independently by -(C₁-C₄)-alkyl, -(C₃-C₆)-cycloalkyl, OH, -O-(C₁-C₆)-alkyl or
-O-(C₃-C₆)-cycloalkyl, where some or all of the hydrogen atoms in the 5- to 8-membered ring formed, and in alkyl or cycloalkyl, may be replaced by fluorine,
R21 and R22 are each independently a hydrogen atom, -(C₁-C₆)-alkyl,
-(C₃-C₆)-cycloalkyl, -(C₀-C₄)-alkylene-(C₆-C₁₄)-aryl where aryl is unsubstituted or mono-, di- or trisubstituted independently by -O-(C₁-C₆)-alkyl, -(C₁-C₄)-alkyl, OH, -(C₃-C₆)-cycloalkyl or
-O-(C₃-C₆)-cycloalkyl,
-(C₀-C₄)-alkylene-(C₄-C₁₅)- Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or
-O-(C₃-C₆)-cycloalkyl,
-SO₂CH₃ or -SO₂CF₃,
where some or all of the hydrogen atoms in alkyl, alkylene or cycloalkyl may be replaced by fluorine, or
R21 and R22 in the "N(R21)-R22" and "N(R21)-C(O)-R22" fragments represent a 5- to 8-membered ring which is formed together with the nitrogen atom "N" or the "N-C(O)" group to form cyclic amines, imides or lactams which contain up to 2 further heteroatoms from the group of N, O and S, where the ring is unsubstituted or mono- or disubstituted independently by -(C₁-C₄)-alkyl, - (C₃-C₆)-cycloalkyl, OH, -O-(C₁-C₆)-alkyl or -O-(C₃-C₆)-cycloalkyl, where some or all of the hydrogen atoms in the 5- to 8-membered ring formed, and in alkyl or cycloalkyl, may be replaced by fluorine.

3. Compound of the formula I according to Claim 1 or 2, wherein
Q1, Q2 and Q3 are the same or different and are each independently a hydrogen atom, -(C₁-C₆)-alkyl or -(C₃-C₆)-cycloalkyl, where some or all of the hydrogen atoms in alkyl or cycloalkyl may be replaced by fluorine,
R1, R2 and R3 are the same or different and are each independently
a hydrogen atom, -(C₁-C₆)-alkyl, -(C₃-C₆)-cycloalkyl, -O-(C₁-C₈)-alkyl, -O-(C₃-C₆)-cycloalkyl, -(C₀-C₄)-alkylene-C(O)-N(R11)-R12, - (C₀-C₄)-alkylene-C (O)-O-R11, -(C₀-C₄)-alkylene-C (O) -R11, -(C₀-C₄)-alkylene-N(R11)-R12, -(C₀-C₄)-alkylene-N(R11)-C(O)-R12,
halogen, OH, -CN, -NO₂, -SO₂CH₃, -Si[-(C₁-C₄)-alkyl]3, -(C₁-C₆)-alkylene-O-(C₁-C₆)-alkyl, -O-(C₁-C₆)-alkylene-O-(C₁-C₆)-alkyl, -O-(C₀-C₄)-alkylene-(C₆-C₁₄)-aryl, -O-(C₁-C₄)-alkylene-(C₃-C₆)-cycloalkyl, -(C₄-C₁₅)-Het or -O-(C₁-C₆)-alkylene-O-(C₁-C₆)-alkylene-O-(C₁-C₆)-alkyl,
where alkyl, alkylene and cycloalkyl are each unsubstituted or mono-, di- or trisubstituted independently by -(C₁-C₄)-alkyl, -(C₃-C₆)-cycloalkyl, OH, -O-(C₁-C₆)-alkyl or -O-(C₃-C₆)-cycloalkyl, or
where some or all of the hydrogen atoms in alkyl, alkylene or cycloalkyl may be replaced by fluorine,
with the proviso that at least one R1, R2 or R3 is not a hydrogen atom or
R1 and R2 or R2 and R3, together with the ring atoms to which they are each bonded, form a ring selected from the group of 2,3,5,6,7,8-hexahydro-1,2,3a,4,5,8-hexaaza-cyclopenta[b]naphthalene; 2,6,7,8-tetrahydro-3H-5-oxa-1,2,3a,4,8-pentaazacyclopenta[b]naphthalene; 2,3,6,7-tetrahydro-5,8-dioxa-1,2,3a,4-tetraaza-cyclopenta[b]naphthalene; 2,3,6,7-tetrahydro-5H-8-oxa-1,2,3a,4,5-pentaazacyclopenta[b]naphthalene; 2,6,7,8-tetrahydro-3H-5-thia-1,2,3a,4,8-pentaaza-cyclopenta[b]naphthalene; 2,3,6,7,8,9-hexahydro-1,2,3a,4,6,9-hexaazacyclopenta[a]naphthalene; 2,3-dihydro-5,7-dioxa-1,2,3a,4-tetraaza-s-indacene; 2,6,7,8-tetrahydro-3H-cyclopenta[e][1,2,4]triazolo[4,3-b]pyridazine; 2,7,8,9-tetrahydro-3H-cyclopenta[d][1,2,4]triazolo[4,3-b]pyridazine and 2,3,6a,9a-tetrahydro-[1,3]dioxolo[4,5-d][1,2,4]triazolo[4,3-b]pyridazine, where the ring is unsubstituted or mono- or disubstituted independently by -(C₁-C₄)-alkyl, -(C₃-C₆)-cycloalkyl, OH, -O-(C₁-C₆)-alkyl or -O-(C₃-C₆)-cycloalkyl, where some or all of the hydrogen atoms in alkyl or cycloalkyl may be replaced by fluorine,
R11 and R12 are each independently a hydrogen atom, -(C₁-C₆)-alkyl, -(C₃-C₆)-cycloalkyl, -(C₀-C₄)-alkylene-(C₆-C₁₄)-aryl, -(C₀-C₄)-alkylene-(C₄-C₁₅)-Het, -SO₂CH₃ or - SO₂CF₃,
where some or all of the hydrogen atoms in alkyl, alkylene or cycloalkyl may be replaced by fluorine, or
R11 and R12 in the "N(R11)-R12" and "N(R11)-C(O)-R12" fragments represent a 5- to 8-membered ring, selected from the group of azetidinyl, pyrrolidinyl, piperidinyl, piperazinyl, azepinyl, morpholinyl, thiomorpholinyl, pyrrolidine-2.5-dionyl, piperidine-2,6-dionyl, piperazine-2,6-dionyl, morpholine-3,5-dionyl, pyrrolidin-2-onyl, piperidin-2-onyl, piperazin-2-onyl and morpholin-3-onyl, where the ring is unsubstituted or mono- or disubstituted independently by -(C₁-C₄)-alkyl, -(C₃-C₆)-cycloalkyl, OH, -O-(C₁-C₆)-alkyl or -O-(C₃-C₆)-cycloalkyl, where some or all of the hydrogen atoms in alkyl or cycloalkyl may be replaced by fluorine,
R4, R5, R6, R7 and R8 are the same or different and are each independently a hydrogen atom, -(C₁-C₆)-alkyl, -(C₃-C₆)-cycloalkyl, OH, -CN, -NO₂, -O-(C₁-C₈)-alkyl, -O-(C₃-C₆)-cycloalkyl, -(C₀-C₄)-alkylene-(CO)-N(R21)-R22, -SO₂CH₃, - SO₂CF₃, -(C₀-C₄)-alkylene-C(O)-O-R21, halogen, -SF₅, -(C₀-C₄)-alkylene-C(O)-R21, (C₀-C₄)-alkylene-N(R21)-R22, -(C₀-C₄)-alkylene-N(R21)-C(O)-R22, -(C₁-C₆)-alkylene-O-(C₁-C₆)-alkyl, -(C₀-C₆)-alkylene-O-(C₁-C₆)-alkylene-O-(C₁-C₆)-alkyl, -Si[-(C₁-C₄)-alkyl]₃, -(C₀-C₆)-alkylene-O-(C₀-C₄)-alkylene-(C₃-C₆)-cycloalkyl, -(C₀-C₆)-alkylene-O-(C₀-C₆)-alkylene-(C₆-C₁₄)-aryl or - (C₄-C₁₅) -Het,
where alkyl, alkylene and cycloalkyl are each unsubstituted or mono-, di- or trisubstituted independently by -(C₁-C₄)-alkyl, -(C₃-C₆)-cycloalkyl, OH, -O-(C₁-C₆)-alkyl, -(C₆-C₁₄)-aryl where aryl is unsubstituted or mono-, di-, tri-, tetra- or pentasubstituted independently by halogen,
-(C₁-C₄)-alkyl, -(C₃-C₆)-cycloalkyl, OH, -O-(C₁-C₆)-alkyl or
-O-(C₃-C₆)-cycloalkyl,
-(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, or -O-(C₃-C₆)-cycloalkyl, or
where some or all of the hydrogen atoms in alkyl, alkylene or cycloalkyl may be replaced by fluorine,
with the proviso that at least one R4, R5, R6, R7 or R8 is not a hydrogen atom, or
R4 and R5, R5 and R6, R6 and R7 or R7 and R8, together with the ring atoms to which they are each bonded, form a 5- to 8-membered ring selected from the group of 2,3-dihydrobenzo[1,4]dioxin; 3,4-dihydro-2H-benzo[1,4]oxazine; 1,2,3,4-tetrahydroquinoxaline; benzo[1,3]dioxole; 3,4-dihydro-2H-benzo[1,4]thiazine and 2,3,4,5-tetrahydro-1H-benzo[b][1,4]diazepine,
where the ring is unsubstituted or mono- or disubstituted independently by -(C₁-C₄)-alkyl, - (C₃-C₆)-cycloalkyl, OH, -O-(C₁-C₆)-alkyl or -0-(C₃-C₆)-cycloalkyl, where some or all of the hydrogen atoms in alkyl or cycloalkyl may be replaced by fluorine,
R21 and R22 are each independently a hydrogen atom, -(C₁-C₆)-alkyl, -(C₃-C₆)-cycloalkyl, -(C₀-C₄)-alkylene-(C₆-C₁₄)-aryl, -(C₀-C₄)-alkylene-(C₄-C₁₅)-Het, -SO₂CH₃ or - SO₂CF₃,
where some or all of the hydrogen atoms in alkyl, alkylene or cycloalkyl may be replaced by fluorine, or
R21 and R22 in the "N(R21)-R22" and "N(R21)-C(O)-R22" fragments represent a 5- to 8-membered ring selected from the group of azetidinyl, pyrrolidinyl, piperidinyl, piperazinyl, azepinyl, morpholinyl, thiomorpholinyl, pyrrolidine-2.5-dionyl, piperidine-2,6-dionyl, piperazine-2,6-dionyl, morpholine-3,5-dionyl, pyrrolidin-2-onyl, piperidin-2-onyl, piperazin-2-onyl and morpholin-3-onyl, where the ring is unsubstituted or mono- or disubstituted independently by -(C₁-C₄)-alkyl, -(C₃-C₆)-cycloalkyl, OH, -O-(C₁-C₆)-alkyl or -O-(C₃-C₆)-cycloalkyl, where some or all of the hydrogen atoms in alkyl or cycloalkyl may be replaced by fluorine.

4. Compound of the formula I according to Claims 1 to 3, wherein
Q1, Q2 and Q3 are the same and are each a hydrogen atom,
R1, R2 and R3 are the same or different and are each independently
a hydrogen atom, -(C₁-C₆)-alkyl; -O-(C₁-C₈)-alkyl, -O-(C₃-C₆)-cycloalkyl, -(C₀-C₄)-alkylene-C(O)-N(R11)-R12, -(C₀-C₄)-alkylene-N(R11)-R12, halogen, -O-(C₁-C₆)-alkylene-O-(C₁-C₆)-alkyl, -O-(C₀-C₄)-alkylenephenyl or -O-(C₁-C₆)-alkylene-O-(C₁-C₆)-alkylene-O-(C₁-C₆)-alkyl,
where alkyl or alkylene is in each case unsubstituted or mono- or disubstituted by -O-(C₁-C₆)-alkyl,
where some or all of the hydrogen atoms in alkyl or alkylene may be replaced by fluorine,
with the proviso that at least one R1, R2 or R3 is not a hydrogen atom,
R11 and R12 are each independently a hydrogen atom or -(C₁-C₆)-alkyl, or
R11 and R12 in the "N(R11)-R12" fragment is a 5-to 8-membered ring selected from the group of azetidinyl, pyrrolidinyl, piperidinyl, piperazinyl, azepinyl, imidazolyl, morpholinyl, thiomorpholinyl, pyrrolidine-2.5-dionyl, piperidine-2,6-dionyl, piperazine-2,6-dionyl, morpholine-3,5-dionyl, pyrrolidin-2-onyl, piperidin-2-onyl, piperazin-2-onyl and morpholin-3-onyl,
R4, R5, R6, R7 and R8 are the same or different and are each independently
a hydrogen atom, -(C₁-C₆)-alkyl, OH, -O-(C₁-C₈)-alkyl, halogen, -SF₅, -(C₀-C₄)-alkylene-N(R21)-R22, -(C₀-C₄)-alkylene-N(R21)-C(O)-R22, -CF₃, -(C₀-C₆)-alkylene-O-(C₁-C₆)-alkylene-O-(C₁-C₆)-alkyl, -(C₀-C₆)-alkylene-O-(C₁-C₄)-alkylene-(C₃-C₆)-cycloalkyl, or -(C₀-C₆)-alkylene-O-(C₀-C₆)-alkylenephenyl,
where alkyl or alkylene is in each case unsubstituted or mono- or disubstituted by -O-(C₁-C₆)-alkyl,
with the proviso that at least one R4, R5, R6, R7 or R8 is not a hydrogen atom, or
R4 and R5, R5 and R6, R6 or R7 or R7 and R8, together with the ring atoms to which they are each bonded, form a five- to eight-membered ring selected from the group of 2,3-dihydrobenzo[1,4]dioxin; 3,4-dihydro-2H-benzo[1,4]oxazine; 1,2,3,4-tetrahydro-quinoxaline; benzo[1,3]dioxole; 3,4-dihydro-2H-benzo[1,4]thiazine and 2,3,4,5-tetrahydro-1H-benzo[b][1,4]diazepine,
where the ring is unsubstituted or mono- or disubstituted by -(C₁-C₄)-alkyl,
R21 and R22 are each independently a hydrogen atom or -(C₁-C₆)-alkyl, or
R21 and R22 in the "N(R21)-R22" fragment represent a 5- to 8-membered ring selected from the group of azetidinyl, pyrrolidinyl, piperidinyl, piperazinyl, azepinyl, imidazolyl, morpholinyl, thiomorpholinyl, pyrrolidine-2.5-dionyl, piperidine-2,6-dionyl, piperazine-2,6-dionyl, morpholine-3,5-dionyl, pyrrolidin-2-onyl, piperidin-2-onyl, piperazin-2-onyl and morpholin-3-onyl.

5. Compound of the formula I according to Claims 1 to 4, wherein the compound of the formula I is selected from the group of 2-(6-chloro-3-imino-[1,2,4]triazolo[4,3-b]pyridazin-2-yl)-1-(3,5-di-tert-butyl-4-hydroxyphenyl)ethanone, 1-(3-tert-butyl-4-methoxy-5-morpholin-4-ylphenyl)-2-(3-imino-6-isopropoxy-[1,2,4]triazolo[4,3-b]-pyridazin-2-yl)ethanone, 1-(3-tert-butyl-4-methoxy-5-morpholin-4-ylphenyl)-2-(3-imino-6-methoxy-[1,2,4]triazolo[4,3-b]pyridazin-2-yl)ethanone, 2-(6-ethoxy-3-imino-[1,2,4]triazolo[4,3-b]pyridazin-2-yl)-1-(4-methoxy-3-morpholin-4-yl-5-trifluoromethylphenyl)ethanone, N-[3-[2-(6-ethoxy-3-imino-[1,2,4]triazolo[4,3-b]pyridazin-2-yl)acetyl]-5-(pentafluorosulfanyl)phenyl]-N-methylacetamide, N-[3-[2-(6-ethoxy-3-imino-[1,2,4]triazolo[4,3-b]pyridazin-2-yl)acetyl]-5-(pentafluorosulfanyl)phenyl]acetamide, 1-(3-tert-butyl-4-methoxy-5-morpholin-4-ylphenyl)-2-(6-ethoxy-3-imino-[1,2,4]triazolo[4,3-b]pyridazin-2-yl)ethanone, 1-(3-tert-butyl-4-methoxy-5-morpholin-4-ylphenyl)-2-(6-cyclopentyloxy-3-imino-[1,2,4]triazolo[4,3-b]pyridazin-2-yl)ethanone, 1-(3-tert-butyl-4-methoxy-5-morpholin-4-ylphenyl)-2-(6-cyclobutoxy-3-imino-[1,2,4]triazolo[4,3-b]pyridazin-2-yl)ethanone, 1-(3-tert-butyl-4-methoxy-5-morpholin-4-ylphenyl)-2-(3-imino-6-phenoxy-[1,2,4]triazolo[4,3-b]pyridazin-2-yl)ethanone, 2-(6-benzyloxy-3-imino-[1,2,4]triazolo[4,3-b]pyridazin-2-yl)-1-(3-tert-butyl-4-methoxy-5-morpholin-4-ylphenyl)ethanone, 1-(3-tert-butyl-4-methoxy-5-morpholin-4-ylphenyl)-2-(6-cyclohexyloxy-3-imino-[1,2,4]triazolo[4,3-b]pyridazin-2-yl)ethanone, 1-(3-tert-butyl-4-methoxy-5-morpholin-4-ylphenyl)-2-[3-imino-6-(2,2,2-trifluoroethoxy)-[1,2,4]triazolo[4,3-b]pyridazin-2-yl]ethanone, 1-(3-tert-butyl-4-methoxy-5-morpholin-4-ylphenyl)-2-[6-(1-ethylpropoxy)-3-imino-[1,2,4]triazolo[4,3-b]pyridazin-2-yl]ethanone, 1-(3-tert-butyl-4-methoxy-5-morpholin-4-ylphenyl)-2-(6-cyclopropylmethoxy-3-imino-[1,2,4]triazolo[4,3-b]pyridazin-2-yl)ethanone, 2-[6-(1-ethylpropoxy)-3-imino-[1,2,4]triazolo[4,3-b]pyridazin-2-yl]-1-[3-methylamino-5-(pentafluorosulfanyl)phenyl]ethanone, 2-[6-(1-ethylpropoxy)-3-imino-[1,2,4]triazolo[4,3-b]pyridazin-2-yl]-1-[3-methoxy-5-(pentafluorosulfanyl)phenyl]ethanone, 1-(3-tert-butyl-5-ethoxymethylphenyl)-2-[6-(1-ethylpropoxy)-3-imino-[1,2,4]triazolo[4,3-b]pyridazin-2-yl]ethanone, 1-(3-tert-butyl-5-cyclopropylmethoxymethylphenyl)-2-[6-(1-ethylpropoxy)-3-imino-[1,2,4]triazolo[4,3-b]pyridazin-2-yl]ethanone, 1-(3-tert-butyl-4,5-diethoxyphenyl)-2-[6-(1-ethylpropoxy)-3-imino-[1,2,4]triazolo[4,3-b]pyridazin-2-yl]ethanone, 1-(3-tert-butyl-5-ethoxyphenyl)-2-[6-(1-ethylpropoxy)-3-imino-[1,2,4]triazolo[4,3-b]pyridazin-2-yl]ethanone, 1-(3-tert-butyl-5-propoxymethylphenyl)-2-[6-(1-ethylpropoxy)-3-imino-[1,2,4]triazolo[4,3-b]pyridazin-2-yl]ethanone, 1-(3-tert-butyl-4,5-bis(cyclopropylmethoxy)phenyl)-2-[6-(1-ethylpropoxy)-3-imino-[1,2,4]triazolo[4,3-b]pyridazin-2-yl]ethanone, 2-[6-(1-ethylpropoxy)-3-imino-[1,2,4]triazolo[4,3-b]pyridazin-2-yl]-.1-(3-methoxy-5-trifluoromethylphenyl)ethanone, 1-(3-tert-butyl-5-methoxyphenyl)-2-[6-(1-ethylpropoxy)-3-imino-[1,2,4]triazolo[4,3-b]pyridazin-2-yl]ethanone, 1-(3-tert-butyl-5-cyclopropylmethoxyphenyl)-2-[6-(1-ethylpropoxy)-3-imino-[1,2,4]triazolo[4,3-b]pyridazin-2-yl]ethanone, 1-(3-tert-butyl-5-cyclobutyl-methoxyphenyl)-2-[6-(1-ethylpropoxy)-3-imino-[1,2,4]triazolo[4,3-b]pyridazin-2-yl]ethanone, 1-(3-benzyloxymethyl-5-tert-butylphenyl)-2-[6-(1-ethylpropoxy)-3-imino-[1,2,4]triazolo[4,3-b]pyridazin-2-yl]ethanone, 1-(3-cyclo-hexylmethoxy-4,5-dimethoxyphenyl)-2-[6-(1-ethylpropoxy)-3-imino-[1,2,4]triazolo[4,3-b]pyridazin-2-yl]ethanone, 2-(6-butoxy-3-imino-[1,2,4]triazolo[4,3-b]pyridazin-2-yl)-1-(3-tert-butyl-5-methoxymethylphenyl)ethanone, 1-(3-chloro-5-methoxyphenyl)-2-[6-(1-ethylpropoxy)-3-imino-[1,2,4]triazolo[4,3-b]pyridazin-2-yl]ethanone, 1-(8-tert-butyl-4-methyl-3,4-dihydro-2H-benzo[1,4]oxazin-6-yl)-2-[6-(1-ethylpropoxy)-3-imino-[1,2,4]triazolo[4,3-b]pyridazin-2-yl]ethanone, 1-(3-tert-butyl-4-methoxy-5-morpholin-4-ylphenyl)-2-(3-imino-6-piperidin-1-yl-[1,2,4]triazolo[4,3-b]pyridazin-2-yl)ethanone, 1-(3-tert-butyl-4-methoxy-5-morpholin-4-ylphenyl)-2-(6-diethylamino-3-imino-[1,2,4]triazolo[4,3-b]pyridazin-2-yl)ethanone, 2-[6-(1-ethylpropoxy)-3-imino-[1,2,4]triazolo[4,3-b]pyridazin-2-yl]-1-(3-isopropyl-5-methoxyphenyl)ethanone, 1-(3-cyclohexylmethoxy-5-ethoxyphenyl)-2-[6-(1-ethylpropoxy)-3-imino-[1,2,4]triazolo[4,3-b]pyridazin-2-yl]ethanone, 1-(3-bromo-5-methoxyphenyl)-2-[6-(1-ethylpropoxy)-3-imino-[1,2,4]triazolo[4,3-b]pyridazin-2-yl]ethanone, 1-[3-(3,3-dimethylbutoxy)-5-methoxyphenyl]-2-[6-(1-ethylpropoxy)-3-imino-[1,2,4]triazolo[4,3-b]pyridazin-2-yl]ethanone, 1-[3-(3,3-dimethylbutoxy)-5-ethoxyphenyl]-2-[6-(1-ethylpropoxy)-3-imino-[1,2,4]triazolo[4,3-b]pyridazin-2-yl]ethanone, 1-(3-cyclohexylmethoxy-5-methoxyphenyl)-2-[6-(1-ethylpropoxy)-3-imino-[1,2,4]triazolo[4,3-b]pyridazin-2-yl]ethanone, 1-(3-bromo-4,5-dimethoxyphenyl)-2-[6-(1-ethylpropoxy)-3-imino-[1,2,4]triazolo[4,3-b]pyridazin-2-yl]ethanone, 2-(6-diethylamino-3-imino-[1,2,4]triazolo[4,3-b]pyridazin-2-yl)-1-[3-methoxy-5-(pentafluorosulfanyl)phenyl]ethanone, 1-(3-tert-butyl-4-methoxy-5-morpholin-4-ylphenyl)-2-(3-imino-6-morpholin-4-yl-[1,2,4]triazolo[4,3-b]pyridazin-2-yl)ethanone, 1-(3-tert-butyl-4-methoxy-5-morpholin-4-ylphenyl)-2-(6-chloro-3-imino-8-methyl-[1,2,4]triazolo[4,3-b]pyridazin-2-yl)ethanone, 1-(3-tert-butyl-4-methoxy-5-morpholin-4-ylphenyl)-2-(6-imidazol-1-yl-3-imino-[1,2,4]triazolo[4,3-b]pyridazin-2-yl)ethanone, 1-(5-bromo-2,3-dimethoxyphenyl)-2-[6-(1-ethylpropoxy)-3-imino-[1,2,4]triazolo[4,3-b]pyridazin-2-yl]ethanone, 1-(3-tert-butyl-5-methoxyphenyl)-2-{3-imino-6-[2-(2-methoxyethoxy)ethoxy]-[1,2,4]triazolo[4,3-b]pyridazin-2-yl}ethanone, 1-(3-chloro-4,5-dimethoxyphenyl)-2-[6-(1-ethylpropoxy)-3-imino-[1,2,4]triazolo[4,3-b]pyridazin-2-yl]ethanone, 1-(3-tert-butyl-4-methoxy-5-morpholin-4-ylphenyl)-2-(6-diethylamino-3-imino-8-methyl-[1,2,4]triazolo[4,3-b]pyridazin-2-yl)ethanone, 1-[3-tert-butyl-5-(2-methoxyethoxy)phenyl]-2-[6-(1-ethylpropoxy)-3-imino-[1,2,4]triazolo[4,3-b]pyridazin-2-yl]ethanone, 1-(3-tert-butyl-5-methoxyphenyl)-2-[3-imino-6-(2-methoxyethoxy)-[1,2,4]triazolo[4,3-b]pyridazin-2-yl]ethanone, 1-(3-tert-butyl-5-methoxymethylphenyl)-2-[3-imino-6-(2-methoxyethoxy)-[1,2,4]triazolo[4,3-b]pyridazin-2-yl]ethanone, 2-[6-(1-ethylpropoxy)-3-imino-[1,2,4]triazolo[4,3-b]pyridazin-2-yl]-1-[3-morpholin-4-yl-5-(pentafluorosulfanyl)phenyl]ethanone, 1-(3-tert-butyl-4-methoxy-5-morpholin-4-ylphenyl)-2-(6-ethoxy-3-imino-8-methyl-[1,2,4]triazolo[4,3-b]pyridazin-2-yl)ethanone, 2-[6-(1-ethylpropoxy)-3-imino-8-methyl-[1,2,4]triazolo[4,3-b]pyridazin-2-yl]-1-(5-methoxy-3-pentafluorosulfanylphenyl)ethanone, 1-(3-tert-butyl-4-methoxy-5-morpholin-4-ylphenyl)-2-(6-chloro-3-imino-[1,2,4]triazolo[4,3-b]-pyridazin-2-yl)ethanone, 1-[3-tert-butyl-5-(2-methoxyethoxymethyl)phenyl]-2-[6-(1-ethylpropoxy)-3-imino-[1,2,4]triazolo[4,3-b]pyridazin-2-yl]ethanone, 2-[6-(1-ethylpropoxy)-3-imino-[1,2,4]triazolo[4,3-b]-pyridazin-2-yl]-1-[3-(2-methoxyethoxy)-5-(pentafluorosulfanyl)phenyl]ethanone, 2-(6-ethoxy-3-imino-8-methyl-[1,2,4]triazolo[4,3-b]pyridazin-2-yl)-1-[3-methoxy-5-(pentafluorosulfanyl)phenyl]ethanone, 2-[6-(1-ethylpropoxy)-3-imino-[1,2,4]triazolo[4,3-b]pyridazin-2-yl]-1-[3-(3-methoxypropoxy)-5-(pentafluorosulfanyl)phenyl]ethanone, N,N-diethyl-2-[2-(3-tert-butyl-4-methoxy-5-morpholin-4-ylphenyl)-2-oxoethyl]-6-chloro-3-imino-2,3-dihydro-[1,2,4]triazolo[4,3-b]pyridazine-8-carboxamide, N,N-diethyl-6-chloro-3-imino-2-{2-[3-methoxy-5-(pentafluorosulfanyl)phenyl]-2-oxoethyl}-2,3-dihydro-[1,2,4]triazolo[4,3-b]pyridazine-8-carboxamide, 2-(6-ethyl-3-imino-[1,2,4]triazolo[4,3-b]pyridazin-2-yl)-1-[3-methoxy-5-(pentafluorosulfanyl)phenyl]ethanone, 1-(3-tert-butyl-4-methoxy-5-morpholin-4-ylphenyl)-2-(6-ethyl-3-imino-[1,2,4]triazolo[4,3-b]pyridazin-2-yl)ethanone, 1-[3-tert-butyl-5-(3-methoxypropoxy)phenyl]-2-[6-(1-ethylpropoxy)-3-imino-8-methyl-[1,2,4]triazolo[4,3-b]pyridazin-2-yl]ethanone, 1-[3-tert-butyl-5-(2-methoxyethoxy)phenyl]-2-[6-(1-ethylpropoxy)-3-imino-8-methyl-[1,2,4]triazolo[4,3-b]pyridazin-2-yl]ethanone, 1-(3-tert-butyl-5-cyclopropylmethoxyphenyl)-2-[6-(1-ethylpropoxy)-3-imino-8-methyl-[1,2,4]triazolo[4,3-b]pyridazin-2-yl]ethanone, 1-(3-tert-butyl-5-methoxyphenyl)-2-[6-(1-ethylpropoxy)-3-imino-8-methyl-[1,2,4]triazolo[4,3-b]pyridazin-2-yl]ethanone, 1-[3-tert-butyl-5-(3-methoxypropoxy)phenyl]-2-[6-(1-ethylpropoxy)-3-imino-7-methyl-[1,2,4]triazolo[4,3-b]pyridazin-2-yl]ethanone, 2-(6-chloro-3-imino-7,8-dimethyl-[1,2,4]triazolo[4,3-b]pyridazin-2-yl)-1-[3-methoxy-5-(pentafluorosulfanyl)phenyl]ethanone, 1-(3-tert-butyl-4-methoxy-5-morpholin-4-ylphenyl)-2-(6-chloro-3-imino-7,8-dimethyl-[1,2,4]triazolo[4,3-b]pyridazin-2-yl)ethanone, N,N-diethyl-6-chloro-3-imino-2-{2-[3-methoxy-5-(pentafluorosulfanyl)-phenyl]-2-oxoethyl}-2,3-dihydro-[1,2,4]triazolo[4,3-b]pyridazine-7-carboxamide, N,N-diethyl-2-[2-(3-tert-butyl-4-methoxy-5-morpholin-4-ylphenyl)-2-oxoethyl]-6-chloro-3-imino-2,3-dihydro-[1,2,4]triazolo[4,3-b]pyridazine-7-carboxamide, 1-[3-tert-butyl-5-(3-methoxypropoxy)phenyl]-2-(6-ethoxy-3-imino-7-methyl-[1,2,4]triazolo[4,3-b]pyridazin-2-yl)ethanone or 1-[3-tert-butyl-5-(2-methoxyethoxy)phenyl]-2-[6-(1-ethylpropoxy)-3-imino-7-methyl-[1,2,4]triazolo[4,3-b]pyridazin-2-yl]ethanone.

6. Medicament, **characterized by** an effective content of at least one compound of the formula I according to one or more of Claims 1 to 5 together with a pharmaceutically suitable and physiologically compatible carrier, additive and/or other active ingredients and excipients.

7. Compound of the formula I according to one or more of Claims 1 to 5 for use in prophylaxis, secondary prevention and treatment of all of those disorders associated with thromboses, embolisms, hypercoagulability, fibrotic changes or inflammatory disorders.

8. Compound according to Claim 7, **characterized in that** the disorders are myocardial infarction, angina pectoris and other types of acute coronary syndrome, stroke, peripheral vascular disorders, deep vein thrombosis, pulmonary embolism, embolic or thrombotic events caused by cardiac arrhythmias, cardiovascular events such as restenosis following revascularization and angioplasty and similar procedures such as stent implantations and bypass operations or reduction of the risk of thrombosis following surgical procedures such as knee and hip joint operations or procedures leading to contact of blood with foreign surfaces, such as for dialysis patients and patients with indwelling catheters or disseminated intravascular coagulation, sepsis and other intravascular events associated with inflammation, atherosclerosis, diabetes and the metabolic syndrome and the sequelae thereof, tumor growth and tumor metastasis, inflammatory and degenerative articular disorders such as rheumatoid arthritis and arthrosis, impairments of the hemostatic system such as fibrin deposits, fibrotic changes in the lung such as chronic obstructive pulmonary disease, adult respiratory distress syndrome or fibrin deposits in the eye following eye operations or prevention and/or treatment of scarring.

9. Process for preparing the compound of the formula I according to one or more of Claims 1 to 5, which comprises
a) reacting a compound of the formula II 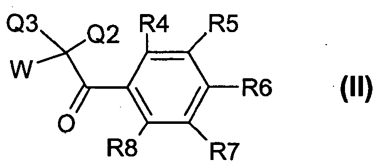 where R4 to R8, Q2 and Q3 are each as defined in formula I and W is chloride, bromide, mesylate or tosylate with a compound of the formula III 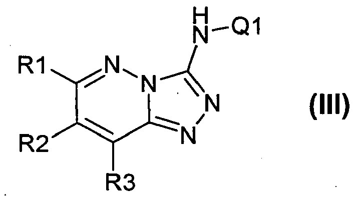 where R1, R2, R3 and Q1 are each as defined in formula I, with or without addition of base, in a solvent to give a compound of the formula I, or
b) reacting a compound of the formula VII 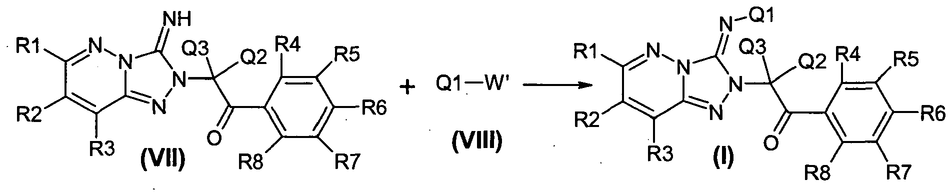 where R1, R2, R3, R4, R5, R6, R7, R8, Q1, Q2 and Q3 are each as defined in formula I with a compound Q-W' where W' is chloride, bromide, mesylate, tosylate, methylsulfate or a similar good leaving group, with or without addition of base, to give a compound of the formula-I, or
c) either isolating the compound of the formula I prepared by method a) or b) in free form or releasing it from physiologically incompatible salts or, in the case of the presence of acidic or basic groups, converting it to physiologically compatible salts, or
d) separating a compound of the formula I prepared by method a) or b), or a suitable precursor of the formula I which, owing to its chemical structure, occurs in enantiomeric or diastereomeric forms, into the pure enantiomers or diastereomers by salt formation with enantiomerically pure acids or bases, chromatography on chiral stationary phases or derivatization by means of chiral enantiomerically pure compounds such as amino acids, separation of the diastereomers thus obtained, and elimination of the chiral auxiliary groups.

## Revendications

1. Composé de formule I 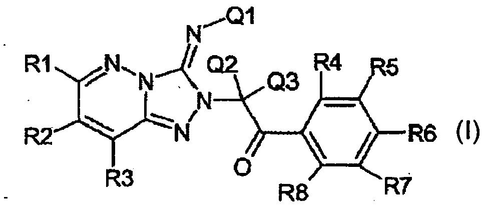 et/ou des formes stéréoisomères ou tautomères quelconques du composé de formule I et/ou des mélanges de ces formes dans un rapport quelconque, et/ou un sel physiologiquement compatible du composé de formule I, où
Q1 est un atome d'hydrogène, -(alkyle en C₁-C₆), -(cycloalkyle en C₃-C₆), -OH, -O-(alkyle en C₁-C₆) ou -O-(cycloalkyle en C₃-C₆), où l'alkyle et le cycloalkyle sont chacun non substitués ou mono-, di- ou trisubstitués indépendamment par -(alkyle en C₁-C₄), -(cycloalkyle en C₃-C₆), OH, -O-(alkyle en C₁-C₆) ou -O-(cycloalkyle en C₃-C₆), où certains ou l'ensemble des atomes d'hydrogène dans l'alkyle ou le cycloalkyle peuvent être remplacés par un fluor,
Q2 et Q3 sont identiques ou différents et sont chacun indépendamment un atome d'hydrogène, -(alkyle en C₁-C₆) ou -(cycloalkyle en C₃-C₆), où certains ou l'ensemble des atomes d'hydrogène dans l'alkyle ou le cycloalkyle peuvent être remplacés par un fluor,
R1, R2 et R3 sont identiques ou différents et sont chacun indépendamment un atome d'hydrogène, -(alkyle en C₁-C₆), -(cycloalkyle en C₃-C₆), -O-(alkyle en C₁-C₈), -O-(cycloalkyle en C₃-C₆), -(alkylène en C₀-C₄)-C(O)-N(R11)-R12, -(alkylène en C₀-C₄)-C(O)-O-R11, -(alkylène en C₀-C₄)-C(O)-R11, -(alkylène en C₀-C₄)-N(R11)-R12, -(alkylène en C₀-C₄)-N(R11 )-C(O)-R12, un halogène, OH, -CN, -NO₂, -SO₂CH₃, -SO₂CF₃, -SF₅, -Si[-(alkyle en C₁-C₄)]₃, -(alkylène en C₁-C₆)-O-(alkyle en C₁-C₆), -O-(alkylène en C₁-C₆)-O-(alkyle en C₁-C₆), -O-(alkylène en C₀-C₄)-(aryle en C₆-C₁₄) où l'aryle est non substitué ou mono-, di- ou trisubstitué indépendamment par -O-(alkyle en C₁-C₆), -(alkyle en C₁-C₄), OH, -(cycloalkyle en C₃-C₆) ou -O-(cycloalkyle en C₃-C₆), -O-(alkylène en C₁-C₄)-(cycloalkyle en C₃-C₆), -(Het en C₄-C₁₅),
où Het est non substitué ou mono-, di- ou trisubstitué indépendamment par -(alkyle en C₁-C₄), -(cycloalkyle en C₃-C₆), OH, -O-(alkyle en C₁-C₆) ou -O-(cycloalkyle en C₃-C₆), ou -O-(alkylène en C₁-C₆)-O-(alkylène en C₁-C₆)-O-(alkyle en C₁-C₆),
où l'alkyle, l'alkylène et le cycloalkyle sont chacun non substitués ou mono-, di- ou trisubstitués indépendamment par -(alkyle en C₁-C₄), -(cycloalkyle en C₃-C₆), OH, -O-(alkyle en C₁-C₆), -(aryle en C₆-C₁₄) où l'aryle est non substitué ou mono-, di-, tri-, tétra- ou pentasubstitué indépendamment par un halogène, -(alkyle en C₁-C₄), -(cycloalkyle en C₃-C₅), OH, -O-(alkyle en C₁-C₆) ou -O-(cycloalkyle en C₃-C₆), -(Het en C₄-C₁₅) où Het est non substitué ou mono-, di-, tri-, tétra- ou pentasubstitué indépendamment par un halogène, -(alkyle en C₁-C₄), -(cycloalkyle en C₃-C₆), OH, -O-(alkyle en C₁-C₆) ou -O-(cycloalkyle en C₃-C₆), ou -O-(cycloalkyle en C₃-C₆), ou
où certains ou l'ensemble des atomes d'hydrogène dans l'alkyle, l'alkylène ou le cycloalkyle peuvent être remplacés par un fluor, ou
R1 et R2 ou R2 et R3, conjointement avec les atomes cycliques auxquels ils sont chacun liés, forment un anneau de 5 à 8 chaînons, où l'anneau est constitué uniquement d'atomes de carbone ou 1, 2 ou 3 de ces atomes sont remplacés par des atomes d'azote, d'oxygène ou de soufre, où l'anneau est non substitué ou mono- ou disubstitué indépendamment par -(alkyle en C₁-C₄), -(cycloalkyle en C₃-C₆), OH, -O-(alkyle en C₁-C₆) ou -O-(cycloalkyle en C₃-C₆), où certains ou l'ensemble des atomes d'hydrogène dans l'anneau de 5 à 8 chaînons formé, dans l'alkyle ou le cycloalkyle, peuvent être remplacés par un fluor,
R11 et R12 sont chacun indépendamment un atome d'hydrogène, -(alkyle en C₁-C₆), -(cycloalkyle en C₃-C₆), -(alkylène en C₀-C₄)-(aryle en C₆-C₁₄) où l'aryle est non substitué ou mono-, di- ou trisubstitué indépendamment par -O-(alkyle en C₁-C₆), -(alkyle en C₁-C₄), OH, -(cycloalkyle en C₃-C₆) ou -O-(cycloalkyle en C₃-C₆), -(alkylène en C₀-C₄)-(Het en C₄-C₁₅) où Het est non substitué ou mono-, di-, tri-, tétra- ou pentasubstitué indépendamment par un halogène, -(alkyle en C₁-C₄), -(cycloalkyle en C₃-C₆), OH, -O-(alkyle en C₁-C₆) ou -O-(cycloalkyle en C₃-C₆), -SO₂CH₃ ou -SO₂CF₃,
où certains ou l'ensemble des atomes d'hydrogène dans l'alkyle, l'alkylène ou le cycloalkyle peuvent être remplacés par un fluor, ou
R11 et R12 dans les fragments « N(R11)-R12 » et « N(R11)-C(O)-R12 » représentent un anneau de 5 à 8 chaînons qui est formé conjointement avec l'atome d'azote « N » ou le groupe « N-C(O) » pour former des amines, imides ou lactames cycliques qui contiennent jusqu'à 2 autres hétéroatomes dans le groupe de N, O et S, où l'anneau est non substitué ou mono- ou disubstitué indépendamment par -(alkyle en C₁-C₄), -(cycloalkyle en C₃-C₆), OH, -O-(alkyle en C₁-C₆) ou -O-(cycloalkyle en C₃-C₆), où certains ou l'ensemble des atomes d'hydrogène dans l'anneau de 5 à 8 chaînons formé, dans l'alkyle ou le cycloalkyle, peuvent être remplacés par un fluor,
R4, R5, R6, R7 et R8 sont identiques ou différents et sont chacun indépendamment un atome d'hydrogène, -(alkyle en C₁-C₆), -(cycloalkyle en C₃-C₆), OH, -CN, -NO₂, -O-(alkyle en C₁-C₈), -O-(cycloalkyle en C₃-C₆), -(alkylène en C₀-C₄)-(CO)-N(R21)-R22, -SO₂CH₃, -SO₂CF₃, -(alkylène en C₀-C₄)-C(O)-O-R21, un halogène, -SF₅, -(alkylène en C₀-C₄)-C(O)-R21, -(alkylène en C₀-C₄)-N(R21)-R22, -(alkylène en C₀-C₄)-N(R21)-C(O)-R22, -(alkylène en C₁-C₆)-O-(alkyle en C₁-C₆), -(alkylène en C₀-C₆)-O-(alkylène en C₁-C₆)-O-(alkyle en C₁-C₆), -Si[-(alkyle en C₁-C₄)]₃, -(alkylène en C₀-C₆)-O-(alkylène en C₁-C₄)-(cycloalkyle en C₃-C₆), -(alkylène en C₀-C₆)-O-(alkylène en C₀-C₆)-(aryle en C₆-C₁₄) où l'aryle est non substitué ou mono-, di- ou trisubstitué indépendamment par -O-(alkyle en C₁-C₆), -(alkyle en C₁-C₄), OH, -(cycloalkyle en C₃-C₆) ou -O-(cycloalkyle en C₃-C₆), ou -(Het en C₄-C₁₅) où Het est non substitué ou mono-, di- ou trisubstitué indépendamment par -O-(alkyle en C₁-C₆), -(alkyle en C₁-C₄), OH, -(cycloalkyle en C₃-C₆) ou -O-(cycloalkyle en C₃-C₆), où l'alkyle, l'alkylène et le cycloalkyle sont chacun non substitués ou mono-, di- ou trisubstitués indépendamment par -(alkyle en C₁-C₄), -(cycloalkyle en C₃-C₆), OH, -O-(alkyle en C₁-C₆), -(aryle en C₆-C₁₄) où l'aryle est non substitué ou mono-, di-, tri-, tétra- ou pentasubstitué indépendamment par un halogène, -(alkyle en C₁-C₄), -(cycloalkyle en C₃-C₆), OH, -O-(alkyle en C₁-C₆) ou -O-(cycloalkyle en C₃-C₆), -(Het en C₄-C₁₅) où Het est non substitué ou mono-, di-, tri-, tétra- ou pentasubstitué indépendamment par un halogène, -(alkyle en C₁-C₄), -(cycloalkyle en C₃-C₆), OH, -O-(alkyle en C₁-C₆) ou -O-(cycloalkyle en C₃-C₆), ou -O-(cycloalkyle en C₃-C₆),
où certains ou l'ensemble des atomes d'hydrogène dans l'alkyle, l'alkylène ou le cycloalkyle peuvent être remplacés par un fluor, ou
R4 et R5, R5 et R6, R6 et R7 ou R7 et R8, conjointement avec les atomes cycliques auxquels ils sont chacun liés, forment un anneau de 5 à 8 chaînons, où l'anneau est constitué uniquement d'atomes de carbone ou 1, 2 ou 3 de ces atomes sont remplacés par des atomes d'azote, d'oxygène ou de soufre, où l'anneau est non substitué ou mono- ou disubstitué indépendamment par -(alkyle en C₁-C₄), -(cycloalkyle en C₃-C₆), OH, -O-(alkyle en C₁-C₆) ou -O-(cycloalkyle en C₃-C₆), où certains ou l'ensemble des atomes d'hydrogène dans l'anneau de 5 à 8 chaînons formé, dans l'alkyle ou le cycloalkyle, peuvent être remplacés par un fluor,
R21 et R22 sont chacun indépendamment un atome d'hydrogène, -(alkyle en C₁-C₆), -(cycloalkyle en C₃-C₆), -(alkylène en C₀-C₄)-(aryle en C₆-C₁₄) où l'aryle est non substitué ou mono-, di- ou trisubstitué indépendamment par -O-(alkyle en C₁-C₆), -(alkyle en C₁-C₄), OH, -(cycloalkyle en C₃-C₆) ou -O-(cycloalkyle en C₃-C₆), -(alkylène en C₀-C₄)-(Het en C₄-C₁₅) où Het est non substitué ou mono-, di-, tri-, tétra- ou pentasubstitué indépendamment par un halogène, -(alkyle en C₁-C₄), -(cycloalkyle en C₃-C₆), OH, -O-(alkyle en C₁-C₆) ou -O-(cycloalkyle en C₃-C₆), -SO₂CH₃ ou -SO₂CF₃,
où certains ou l'ensemble des atomes d'hydrogène dans l'alkyle, l'alkylène ou le cycloalkyle peuvent être remplacés par un fluor, ou
R21 et R22 dans les fragments « N(R21)-R22 » et « N(R21)-C(O)-R22 » représentent un anneau de 5 à 8 chaînons qui est formé conjointement avec l'atome d'azote « N » ou le groupe « N-C(O) » pour former des amines, imides ou lactames cycliques qui contiennent jusqu'à 2 autres hétéroatomes dans le groupe de N, O et S, où l'anneau est non substitué ou mono- ou disubstitué indépendamment par -(alkyle en C₁-C₄), -(cycloalkyle en C₃-C₆), OH, -O-(alkyle en C₁-C₆) ou -O-(cycloalkyle en C₃-C₆), où certains ou l'ensemble des atomes d'hydrogène dans l'anneau de 5 à 8 chaînons formé, dans l'alkyle ou le cycloalkyle, peuvent être remplacés par un fluor.

2. Composé de formule I selon la revendication 1, où
Q1, Q2 et Q3 sont identiques ou différents et sont chacun indépendamment un atome d'hydrogène, -(alkyle en C₁-C₆) ou -(cycloalkyle en C₃-C₆), où certains ou l'ensemble des atomes d'hydrogène dans l'alkyle ou le cycloalkyle peuvent être remplacés par un fluor,
R1, R2 et R3 sont identiques ou différents et sont chacun indépendamment un atome d'hydrogène, -(alkyle en C₁-C₆), -(cycloalkyle en C₃-C₆), -O-(alkyle en C₁-C₈), -O-(cycloalkyle en C₃-C₆), -(alkylène en C₀-C₄)-C(O)-N(R11)-R12, -(alkylène en C₀-C₄)-C(O)-O-R11, -(alkylène en C₀-C₄)-C(O)-R11, -(alkylène en C₀-C₄)-N(R11)-R12, -(alkylène en C₀-C₄)-N(R11)-C(O)-R12, un halogène, OH, -CN, -NO₂, -SO₂CH₃, -SO₂CF₃, -SF₅, -Si[-(alkyle en C₁-C₄)]₃, -(alkylène en C₁-C₆)-O-(alkyle en C₁-C₆), -O-(alkylène en C₁-C₆)-O-(alkyle en C₁-C₆), -O-(alkylène en C₀-C₄)-(aryle en C₆-C₁₄) où l'aryle est non substitué ou mono-, di- ou trisubstitué indépendamment par -O-(alkyle en C₁-C₆), -(alkyle en C₁-C₄), OH, -(cycloalkyle en C₃-C₆) ou -O-(cycloalkyle en C₃-C₆), -O-(alkylène en C₁-C₄)-(cycloalkyle en C₃-C₆), -(Het en C₄-C₁₅),
où Het est non substitué ou mono-, di- ou trisubstitué indépendamment par -(alkyle en C₁-C₄), -(cycloalkyle en C₃-C₆), OH, -O-(alkyle en C₁-C₆) ou -O-(cycloalkyle en C₃-C₆), ou -O-(alkylène en C₁-C₆)-O-(alkylène en C₁-C₆)-O-(alkyle en C₁-C₆),
où l'alkyle, l'alkylène et le cycloalkyle sont chacun non substitués ou mono-, di- ou trisubstitués indépendamment par -(alkyle en C₁-C₄), -(cycloalkyle en C₃-C₆), OH, -O-(alkyle en C₁-C₆), -(aryle en C₆-C₁₄) où l'aryle est non substitué ou mono-, di-, tri-, tétra- ou pentasubstitué indépendamment par un halogène, -(alkyle en C₁-C₄), -(cycloalkyle en C₃-C₆), OH, -O-(alkyle en C₁-C₆) ou -O-(cycloalkyle en C₃-C₆), -(Het en C₄-C₁₅) où Het est non substitué ou mono-, di-, tri-, tétra- ou pentasubstitué indépendamment par un halogène, -(alkyle en C₁-C₄), -(cycloalkyle en C₃-C₆), OH, -O-(alkyle en C₁-C₆) ou -O-(cycloalkyle en C₃-C₆), ou -O-(cycloalkyle en C₃-C₆), ou où certains ou l'ensemble des atomes d'hydrogène dans l'alkyle, l'alkylène ou le cycloalkyle peuvent être remplacés par un fluor,
à condition qu'au moins un R1, R2 ou R3 ne soit pas un atome d'hydrogène ou
R1 et R2 ou R2 et R3, conjointement avec les atomes cycliques auxquels ils sont chacun liés, forment un anneau de 5 à 8 chaînons, où l'anneau est constitué uniquement d'atomes de carbone ou 1, 2 ou 3 de ces atomes sont remplacés par des atomes d'azote, d'oxygène ou de soufre, où l'anneau est non substitué ou mono- ou disubstitué indépendamment par -(alkyle en C₁-C₄), -(cycloalkyle en C₃-C₆), OH, -O-(alkyle en C₁-C₆) ou -O-(cycloalkyle en C₃-C₆), où certains ou l'ensemble des atomes d'hydrogène dans l'anneau de 5 à 8 chaînons, dans l'alkyle ou le cycloalkyle peuvent être remplacés par un fluor,
R11 et R12 sont chacun indépendamment un atome d'hydrogène, -(alkyle en C₁-C₆), -(cycloalkyle en C₃-C₆), -(alkylène en C₀-C₄)-(aryle en C₆-C₁₄) où l'aryle est non substitué ou mono-, di- ou trisubstitué indépendamment par -O-(alkyle en C₁-C₆), -(alkyle en C₁-C₄), OH, -(cycloalkyle en C₃-C₆) ou -O-(cycloalkyle en C₃-C₆), -(alkylène en C₀-C₄)-(Het en C₄-C₁₅) où Het est non substitué ou mono-, di-, tri-, tétra- ou pentasubstitué indépendamment par un halogène, -(alkyle en C₁-C₄), -(cycloalkyle en C₃-C₆), OH, -O-(alkyle en C₁-C₆) ou -O-(cycloalkyle en C₃-C₆), -SO₂CH₃ ou -SO₂CF₃,
où certains ou l'ensemble des atomes d'hydrogène dans l'alkyle, l'alkylène ou le cycloalkyle peuvent être remplacés par un fluor, ou
R11 et R12 dans les fragments « N(R11)-R12 » et « N(R11)-C(O)-R12 » représentent un anneau de 5 à 8 chaînons qui est formé conjointement avec l'atome d'azote « N » ou le groupe « N-C(O) » pour former des amines, imides ou lactames cycliques qui contiennent jusqu'à 2 autres hétéroatomes dans le groupe de N, O et S, où l'anneau est non substitué ou mono- ou disubstitué indépendamment par -(alkyle en C₁-C₄), -(cycloalkyle en C₃-C₆), OH, -O-(alkyle en C₁-C₆) ou -O-(cycloalkyle en C₃-C₆), où certains ou l'ensemble des atomes d'hydrogène dans l'alkyle ou le cycloalkyle, peuvent être remplacés par un fluor,
R4, R5, R6, R7 et R8 sont identiques ou différents et sont chacun indépendamment un atome d'hydrogène, -(alkyle en C₁-C₆), -(cycloalkyle en C₃-C₆), OH, -CN, -NO₂, -O-(alkyle en C₁-C₈), -O-(cycloalkyle en C₃-C₆), -(alkylène en C₀-C₄)-(CO)-N(R21)-R22, -SO₂CH₃, -SO₂CF₃, -(alkylène en C₀-C₄)-C(O)-O-R21, un halogène, -SF₅, -(alkylène en C₀-C₄)-C(O)-R21, -(alkylène en C₀-C₄)-N(R21)-R22, -(alkylène en C₀-C₄)-N(R21)-C(O)-R22, -(alkylène en C₁-C₆)-O-(alkyle en C₁-C₆), -(alkylène en C₀-C₆)-O-(alkylène en C₁-C₆)-O-(alkyle en C₁-C₆), -Si[-(alkyle en C₁-C₄)]₃, -(alkylène en C₀-C₆)-O-(alkylène en C₁-C₄)-(cycloalkyle en C₃-C₆), -(alkylène en C₀-C₆)-O-(alkylène en C₀-C₆)-(aryle en C₆-C₁₄) où l'aryle est non substitué ou mono-, di- ou trisubstitué indépendamment par -O-(alkyle en C₁-C₆), -(alkyle en C₁-C₄), OH, -(cycloalkyle en C₃-C₆) ou -O-(cycloalkyle en C₃-C₆), ou -(Het en C₄-C₁₅) où Het est non substitué ou mono-, di- ou trisubstitué indépendamment par -O-(alkyle en C₁-C₆), -(alkyle en C₁-C₄), OH, -(cycloalkyle en C₃-C₆) ou -O-(cycloalkyle en C₃-C₆), où l'alkyle, l'alkylène et le cycloalkyle sont chacun non substitués ou mono-, di- ou trisubstitués indépendamment par -(alkyle en C₁-C₄), -(cycloalkyle en C₃-C₆), OH, -O-(alkyle en C₁-C₆), -(aryle en C₆-C₁₄) où l'aryle est non substitué ou mono-, di-, tri-, tétra- ou pentasubstitué indépendamment par un halogène, -(alkyle en C₁-C₄), -(cycloalkyle en C₃-C₆), OH, -O-(alkyle en C₁-C₆) ou -O-(cycloalkyle en C₃-C₆), -(Het en C₄-C₁₅) où Het est non substitué ou mono-, di-, tri-, tétra- ou pentasubstitué indépendamment par un halogène, -(alkyle en C₁-C₄), -(cycloalkyle en C₃-C₆), OH, -O-(alkyle en C₁-C₆) ou -O-(cycloalkyle en C₃-C₆), ou -O-(cycloalkyle en C₃-C₆), ou
où certains ou l'ensemble des atomes d'hydrogène dans l'alkyle, l'alkylène ou le cycloalkyle peuvent être remplacés par un fluor,
à condition qu'au moins un R4, R5, R6, R7 ou R8 ne soit pas un atome d'hydrogène, ou
R4 et R5, R5 et R6, R6 et R7 ou R7 et R8, conjointement avec les atomes cycliques auxquels ils sont chacun liés, forment un anneau de 5 à 8 chaînons, où l'anneau est constitué uniquement d'atomes de carbone ou 1, 2 ou 3 de ces atomes sont remplacés par des atomes d'azote, d'oxygène ou de soufre, où l'anneau est non substitué ou mono- ou disubstitué indépendamment par -(alkyle en C₁-C₄), -(cycloalkyle en C₃-C₆), OH, -O-(alkyle en C₁-C₆) ou -O-(cycloalkyle en C₃-C₆), où certains ou l'ensemble des atomes d'hydrogène dans l'anneau de 5 à 8 chaînons formé, dans l'alkyle ou le cycloalkyle, peuvent être remplacés par un fluor,
R21 et R22 sont chacun indépendamment un atome d'hydrogène, -(alkyle en C₁-C₆), -(cycloalkyle en C₃-C₆), -(alkylène en C₀-C₄)-(aryle en C₆-C₁₄) où l'aryle est non substitué ou mono-, di- ou trisubstitué indépendamment par -O-(alkyle en C₁-C₆), -(alkyle en C₁-C₄), OH, -(cycloalkyle en C₃-C₆) ou -O-(cycloalkyle en C₃-C₆), -(alkylène en C₀-C₄)-(Het en C₄-C₁₅) où Het est non substitué ou mono-, di-, tri-, tétra- ou pentasubstitué indépendamment par un halogène, -(alkyle en C₁-C₄), -(cycloalkyle en C₃-C₆), OH, -O-(alkyle en C₁-C₆) ou -O-(cycloalkyle en C₃-C₆), -SO₂CH₃ ou -SO₂CF₃,
où certains ou l'ensemble des atomes d'hydrogène dans l'alkyle, l'alkylène ou le cycloalkyle peuvent être remplacés par un fluor, ou
R21 et R22 dans les fragments « N(R21)-R22 » et « N(R21)-C(O)-R22 » représentent un anneau de 5 à 8 chaînons qui est formé conjointement avec l'atome d'azote « N » ou le groupe « N-C(O) » pour former des amines, imides ou lactames cycliques qui contiennent jusqu'à 2 autres hétéroatomes dans le groupe de N, O et S, où l'anneau est non substitué ou mono- ou disubstitué indépendamment par -(alkyle en C₁-C₄), -(cycloalkyle en C₃-C₆), OH, -O-(alkyle en C₁-C₆) ou -O-(cycloalkyle en C₃-C₆), où certains ou l'ensemble des atomes d'hydrogène dans l'anneau de 5 à 8 chaînons formé, dans l'alkyle ou le cycloalkyle, peuvent être remplacés par un fluor.

3. Composé de formule I selon la revendication 1 ou 2, où
Q1, Q2 et Q3 sont identiques ou différents et sont chacun indépendamment un atome d'hydrogène, -(alkyle en C₁-C₆) ou -(cycloalkyle en C₃-C₆), où certains ou l'ensemble des atomes d'hydrogène dans l'alkyle ou le cycloalkyle peuvent être remplacés par un fluor,
R1, R2 et R3 sont identiques ou différents et sont chacun indépendamment un atome d'hydrogène, -(alkyle en C₁-C₆), -(cycloalkyle en C₃-C₆), -O-(alkyle en C₁-C₈), -O-(cycloalkyle en C₃-C₆), -(alkylène en C₀-C₄)-C(O)-N(R11)-R12, -(alkylène en C₀-C₄)-C(O)-O-R11, -(alkylène en C₀-C₄)-C(O)-R11, -(alkylène en C₀-C₄)-N(R11)-R12, -(alkylène en C₀-C₄)-N(R11)-C(O)-R12, un halogène, OH, -CN, -NO₂, -SO₂CH₃, -Si[-(alkyle en C₁-C₄)]₃, -(alkylène en C₁-C₆)-O-(alkyle en C₁-C₆), -O-(alkylène en C₁-C₆)-O-(alkyle en C₁-C₆), -O-(alkylène en C₀-C₄)-(aryle en C₆-C₁₄), -O-(alkylène en C₁-C₄)-(cycloalkyle en C₃-C₆), -(Het en C₄-C₁₅) ou -O-(alkylène en C₁-C₆)-O-(alkylène en C₁-C₆)-O-(alkyle en C₁-C₆),
où l'alkyle, l'alkylène et le cycloalkyle sont chacun non substitués ou mono-, di- ou trisubstitués indépendamment par -(alkyle en C₁-C₄), -(cycloalkyle en C₃-C₆), OH, -O-(alkyle en C₁-C₆) ou -O-(cycloalkyle en C₃-C₆), ou
où certains ou l'ensemble des atomes d'hydrogène dans l'alkyle, l'alkylène ou le cycloalkyle peuvent être remplacés par un fluor,
à condition qu'au moins un R1, R2 ou R3 ne soit pas un atome d'hydrogène ou
R1 et R2 ou R2 et R3, conjointement avec les atomes cycliques auxquels ils sont chacun liés, forment un anneau choisi dans le groupe de 2,3,5,6,7,8-hexahydro-1,2,3a,4,5,8-hexaaza-cyclopenta[b]naphtalène ; 2,6,7,8-tétrahydro-3H-5-oxa-1,2,3a,4,8-pentaaza-cyclopenta[b]naphtalène ; 2,3,6,7-tétrahydro-5,8-dioxa-1,2,3a,4-tétraaza-cyclopenta[b]naphtalène ; 2,3,6,7-tétrahydro-5H-8-oxa-1,2,3a,4,5-pentaazacyclopenta[b]naphtalène ; 2,6,7,8-tétrahydro-3H-5-thia-1,2,3a,4,8-pentaazacyclopenta[b]naphtalène ; 2,3,6,7,8,9-hexahydro-1,2,3a,4,6,9-hexaaza-cyclopenta-[a]naphtalène ; 2,3-dihydro-5,7-dioxa-1,2,3a,4-tétraaza-s-indacène ; 2,6,7,8-tétrahydro-3H-cyclopenta[e][1,2,4]triazolo[4,3-b]pyridazine ; 2,7,8,9-tétrahydro-3H-cyclopenta[d][1,2,4]triazolo[4,3-b]pyridazine et 2,3,6a,9a-tétrahydro-[1,3]dioxolo[4,5-d][1,2,4]triazolo[4,3-b]pyridazine, où l'anneau est non substitué ou mono- ou disubstitué indépendamment par -(alkyle en C₁-C₄), -(cycloalkyle en C₃-C₆), OH, -O-(alkyle en C₁-C₆) ou -O-(cycloalkyle en C₃-C₆), où certains ou l'ensemble des atomes d'hydrogène dans l'alkyle ou le cycloalkyle peuvent être remplacés par un fluor,
R11 et R12 sont chacun indépendamment un atome d'hydrogène, -(alkyle en C₁-C₆), -(cycloalkyle en C₃-C₆), -(alkylène en C₀-C₄)-(aryle en C₆-C₁₄), -(alkylène en C₀-C₄)-(Het en C₄-C₁₅), -SO₂CH₃ ou -SO₂CF₃,
où certains ou l'ensemble des atomes d'hydrogène dans l'alkyle, l'alkylène ou le cycloalkyle peuvent être remplacés par un fluor, ou
R11 et R12 dans les fragments « N(R11)-R12 » et « N(R11)-C(O)-R12 » représentent un anneau de 5 à 8 chaînons choisi dans le groupe des azétidinyle, pyrrolidinyle, pipéridinyle, pipérazinyle, azépinyle, morpholinyle, thiomorpholinyle, pyrrolidine-2,5-dionyle, pipéridine-2,6-dionyle, pipérazine-2,6-dionyle, morpholine-3,5-dionyle, pyrrolidin-2-onyle, pipéridin-2-onyle, pipérazin-2-onyle et morpholin-3-onyle, où l'anneau est non substitué ou mono- ou disubstitué indépendamment par -(alkyle en C₁-C₄), -(cycloalkyle en C₃-C₆), OH, -O-(alkyle en C₁-C₆) ou -O-(cycloalkyle en C₃-C₆) où certains ou l'ensemble des atomes d'hydrogène dans l'alkyle ou le cycloalkyle peuvent être remplacés par un fluor,
R4, R5, R6, R7 et R8 sont identiques ou différents et sont chacun indépendamment un atome d'hydrogène, -(alkyle en C₁-C₆), -(cycloalkyle en C₃-C₆), OH, -CN, -NO₂, -O-(alkyle en C₁-C₈), -O-(cycloalkyle en C₃-C₆), -(alkylène en C₀-C₄)-C(O)-N(R21)-R22, -SO₂CH₃, -SO₂CF₃, -(alkylène en C₀-C₄)-C(O)-O-R21, un halogène, -SF₅, -(alkylène en C₀-C₄)-C(O)-R21, -(alkylène en C₀-C₄)-N(R21)-R22, -(alkylène en C₀-C₄)-N(R21)-C(O)-R22, -(alkylène en C₁-C₆)-O-(alkyle en C₁-C₆), -(alkylène en C₀-C₆)-O-(alkylène en C₁-C₆)-O-(alkyle en C₁-C₆), -Si[-(alkyle en C₁-C₄)]₃, -(alkylène en C₀-C₆)-O-(alkylène en C₁-C₄)-(cycloalkyle en C₃-C₆), -(alkylène en C₀-C₆)-O-(alkylène en C₀-C₆)-(aryle en C₆-C₁₄) ou -(Het en C₄-C₁₅),
où l'alkyle, l'alkylène et le cycloalkyle sont chacun non substitués ou mono-, di- ou trisubstitués indépendamment par -(alkyle en C₁-C₄), -(cycloalkyle en C₃-C₆), OH, -O-(alkyle en C₁-C₆), -(aryle en C₆-C₁₄) où l'aryle est non substitué ou mono-, di-, tri-, tétra- ou pentasubstitué indépendamment par un halogène, -(alkyle en C₁-C₄), -(cycloalkyle en C₃-C₆), OH, -O-(alkyle en C₁-C₆) ou -O-(cycloalkyle en C₃-C₆), -(Het en C₄-C₁₅) où Het est non substitué ou mono-, di-, tri-, tétra- ou pentasubstitué indépendamment par un halogène, -(alkyle en C₁-C₄), -(cycloalkyle en C₃-C₆), OH, -O-(alkyle en C₁-C₆) ou -O-(cycloalkyle en C₃-C₆), ou -O-(cycloalkyle en C₃-C₆), ou
où certains ou l'ensemble des atomes d'hydrogène dans l'alkyle, l'alkylène ou le cycloalkyle peuvent être remplacés par un fluor,
à condition qu'au moins un R4, R5, R6, R7 ou R8 ne soit pas un atome d'hydrogène, ou
R4 et R5, R5 et R6, R6 et R7 ou R7 et R8, conjointement avec les atomes cycliques auxquels ils sont chacun liés, forment un anneau de 5 à 8 chaînons choisi dans le groupe des 2,3-dihydrobenzo[1,4]dioxine; 3,4-dihydro-2H-benzo[1,4]oxazine; 1,2,3,4-tétrahydro-quinoxaline ; benzo[1,3]dioxole ; 3,4-dihydro-2H-benzo[1,4]thiazine et 2,3,4,5-tétrahydro-1H-benzo[b][1,4]diazépine,
où l'anneau est non substitué ou mono- ou disubstitué indépendamment par -(alkyle en C₁-C₄), -(cycloalkyle en C₃-C₆), OH, -O-(alkyle en C₁-C₆) ou -O-(cycloalkyle en C₃-C₆), où certains ou l'ensemble des atomes d'hydrogène dans l'alkyle ou le cycloalkyle peuvent être remplacés par un fluor,
R21 et R22 sont chacun indépendamment un atome d'hydrogène, -(alkyle en C₁-C₆), -(cycloalkyle en C₃-C₆), -(alkylène en C₀-C₄)-(aryle en C₆-C₁₄), -(alkylène en C₀-C₄)-(Het en C₄-C₁₅), -SO₂CH₃ ou -SO₂CF₃,
où certains ou l'ensemble des atomes d'hydrogène dans l'alkyle, l'alkylène ou le cycloalkyle peuvent être remplacés par un fluor, ou
R21 et R22 dans les fragments « N(R21)-R22 » et « N(R21)-C(O)-R22 » représentent un anneau de 5 à 8 chaînons choisi dans le groupe des azétidinyle, pyrrolidinyle, pipéridinyle, pipérazinyle, azépinyle, morpholinyle, thiomorpholinyle, pyrrolidine-2,5-dionyle, pipéridine-2,6-dionyle, pipérazine-2,6-dionyle, morpholine-3,5-dionyle, pyrrolidin-2-onyle, pipéridin-2-onyle, pipérazin-2-onyle et morpholin-3-onyle, où l'anneau est non substitué ou mono- ou disubstitué indépendamment par -(alkyle en C₁-C₄), -(cycloalkyle en C₃-C₆), OH, -O-(alkyle en C₁-C₆) ou -O-(cycloalkyle en C₃-C₆) où certains ou l'ensemble des atomes d'hydrogène dans l'alkyle ou le cycloalkyle peuvent être remplacés par un fluor.

4. Composé de formule I selon les revendications 1 à 3, où
Q1, Q2 et Q3 sont identiques et sont chacun un atome d'hydrogène,
R1, R2 et R3 sont identiques ou différents et sont chacun indépendamment un atome d'hydrogène, -(alkyle en C₁-C₆), -O-(alkyle en C₁-C₈), -O-(cycloalkyle en C₃-C₆), -(alkylène en C₀-C₄)-C(O)-N(R11)-R12, -(alkylène en C₀-C₄)-N(R11)-R12, un halogène, -O-(alkylène en C₁-C₆)-O-(alkyle en C₁-C₆), -O-(alkylène en C₀-C₄)phényle ou -O-(alkylène en C₁-C₆)-O-(alkylène en C₁-C₆)-O-(alkyle en C₁-C₆),
où l'alkyle ou l'alkylène est dans chaque cas non substitué ou mono- ou disubstitué par -O-(alkyle en C₁-C₆),
où certains ou l'ensemble des atomes d'hydrogène dans l'alkyle ou l'alkylène peuvent être remplacés par un fluor,
à condition qu'au moins un R1, R2 ou R3 ne soit pas un atome d'hydrogène,
R11 et R12 sont chacun indépendamment un atome d'hydrogène ou -(alkyle en C₁-C₆), ou
R11 et R12 dans le fragment « N(R11)-R12 » est un anneau de 5 à 8 chaînons choisi dans le groupe des azétidinyle, pyrrolidinyle, pipéridinyle, pipérazinyle, azépinyle, imidazolyle, morpholinyle, thiomorpholinyle, pyrrolidine-2,5-dionyle, pipéridine-2,6-dionyle, pipérazine-2,6-dionyle, morpholine-3,5-dionyle, pyrrolidin-2-onyle, pipéridin-2-onyle, pipérazin-2-onyle et morpholin-3-onyle, R4, R5, R6, R7 et R8 sont identiques ou différents et sont chacun indépendamment un atome d'hydrogène, -(alkyle en C₁-C₆), OH, -O-(alkyle en C₁-C₈), un halogène, -SF₅, -(alkylène en C₀-C₄)-N(R21)-R22, -(alkylène en C₀-C₄)-N(R21)-C(O)-R22, -CF₃, -(alkylène en C₀-C₆)-O-(alkylène en C₁-C₆)-O-(alkyle en C₁-C₆), -(alkylène en C₀-C₆)-O-(alkylène en C₁-C₄)-(cycloalkyle en C₃-C₆), ou -(alkylène en C₀-C₆)-O-(alkylène en C₀-C₆)phényle,
où l'alkyle ou l'alkylène est dans chaque cas non substitué ou mono- ou disubstitué par -O-(alkyle en C₁-C₆),
à condition qu'au moins un R4, R5, R6, R7 ou R8 ne soit pas un atome d'hydrogène, ou
R4 et R5, R5 et R6, R6 ou R7 ou R7 et R8, conjointement avec les atomes cycliques auxquels ils sont chacun liés, forment un anneau de cinq à huit chaînons choisi dans le groupe des 2,3-dihydrobenzo[1,4]dioxine; 3,4-dihydro-2H-benzo[1,4]oxazine ; 1,2,3,4-tétrahydro-quinoxaline ; benzo[1,3]dioxole ; 3,4-dihydro-2H-benzo[1,4]thiazine et 2,3,4,5-tétrahydro-1H-benzo[b][1,4]diazépine,
où l'anneau est non substitué ou mono- ou disubstitué par -(alkyle en C₁-C₄),
R21 et R22 sont chacun indépendamment un atome d'hydrogène ou -(alkyle en C₁-C₆), ou
R21 et R22 dans le fragment « N(R21)-R22 » représentent un anneau de 5 à 8 chaînons choisi dans le groupe des azétidinyle, pyrrolidinyle, pipéridinyle, pipérazinyle, azépinyle, imidazolyle, morpholinyle, thiomorpholinyle, pyrrolidine-2,5-dionyle, pipéridine-2,6-dionyle, pipérazine-2,6-dionyle, morpholine-3,5-dionyle, pyrrolidin-2-onyle, pipéridin-2-onyle, pipérazin-2-onyle et morpholin-3-onyle.

5. Composé de formule I selon les revendications 1 à 4, où le composé de formule I est choisi dans le groupe des 2-(6-chloro-3-imino-[1,2,4]triazolo[4,3-b]pyridazin-2-yl)-1-(3,5-di-tert-butyl-4-hydroxyphényl)éthanone, 1-(3-tert-butyl-4-méthoxy-5-morpholin-4-ylphényl)-2-(3-imino-6-isopropoxy-[1,2,4]triazolo[4,3-b]pyridazin-2-yl)éthanone, 1-(3-tert-butyl-4-méthoxy-5-morpholin-4-ylphényl)-2-(3-imino-6-méthoxy-[1,2,4]triazolo[4,3-b]pyridazin-2-yl)éthanone, 2-(6-éthoxy-3-imino-[1,2,4]triazolo[4,3-b]pyridazin-2-yl)-1-(4-méthoxy-3-morpholin-4-yl-5-trifluorométhyl-phényl)éthanone, N-[3-[2-(6-éthoxy-3-imino-[1,2,4]triazolo[4,3-b]pyridazin-2-yl)-acétyl]-5-(pentafluorosulfanyl)phényl]-N-méthylacétamide, N-[3-[2-(6-éthoxy-3-imino-[1,2,4]triazolo[4,3-b]pyridazin-2-yl)-acétyl]-5-(pentafluorosulfanyl)phényl]acétamide, 1-(3-tert-butyl-4-méthoxy-5-morpholin-4-ylphényl)-2-(6-éthoxy-3-imino-[1,2,4]triazolo-[4,3-b]pyridazin-2-yl)éthanone, 1-(3-tert-butyl-4-méthoxy-5-morpholin-4-ylphényl)-2-(6-cyclopentyloxy-3-imino-[1,2,4]triazolo[4,3-b]pyridazin-2-yl)éthanone, 1-(3-tert-butyl-4-méthoxy-5-morpholin-4-ylphényl)-2-(6-cyclobutoxy-3-imino-[1,2,4]triazolo(4,3-b]pyridazin-2-yl)éthanone, 1-(3-tert-butyl-4-méthoxy-5-morpholin-4-ylphényl)-2-(3-imino-6-phénoxy-[1,2,4]triazolo[4,3-b]pyridazin-2-yl)éthanone, 2-(6-benzyloxy-3-imino-[1,2,4]triazolo[4,3-b]pyridazin-2-yl)-1-(3-tert-butyl-4-méthoxy-5-morpholin-4-ylphényl)éthanone, 1-(3-tert-butyl-4-méthoxy-5-morpholin-4-ylphényl)-2-(6-cyclohexyloxy-3-imino-[1,2,4]triazolo[4,3-b]pyridazin-2-yl)éthanone, 1-(3-tert-butyl-4-méthoxy-5-morpholin-4-ylphényl)-2-[3-imino-6-(2,2,2-trifluoroéthoxy)-[1,2,4]triazolo-[4,3-b]pyridazin-2-yl]éthanone, 1-(3-tert-butyl-4-méthoxy-5-morpholin-4-ylphényl)-2-[6-(1-éthylpropoxy)-3-imino-[1,2,4]triazolo[4,3-b]pyridazin-2-yl]éthanone, 1-(3-tert-butyl-4-méthoxy-5-morpholin-4-ylphényl)-2-(6-cyclopropylméthoxy-3-imino-[1,2,4]-triazolo[4,3-b]pyridazin-2-yl)éthanone, 2-[6-(1-éthylpropoxy)-3-imino-[1,2,4]triazolo-[4,3-b]pyridazin-2-yl]-1-[3-méthylamino-5-(pentafluorosulfanyl)phényl]éthanone, 2-[6-(1-éthylpropoxy)-3-imino-[1,2,4]triazolo[4,3-b]pyridazin-2-yl]-1-[3-méthoxy-5-(pentafluorosulfanyl)phényl]éthanone, 1-(3-tert-butyl-5-éthoxyméthylphényl)-2-[6-(1-éthylpropoxy)-3-imino-[1,2,4]triazolo[4,3-b]pyridazin-2-yl]éthanone, 1-(3-tert-butyl-5-cyclopropylméthoxyméthylphényl)-2-[6-(1-éthylpropoxy)-3-imino-[1,2,4]triazolo[4,3-b]pyridazin-2-yl]éthanone, 1-(3-tert-butyl-4,5-diéthoxyphényl)-2-[6-(1-éthylpropoxy)-3-imino-[1,2,4]triazolo[4,3-b]pyridazin-2-yl]éthanone, 1-(3-tert-butyl-5-éthoxyphényl)-2-[6-(1-éthylpropoxy)-3-imino-[1,2,4]triazolo[4,3-b]pyridazin-2-yl]-éthanone, 1-(3-tert-butyl-5-propoxyméthylphényl)-2-[6-(1-éthylpropoxy)-3-imino-[1,2,4]triazolo[4,3-b]pyridazin-2-yl]éthanone, 1-(3-tert-butyl-4,5-bis(cyclopropylméthoxy)phényl)-2-[6-(1-éthylpropoxy)-3-imino-[1,2,4]triazolo[4,3-b]pyridazin-2-yl]éthanone, 2-[6-(1-éthylpropoxy)-3-imino-[1,2,4]triazolo[4,3-b]pyridazin-2-yl]-1-(3-méthoxy-5-trifluorométhylphényl)éthanone, 1-(3-tert-butyl-5-méthoxyphényl)-2-[6-(1-éthyl-propoxy)-3-imino-[1,2,4]triazolo[4,3-b]pyridazin-2-yl]éthanone, 1-(3-tert-butyl-5-cyclopropylméthoxyphényl)-2-[6-(1-éthylpropoxy)-3-imino-[1,2,4]-triazolo[4,3-b]pyridazin-2-yl]éthanone, 1-(3-tert-butyl-5-cyclobutylméthoxyphényl)-2-[6-(1-éthylpropoxy)-3-imino-[1,2,4]triazolo[4,3-b]pyridazin-2-yl]éthanone, 1-(3-benzyloxyméthyl-5-tert-butylphényl)-2-[6-(1-éthylpropoxy)-3-imino-[1,2,4]triazolo[4,3-b]pyridazin-2-yl]éthanone, 1-(3-cyclohexylméthoxy-4,5-diméthoxyphényl)-2-[6-(1-éthylpropoxy)-3-imino-[1,2,4]triazolo[4,3-b]pyridazin-2-yl]éthanone, 2-(6-butoxy-3-imino-[1,2,4]triazolo[4,3-b]pyridazin-2-yl)-1-(3-tert-butyl-5-méthoxyméthylphényl)-éthanone, 1-(3-chloro-5-méthoxyphényl)-2-[6-(1-éthylpropoxy)-3-imino-[1,2,4]-triazolo[4,3-b]pyridazin-2-yl]éthanone, 1-(8-tert-butyl-4-méthyl-3,4-dihydro-2H-benzo[1,4]oxazin-6-yl)-2-[6-(1-éthylpropoxy)-3-imino-[1,2,4]tria zolo[4,3-b]pyridazin-2-yl]éthanone, 1-(3-tert-butyl-4-méthoxy-5-morpholin-4-ylphényl)-2-(3-imino-6-pipéridin-1-yl-[1,2,4]triazolo[4,3-b]pyridazin-2-yl)éthanone, 1-(3-tert-butyl-4-méthoxy-5-morpholin-4-ylphényl)-2-(6-diéthylamino-3-imino-[1,2,4]triazolo[4,3-b]pyridazin-2-yl)éthanone, 2-[6-(1-éthylpropoxy)-3-imino-[1,2,4]triazolo[4,3-b]pyridazin-2-yl]-1-(3-isopropyl-5-méthoxyphényl)éthanone, 1-(3-cyclohexylméthoxy-5-éthoxyphényl)-2-[6-(1-éthylpropoxy)-3-imino-[1,2,4]triazolo[4,3-b]pyridazin-2-yl]éthanone, 1-(3-bromo-5-méthoxyphényl)-2-[6-(1-éthylpropoxy)-3-imino-[1,2,4]triazolo[4,3-b]pyridazin-2-yl]éthanone, 1-[3-(3,3-diméthylbutoxy)-5-méthoxyphényl]-2-[6-(1-éthylpropoxy)-3-imino-[1,2,4]-triazolo[4,3-b]pyridazin-2-yl]éthanone, 1-[3-(3,3-diméthylbutoxy)-5-éthoxyphényl]-2-[6-(1-éthylpropoxy)-3-imino-[1,2,4]-triazolo[4,3-b]pyridazin-2-yl]éthanone, 1-(3-cyclohexylméthoxy-5-méthoxyphényl)-2-[6-(1-éthylpropoxy)-3-imino-[1,2,4]-triazolo[4,3-b]pyridazin-2-yl]éthanone, 1-(3-bromo-4,5-diméthoxyphényl)-2-[6-(1-éthylpropoxy)-3-imino-[1,2,4]triazolo[4,3-b]pyridazin-2-yl]éthanone, 2-(6-diéthylamino-3-imino-[1,2,4]triazolo[4,3-b]pyridazin-2-yl)-1-[3-méthoxy-5-(pentafluorosulfanyl)phényl]éthanone, 1-(3-tert-butyl-4-méthoxy-5-morpholin-4-ylphényl)-2-(3-imino-6-morpholin-4-yl-[1,2,4]triazolo[4,3-b]pyridazin-2-yl)éthanone, 1-(3-tert-butyl-4-méthoxy-5-morpholin-4-ylphényl)-2-(6-chloro-3-imino-8-méthyl-[1,2,4]triazolo[4,3-b]pyridazin-2-yl)éthanone, 1-(3-tert-butyl-4-méthoxy-5-morpholin-4-ylphényl)-2-(6-imidazol-1-yl-3-imino-[1,2,4]-triazolo[4,3-b]pyridazin-2-yl)éthanone, 1-(5-bromo-2,3-diméthoxyphényl)-2-[6-(1-éthylpropoxy)-3-imino-[1,2,4]triazolo[4,3-b]pyridazin-2-yl]éthanone, 1-(3-tert-butyl-5-méthoxyphényl)-2-{3-imino-6-[2-(2-méthoxyéthoxy)éthoxy]-[1,2,4]-triazolo(4,3-b]pyridazin-2-yl}éthanone, 1-(3-chloro-4,5-diméthoxyphényl)-2-[6-(1-éthylpropoxy)-3-imino-[1,2,4]triazolo[4,3-b]pyridazin-2-yl]éthanone, 1-(3-tert-butyl-4-méthoxy-5-morpholin-4-ylphényl)-2-(6-diéthylamino-3-imino-8-méthyl-[1,2,4]triazolo[4,3-b]pyridazin-2-yl)éthanone, 1-[3-tert-butyl-5-(2-méthoxyéthoxy)phényl]-2-[6-(1-éthylpropoxy)-3-imino-[1,2,4]-triazolo(4,3-b]pyridazin-2-yl]éthanone, 1-(3-tert-butyl-5-méthoxyphényl)-2-[3-imino-6-(2-méthoxyéthoxy)-[1,2,4]triazolo[4,3-b]pyridazin-2-yl]éthanone, 1-(3-tert-butyl-5-méthoxyméthylphényl)-2-[3-imino-6-(2-méthoxyéthoxy)-[1,2,4]-triazolo[4,3-b]pyridazin-2-yl]éthanone, 2-[6-(1-éthylpropoxy)-3-imino-[1,2,4]triazolo[4,3-b]pyridazin-2-yl]-1-[3-morpholin-4-yl-5-(pentafluorosulfanyl)phényl]éthanone, 1-(3-tert-butyl-4-méthoxy-5-morpholin-4-ylphényl)-2-(6-éthoxy-3-imino-8-méthyl-[1,2,4]triazolo[4,3-b]pyridazin-2-yl)éthanone, 2-[6-(1-éthylpropoxy)-3-imino-8-méthyl-[1,2,4]triazolo[4,3-b]pyridazin-2-yl]-1-(5-méthoxy-3-pentafluorosulfanylphényl)éthanone, 1-(3-tert-butyl-4-méthoxy-5-morpholin-4-ylphényl)-2-(6-chloro-3-imino-[1,2,4]triazolo-[4,3-b]pyridazin-2-yl)éthanone, 1-[3-tert-butyl-5-(2-méthoxyéthoxyméthyl)phényl]-2-[6-(1-éthylpropoxy)-3-imino-[1,2,4]triazolo[4,3-b]pyridazin-2-yl]éthanone, 2-[6-(1-éthylpropoxy)-3-imino-[1,2,4]triazolo[4,3,b]pyridazin-2-yl]-1-[3-(2-méthoxyéthoxy)-5-(pentafluorosulfanyl)phényl]éthanone, 2-(6-éthoxy-3-imino-8-méthyl-[1,2,4]triazolo[4,3-b]pyridazin-2-yl)-1-[3-méthoxy-5-(pentafluorosulfanyl)phényl]éthanone, 2-[6-(1-éthylpropoxy)-3-imino-[1,2,4]triazolo[4,3-b]pyridazin-2-yl]-1-[3-(3-méthoxypropoxy)-5-(pentafluorosulfanyl)phényl]éthanone, N,N-diéthyl-2-[2-(3-tert-butyl-4-méthoxy-5-morpholin-4-ylphényl)-2-oxoéthyl]-6-chloro-3-imino-2,3-dihydro-[1,2,4]triazolo[4;3-b]pyridazine-8-carboxamide, N,N-diéthyl-6-chloro-3-imino-2-{2-[3-méthoxy-5-(pentafluorosulfanyl)phényl]-2-oxoéthyl}-2,3-dihydro-[1,2,4]triazolo[4,3-b]pyridazine-8-carboxamide, 2-(6-éthyl-3-imino-[1,2,4]triazolo[4,3-b]pyridazin-2-yl)-1-[3-méthoxy-5-(pentafluoro-sulfanyl)phényl]éthanone, 1-(3-tert-butyl-4-méthoxy-5-morpholin-4-ylphényl)-2-(6-éthyl-3-imino-[1,2,4]triazolo-[4,3-b]pyridazin-2-yl)éthanone, 1-[3-tert-butyl-5-(3-méthoxypropoxy)phényl]-2-[6-(1-éthylpropoxy)-3-imino-8-méthyl-[1,2,4]triazolo[4,3-b]pyridazin-2-yl]éthanone, 1-[3-tert-butyl-5-(2-méthoxyéthoxy)phényl]-2-[6-(1-éthylpropoxy)-3-imino-8-méthyl-[1,2,4]triazolo[4,3-b]pyridazin-2-yl]éthanone, 1-(3-tert-butyl-5-cyclopropylméthoxyphényl)-2-[6-(1-éthylpropoxy)-3-imino-8-méthyl-[1,2,4]triazolo[4,3-b]pyridazin-2-yl]éthanone, 1-(3-tert-butyl-5-méthoxyphényl)-2-[6-(1-éthylpropoxy)-3-imino-8-méthyl-[1,2,4]-triazolo[4,3-b]pyridazin-2-yl]éthanone, 1-[3-tert-butyl-5-(3-méthoxypropoxy)phényl]-2-[6-(1-éthylpropoxy)-3-imino-7-méthyl-[1,2,4]triazolo[4,3-b]pyridazin-2-yl]éthanone, 2-(6-chloro-3-imino-7,8-diméthyl-[1,2,4]triazolo[4,3-b]pyridazin-2-yl)-1-[3-méthoxy-5-(pentafluorosulfanyl)phényl]éthanone, 1-(3-tert-butyl-4-méthoxy-5-morpholin-4-ylphényl)-2-(6-chloro-3-imino-7,8-diméthyl-[1,2,4]triazolo[4,3-b]pyridazin-2-yl)éthanone, N,N-diéthyl-6-chloro-3-imino-2-{2-[3-méthoxy-5-(pentafluorosulfanyl)phényl]-2-oxoéthyl}-2,3-dihydro-[1,2,4]triazolo[4,3-b]pyridazine-7-carboxamide, N,N-diéthyl-2-[2-(3-tert-butyl-4-méthoxy-5-morpholin-4-ylphényl)-2-oxoéthyl]-6-chloro-3-imino-2,3-dihydro-[1,2,4]triazolo[4,3-b]pyridazine-7-carboxamide, 1-[3-tert-butyl-5-(3-méthoxypropoxy)phényl]-2-(6-éthoxy-3-imino-7-méthyl-[1,2,4]-triazolo[4,3-b]pyridazin-2-yl)éthanone ou 1-[3-tert-butyl-5-(2-méthoxyéthoxy)phényl]-2-[6-(1-éthylpropoxy)-3-imino-7-méthyl-[1,2,4]triazolo[4,3-b]pyridazin-2-yl]éthanone.

6. Médicament, **caractérisé par** une teneur efficace d'au moins un composé de formule I selon une ou plusieurs des revendications 1 à 5 conjointement avec un véhicule, additif et/ou d'autres substances actives et excipients pharmaceutiquement adaptés et physiologiquement compatibles.

7. Composé de formule I selon une ou plusieurs des revendications 1 à 5 pour utilisation dans la prophylaxie, la prévention secondaire et le traitement de tous les troubles associés aux thromboses, embolies, hypercoagulabilité, changements fibrotiques ou troubles inflammatoires.

8. Composé selon la revendication 7, **caractérisé en ce que** les troubles sont l'infarctus du myocarde, l'angine de poitrine et d'autres types de syndrome coronarien aigu, un accident vasculaire cérébral, des troubles vasculaires périphériques, la thrombose veineuse profonde, l'embolie pulmonaire, des événements emboliques ou thrombotiques causés par des arythmies cardiaques, des événements cardiovasculaires tels qu'une resténose après une revascularisation et une angioplastie et des procédures similaires telles que des implantations d'endoprothèse et des opérations de pontage ou la réduction du risque de thrombose après des procédures chirurgicales telles que des opérations des articulations du genou et de la hanche ou des procédures conduisant au contact du sang avec des surfaces étrangères, par exemple pour des patients dialysés et des patients avec des cathéters à demeure ou la coagulation intravasculaire disséminée, la sepsie et d'autres événements intravasculaires associés à l'inflammation, l'athérosclérose, le diabète et le syndrome métabolique et les séquelles de ceux-ci, la croissance de tumeur et la métastase de tumeur, des troubles articulaires inflammatoires et dégénératifs tels que la polyarthrite rhumatoïde et l'arthrose, des altérations du système hémostatique telles que des dépôts de fibrine, des changements fibrotiques dans les poumons tels que la bronchopneumopathie chronique obstructive, le syndrome de détresse respiratoire de l'adulte ou des dépôts de fibrine dans les yeux après des opérations des yeux ou la prévention et/ou le traitement de la cicatrisation.

9. Procédé pour préparer le composé de formule I selon une ou plusieurs des revendications 1 à 5, qui comprend
a) la réaction d'un composé de formule II 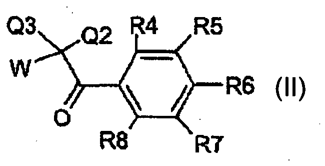 où R4 à R8, Q2 et Q3 sont chacun tels que définis dans la formule I et W est un chlorure, un bromure, un mésylate ou un tosylate avec un composé de formule III 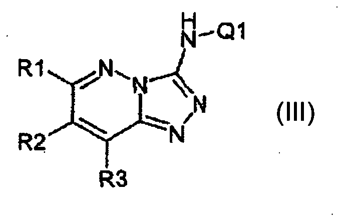 où R1, R2, R3 et Q1 sont chacun tels que définis dans la formule I, avec ou sans ajout de base, dans un solvant pour obtenir un composé de formule I, ou
b) la réaction d'un composé de formule VII 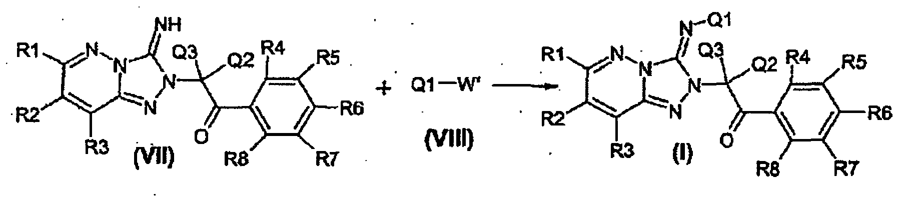 où R1, R2, R3, R4, R5, R6, R7, R8, Q1, Q2 et Q3 sont chacun tels que définis dans la formule I avec un composé Q-W' où W' est un chlorure, un bromure, un mésylate, un tosylate, un méthylsulfate ou un bon groupe partant similaire, avec ou sans ajout de base, pour obtenir un composé de formule I, ou
c) l'isolement du composé de formule I préparé par le procédé a) ou b) sous forme libre ou la libération de celui-ci à partir de sels physiologiquement incompatibles ou, dans le cas de la présence de groupes acides ou basiques, la conversion de celui-ci en sels physiologiquement compatibles, ou
d) la séparation d'un composé de formule I préparé par le procédé a) ou b), ou un précurseur adapté de formule I qui, en raison de sa structure chimique, est produit sous des formes énantiomériques ou diastéréoisomériques, en énantiomères ou diastéréoisomères purs par formation de sel avec des acides ou bases énantiomériquement purs, chromatographie sur des phases stationnaires chirales ou dérivation au moyen de composés chiraux énantiomériquement purs tels que des acides aminés, séparation des diastéréoisomères ainsi obtenus, et élimination des groupes auxiliaires chiraux.
